# EUROPEAN PATENT SPECIFICATION

(11) **EP 0 448 726 B1**
(45) Date of publication and mention of the grant of the patent: **24.04.1996**
(21) Application number: 90915190.4
(22) Date of filing: 18.10.1990
(51) Int. Cl.: C07C 405/00, A61K 31/557

(54) **2-SUBSTITUTED 2-CYCLOPENTENONE AND CARCINOSTATIC AGENT AND OSTEOGENESIS PROMOTER CONTAINING THE SAME AS ACTIVE INGREDIENT**
2-SUBSTITUIERTE-2-CYCLOPENTENONE UND DIESE ALS AKTIVE SUBSTANZEN ENTHALTENDE CARCINOSTATIKA UND OSTEOGENESIS-PROMOTER
2-CYCLPENTENONE SUBSTITUE EN 2 ET AGENT CARCINOSTATIQUE ET STIMULATEUR D'OSTEOGENESE LE CONTENANT EN TANT QU'INGREDIENT ACTIF

(30) Priority: 19.10.1989 JP 272296/89
(43) Date of publication of application: 02.10.1991
(73) Proprietor: TEIJIN LIMITED, Osaka-shi Osaka 541 (JP)
(72) Inventor: SUGIURA, Satoshi, Hino-shi Tokyo 191 (JP); MINOSHIMA, Toru, Hino-shi Tokyo 191 (JP); HAZATO, Atsuo, Hino-shi Tokyo 191 (JP); KATO, Yoshinori, Hino-shi Tokyo 191 (JP)
(74) Representative: Votier, Sidney David
(86) International application number: JP9001343
(87) International publication number: WO9105766

(56) References cited:
- EP-A- 0 131 441
- FR-A- 2 357 542
- JP-A- 2 275 849
- JP-A-58 109 468
- CHEMISTRY LETTERS, 1977, pages 331-334, Chemical Society of Japan; S. KUROZUMI et al.: "Synthesis of e-type 7-thiaprostaglandins"
- Chemical and Pharmaceutical Bulletin, Vol. 33, No. 7, (1985), KATSUHIDE MATOBA et al. (Reduction of Vinylogous Thioesters with Lithium Aluminum Hydride. II.), p. 3001-3005 Particularly, refer to compound Vb on page 3002.
- Slides used during the presentation associated with preprint on page 117 of the 109th Annual Meeting of the Pharmaceutical Society of Japan

## Description

The present invention relates to a 2-substituted-2-cyclopentenone compound, and more particularly, to a 2-substituted-2-cyclopentenone compound having superior pharmacological activities, such as an anticancer activity and bone formation activity, and an anticancer agent and a bone formation accelerator comprising the same as an active ingredient.

### BACKGROUND ART

A prostaglandin is a compound having specific biological activities, such as a platelet agglutination inhibitory activity and a vasodepressor activity, and is a useful naturally occurring substance which is now used in the medical field as a therapeutic agent for diseases of the peripheral cardiovascular system. Among the prostaglandins, prostaglandin A compounds are known as a prostaglandin having a double bond in its cyclopentane ring. For example, European Unexamined Patent Publication No. 0106576 (publication date: April 25, 1984) discloses 4,5-substituted-2-cyclopentenone compounds embracing the prostaglandin A compounds, which include 5-alkylidene-4-substituted-2-cyclopentene compounds represented by the following formula: 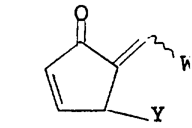 wherein W stands for a substituted or unsubstituted hydrocarbon group having 1 to 12 carbon atoms; and Y stands for a substituted or unsubstituted hydrocarbon group having 1 to 12 carbon atoms, and 5-(1-hydroxy-hydrocarbon)-4-substituted-2-cyclopentenone compounds represented by the following formula: 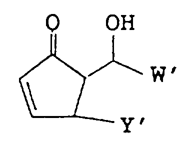 wherein W' and Y' are the same as W and Y, respectively.

Further, the above publication also states that the above-described compounds are useful for treating a malignant tumor.

European Unexamined Patent Publication No. 0131441 (publication date: January 16, 1985) discloses 5-alkylidene-2-halo-4-substituted-2-cyclopentenone compounds represented by the following formula: 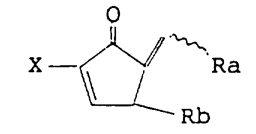 wherein Ra stands for a substituted or unsubstituted hydrocarbon group having 1 to 12 carbon atoms or a substituted or unsubstituted phenyl group; Rb stands for a substituted or unsubstituted hydrocarbon having 1 to 12 carbon atoms; and X stands for a halogen atom,
and further discloses that the above-described compounds are similarly useful for treating a malignant tumor.

Further, it is also known that prostaglandin D compounds and prostaglandin J compounds, although different from the prostaglandin A compounds, are useful as an antitumor agent [see Japanese Unexamined Patent Publication (Kokai) No. 58-216155 and Proc. Natl. Acad. Sci., U.S.A., 81, 1317 - 1321 (1984)].

Japanese Unexamined Patent Publication (Kokai) No. 62-96438 discloses 4-hydroxy-2-cyclopentenone compounds represented by the general formula: 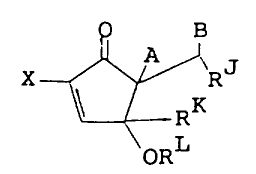 wherein X stands for a hydrogen atom or a halogen atom; A and B stand for a hydrogen atom and a hydroxyl group, respectively, or are combined with each other to form a single bond; R^{J} stands for a substituted or unsubstituted alkyl, alkenyl or alkynyl group having 1 to 10 carbon atoms; R^{K} stands for a substituted or unsubstituted alkyl, alkenyl or alkynyl group having 1 to 10 carbon atoms; and R^{L} stands for a hydrogen atom or a protecting group for a hydroxyl group, provided that R^{K} is not 2-octenyl, 8-acetoxy-2-octenyl or 2,5-octadienyl,
and states that the above-described compounds are useful for the treatment of a malignant tumor.

The present inventors have reported, in The 109th Annual Meeting of the Pharmaceutical Society of Japan (Preprints on page 117), that 2-methylthio-2-cyclopentenone compounds represented by the following formula have an anticancer activity: 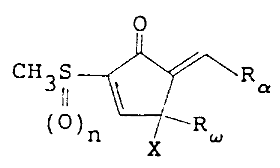 wherein in the specific compounds reported, n is 0, 1 or 2, and either X is OH, R_{α} is -CH=CH-CH(OH)(CH₂)₃OH and R_{ω} is -(CH₂)₄OPh, or X is H, R_{α} is -(CH₂)₅CO₂CH₃ and R_{ω} is -CH=CH-CH(OH)(CH₂)₄CH₃.

It is commonly recognized that the metabolism of the bone of healthy persons is established by a good balance of the repetition of the absorption of bone by osteoclasts and the formation of bone by osteoblasts, and it is considered that the breaking of the balance between-the absorption of bone and the formation of bone leads to diseases such as osteoporosis and osteomalalacia. Active vitamin preparations, calcitonin preparations, diphosphonic acid preparations, estrogen preparations, calcium preparations, etc. are used as a therapeutic agent for these diseases of bone. Nevertheless, although it has been reported that many of these preparations have a bone absorption inhibitory activity, there is no report clearly showing that they exhibit a bone formation accelerative activity. Further, since the effect of these preparations is not clear, the development of a preparation capable of more surely attaining the effect and having an activity through which the formation of bone by the osteoblasts is accelerated has been desired in the art.

Koshihara et al. reported in Biochemical Society of Japan (Preprints, 1988, p.767) that prostaglandin D₂ has an activity through which the calcification by human osteoblasts is accelerated, and suggested that this activity is derived from the action of ¹Δ-prostaglandin J₂ , which is a decomposition product of the prostaglandin D₂.

### Problem to be Solved by the Invention

As described in European Unexamined Patent Publication No. 338796 (publication date: October 25, 1989), the present inventors found that 2-cyclopentenone compounds having in their 2-position a group bonded to the skeleton through a sulfur atom represented by the following formula have an antitumor activity and a bone formation accelerative activity: 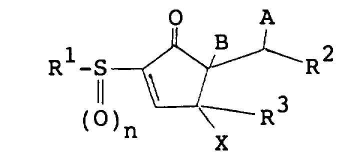 wherein A stands for a hydroxyl group or 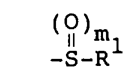 and B stands for a hydrogen atom, or A and B are combined with each other to form a bond line;
R₁ stands for a substituted or unsubstituted hydrocarbon group having 1 to 10 carbon atoms;
R₂ stands for a substituted or unsubstituted aliphatic hydrocarbon group having 1 to 10 carbon atoms;
R₃ stands for a substituted or unsubstituted aliphatic hydrocarbon group having 1 to 10 carbon atoms, provided that when R₃ is bonded to the cyclopentene skeleton through a single bond, X stands for a hydrogen atom, a hydroxyl group or a protected hydroxyl group, and when R₃ is bonded to the cyclopentene skeleton through a double bond, X stands for a bond constituting part of the double bond; and
m and n, which may be the same or different from each other, stand for 0, 1 or 2.

Further, the present inventors have made extensive and intensive studies, and as a result, have found that not only the above-described compounds but also a wider range of 2-cyclopentenone compounds having in their 2-position a group attached to the skeleton through a sulfur atom have a similar activity.

### Disclosure of the Invention

An object of the present invention is to provide a novel 2-substituted-2-cyclopentenone compound, that is, a 2-cyclopentenone compound having in its 2-position a group bonded to the skeleton through a sulfur atom.

Another object of the present invention is to provide a bone formation accelerator comprising a 2-substituted-2-cyclopentenone compound as an active ingredient.

A further object of the present invention is to provide a 2,3-epoxycyclopentanone compound or a 2-substituted-2-cyclopentenone compound useful as a starting compound for producing the 2-substituted-2-cyclopentenone compound of the present invention.

Other objects and advantages of the present invention will be apparent from the following description.

According to the present invention, there is provided a 2-substituted-2-cyclopentenone compound represented by the following formula (I): 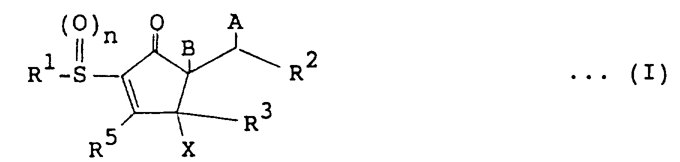 wherein
R¹ stands for a substituted or unsubstituted (i) acyclic aliphatic hydrocarbon group having 1 to 10 carbon atoms, (ii) alicyclic hydrocarbon group having 4 to 10 carbon atoms, (iii) aromatic hydrocarbon group having 6 to 10 carbon atoms or (iv) heterocyclic group having 1 to 9 carbon atoms;
R stands for a substituted or unsubstituted (i) acyclic aliphatic hydrocarbon group having 1 to 10 carbon atoms, (ii) alicyclic hydrocarbon group having 4 to 10 carbon atoms, (iii) aromatic hydrocarbon group having 6 to 10 carbon atoms or (iv) heterocyclic group having 1 to 9 carbon atoms;
R³ stands for a hydrogen atom or a substituted or unsubstituted (i) acyclic aliphatic hydrocarbon group having 1 to 10 carbon atoms, (ii) alicyclic hydrocarbon group having 4 to 10 carbon atoms or (iii) aromatic hydrocarbon group having 6 to 10 carbon atoms;
X stands for a hydrogen atom, or -OR⁴ (wherein R⁴ stands for a hydrogen atom, an alkyl group having 1 to 4 carbon atoms, an acyl group having 2 to 7 carbon atoms, an alkoxycarbonyl group having 2 to 5 carbon atoms, a tri(C₁ - C₇) hydrocarbon silyl group or a group capable of forming an acetal bond together with the oxygen atom attached to the R⁴), provided that X is absent when R³ is bonded to the carbon atom bonding thereto through a double bond;
R⁵ stands for a hydrogen atom, or a substituted or unsubstituted (i) acyclic aliphatic hydrocarbon group having 1 to 10 carbon atoms or (ii) an alicyclic hydrocarbon group having 4 to 10 carbon atoms;
B stands for a hydrogen atom when A stands for a hydrogen atom, a hydroxyl group, an acyloxy group having 2 to 7 carbon atoms, an alkoxycarbonyloxy group having 2 to 5 5 carbon atoms, a sulfonyloxy group having 1 to 7 carbon atoms or 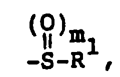 or A and B are combined with each other to form a bond line;
m and n, which may be the same or different from each other, stand for 0, 1 or 2,
exclusive of the cases where R¹ stands fora substituted or unsubstituted acyclic aliphatic hydrocarbon group, a substituted or unsubstituted alicyclic hydrocarbon group, or a substituted or unsubstituted aromatic hydrocarbon group, and R stands for a substituted or unsubstituted acyclic aliphatic hydrocarbon group, and R³ does not stand for a hydrogen atom, and R⁵ stands for a hydrogen atom, and either i) A stands for a hydroxyl group, a sulfonyloxy group or 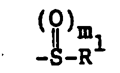 and B is a hydrogen atom or ii) A and B are combined with each other to form a bond line.

The term "aliphatic hydrocarbon group" as used hereinafter is intended to mean "acyclic aliphatic hydrocarbon group".

In the above-described formula (I), regarding A and B, when A is a hydrogen atom, a hydroxyl group, an acyloxy group having 2 to 7 carbon atoms, an alkoxy-carbonyloxy group having 2 to 5 carbon atoms, a sulfonyloxy group having 1 to 7 carbon atoms or 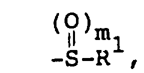 B is absent and a double bond exists between carbon atoms to which A and B are respectively bonded.

That is, when A stands for a hydrogen atom, a hydroxyl 5 group, an acyloxy group having 2 to 7 carbon atoms, an alkoxycarbonyloxy group having 2 to 5 carbon atoms, a sulfonyloxy group having 1 to 7 carbon atoms or 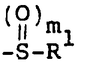 and B stands for a hydrogen atom, the above-described formula (I) represents 2-substituted-2-cyclopentenone compounds represented by the following formula (I-B'): 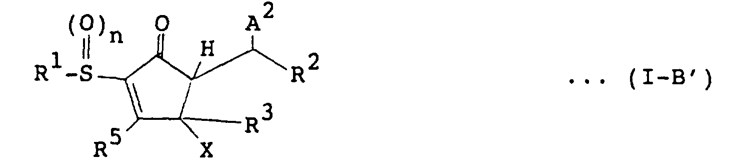 wherein R¹, R, R³, R⁵, X and n are as defined above, and
A stands for a hydrogen atom, a hydroxyl group, an acyloxy group having 2 to 7 carbon atoms, an alkoxycarbonyloxy group having 2 to 5 carbon atoms or a sulfonyloxy group having 1 to 7 carbon atoms;

When A stands for 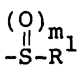 and B stands for a hydrogen atom, the above-described formula (I) represents 2-substituted-2-cyclopentenone compounds represented by the following formula (I-B'') 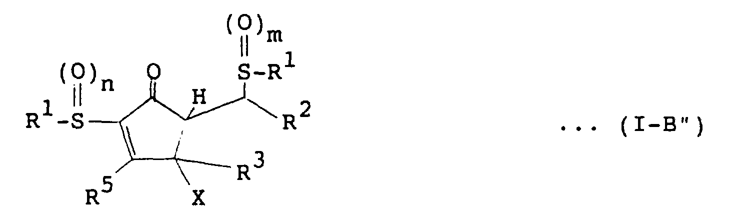 wherein R¹, R, R³, R⁵, X, m and n are as defined above; and

When A and B are combined with each other to form a bond line, the above-described formula (I) represents 2-substituted-2-cyclopentenone compounds represented by the following formula (I-A): 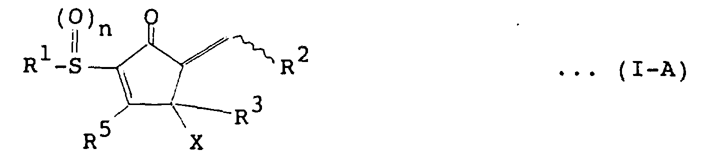 wherein R¹, R, R³, R⁵, X and n are as defined above and represents that the substituent attached to the double bond is in an E-configuration or a Z-configuration or a mixture thereof in any proportion, and further there are provided anticancer agents and bone formation accelerators comprising the above compounds as an active ingredient.

In the above-described formula (I), when A stands for an acyloxy group having 2 to 7 carbon atoms, examples of the acyloxy group having 2 to 7 carbon atoms include acetoxy, propionyloxy, isopropionyloxy, butyryloxy, isobutyryloxy, s-butyryloxy, valeryloxy, isovaleryloxy, hexanoyloxy, heptanoyloxy and benzoyloxy.

In the above-described formula (I), when A stands for an alkoxycarbonyloxy group having 2 to 5 carbon atoms, examples of the alkoxycarbonyloxy group having 2 to 5 carbon atoms include methoxycarbonyloxy, ethoxycarbonyloxy, propoxycarbonyloxy, isopropoxycarbonyloxy, butoxycarbonyloxy, isobutoxycarbonyloxy, s-butoxycarbonyloxy and t-butoxycarbonyloxy.

In the above-described formula (I), when A stands for a sulfonyloxy group having 1 to 7 carbon atoms, examples of the sulfonyloxy group having 1 to 7 carbon atoms include an alkylsulfonyloxy group having 1 to 4 carbon atoms which may be substituted with a halogen atom, a substituted or unsubstituted phenylsulfonyloxy group and a substituted or unsubstituted phenyl (C₁ - C₂) alkylsulfonyloxy group.

Examples of the alkylsulfonyloxy group having 1 to 4 carbon atoms which may be substituted with a halogen atom include methanesulfonyloxy, ethanesulfonyloxy, butanesulfonyloxy, t-butanesulfonyloxy, chloromethanesulfonyloxy, dichloromethanesulfonyloxy, trifluoromethanesulfonyloxy and nonafluorobutanesulfonyloxy groups. Examples of the substituted or unsubstituted phenylsulfonyloxy group include benzenesulfonyloxy, p-bromobenzenesulfonyloxy and toluenesulfonyloxy groups. Examples of the substituted or unsubstituted phenyl (C₁ - C₂) alkylsulfonyloxy group include benzylsulfonyloxy, α-phenetylsulfonyloxy and β-phenetylsulfonyloxy groups.

In the above-described formula (I), R¹ stands for a substituted or unsubstituted aliphatic hydrocarbon group having 1 to 10 carbon atoms, an alicyclic hydrocarbon group having 4 to 10 carbon atoms, an aromatic hydrocarbon group having 6 to 10 carbon atoms or a heterocyclic group having 1 to 9 carbon atoms.

Examples of the unsubstituted aliphatic hydrocarbon group having 1 to 10 carbon atoms in the R¹ include alkyl groups such as methyl, ethyl, propyl, isopropyl, butyl, isobutyl, s-butyl, t-butyl, pentyl, isopentyl, neopentyl, t-pentyl, hexyl, heptyl, octyl, 3,7-dimethyloctyl, nonyl and decyl groups; alkenyl groups such as 2-propenyl, 2-butenyl, 3-butenyl, 2-pentenyl, 4-pentenyl, 2-hexenyl, 3-hexenyl and 5-hexenyl; and alkynyl groups such as 2-propynyl, 2-butynyl, 2-pentynyl, 2-hexynyl and 3-hexynyl.

Examples of the unsubstituted alicyclic hydrocarbon groups having 4 to 10 carbon atoms in the R¹ include cyclobutyl, cyclopentyl, cyclohexyl, 3-cyclohexenyl, 4-cyclohexenyl, cycloheptyl, cyclooctyl and bicyclo[4.4.0]decan-2-yl groups.

Examples of the unsubstituted aromatic hydrocarbon group having 6 to 10 carbon groups in the R¹ include phenyl,1-naphthyl and 2-naphthyl groups.

Examples of the unsubstituted heterocyclic group having 1 to 9 carbon atoms in the R¹ include monocyclic or bicyclic groups having an oxygen, nitrogen or sulfur atom, such as furyl, thienyl, pyrrolyl, oxazolyl, isooxazolyl, thiazolyl, isothiazolyl, imidazolyl, pyrazolyl, triazolyl, tetrazolyl, pyranyl, pyridyl, pyrazinyl, pyrimidinyl, benzofuranyl, indolyl, benzimidazolyl, benzothiazolyl, benzoxazolyl, quinolyl, isoquinolyl, quinazolyl, purinyl, pteridinyl, morpholinyl and piperidinyl groups.

R¹ may be a group comprising, attached to each other, any combination of the above-described aliphatic hydrocarbon group having 1 to 10 carbon atoms, alicyclic hydrocarbon group having 4 to 10 carbon atoms, aromatic hydrocarbon group having 6 to 10 carbon atoms and heterocyclic group having 1 to 9 carbon atoms. Among them, preferred examples of the R¹ include a substituted or unsubstituted
(R¹-a) aliphatic hydrocarbon group having 1 to 10 carbon atoms which may be substituted with one or a plurality of alkoxy groups having 1 to 4 carbon atoms;
(R¹-b) alicyclic hydrocarbon group having 4 to 10 carbon atoms which may be substituted with one or a plurality of alkyl groups having 1 to 4 carbon atoms or alkoxy groups having 1 to 4 carbon atoms;
(R¹-c) aromatic hydrocarbon group having 6 to 10 carbon atoms which may be substituted with one or a plurality of alkyl groups having 1 to 4 carbon atoms or alkoxy groups having 1 to 4 carbon atoms;
(R¹-d) heterocyclic group having 1 to 9 carbon atoms which may be substituted with one or a plurality of alkyl groups having 1 to 4 carbon atoms or alkoxy groups having 1 to 4 carbon atoms;
(R¹-e) aliphatic hydrocarbon group having 1 to 10 carbon atoms substituted with an alicyclic hydrocarbon group having 4 to 10 carbon atoms and which may be substituted with one or a plurality of alkyl groups having 1 to 4 carbon atoms or alkoxy groups having 1 to 4 carbon atoms;
(R¹-f) aliphatic hydrocarbon group having 1 to 10 carbon atoms substituted with an aromatic hydrocarbon group having 6 to 10 carbon atoms and which may be substituted with one or a plurality of alkyl groups having 1 to 4 carbon atoms or alkoxy groups having 1 to 4 carbon atoms; and
(R¹-g) aliphatic hydrocarbon group having 1 to 10 carbon atoms substituted with a heterocyclic group having 1 to 9 carbon atoms and which may be substituted with one or a plurality of alkyl groups having 1 to 4 carbon atoms or alkoxy groups having 1 to 4 carbon atoms.

Preferred examples of the unsubstituted group (r¹-a) include methyl, ethyl, propyl, isopropyl, butyl, isobutyl, s-butyl, t-butyl, pentyl, hexyl, octyl, decyl, 2-propenyl and 4-pentenyl groups. Preferred examples of the unsubstituted group (r¹-b) include cyclopentyl, cyclohexyl, cyclooctyl, 3-methylcyclopentyl, 4-methylcyclohexyl, 4-butylcyclohexyl, 3,4-dimethylcyclohexyl and 4-methoxycyclohexyl groups. Preferred examples of the unsubstituted group (r¹-c) include phenyl, 1-naphthyl, 2-naphthyl, 2-methylphenyl, 3-methylphenyl, 4-methylphenyl, 2,4-dimethylphenyl, 3,5-dimethylphenyl, 4-ethylphenyl, 4-butylphenyl, 3-t-butylphenyl, 2-methoxyphenyl, 3-methoxyphenyl, 4-methoxyphenyl, 4-ethoxyphenyl, 6-methoxy-2-naphthyl, 7-methoxy-2-naphthyl and 6,7-dimethoxy-2-naphthyl groups. Preferred examples of the unsubstituted group (r¹-d) include 2-oxazolyl, 2-thiazolyl, 2-imidazolyl, 2-pyridyl, 4-pyridyl, 2-pyrimidinyl, 2-benzimidazolyl, 2-benzoxazolyl, 2-benzothiazolyl, 6-purinyl, 1-methylimidazol-2-yl, 4-methyl-1,2,4-triazol-3-yl, 1-methyl-5-tetrazolyl, 5-methyl-2-benzimidazolyl, 6-ethoxy-2-benzothiazolyl, 4-methyl-2-pyrimidinyl, 4,6-dimethyl-2-pyrimidinyl and 6-propyl-2-pyrimidinyl groups.

Preferred examples of the unsubstituted group (r¹-e) include cyclopentylmethyl, cyclohexylmethyl, 2-cyclohexylethyl, 2-cyclohexylpropyl, 3-cyclohexylpropyl, 4-cyclohexylbutyl, 1-cyclohexyl-1-methylethyl, (4-t-butylcyclohexyl)methyl and (4-methoxycyclohexyl)methyl groups. Preferred examples of the unsubstituted group (r¹-f) include benzyl, 2-phenylethyl, 3-phenylpropyl, 4-phenylbutyl, (1-naphthyl)methyl, (2-naphthyl)methyl, 2-(2-naphthyl)ethyl, (4-methylphenyl)methyl, (3-methylphenyl)methyl, (4-ethylphenyl)methyl, (4-butylphenyl)methyl, (4-methoxyphenyl)methyl, 2-(3,4-dimethoxyphenyl)ethyl and (6-methoxynaphthyl)methyl groups. Preferred examples of the unsubstituted group (r¹-g) include furfuryl and 3-(4-morpholinyl)propyl groups.

The above-described groups (r¹-a) to (r¹-g) may be substituted with a plurality of different groups, and examples of the substituent include (i) a halogen atom; (ii) an oxo group; (iii) a cyano group; (iv) a nitro group; (v) -COOR⁶¹ (wherein R⁶¹ stands for a hydrogen atom; one equivalent of cation; a residue of a saccharide; or an aliphatic hydrocarbon group having 1 to 10 carbon atoms which may be substituted with a halogen atom, an oxo group, a hydroxyl group, a tri(C₁ - C₇) hydrocarbon silyloxy group, an acyloxy group having 2 to 7 carbon atoms, an alkoxycarbonyloxy group having 2 to 5 carbon atoms, an alkoxy group having 1 to 4 carbon atoms, an alicyclic hydrocarbon group having 4 to 10 carbon atoms or an aromatic hydrocarbon group having 6 to 10 carbon atoms); (vi) -OR⁷¹ (wherein R⁷¹ stands for a hydrogen atom; an alkyl group having 1 to 4 carbon atoms; an acyl group having 2 to 7 carbon atoms; an alkoxycarbonyl group having 2 to 5 carbon atoms; a tri(C₁ - C₇) hydrocarbon silyl group; a group capable of forming an acetal bond together with the oxygen atom attached to the R⁷¹; an aliphatic hydrocarbon group having 1 to 10 carbon atoms or an alicyclic hydrocarbon group having 4 to 10 carbon atoms which may be substituted with a halogen atom, an oxo group, a hydroxyl group, a carboxyl group, a tri(C₁ - C₇) hydrocarbon silyloxy group, an acyloxy group having 2 to 7 carbon atoms, an alkoxycarbonyloxy group having 2 to 5 carbon atoms, an alkoxy group having 1 to 4 carbon atoms, an alkoxycarbonyl group having 2 to 5 carbon atoms or an aromatic hydrocarbon group having 6 to 10 carbon atoms; or an aromatic hydrocarbon group having 6 to 10 carbon atoms which may be substituted with a halogen atom, a hydroxyl group, a nitro group, a tri(C₁ - C₇) hydrocarbon silyloxy group, an alkoxy group having 1 to 4 carbon atoms, an acyloxy group having 2 to 7 carbon atoms, an alkoxycarbonyloxy group having 2 to 5 carbon atoms, an acyl group having 2 to 7 carbon atoms, a carboxyl group, an alkoxycarbonyl group having 2 to 5 carbon atoms or an alkyl group having 1 to 4 carbon atoms); (vii) -CONR⁸¹R⁸¹⁰ (wherein R⁸¹ and R⁸¹⁰ which may be the same or different from each other stand for a hydrogen atom; an aliphatic hydrocarbon group having 1 to 10 carbon atoms or an alicyclic hydrocarbon group having 4 to 10 carbon atoms which may be substituted with a halogen atom, an oxo group, a hydroxyl group, a tri(C₁ - C₇) hydrocarbon silyloxy group, an acyloxy group having 2 to 7 carbon atoms, an alkoxycarbonyloxy group having 2 to 5 carbon atoms, an alkoxy group having 1 to 4 carbon atoms or an aromatic hydrocarbon group having 6 to 10 carbon atoms; an aromatic hydrocarbon group having 6 to 10 carbon atoms which may be substituted with a halogen atom, a hydroxyl group, a nitro group, a tri(C₁ - C₇) hydrocarbon silyloxy group, an acyloxy group having 2 to 7 carbon atoms, an alkoxycarbonyloxy group having 2 to 5 carbon atoms, an alkoxy group having 1 to 4 carbon atoms, an acyl group having 2 to 7 carbon atoms, a carboxyl group, an alkoxycarbonyl group having 2 to 5 carbon atoms or an alkyl group having 1 to 4 carbon atoms; or a group wherein R⁸¹ and R⁸¹⁰ are combined with each other to form a five- or six-membered ring); and (viii) -NR⁹¹R⁹¹⁰ (wherein R⁹¹ and R⁹¹⁰ which may be the same or different from each other stand for a hydrogen atom; an aliphatic hydrocarbon group having 1 to 10 carbon atoms or an alicyclic hydrocarbon group having 4 to 10 carbon atoms which may be substituted with a halogen atom, an oxo group, a hydroxyl group, a tri(C₁ - C₇) hydrocarbon silyloxy group, an acyloxy group having 2 to 7 carbon atoms, an alkoxycarbonyloxy group having 2 to 5 carbon atoms, an alkoxy group having 1 to 4 carbon atoms or an aromatic hydrocarbon group having 6 to 10 carbon atoms; an aromatic hydrocarbon group having 6 to 10 carbon atoms which may be substituted with a halogen atom, a hydroxyl group, a nitro group, a tri(C₁ - C₇) hydrocarbon silyloxy group, an acyloxy group having 2 to 7 carbon atoms, an alkoxycarbonyloxy group having 2 to 5 carbon atoms, an alkoxy group having 1 to 4 carbon atoms or an alkyl group having 1 to 4 carbon atoms; or a group wherein R⁹¹ and R⁹¹⁰ are combined with each other to form a five- or six-membered ring).

Preferred examples of the halogen atom as the substituent (i) include fluorine, chlorine and bromine atoms.

Examples of the R⁶¹ in the group represented by the formula -COOR⁶¹ as the substituent (v) include a hydrogen atom; cations such as ammonium, tetramethylammonium, cyclohexylammonium, benzylammonium and phenetyl ammonium, a morpholinium cation, a piperidinium cation and one equivalent of cations such as Na⁺, K⁺, 1/2Ca⁺, 1/2Mg⁺ and 1/3Al³⁺; residues of saccharides, for example, monosaccharides such as altrose, glucose, mannose, galactose, ribose, arabinose, xylose and fructose, or deoxy saccharides thereof; and aliphatic hydrocarbon groups having 1 to 10 carbon atoms, i.e., alkyl groups such as methyl, ethyl, propyl, isopropyl, butyl, isobutyl, s-butyl, t-butyl, pentyl, isopentyl, neopentyl, hexyl, octyl and decyl groups, alkenyl groups such as 2-propenyl, 2-butenyl, 2-pentenyl, 2-hexenyl and 5-hexenyl groups and alkynyl group such as 2-butynyl, 2-pentynyl and 3-hexynyl groups, which may be substituted with a halogen atom such as fluorine, chlorine or bromine, an oxy group, a hydroxyl group, a tri(C₁ - C₇) hydrocarbon silyloxy group such as a trimethylsilyloxy, triethylsilyloxy or t-butyldimethylsilyloxy group, an acyloxy group having 2 to 7 carbon atoms such as an acetoxy, propionyloxy, a butyryloxy, isobutyryloxy, valeryloxy or benzoyloxy group, an alkoxycarbonyloxy group having 2 to 5 carbon atoms such as a methoxycarbonyloxy, ethoxycarbonyloxy, propoxycarbonyloxy, isopropoxycarbonyloxy or butoxycarbonyloxy group, an alkoxy group having 1 to 4 carbon atoms such as a methoxy, ethoxy, propoxy, isopropoxy, butoxy, isobutoxy, s-butoxy or t-butoxy group an alicyclic hydrocarbon group having 4 to 10 carbon atoms such as a cyclobutyl, cyclopentyl or cyclohexyl group, or an aromatic hydrocarbon group having 6 to 10 carbon atoms such as a phenyl, 1-naphthyl or 2-naphthyl group.

Examples of the R⁷¹ in the group represented by the formula -OR⁷¹ as the substituent (iv) include a hydrogen atom; alkyl groups having 1 to 4 carbon atoms such as methyl, ethyl, propyl, isopropyl, butyl, isobutyl, s-butyl and t-butyl groups; acyl groups having 2 to 7 carbon atoms such as acetyl, propionyl, butyryl, isobutyryl, valeryl, isovaleryl, hexanoyl, heptanoyl and benzoyl groups; alkoxycarbonyl groups having 2 to 5 carbon atoms such as methoxycarbonyl, ethoxycarbonyl, propoxycarbonyl, isopropoxycarbonyl, butoxycarbonyl, isobutoxycarbonyl and t-butoxycarbonyl groups; tri(C₁ - C₇) hydrocarbon silyl groups such as trimethylsilyl, triethylsilyl, t-butyldimethylsilyl, t-butyldiphenylsilyl and tribenzylsilyl groups; a group capable of forming acetal bond together with the oxygen atom attached to the R⁷¹, such as a methoxymethyl, 1-ethoxyethyl, 1-methoxy-1-methylethyl, 2-ethoxy-1-methylethyl, 2-methoxyethoxymethyl, tetrahydropyran-2-yl or tetrahydrofuran-2-yl, is attached; aliphatic hydrocarbon groups having 1 to 10 carbon atoms, i.e., alkyl groups such as methyl, ethyl, propyl, isopropyl, butyl, s-butyl, t-butyl, pentyl, isopentyl, neopentyl, t-pentyl, hexyl, heptyl, octyl, nonyl and decyl groups, alkenyl groups such as 2-propenyl, 2-butenyl, 3-butenyl and 3-hexenyl groups and alkynyl groups such as 2-propynyl, 2-butynyl and 3-hexynyl groups, or alicyclic hydrocarbon groups having 4 to 10 carbon atoms such as cyclobutyl, cyclopentyl, cyclohexyl, 3-cyclohexenyl, 4-cyclohexenyl, cyclooctyl and bicyclo[4.4.0]decan-2-yl groups, which may be substituted with a halogen atom such as fluorine, chlorine or bromine, an oxo group, a hydroxyl group, a carboxyl group, a tri(C₁ - C₇) hydrocarbon silyloxy group such as a trimethylsilyloxy, triethylsilyloxy or t-butyldimethylsilyloxy group, an acyloxy group having 2 to 7 carbon atoms such as an acetoxy, propionyloxy, butyryloxy, isobutyryloxy, valeryloxy, isovaleryloxy or benzoyloxy group, an alkoxycarbonyloxy group having 2 to 5 carbon atoms such as a methoxycarbonyloxy, ethoxycarbonyloxy, propoxycarbonyloxy, isopropoxycarbonyloxy or butoxycarbonyloxy group, an alkoxy group having 1 to 4 carbon atoms such as a methoxy, ethoxy propoxy, isopropoxy, butoxy, isobutoxy or t-butoxy group, an alkoxycarbonyl group having 2 to 5 carbon atoms such as a methoxycarbonyl, ethoxycarbonyl, isobutoxycarbonyl or t-butoxycarbonyl group, or an aromatic hydrocarbon group having 6 to 10 carbon atoms such as a phenyl, 1-naphthyl or 2-naphthyl group; and aromatic hydrocarbon groups having 6 to 10 carbon atoms such as phenyl, 1-naphthyl and 2-naphthyl groups, which may be substituted with a halogen atom such as fluorine, chlorine or bromine, an oxo group, a hydroxyl group, a nitro group, a tri(C₁ - C₇) hydrocarbon silyloxy group such as a trimethylsilyloxy, triethylsilyloxy or t-butyldimethylsilyloxy group, an alkoxy group having 1 to 4 carbon atoms such as a methoxy, ethoxy, propoxy, isopropoxy, butoxy, isobutoxy, s-butoxy or t-butoxy group, an acyloxy group having 2 to 7 carbon atoms such as an acetoxy, propionyloxy, butyryloxy, isobutyryloxy, valeryloxy, hexanoyloxy or benzoyloxy group, an alkoxycarbonyloxy group having 2 to 5 carbon atoms such as a methoxycarbonyloxy, ethoxycarbonyloxy, propoxycarbonyloxy, isopropoxycarbonyloxy, butoxycarbonyloxy group or t-butoxycarbonyloxy group, an acyl group having 2 to 7 carbon atoms such as an acetyl, propionyl, butyryl, isobutyryl, valeryl, hexanoyl, heptanoyl or benzoyl group, a carboxyl group, an alkoxycarbonyl group having 2 to 5 carbon atoms such as a methoxycarbonyl, ethoxycarbonyl, propoxycarbonyl, isopropoxycarbonyl, butoxycarbonyl, isobutoxycarbonyl or t-butoxycarbonyl group, or an alkyl group such as a methyl, ethyl, propyl, isopropyl, butyl, isobutyl, s-butyl or t-butyl group.

Examples of the R⁸¹ and R⁸¹⁰ in the group represented by the formula -CONR⁸¹R⁸¹⁰ as the substituent (vii) include a hydrogen atom; aliphatic hydrocarbon groups having 1 to 10 carbon atoms, i.e., alkyl groups such as methyl, ethyl, propyl, isopropyl, butyl, isobutyl, s-butyl, t-butyl, pentyl, isopentyl, neopentyl, t-pentyl, hexyl, heptyl, octyl, nonyl and decyl groups, alkenyl groups such as 2-propenyl, 2-butenyl, 2-pentenyl and 3-hexenyl groups and alkynyl groups such as 2-propynyl, 2-butynyl, 2-pentynyl and 3-hexynyl groups, or alicyclic hydrocarbon groups having 4 to 10 carbon atoms such as cyclobutyl, cyclopentyl, cyclohexyl, cyclooctyl and bicyclo[4.4.0]decan-2-yl groups, which may be substituted with a halogen atom such as fluorine, chlorine or bromine, an oxo group, a hydroxyl group, a tri(C₁ - C₇) hydrocarbon silyloxy group such as a trimethylsilyloxy, triethylsilyloxy or t-butyldimethylsilyloxy group, an acyloxy group having 2 to 7 carbon atoms such as an acetoxy, propionyloxy, butyryloxy, isobutyryloxy, valeryloxy, hexanoyloxy or benzoyloxy group, an alkoxycarbonyloxy group having 2 to 5 carbon atoms such as a methoxycarbonyloxy, ethoxycarbonyloxy, propoxycarbonyloxy, isopropoxycarbonyloxy, butoxycarbonyloxy group or t-butoxycarbonyloxy group, an alkoxy group having 1 to 4 carbon atoms such as a methoxy, ethoxy, propoxy, isopropoxy, butoxy, isobutoxy, s-butoxy or t-butoxy group, or an aromatic hydrocarbon group having 6 to 10 carbon atoms such as a phenyl, 1-naphthyl or 2-naphthyl group; aromatic hydrocarbon groups having 6 to 10 carbon atoms such as phenyl, 1-naphthyl and 2-naphthyl groups, which may be substituted with a halogen atom such as fluorine, chlorine or bromine, a hydroxyl group, a nitro group, a tri(C₁ - C₇) hydrocarbon silyloxy group such as a trimethylsilyloxy, triethylsilyloxy or t-butyldimethylsilyloxy group, an acyloxy group having 2 to 7 carbon atoms such as an acetoxy, propionyloxy, butyryloxy, isobutyryloxy, valeryloxy, hexanoyloxy or benzoyloxy group, an alkoxycarbonyloxy group having 2 to 5 carbon atoms such as a methoxycarbonyloxy, ethoxycarbonyloxy, propoxycarbonyloxy, isopropoxycarbonyloxy, butoxycarbonyloxy group or t-butoxycarbonyloxy group, an alkoxy group having 1 to 4 carbon atoms such as a methoxy, ethoxy, propoxy, isopropoxy, butoxy, isobutoxy, s-butoxy or t-butoxy group, an acyl group having 2 to 7 carbon atoms such as an acetyl, propionyl, butyryl, isobutyryl, valeryl, hexanoyl or benzoyl group, a carboxyl group, an alkoxycarbonyl group having 2 to 5 carbon atoms such as a methoxycarbonyl, ethoxycarbonyl, propoxycarbonyl, isopropoxycarbonyl, butoxycarbonyl, isobutoxycarbonyl or t-butoxycarbonyl group, or an alkyl group having 1 to 4 carbon atoms such as a methyl, ethyl, propyl, isopropyl, butyl, isobutyl, s-butyl or t-butyl group; or a group wherein the R⁸¹ and R⁸¹⁰ are combined with each other to form a five- or six-membered ring together with a nitrogen atom intervening between the R⁸¹ and the R⁸¹⁰, for example, 1-pyrrolidinyl, 1-piperidinyl, 1-imidazolidinyl, 1-piperazinyl, 4-morpholinyl and 2-thioxo-3-thiazolidinyl groups, which may be substituted with an alkoxy group having 1 to 4 carbon atoms such as methoxy, ethoxy, propoxy, isopropoxy, butoxy, isobutoxy, s-butoxy or t-butoxy group, or an alkyl group having 1 to 4 carbon atoms such as a methyl, ethyl, propyl, isopropyl, butyl, isobutyl, s-butyl or t-butyl group.

Examples of the R⁹¹ and R⁹¹⁰ in the group represented by the formula -NR⁹¹R⁹¹⁰ as the substituent (viii) include a hydrogen atom; aliphatic hydrocarbon groups having 1 to 10 carbon atoms, i.e., alkyl groups such as methyl, ethyl, propyl, isopropyl, butyl, isobutyl, s-butyl, t-butyl, pentyl, isopentyl, neopentyl, hexyl, octyl and decyl groups, alkenyl groups such as 2-propenyl, 2-butenyl, 2-pentenyl, 2-hexenyl and 5-hexenyl groups and alkynyl groups such as 2-butynyl, 2-pentynyl and 3-hexynyl groups, or alicyclic hydrocarbon groups having 4 to 10 carbon atoms such as cyclobutyl, cyclopentyl, cyclohexyl, cycloheptyl, cyclooctyl and bicyclo[4.4.0]decan-2-yl groups, which may be substituted with a halogen atom such as fluorine, chlorine or bromine, an oxo group, a hydroxyl group, a tri(C₁ - C₇) hydrocarbon silyloxy group such as a trimethylsilyloxy, triethylsilyloxy or t-butyldimethylsilyloxy group, an acyloxy group having 2 to 7 carbon atoms such as an acetoxy, propionyloxy, butyryloxy, isobutyryloxy, valeryloxy, hexanoyloxy or benzoyloxy group, an alkoxycarbonyloxy group having 2 to 5 carbon atoms such as a methoxycarbonyloxy, ethoxycarbonyloxy, propoxycarbonyloxy, isopropoxycarbonyloxy, butoxycarbonyloxy or t-butoxycarbonyloxy group, an alkoxy group having 1 to 4 carbon atoms such as a methoxy, ethoxy, propoxy, isopropoxy, butoxy, s-butoxy or t-butoxy group, or an aromatic hydrocarbon group having 6 to 10 carbon atoms such as a phenyl, 1-naphthyl or 2-naphthyl group; aromatic hydrocarbon groups having 6 to 10 carbon atoms such as phenyl, 1-naphthyl and 2-naphthyl groups, which may be substituted with a halogen atom such as fluorine, chlorine or bromine, a hydroxyl group, a nitro group, a tri(C₁ - C₇) hydrocarbon silyloxy group such as a trimethylsilyloxy, triethylsilyloxy or t-butyldimethylsilyloxy group, an acyloxy group having 2 to 7 carbon atoms such as an acetoxy, propionyloxy, butyryloxy, isobutyryloxy, valeryloxy, hexanoyloxy or benzoyloxy group, an alkoxycarbonyloxy group having 2 to 5 carbon atoms such as a methoxycarbonyloxy, ethoxycarbonyloxy, propoxycarbonyloxy, isopropoxycarbonyloxy or butoxycarbonyloxy group, an alkoxy group having 1 to 4 carbon atoms such as a methoxy, ethoxy, propoxy, isopropoxy, butoxy, isobutoxy or t-butoxy group, or an alkyl group such as a methyl, ethyl, propyl, isopropyl, butyl, isobutyl, s-butyl or t-butyl group; or a group wherein the R⁹¹ and R⁹¹⁰ are combined with each other to form a five- or six-membered ring together with a nitrogen atom intervening between the R⁹¹ and the R⁹¹⁰, for example, 1-pyrrolidinyl, 1-piperidinyl, 1-imidazolidinyl, 1-piperazinyl, 4-morpholinyl and 2-thioxo-3-thiazolidinyl groups, which may be substituted with an alkoxy group having 1 to 4 carbon atoms, such as methoxy, ethoxy, propoxy, isopropoxy, butoxy, isobutoxy or t-butoxy group, or an alkyl group having 1 to 4 carbon atoms such as a methyl, ethyl, propyl, isopropyl, butyl, isobutyl or t-butyl group.

Preferred examples of the R¹ include methyl, ethyl, propyl, 2-propenyl, isopropyl, butyl, isobutyl, s-butyl, t-butyl, hexyl, octyl, 2,3-dihydroxypropyl, 2,3-diacetoxypropyl, 3,4-dimethoxyphenylpropyl, 4-phenoxybutyl, carboxymethyl, methoxycarbonylmethyl, ethoxycarbonylmethyl, 2-carboxyethyl, 5-carboxypentyl, 5-methoxycarbonylpentyl, 5-butoxycarbonylpentyl, 5-decyloxycarbonylpentyl, 5-carboxy-4-pentenyl, 5-methoxycarbonyl-4-pentenyl, 5-methoxycarbonyl-5,5-difluoropentyl, 5-(2-thioxo-3-thiazolidinylcarbonyl)pentyl, 5-(6-D-glucosylcarbonyl)pentyl, 5-(1-D-xylosylcarbonyl)pentyl, 5-(5-D-ribosylcarbonyl)pentyl, 2-(butylamino)ethyl, 2-(4-fluorophenylamino)ethyl, 2-(2-phenylethylamino)ethyl, cyclohexyl, phenyl, 3-methylphenyl, 4-methylphenyl, 3-methoxyphenyl, 4-methoxyphenyl, 4-fluorophenyl, 4-chlorophenyl, 4-bromophenyl, 2,5-dichlorophenyl, 4-bromo-3-methylphenyl, 2,3,5,6-tetrafluorophenyl, 2,4,5-trichlorophenyl, 3-aminophenyl, 4-aminophenyl, 4-nitrophenyl, 2-pyridinyl, 4-pyridinyl, 3-hydroxy-2-pyridinyl, 4-hydroxy-2-pyrimidinyl, 4,6-dimethyl-2-pyrimidinyl, 4,6-dichloro-2-pyrimidinyl, 4-hydroxy-6-propyl-2-pyrimidinyl, 4,5-diamino-2-pyrimidinyl, 4,6-diamino-2-pyrimidinyl, 4-amino-6-hydroxy-2-pyrimidinyl, 1-methyl-2-imidazolyl, 4-methyl-1,2,4-triazole-3-yl, 1-methyl-5-tetrazolyl, 2-benzimidazolyl, 2-benzoxazolyl, 2-benzothiazolyl, 5-chloro-2-benzothiazolyl, 6-ethoxy-2-benzothiazolyl, 2-quinolinyl, 4-hydroxy-pteridinyl, 6-hydroxy-8-purinyl, 6-purinyl, 4-pyrazolo[3,4-d]pyrimidinyl, 2-amino-6-purinyl, 6-hydroxy-2-purinyl, 2-hydroxy-6-purinyl, 3-cyclohexylpropyl, benzyl, (2-chlorophenyl)methyl, (4-chlorophenyl)methyl, 2-(3,4-dichlorophenyl)ethyl, (3-fluorophenyl)methyl, (4-methoxyphenyl)methyl, (3-trifluoromethylphenyl)methyl and 3-phenylpropyl, 2-furanylmethyl, 3-(4-morpholinyl)propyl.

In the above-described formula (I), the R stands for a substituted or unsubstituted aliphatic hydrocarbon group having 1 to 10 carbon atoms, alicyclic hydrocarbon group having 4 to 10 carbon atoms, aromatic hydrocarbon having 6 to 10 carbon atoms or heterocyclic group having 1 to 9 carbon atoms. Examples of the unsubstituted aliphatic hydrocarbon group having 1 to 10 carbon atoms in the R include alkyl groups such as methyl, ethyl, propyl, isopropyl, butyl, isobutyl, s-butyl, t-butyl, pentyl, isopentyl, neopentyl, 4-methylpentyl, hexyl, heptyl, octyl, 3,7-dimethyloctyl, nonyl and decyl groups; alkenyl groups such as vinyl, 1-methylvinyl, 1-ethylvinyl, 1-propenyl, 2-propenyl, 2-methyl-1-propenyl, 1-butenyl, 2-butenyl, 2-methyl-1-butenyl, 1,3-butadienyl, 1-pentenyl, 2-pentenyl, 4-methyl-1-pentenyl, 1-hexenyl, 2-hexenyl, 3-hexenyl, 5-hexenyl, 1,5-hexadienyl, 1-heptenyl, 1-octenyl, 6-methyl-1-heptenyl, 1-nonenyl and 1-decenyl groups; and alkynyl groups such as ethynyl, 1-propynyl, 2-propynyl, 1-butynyl, 3-methyl-1-butynyl, 3,3-dimethyl-1-butynyl, 1-pentynyl, 2-pentynyl, 1-hexynyl, 2-hexynyl, 5-hexen-1-yl, 1-heptynyl, 1-nonynyl and 1-decynyl groups.

Examples of the unsubstituted alicyclic hydrocarbon group having 4 to 10 carbon atoms in the R include cyclobutyl, cyclopentyl, cyclohexyl, 1-cyclohexenyl, 3-cyclohexenyl, 4-cyclohexenyl, cycloheptyl, cyclooctyl and bicyclo[4.4.0]decan-2-yl. Examples of the unsubstituted aromatic hydrocarbon group having 6 to 10 carbon atoms in the R include phenyl, 1-naphthyl and 2-naphthyl groups. Examples of the unsubstituted heterocyclic hydrocarbon group having 1 to 9 carbon atoms in the R include monocyclic or bicyclic groups having an oxygen, nitrogen or sulfur atom, such as furyl, thienyl, pyrolyl, oxazolyl, isooxazolyl, thiazolyl, isothiazolyl, imidazolyl, pyrazolyl, triazolyl, tetrazolyl, pyranyl, pyridyl, pyrazinyl, pyrimidinyl, benzofuranyl, indolyl, benzimidazolyl, benzothiazolyl, benzoxazolyl, quinolyl, isoquinolyl, quinazolyl, purinyl, pteridinyl, morpholinyl and piperidinyl groups.

The R may be a group comprising, attached to each other, any combination of the above-described aliphatic hydrocarbon group having 1 to 10 carbon atoms, alicyclic hydrocarbon group having 4 to 10 carbon atoms, aromatic hydrocarbon group having 6 to 10 carbon atoms and heterocyclic group having 1 to 9 carbon atoms. Among them, preferred examples of the R include a substituted or unsubstituted
(r-a) aliphatic hydrocarbon group having 1 to 10 carbon atoms which may be substituted with one or a plurality of alkoxy groups having 1 to 4 carbon atoms;
(r-b) alicyclic hydrocarbon group having 4 to 10 carbon atoms which may be substituted with one or a plurality of alkyl groups having 1 to 4 carbon atoms or alkoxy groups having 1 to 4 carbon atoms;
(r-c) aromatic hydrocarbon group having 6 to 10 carbon atoms which may be substituted with one or a plurality of alkyl groups having 1 to 4 carbon atoms or alkoxy groups having 1 to 4 carbon atoms;
(r-d) heterocyclic group having 1 to 9 carbon atoms which may be substituted with one or a plurality of alkyl groups having 1 to 4 carbon atoms or alkoxy groups having 1 to 4 carbon atoms;
(r-e) aliphatic hydrocarbon group having 1 to 10 carbon atoms substituted with an alicyclic hydrocarbon group having 4 to 10 carbon atoms and which may be substituted with one or a plurality of alkyl groups having 1 to 4 carbon atoms or alkoxy groups having 1 to 4 carbon atoms;
(r-f) aliphatic hydrocarbon group having 1 to 10 carbon atoms substituted with an aromatic hydrocarbon group having 6 to 10 carbon atoms and which may be substituted with one or a plurality of alkyl groups having 1 to 4 carbon atoms or alkoxy groups having 1 to 4 carbon atoms; and
(r-g) aliphatic hydrocarbon group having 1 to 10 carbon atoms substituted with a heterocyclic group having 1 to 9 carbon atoms and which may be substituted with one or a plurality of alkyl groups having 1 to 4 carbon atoms or alkoxy groups having 1 to 4 carbon atoms.

Preferred examples of the unsubstituted group (r-a) include methyl, ethyl, propyl, isopropyl, butyl, isobutyl, s-butyl, pentyl, hexyl, octyl, nonyl, decyl, vinyl, 1-propenyl, 1-pentenyl, 4-pentenyl, 6-methyl-1-heptenyl, ethynyl, 1-propynyl, 1-pentynyl, 3-methoxypropyl and 1-(2-methoxyethyl)vinyl groups. Preferred examples of the unsubstituted group (r-b) include cyclopentyl, cyclohexyl, cyclooctyl, 3-methylcyclopentyl, 4-methylcyclohexyl, 4-butylcyclohexyl, 3,4-dimethylcyclohexyl and 4-methoxycyclohexyl groups. Preferred examples of the unsubstituted group (r-c) include phenyl, 1-naphthyl, 2-methylphenyl, 3-methylphenyl, 4-methylphenyl, 2,4-dimethylphenyl, 3,5-dimethylphenyl, 4-ethylphenyl, 4-isobutylphenyl, 3-t-butylphenyl, 2-methoxyphenyl, 3-methoxyphenyl, 4-methoxyphenyl, 4-ethoxyphenyl, 6-methoxy-2-naphthyl, 7-methoxy-2-naphthyl and 6,7-dimethoxy-2-naphthyl groups. Preferred examples of the unsubstituted group (r-d) include 2-oxazolyl, 2-thiazolyl, 2-imidazolyl, 2-pyridyl, 4-pyridyl, 2-pyrimidinyl, 2-benzimidazolyl, 2-benzoxazolyl, 2-benzothiazolyl, 6-purinyl, 1-methylimidazol-2-yl, 4-methyl-1,2,4-triazol-3-yl, 1-methyl-5-tetrazolyl, 5-methyl-2-benzimidazolyl, 4-methyl-2-pyrimidinyl and 6-propyl-2-pyrimidinyl groups.

Preferred examples of the unsubstituted group (r-e) include cyclopentylmethyl, cyclohexylmethyl, 2-cyclohexylmethyl, 2-cyclohexylpropyl, 3-cyclohexylpropyl, 4-cyclohexylbutyl, (4-t-butylcyclohexyl)methyl and (4-methoxycyclohexyl)methyl groups. Preferred examples of the unsubstituted group (r-f) include benzyl, 2-phenylethyl, 3-phenylpropyl, 4-phenylbutyl, (1-naphthyl)methyl, (2-naphthyl)methyl, 2-phenylvinyl, 2-phenyl-1-propenyl, (4-methylphenyl)methyl, (3-methylphenyl)methyl, (4-ethylphenyl)methyl, (4-butylphenyl)methyl, (4-methoxyphenyl)methyl and 2-(3,4-dimethoxyphenyl)ethyl groups. Preferred examples of the unsubstituted group (r-g) include furfuryl and 3-(4-morpholinyl)propyl groups.

The above-described groups (r-a) to (r-g) may be substituted with a plurality of different groups, and examples of the substituent include (i) a halogen atom; (ii) an oxo group; (iii) a cyano group; (iv) a nitro group; (v) -COOR⁶ (wherein R⁶ stands for a hydrogen atom; one equivalent of cation; a residue of a saccharide; or an aliphatic hydrocarbon group having 1 to 10 carbon atoms which may be substituted with a halogen atom, an oxo group, a hydroxyl group, a nitro group, a tri(C₁ - C₇) hydrocarbon silyloxy group, an acyloxy group having 2 to 7 carbon atoms, an alkoxycarbonyloxy group having 2 to 5 carbon atoms, an alkoxy group having 1 to 4 carbon atoms, an alicyclic hydrocarbon group having 4 to 10 carbon atoms or an aromatic hydrocarbon group having 6 to 10 carbon atoms); (vi) -OR⁷ (wherein R⁷ stands for a hydrogen atom; an alkyl group having 1 to 4 carbon atoms; an acyl group having 2 to 7 carbon atoms; an alkoxycarbonyl group having 2 to 5 carbon atoms; a tri(C₁ - C₇) hydrocarbon silyl group; a group capable of forming an acetal bond together with the oxygen atom attached to the R⁷; an aliphatic hydrocarbon group having 1 to 10 carbon atoms or an alicyclic hydrocarbon group having 4 to 10 carbon atoms which may be substituted with a halogen atom, an oxo group, a hydroxyl group, a carboxyl group, a tri(C₁ - C₇) hydrocarbon silyloxy group, an acyloxy group having 2 to 7 carbon atoms, an alkoxycarbonyloxy group having 2 to 5 carbon atoms, an alkoxy group having 1 to 4 carbon atoms, an alkoxycarbonyl group having 2 to 5 carbon atoms or an aromatic hydrocarbon group having 6 to 10 carbon atoms; or an aromatic hydrocarbon group having 6 to 10 carbon atoms which may be substituted with a halogen atom, hydroxyl group, a nitro group, a tri(C₁ - C₇) hydrocarbon silyloxy group, an alkoxycarbonyloxy group having 2 to 5 carbon atoms, an acyl group having 2 to 7 carbon atoms, a carboxyl group, an alkoxycarbonyl group having 2 to 5 carbon atoms or an alkyl group having 1 to 4 carbon atoms); (vii) -CONR⁸R⁸⁰ (wherein R⁸ and R⁸⁰ which may be the same or different from each other stand for a hydrogen atom; an aliphatic hydrocarbon group having 1 to 10 carbon atoms or an alicyclic hydrocarbon group having 4 to 10 carbon atoms which may be substituted with a halogen atom, an oxo group, a hydroxyl group, a tri(C₁ - C₇) hydrocarbon silyloxy group, an acyloxy group having 2 to 7 carbon atoms, an alkoxycarbonyloxy group having 2 to 5 carbon atoms, an alkoxy group having 1 to 4 carbon atoms or an aromatic hydrocarbon group having 6 to 10 carbon atoms; an aromatic hydrocarbon group having 6 to 10 carbon atoms which may be substituted with a halogen atom, a hydroxyl group, a nitro group, a tri(C₁ - C₇) hydrocarbon silyloxy group, an acyloxy group having 2 to 7 carbon atoms, an alkoxycarbonyloxy group having 2 to 5 carbon atoms, an alkoxy group having 1 to 4 carbon atoms, an acyl group having 2 to 7 carbon atoms, a carboxyl group, an alkoxycarbonyl group having 2 to 5 carbon atoms or an alkyl group having 1 to 4 carbon atoms; or a group wherein the R⁸ and R⁸⁰ are combined with each other to form a five- or six-membered ring); (viii) -NR⁹R⁹⁰ (wherein R⁹ and R⁹⁰ which may be the same or different from each other stand for a hydrogen atom; an aliphatic hydrocarbon group having 1 to 10 carbon atoms or an alicyclic hydrocarbon group having 4 to 10 carbon atoms which may be substituted with a halogen atom, an oxo group, a hydroxyl group, a tri(C₁ - C₇) hydrocarbon silyloxy group, an acyloxy group having 2 to 7 carbon atoms, an alkoxycarbonyloxy group having 2 to 5 carbon atoms, an alkoxy group having 1 to 4 carbon atoms or an aromatic hydrocarbon group having 6 to 10 carbon atoms; an aromatic hydrocarbon group having 6 to 10 carbon atoms which may be substituted with a halogen atom, a hydroxyl group, a nitro group, a tri(C₁ - C₇) hydrocarbon silyloxy group, an acyloxy group having 2 to 7 carbon atoms, an alkoxycarbonyl group having 2 to 5 carbon atoms, an alkoxy group having 1 to 4 carbon atoms, an acyl group having 2 to 7 carbon atoms, a carboxyl group, an alkoxycarbonyl group having 2 to 5 carbon atoms or an alkyl group having 1 to 4 carbon atoms; or a group wherein the R⁹ and R⁹⁰ are combined with each other to form a five- or six-membered ring); and (ix) -SR⁷⁶ (wherein R⁷⁶ stands for an aliphatic hydrocarbon group having 1 to 10 carbon atoms or an alicyclic hydrocarbon group having 4 to 10 carbon atoms which may be substituted with a halogen atom, an oxo group, a hydroxyl group, a carboxyl group, a tri(C₁ - C₇) hydrocarbon silyloxy group, an acyloxy group having 2 to 7 carbon atoms, an alkoxycarbonyloxy group having 2 to 5 carbon atoms, an alkoxy group having 1 to 4 carbon atoms, an alkylcarbonyl group having 2 to 5 carbon atoms or an aromatic hydrocarbon group having 6 to 10 carbon atoms; an aromatic hydrocarbon group having 6 to 10 carbon atoms which may be substituted with a halogen atom, a hydroxyl group, a tri(C₁ - C₇) hydrocarbon silyloxy group, a nitro group, an acyloxy group having 2 to 7 carbon atoms, an alkoxycarbonyl group having 2 to 5 carbon atoms, an alkoxy group having 1 to 4 carbon atoms, an acyl group having 2 to 7 carbon atoms, a carboxyl group, an alkoxycarbonyl group having 2 to 5 carbon atoms or an alkyl group having 1 to 4 carbon atoms.

Examples of the substituents (i) to (viii) of the groups (r-a) to (r-g) include the same substituents as those of the groups (r¹-a) to (r¹-g) described above in connection with the R¹. Examples of the -SR⁷⁶ in the substituent (ix) of the groups (r-a) to (r-g) include aliphatic hydrocarbon groups having 1 to 10 carbon atoms, i.e., alkyl groups such as methyl, ethyl, propyl, isopropyl, butyl, isobutyl, s-butyl, t-butyl, pentyl, isopentyl, neopentyl, t-pentyl, hexyl, heptyl, octyl and decyl groups, alkenyl groups such as 2-propenyl, 2-butenyl, 3-hexenyl and 5-hexenyl groups and alkynyl groups such as 2-propynyl, 2-butynyl and 3-hexynyl groups, or alicyclic hydrocarbon groups having 4 to 10 carbon atoms such as cyclobutyl, cyclopentyl, cyclohexyl and bicyclo[4.4.0]decan-2-yl groups, which may be substituted with a halogen atom such as fluorine, chlorine or bromine, an oxo group, a hydroxyl group, a carboxyl group, a tri(C₁ - C₇) hydrocarbon silyloxy group such as a trimethylsilyloxy, triethylsilyloxy, t-butyldimethylsilyloxy or t-butyldiphenylsilyloxy group, an acyloxy group having 2 to 7 carbon atoms such as an acetoxy, propionyloxy, butyryloxy, isobutyryloxy, valeryloxy, hexanoyloxy or benzoyloxy group, an alkoxycarbonyloxy group having 2 to 5 carbon atoms such as a methoxycarbonyloxy, ethoxycarbonyloxy, propoxycarbonyloxy, isopropoxycarbonyloxy, butoxycarbonyloxy group or t-butoxycarbonyloxy group, an alkoxy group having 1 to 4 carbon atoms such as a methoxy, ethoxy, propoxy, isopropoxy, butoxy, isobutoxy, or t-butoxy group, an alkoxycarbonyl group having 2 to 5 carbon atoms such as a methoxycarbonyl, ethoxycarbonyl, propoxycarbonyl, isopropoxycarbonyl, butoxycarbonyl or t-butoxycarbonyl group, or an aromatic hydrocarbon group having 6 to 10 carbon atoms such as a phenyl, 1-naphthyl or 2-naphthyl group; and aromatic hydrocarbon groups having 6 to 10 carbon atoms such as phenyl, 1-naphthyl and 2-naphthyl groups, which may be substituted with a halogen atom such as fluorine, chlorine or bromine, a hydroxyl group, a tri(C₁ - C₇) hydrocarbon silyloxy group such as a trimethylsilyloxy, triethylsilyloxy or t-butyldimethylsilyloxy group, a nitro group, an acyloxy group having 2 to 7 carbon atoms such as an acetoxy, propionyloxy, butyryloxy, isobutyryloxy, valeryloxy, hexanoyloxy, heptanoyloxy or benzoyloxy group, an alkoxycarbonyloxy group having 2 to 5 carbon atoms such as a methoxycarbonyloxy, ethoxycarbonyloxy, propoxycarbonyloxy, isopropoxycarbonyloxy, butoxycarbonyloxy or t-butoxycarbonyloxy group, an alkoxy group having 1 to 4 carbon atoms such as a methoxy, ethoxy, propoxy, isopropoxy, butoxy, isobutoxy, s-butoxy or t-butoxy group, an acyl group having 2 to 7 carbon atoms such as an acetyl, propionyl, butyryl, isobutyryl, valeryl, hexanoyl or benzoyl group, an alkoxycarbonyl group having 2 to 5 carbon atoms such as a carboxyl, methoxycarbonyl, ethoxycarbonyl, propoxycarbonyl, isopropoxycarbonyl, butoxycarbonyl, isobutoxycarbonyl or t-butoxycarbonyl group, or an alkyl group such as a methyl, ethyl, propyl, isopropyl, butyl, isobutyl, s-butyl or t-butyl group.

Preferred examples of the R include methyl, ethyl, propyl, nonyl, 3-carboxypropyl, 3-methoxycarbonylpropyl, 3-(2-thioxo-3-thiazolidinylcarbonyl)propyl, 3-(6-D-glucosylcarbonyl)propyl, 5-carboxypentyl, 5-methoxycarbonylpentyl, 5-ethoxycarbonylpentyl, 5-butoxycarbonylpentyl, 5-decyloxycarbonylpentyl, 5-(2-thioxo-3-thiazolidinylcarbonyl)pentyl, 5-(6-D-glucosylcarbonyl)pentyl, 5-(1-D-xylosylcarbonyl)pentyl, 5-(5-D-ribosylcarbonyl)pentyl, 5-cyanopentyl, 5-methoxycarbonyl-5,5-difluoropentyl, 5-methoxycarbonyl-4-pentenyl, 3,6-dihydroxyhexyl, 3,6-dihydroxy-l-hexenyl, 3,6-diacetoxy-1-hexenyl, 3,6-bis-t-butyldimethylsilyloxy-1-hexenyl, 3,6-bismethoxycarbonyloxy-1-hexenyl, 3,5-diacetoxy-4-(1-methoxy-1-methylethoxy-1-pentenyl, 3,5-diacetoxy-4-hydroxy-1-hexenyl, 7-hydroxy-6-hydroxymethyl-l-heptenyl, 3,4,5-triacetoxy-1-pentenyl, 5-methoxycarbonyl-1-pentynyl, 3-methoxycarbonylpropylthiomethyl, cyclohexyl, phenyl, 4-dimethylaminophenyl, 4-methoxycarbonylphenyl, 4-(3-hydroxy-2-hydroxymethylpropyl)phenyl, 4-pyridinyl, 5-methyl-2-furanyl, 2-cyclohexylethyl, 4-oxo-4-phenylbutyl, 2-phenylvinyl, 2-(3,4-dimethoxyphenyl)ethyl, 2-(4-dimethylaminophenyl)vinyl, 2-(1-piperidinyl)ethyl, 2-(4-morpholinyl)ethyl and 2-(4-methyl-1-piperazinyl)ethyl.

In the above-described formula (I), the R⁵ stands for a hydrogen atom or a substituted or unsubstituted aliphatic hydrocarbon group having 1 to 10 carbon atoms or alicyclic hydrocarbon group having 4 to 10 carbon atoms. Examples of the unsubstituted aliphatic hydrocarbon group having 1 to 10 carbon atoms in the R⁵ include alkyl groups such as methyl, ethyl, propyl, isopropyl, butyl, isobutyl, s-butyl, t-butyl, pentyl, isopentyl, neopentyl, hexyl, heptyl, octyl, 3,7-dimethyloctyl, nonyl and decyl groups; alkenyl groups such as 2-propenyl, 2-butenyl, 3-butenyl and 3-hexenyl groups; and alkynyl groups such as 2-propynyl and 2-butynyl groups.

Examples of the unsubstituted alicyclic hydrocarbon group having 4 to 10 carbon atoms in the R⁵ include cyclobutyl, cyclopentyl, cyclohexyl, 3-cyclohexenyl, cyclooctyl and bicyclo[4.4.0]decan-2-yl.

The R⁵ may be a group comprising, attached to each other, any combination of the above-described aliphatic hydrocarbon group having 1 to 10 carbon atoms and alicyclic hydrocarbon group having 4 to 10 carbon atoms. Among them, preferred examples of the R⁵ include a substituted or unsubstituted
(r⁵-a) aliphatic hydrocarbon group having 1 to 10 carbon atoms which may be substituted with one or a plurality of phenyl groups;
(r⁵-b) alicyclic hydrocarbon group having 4 to 10 carbon atoms which may be substituted with one or a plurality of alkyl groups having 1 to 4 carbon atoms, alkoxy groups having 1 to 4 carbon atoms or phenyl groups; and
(r⁵-c) aliphatic hydrocarbon group having 1 to 10 carbon atoms substituted with an alicyclic hydrocarbon group having 4 to 10 carbon atoms and which may be substituted with one or a plurality of alkyl groups having 1 to 4 carbon atoms, alkoxy groups having 1 to 4 carbon atoms or phenyl groups.

Preferred examples of the unsubstituted group (r⁵-a) include methyl, ethyl, propyl, isopropyl, butyl, isobutyl, s-butyl, t-butyl, pentyl, hexyl, octyl, decyl, 3,7-dimethyloctyl, benzyl, 2-phenylethyl, 3-phenylpropyl, 4-phenylbutyl and 5-phenylpentyl groups. Preferred examples of the unsubstituted group (r⁵-b) include cyclopentyl, cyclohexyl, cyclooctyl, 3-methylcyclopentyl, 4-methylcyclohexyl, 4-butylcyclohexyl, 3,4-dimethylcyclohexyl, 4-methoxycyclohexyl and 4-phenylcyclohexyl groups. Preferred examples of the unsubstituted group (r⁵-c) include 3-cyclopentylpropyl, 3-cyclohexylpropyl, 4-cyclopentylbutyl and 4-cyclohexylbutyl groups.

The above-described groups (r⁵-a) to (r⁵-c) may be substituted with a plurality of different groups, and examples of the substituent include (i) a halogen atom; (ii) an oxo group; (iii) a cyano group; (iv) a nitro group; (v)-COOR⁶⁵ (wherein R⁶⁵ stands for a hydrogen atom; one equivalent of cation; a residue of a saccharide; or an aliphatic hydrocarbon group having 1 to 10 carbon atoms which may be substituted with a halogen atom, an oxo group, a hydroxyl group, a nitro group, a tri(C₁ - C₇) hydrocarbon silyloxy group, an acyloxy group having 2 to 7 carbon atoms, an alkoxycarbonyloxy group having 2 to 5 carbon atoms, an alkoxy group having 1 to 4 carbon atoms, an alicyclic hydrocarbon group having 4 to 10 carbon atoms or an aromatic hydrocarbon group having 6 to 10 carbon atoms); (vi) -OR⁷⁵ (wherein R⁷⁵ stands for a hydrogen atom; an alkyl group having 1 to 4 carbon atoms; an acyl group having 2 to 7 carbon atoms; an alkoxycarbonyl group having 2 to 5 carbon atoms; a tri(C₁ - C₇) hydrocarbon silyl group; a group capable of forming an acetal bond together with the oxygen atom attached to the R⁷⁵; an aliphatic hydrocarbon group having 1 to 10 carbon atoms or an alicyclic hydrocarbon group having 4 to 10 carbon atoms which may be substituted with a halogen atom, an oxo group, a hydroxyl group, a carboxyl group, a tri(C₁ - C₇) hydrocarbon silyloxy group, an acyloxy group having 2 to 7 carbon atoms, an alkoxycarbonyloxy group having 2 to 5 carbon atoms, an alkoxy group having 1 to 4 carbon atoms, an alkoxycarbonyl group having 2 to 5 carbon atoms or an aromatic hydrocarbon group having 6 to 10 carbon atoms; or an aromatic hydrocarbon group having 6 to 10 carbon atoms which may be substituted with a halogen atom, hydroxyl group, a nitro group, a tri(C₁ - C₇) hydrocarbon silyloxy group, an alkoxy group having 1 to 4 carbon atoms, an acyloxy group having 2 to 7 carbon atoms, an alkoxycarbonyloxy group having 2 to 5 carbon atoms, an acyl group having 2 to 7 carbon atoms, a carboxyl group, an alkoxycarbonyl group having 2 to 5 carbon atoms or an alkyl group having 1 to 4 carbon atoms); (vii) -CONR⁸⁵R⁸⁵⁰ (wherein R⁸⁵ and R⁸⁵⁰ which may be the same or different from each other stand for a hydrogen atom; an aliphatic hydrocarbon group having 1 to 10 carbon atoms or an alicyclic hydrocarbon group having 4 to 10 carbon atoms which may be substituted with a halogen atom, an oxo group, a hydroxyl group, a tri(C₁ - C₇) hydrocarbon silyloxy group, an acyloxy group having 2 to 7 carbon atoms, an alkoxycarbonyloxy group having 2 to 5 carbon atoms, an alkoxy group having 1 to 4 carbon atoms or an aromatic hydrocarbon group having 6 to 10 carbon atoms; an aromatic hydrocarbon group having 6 to 10 carbon atoms which may be substituted with a halogen atom, a hydroxyl group, a nitro group, a tri(C₁ - C₇) hydrocarbon silyloxy group, an acryloxy group having 2 to 7 carbon atoms, an alkoxycarbonyloxy group having 2 to 5 carbon atoms, an alkoxy group having 1 to 4 carbon atoms, an acyl group having 2 to 7 carbon atoms, a carboxyl group, an alkoxycarbonyl group having 2 to 5 carbon atoms or an alkyl group having 1 to 4 carbon atoms; or a group wherein the R⁸⁵ and R⁸⁵⁰ are combined with each other to form a five- or six-membered ring); (viii) -NR⁹⁵R⁹⁵⁰ (wherein R⁹⁵ and R⁹⁵⁰ which may be the same or different from each other stand for a hydrogen atom; an aliphatic hydrocarbon group having 1 to 10 carbon atoms or an alicyclic hydrocarbon group having 4 to 10 carbon atoms which may be substituted with a halogen atom, an oxo group, a hydroxyl group, a tri(C₁ - C₇) hydrocarbon silyloxy group, an acyloxy group having 2 to 7 carbon atoms, an alkoxycarbonyloxy group having 2 to 5 carbon atoms, an alkoxy group having 1 to 4 carbon atoms or an aromatic hydrocarbon group having 6 to 10 carbon atoms; and an aromatic hydrocarbon group having 6 to 10 carbon atoms which may be substituted with a halogen atom, a hydroxyl group, a nitro group, a tri(C₁ - C₇) hydrocarbon silyloxy group, an acyloxy group having 2 to 7 carbon atoms, an alkoxycarbonyloxy group having 2 to 5 carbon atoms, an alkoxy group having 1 to 4 carbon atoms, an acyl group having 2 to 7 carbon atoms, a carboxyl group, an alkoxycarbonyl group having 2 to 5 carbon atoms or an alkyl group having 1 to 4 carbon atoms; or a group wherein the R⁹⁵ and R⁹⁵⁰ are combined with each other to form a five- or six-membered ring).

Examples of the substituents (i) to (viii) of the groups (r⁵-a) to (r⁵-c) include the same substituents as those of the groups (r¹-a) to (r¹-g) described above in connection with the R¹.

Preferred examples of the r⁵ include methyl, ethyl, propyl, isopropyl, butyl, t-butyl, hexyl, octyl, 3,7-dimethyloctyl, 3,7-dimethyl-6-octenyl, benzyl, 3-(3,4-dimethoxyphenyl)propyl, 5-phenylpentyl, cyclohexyl, 3-cyclohexylpropyl, 4-cyclohexylbutyl, 6-hydroxyhexyl, 6-t-butyldimethylsilyloxyhexyl, 6-acetoxyhexyl, 6-(1-ethoxyethoxy)hexyl, 5-carboxypentyl, 5-methoxycarbonylpentyl, 5-(6-D-glucosylcarbonyl)pentyl and 4-phenoxybutyl.

In the above-described formula (I), the R³ stands for a hydrogen atom, a substituted or unsubstituted aliphatic hydrocarbon group having 1 to 10 carbon atoms, alicyclic hydrocarbon group having 4 to 10 carbon atoms or aromatic hydrocarbon group having 6 to 10 carbon atoms. When the R³ is attached to the carbon atom of the cyclopentene skeleton through a single-bond, X stands for a hydrogen atom, -OR⁴ (wherein R⁴ stands for a hydrogen atom, an alkyl group having 1 to 4 carbon atoms, an acyl group having 2 to 7 carbon atoms, an alkoxycarbonyl group having 2 to 5 carbon atoms, a tri(C₁ - C₇) hydrocarbon silyl group or a group capable of forming an acetal bond together with the oxygen atom attached to the R⁴) or is absent when the R³ is attached to the carbon atom through a double bond. Specifically, when the R³ is attached to the cyclopentene skeleton through a single bond, the above-described formula (I) represents 2-substituted-2-cyclopentenones represented by the following formula (I'): 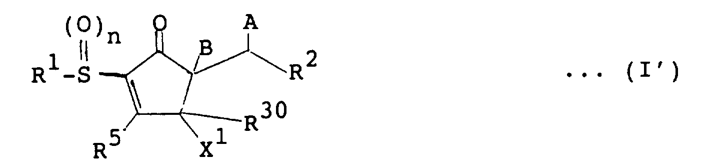 wherein A, B, R¹ R R⁵ and n are as defined above;
R³⁰ stands for a hydrogen atom or a substituted or unsubstituted aliphatic hydrocarbon group having 1 to 10 carbon atoms, alicyclic hydrocarbon group having 4 to 10 carbon atoms or aromatic hydrocarbon group having 6 to 10 carbon atoms; and
X¹ stands for a hydrogen atom or OR⁴ (wherein R⁴ is as defined above). When the R³ is attached to the carbon atom of the cyclopentene skeleton through a double bond and X is absent, the above-described formula (I) represents 2-substituted-2-cyclopentenones represented by the following formula (I''): 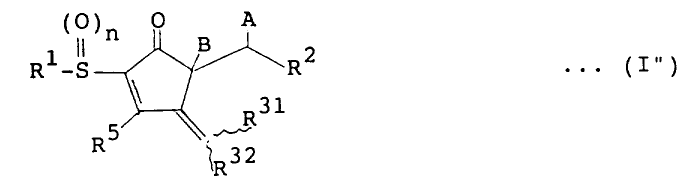 wherein A, B, R¹, R, R⁵ , n and are as defined above;
R³¹ and R³ which may be the same or different from each other stand for a hydrogen atom, a substituted or unsubstituted aliphatic hydrocarbon group having 1 to 9 carbon atoms, alicyclic hydrocarbon group having 4 to 10 carbon atoms or aromatic hydrocarbon group having 6 to 10 carbon atoms, or a group wherein the R³¹ and R³ are combined with each other to form an alicyclic hydrocarbon group having 4 to 10 carbon atoms.

The R³⁰ in the above-described formula (I') stands for a hydrogen atom, a substituted or unsubstituted aliphatic hydrocarbon group having 1 to 10 carbon atoms, alicyclic hydrocarbon group having 4 to 10 carbon atoms or aromatic hydrocarbon group having 6 to 10 carbon atoms. Examples of the unsubstituted aliphatic hydrocarbon group having 1 to 10 carbon atoms in the R³⁰ include alkyl groups such as methyl, ethyl, propyl, isopropyl, butyl, s-butyl, t-butyl, pentyl, isopentyl, neopentyl, hexyl, heptyl, octyl, 3,7-dimethyloctyl, nonyl and decyl groups; alkenyl groups such as vinyl, 1-methylvinyl, 1-propenyl, 2-propenyl, 2-methyl-1-propenyl, 1-butenyl, 2-butenyl, 2-methyl-1-butenyl, 1-pentenyl, 2-pentenyl, 1-hexenyl, 2-hexenyl, 3,3-dimethyl-1-butenyl, 5-hexenyl, 1,5-hexadienyl, 1-heptenyl, 1-octenyl, 3-methyl-1-octenyl, 4,4-dimethyl-1-octenyl, 1,7-octadienyl, 1-nonenyl, 5-methyl-1-nonenyl and 1-decenyl groups; and alkynyl groups such as ethynyl, 1-propynyl, 2-propynyl, 1-butynyl, 3-methyl-1-butynyl, 3,3-dimethyl-1-butynyl, 1-pentynyl, 2-pentynyl, 1-hexynyl, 2-hexynyl, 5-hexen-1-yl, 1-heptynyl, 1-nonynyl and 1-decynyl groups.

Examples of the unsubstituted alicyclic hydrocarbon groups having 4 to 10 carbon atoms in the R³⁰ include cyclobutyl, cyclopentyl, cyclohexyl, 1-cyclohexenyl, 3-cyclohexenyl, 4-cyclohexenyl, cycloheptyl, cyclooctyl and bicyclo[4.4.0]decan-2-yl groups.

Examples of the unsubstituted aromatic hydrocarbon group having 6 to 10 carbon groups in the R³⁰ include phenyl, 1-naphthyl and 2-naphthyl groups.

The R³⁰ may be a group comprising, attached to each other, any combination of the above-described aliphatic hydrocarbon group having 1 to 10 carbon atoms, alicyclic hydrocarbon group having 4 to 10 carbon atoms and aromatic hydrocarbon group having 6 to 10 carbon atoms. Among them, preferred examples of the R³⁰ include a substituted or unsubstituted
(r³⁰-a) aliphatic hydrocarbon group having 1 to 10 carbon atoms which may be substituted with one or a plurality of alkoxy groups having 1 to 4 carbon atoms;
(r³⁰-b) alicyclic hydrocarbon group having 4 to 10 carbon atoms which may be substituted with one or a plurality of alkyl groups having 1 to 4 carbon atoms and alkoxy groups having 1 to 4 carbon atoms;
(r³⁰-c) aromatic hydrocarbon group having 6 to 10 carbon atoms which may be substituted with one or a plurality of alkyl groups having 1 to 4 carbon atoms and alkoxy groups having 1 to 4 carbon atoms;
(r³⁰-d) aliphatic hydrocarbon group having 1 to 10 carbon atoms substituted with an alicyclic hydrocarbon group having 4 to 10 carbon atoms and which may be substituted with one or a plurality of alkyl groups having 1 to 4 carbon atoms and alkoxy groups having 1 to 4 carbon atoms; and
(r³⁰-e) aliphatic hydrocarbon group having 1 to 10 carbon atoms substituted with an aromatic hydrocarbon group having 6 to 10 carbon atoms and which may be substituted with one or a plurality of alkyl groups having 1 to 4 carbon atoms and alkoxy groups having 1 to 4 carbon atoms.

Preferred examples of the unsubstituted group (r³⁰-a) include methyl, ethyl, propyl, isopropyl, butyl, isobutyl, t-butyl, pentyl, hexyl, octyl, decyl, 3,7-dimethyloctyl, vinyl, 1-propenyl, 1-methylvinyl, 1-butenyl, 1-octenyl, 3,3-dimethyl-1-butenyl, 3-methyl-1-octenyl, 4,4-dimethyl-1-octenyl, nona-7-yne-1-enyl, 5-methyl-1-nonenyl, 1-propynyl, 3,3-dimethyl-1-butynyl, 1-pentynyl, 3,3-dimethyl-1-hexynyl and 2-hexenyl. Preferred examples of the unsubstituted group (r³⁰-b) include cyclopentyl, cyclohexyl, cyclooctyl, 3-methylcyclopentyl, 4-methylcyclohexyl, 4-butylcyclohexyl, 3,4-dimethylcyclohexyl and 4-methoxycyclohexyl groups. Preferred examples of the unsubstituted group (r³⁰-c) include phenyl, 1-naphthyl, 4-methylphenyl, 3,5-dimethylphenyl, 4-ethylphenyl, 4-butylphenyl, 4-methoxyphenyl, 4-ethoxyphenyl, 6-methoxy-2-naphthyl and 6,7-dimethoxy-2-naphthyl groups. Preferred examples of the unsubstituted group (r³⁰-d) include 3-cyclopentyl-1-propenyl, 3-cyclohexylpropyl, 3-cyclopentyl-3,3-dimethyl-1-propenyl, 4-cyclohexyl-1-propenyl and 3-(3-methylcyclopentyl)-1-propenyl groups. Preferred examples of the unsubstituted group (r³⁰-e) include benzyl, 2-phenylethyl, 3-phenylpropyl, 4-phenylbutyl, 1-naphthylmethyl, 2-naphthylmethyl, 4-butylbenzyl, 4-methoxybenzyl, 3,4-dimethoxybenzyl and 3,3-dimethyl-4-phenyl-1-butenyl groups.

The X¹ in the above-described formula (I') stands for a hydrogen atom or -OR⁴ wherein R⁴ stands for a hydrogen atom, an alkyl group having 1 to 4 carbon atoms, an acyl group having 2 to 7 carbon atoms, an alkoxycarbonyl group having 2 to 5 carbon atoms, a tri (C₁ - C₇) hydrocarbon silyl group or a group combining with the oxygen atom attached to the R⁴ to form an acetal bond. Examples of the alkyl group having 1 to 4 carbon atoms in the R⁴ include methyl, ethyl, propyl, isopropyl, butyl, isobutyl and t-butyl groups. Examples of the acyl group having 2 to 7 carbon atoms include acetyl, propionyl, butyryl, isobutyryl, valeryl, hexanoyl and benzoyl groups. Examples of the alkoxycarbonyl group having 2 to 5 carbon atoms include methoxycarbonyl, ethoxycarbonyl, propoxycarbonyl, isopropoxycarbonyl, butoxycarbonyl and t-butoxycarbonyl groups. Examples of the tri(C₁ - C₇) hydrocarbon silyl group include trimethylsilyl, triethylsilyl, t-butyldimethylsilyl, t-butyldiphenylsilyl and tribenzylsilyl groups. Examples of the group combining with an oxygen atom attached to the R⁴ to form an acetal bond include methoxymethyl, 1-ethoxyethyl, 1-methoxy-1-methylethyl, 2-ethoxy-1-methylethyl, 2-methoxyethoxymethyl, tetrahydropyran-2-yl, 6,6-dimethyl-3-oxa-2-oxo-bicyclo[3.1.0]hexane-4-yl groups.

Examples of the X¹ include a hydrogen atom and hydroxyl, methoxy, ethoxy, trimethylsilyloxy, acetoxy, methoxycarbonyloxy and isopropoxycarbonyloxy groups.

The R³¹ and R³ in the above-described formula (II') each stand for a hydrogen atom, a substituted or unsubstituted aliphatic hydrocarbon group having 1 to 9 carbon atoms, alicyclic hydrocarbon group having 4 to 10 carbon atoms or aromatic hydrocarbon group having 6 to 10 carbon atoms, or a group wherein the R³¹ and R³ are combined with each other to form an alicyclic hydrocarbon group having a four to ten-membered ring. Examples of the unsubstituted aliphatic hydrocarbon group having 1 to 9 carbon atoms in the R³¹ and R³ include alkyl groups such as methyl, ethyl, isopropyl, butyl, pentyl, heptyl, octyl, nonyl and 2,6-dimethylheptyl groups; alkenyl groups such as vinyl, 1-propenyl, 1-pentenyl and 1-hexenyl groups; and alkynyl groups such as 1-propynyl and 1-pentynyl groups.

Examples of the unsubstituted alicyclic hydrocarbon group having 4 to 10 carbon atoms and aromatic hydrocarbon having 6 to 10 carbon atoms in the R³¹ and R³ include those described above in connection with the R³⁰. Examples of the unsubstituted group which is an alicyclic hydrocarbon group having a four to ten-membered ring formed by combining R³¹ with R³ to each other together with a carbon atom intervening between the R³¹ and the R³ include cyclobutylidene, cyclopentylidene, cyclohexylidene, 2-cyclohexenylidene and bicyclo[4.4.0]decan-2-ylidene groups. The R³¹ and R³ may also be one comprising, attached to each other, any combination of the above-described aliphatic hydrocarbon group having 1 to 9 carbon atoms, alicyclic hydrocarbon group having 4 to 10 carbon atoms and aromatic hydrocarbon group having 6 to 10 carbon atoms; or a group wherein the R³¹ and R³ are combined with each other to form an alicyclic hydrocarbon group having a four to ten-membered ring to which the above-described aliphatic hydrocarbon having 1 to 9 carbon atoms or aromatic hydrocarbon group having 6 to 10 carbon atoms is attached. Among them, preferred examples of the R³¹ and R³ include a substituted or unsubstituted
(r³¹-a) aliphatic hydrocarbon group having 1 to 10 carbon atoms which may be substituted with one or a plurality of alkoxy groups having 1 to 4 carbon atoms;
(r³¹-b) alicyclic hydrocarbon group having 4 to 10 carbon atoms which may be substituted with one or a plurality of alkyl groups having 1 to 4 carbon atoms and alkoxy groups having 1 to 4 carbon atoms;
(r³¹-c) aromatic hydrocarbon group having 6 to 10 carbon atoms which may be substituted with one or a plurality of alkyl groups having 1 to 4 carbon atoms and alkoxy groups having 1 to 4 carbon atoms;
(r³¹-d) aliphatic hydrocarbon group having 1 to 10 carbon atoms substituted with an alicyclic hydrocarbon group having 4 to 10 carbon atoms and which may be substituted with one or a plurality of alkyl groups having 1 to 4 carbon atoms and alkoxy groups having 1 to 4 carbon atoms;
(r³¹-e) aliphatic hydrocarbon group having 1 to 10 carbon atoms substituted with an aromatic hydrocarbon group having 6 to 10 carbon atoms and which may be substituted with one or a plurality of alkyl groups having 1 to 4 carbon atoms and alkoxy groups having 1 to 4 carbon atoms; and
(r³¹-f) group wherein R³¹ and R³ are combined with each other to form an alicyclic hydrocarbon having a four to ten-membered ring, which may be substituted with one or a plurality of alkyl groups having 1 to 4 carbon atoms and alkoxy groups having 1 to 4 carbon atoms.

Preferred examples of the unsubstituted group (r³¹-a) include methyl, ethyl, propyl, isopropyl, butyl, isobutyl, t-butyl, pentyl, heptyl, nonyl, 2,6-dimethylheptyl, vinyl, 1-propenyl and 1-pentenyl groups. Preferred examples of the unsubstituted groups (r³¹-b) and (r³¹-c) include respectively those of the (r³⁰-b) and (r³⁰-c) described above in connection with the R³⁰. Preferred examples of the unsubstituted group (r³¹-f) include cyclopentylidene and cyclohexylidene.

The above-described groups (r³⁰-a) to (r³⁰-e) or (r³¹-a) to (r³¹-f) may be substituted with a plurality of different groups, and examples of the substituent include (i) a halogen atom; (ii) an oxo group; (iii) a cyano group; (iv) a nitro group; (v) -COOR⁶³ (wherein R⁶³ stands for a hydrogen atom; one equivalent of cation; a residue of a saccharide; or an aliphatic hydrocarbon group having 1 to 10 carbon atoms which may be substituted with a halogen atom, an oxo group, a hydroxyl group, a nitro group, a tri(C₁ - C₇) hydrocarbon silyloxy group, an acyloxy group having 2 to 7 carbon atoms, an alkoxycarbonyloxy group having 2 to 5 carbon atoms, an alkoxy group having 1 to 4 carbon atoms, an alicyclic hydrocarbon group having 4 to 10 carbon atoms or an aromatic hydrocarbon group having 6 to 10 carbon atoms); (vi) -OR⁷³ (wherein R⁷³ stands for a hydrogen atom; an alkyl group having 1 to 4 carbon atoms; an acyl group having 2 to 7 carbon atoms; an alkoxycarbonyl group having 2 to 5 carbon atoms; a tri(C₁ - C₇) hydrocarbon silyl group; a group capable of forming an acetal bond together with the oxygen atom attached to the R⁷³; an aliphatic hydrocarbon group having 1 to 10 carbon atoms or an alicyclic hydrocarbon group having 4 to 10 carbon atoms which may be substituted with a halogen atom, an oxo group, a hydroxyl group, a carboxyl group, a tri(C₁ - C₇) hydrocarbon silyloxy group, an acyloxy group having 2 to 7 carbon atoms, an alkoxycarbonyloxy group having 2 to 5 carbon atoms, an alkoxy group having 1 to 4 carbon atoms, an alkoxycarbonyl group having 2 to 5 carbon atoms or an aromatic hydrocarbon group having 6 to 10 carbon atoms; or an aromatic hydrocarbon group having 6 to 10 carbon atoms which may be substituted with a halogen atom, hydroxyl group, a nitro group, a tri(C₁ - C₇) hydrocarbon silyloxy group, an alkoxy group having 1 to 4 carbon atoms, an acyloxy group having 2 to 7 carbon atoms, an alkoxycarbonyloxy group having 2 to 5 carbon atoms, an acyl group having 2 to 7 carbon atoms, a carboxyl group, an alkoxycarbonyl group having 2 to 5 carbon atoms or an alkyl group having 1 to 4 carbon atoms); (vii) -CONR⁸³R⁸³⁰ (wherein R⁸³ and R⁸³⁰ which may be the same or different from each other stand for a hydrogen atom; an aliphatic hydrocarbon group having 1 to 10 carbon atoms or an alicyclic hydrocarbon group having 4 to 10 carbon atoms which may be substituted with a halogen atom, an oxo group, a hydroxyl group, a tri(C₁ - C₇) hydrocarbon silyloxy group, an acyloxy group having 2 to 7 carbon atoms, an alkoxycarbonyloxy group having 2 to 5 carbon atoms, an alkoxy group having 1 to 4 carbon atoms or an aromatic hydrocarbon group having 6 to 10 carbon atoms; an aromatic hydrocarbon group having 6 to 10 carbon atoms which may be substituted with a halogen atom, a hydroxyl group, a nitro group, a tri(C₁ - C₇) hydrocarbon silyloxy group, an acyloxy group having 2 to 7 carbon atoms, an alkoxycarbonyloxy group having 2 to 5 carbon atoms, an alkoxy group having 1 to 4 carbon atoms, an acyl group having 2 to 7 carbon atoms, a carboxyl group, an alkoxycarbonyl group having 2 to 5 carbon atoms or an alkyl group having 1 to 4 carbon atoms; or a group wherein R⁸³ and R⁸³⁰ are combined with each other to form a five- or six-membered ring); and (viii) -NR⁹³R⁹³⁰ (wherein R⁹³ and R⁹³⁰ which may be the same or different from each other stand for a hydrogen atom; an aliphatic hydrocarbon group having 1 to 10 carbon atoms or an alicyclic hydrocarbon group having 4 to 10 carbon atoms which may be substituted with a halogen atom, an oxo group, a hydroxyl group, a tri(C₁ - C₇) hydrocarbon silyloxy group, an acyloxy group having 2 to 7 carbon atoms, an alkoxycarbonyloxy group having 2 to 5 carbon atoms, an alkoxy group having 1 to 4 carbon atoms or an aromatic hydrocarbon group having 6 to 10 carbon atoms; and an aromatic hydrocarbon group having 6 to 10 carbon atoms which may be substituted with a halogen atom, a hydroxyl group, a nitro group, a tri(C₁ - C₇) hydrocarbon silyloxy group, an acyloxy group having 2 to 7 carbon atoms, an alkoxycarbonyloxy group having 2 to 5 carbon atoms, an alkoxy group having 1 to 4 carbon atoms, an acyl group having 2 to 7 carbon atoms, a carboxyl group, an alkoxycarbonyl group having 2 to 5 carbon atoms or an alkyl group having 1 to 4 carbon atoms; or a group wherein R⁹³ and R⁹³⁰ are combined with each other to form a five- or six-membered ring).

Examples of the substituents (i) to (viii) of the groups (r³⁰-a) to (r³⁰-e) or (r³¹-a) to (r³¹-f) include the same substituents as those of the groups (r¹-a) to (r¹-g) described above in connection with the R1.

Preferred examples of the R³⁰ include methyl, ethyl, propyl, isopropyl, butyl, isobutyl, butyl, t-butyl, octyl, 3,7-dimethyloctyl, 1-methylvinyl, 1-octenyl, 3,3-dimethyl-4-phenyl-1-butenyl, 3,3-dimethyl-1-butynyl, 1-pentynyl, 1-hexynyl, 3-t-butyldimethylsilyloxy-1-octenyl, 3-hydroxy-1-octenyl, 3-acetoxy-1-octenyl, 3-methoxycarbonyloxy-1-octenyl, 3-trimethylsilyloxy-3-methyl-1-octenyl, 3-hydroxy-3-methyl-1-octenyl, 3-t-butyldimethylsilyloxy-5-methyl-1-nonenyl, 3-hydroxy-5-methyl-1-nonenyl, 6-carboxyhexyl, 6-methoxycarbonylhexyl, 6-(2-thioxo-3-thiazolidinyl-carbonyl)hexyl, 6-(6-D-glucosylcarbonyl)hexyl, 6-(1-D-xylosylcarbonyl)hexyl, 6-(5-D-ribosylcarbonyl)hexyl, 6-hydroxyhexyl, 6-t-butyldimethylsilyloxyhexyl, 6-acetoxyhexyl, 6-hydroxy-2-hexenyl, 6-carboxy-2-hexenyl, 6-methoxycarbonyl-2-hexenyl, 3-cyclohexylpropyl, 3-hydroxy-3-cyclopentyl-1-propenyl, 3-methoxycarbonyloxy-3-cyclopentyl-1-propenyl, 3-isopropoxycarbonyloxy-3-cyclopentyl-1-propenyl, 3-t-butyldimethylsilyloxy-3-cyclopentyl-1-propenyl, 3-hydroxy-3-cyclohexyl-1-propenyl, 3-t-butyldimethylsilyloxy-3-cyclohexyl-1-propenyl, 3-hydroxy-4-cyclohexyl-1-butenyl, 4-phenoxybutyl, 3-(3,4-dimethoxyphenyl) propyl, benzyl, 2-phenylethyl, 5-phenylpentyl, cyclohexyl and phenyl.

Preferred examples of the R³¹ and R³¹⁰ include a hydrogen atom, methyl, ethyl, propyl, 1-heptenyl, 5-methoxycarbonylpentyl, 5-methoxycarbonyl-1-pentenyl and 3-phenoxypropyl.

Among the 2-substituted-2-cyclopentenone compounds represented by the above-described formula (I), a 2-substituted-2-cyclopentenone compound represented by the formula (I-b-1-1) can be produced through the following scheme 1 by subjecting a 2-cyclopentenone compound represented by the formula (III-b) to an epoxidation reaction to prepare a 2,3-epoxycyclopentanone compound represented by the formula (IV-b), reacting the 2,3-epoxycyclopentanone compound with a thiol compound represented by the formula (V) in the presence of a basic compound to prepare a 2-substituted-2-cyclopentenone compound represented by the formula (I-b-10) and then subjecting the 2-substituted-2-cyclopentenone compound to the following optional reactions: an oxidation reaction; a deblocking reaction for a protecting group of a hydroxyl group; a protecting reaction for a hydroxyl group; a hydrolysis reaction of an ester; and an esterification reaction, an amidation reaction, a condensation reaction of a sugar residue and/or a salt forming reaction of a carboxyl group.

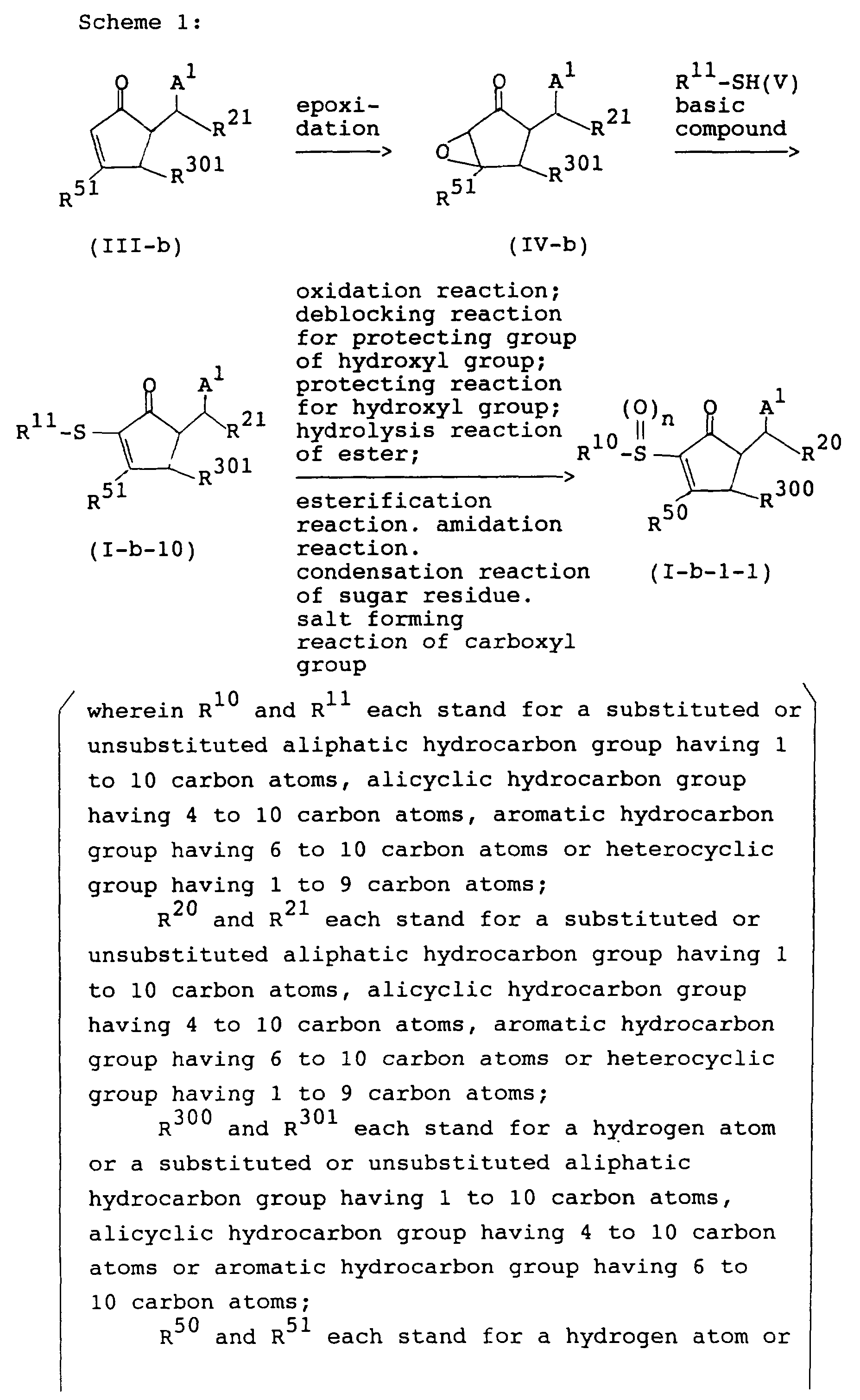

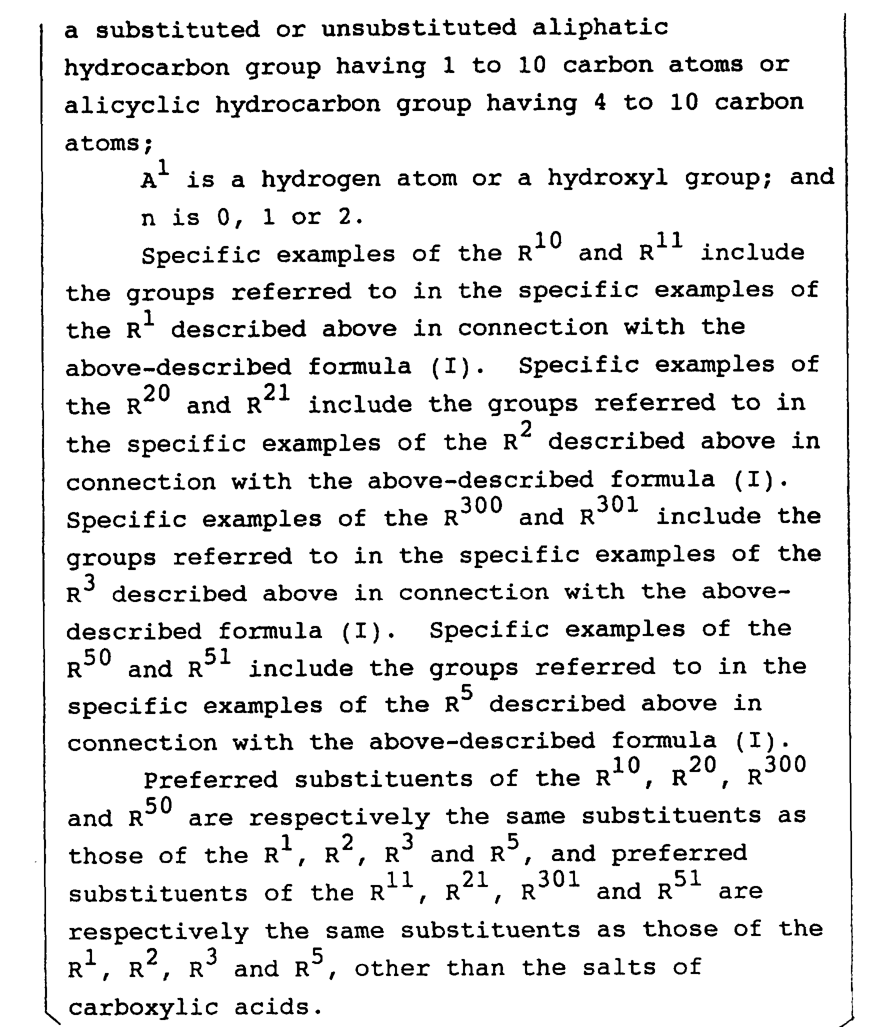

The starting compound represented by the formula (III-b) can be prepared, for example, through the following scheme comprising a combination of processes described in Japanese Unexamined Patent Publication (Kokai) Nos. 59-164747 and 62-81344.

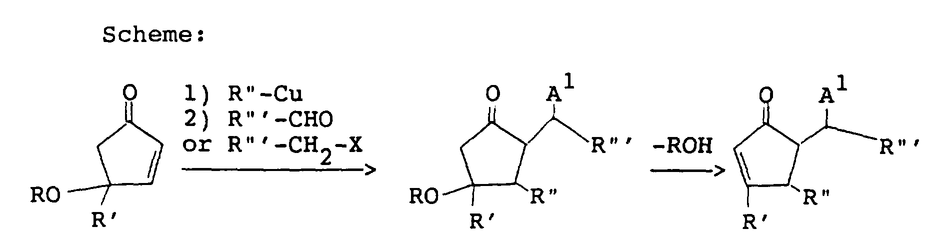

wherein A¹ is as defined above.

Among the 2-substituted-2-cyclopentenone compounds represented by the above-described formula (I), a 2-substituted-2-cyclopentenone compound represented by the formula (I-b-1-2) can be produced through the following scheme 2 by subjecting a 2-substituted-2-cyclopentenone compound represented by the formula (I-b-11) belonging to the 2-substituted-2-cyclopentenone compound represented by the above-described formula (I-b-10) to an acylation reaction or an alkoxycarbonylation reaction to prepare a 2-substituted-2-cyclopentenone compound represented by the formula (I-b-12) and then subjecting the 2-substituted-2-cyclopentenone compound to the following optional reactions: an oxidation reaction; a deblocking reaction for a protecting group of a hydroxyl group; a protecting reaction for a hydroxyl group; a hydrolysis reaction of an ester; and an esterification reaction, an amidation reaction, a condensation reaction of a sugar residue and/or a salt forming reaction of a carboxyl group.

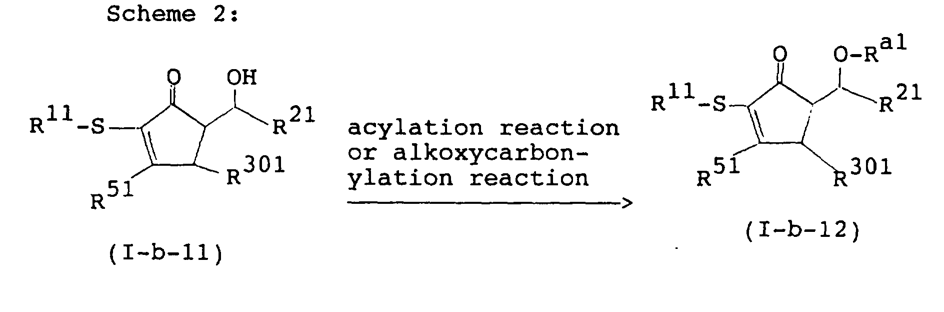

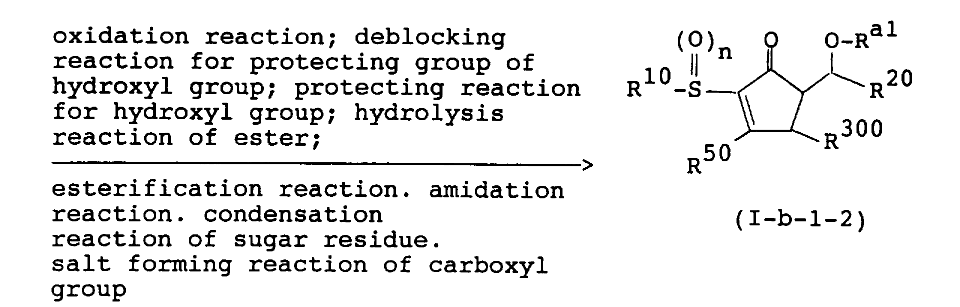

wherein R¹⁰, R¹¹, R⁰, R¹, R³⁰⁰, R³⁰¹, R⁵⁰, R⁵¹ and n are each as defined above; and
R^{a1} stands for an acyl group having 2 to 7 carbon atoms or an alkoxycarbonyl group having 2 to 5 carbon atoms.

Specific examples of the R^{a1} in the form of -O-R^{a1} include the groups referred to in the specific examples of the acyloxy group having 2 to 7 carbon atoms and alkoxycarbonyloxy group having 2 to 5 carbon atoms in the A described above in connection with the above-described formula (I).

Among the 2-substituted-2-cyclopentenone compounds represented by the above-described formula (I), a 2-substituted-2-cyclopentenone compound represented by the formula (I-a-1) can be produced through the following scheme 3 by subjecting a 2-substituted-2-cyclopentenone compound represented by the formula (I-b-11) to a sulfonylation reaction to prepare a 2-substituted-2-cyclopentenone compound represented by the formula (I-b-13), subjecting the 2-substituted-2-cyclopentenone compound to a desulfonation reaction to prepare a 2-substituted-2-cyclopentenone compound represented by the formula (I-a-10) and then subjecting the 2-substituted-2-cyclopentenone compound to the following optional reactions: an oxidation reaction; a deblocking reaction for a protecting group of a hydroxyl group; a protecting reaction for a hydroxyl group; a hydrolysis reaction of an ester; and an esterification reaction, an amidation reaction, a condensation reaction of a sugar residue and/or a salt forming reaction of a carboxyl group. 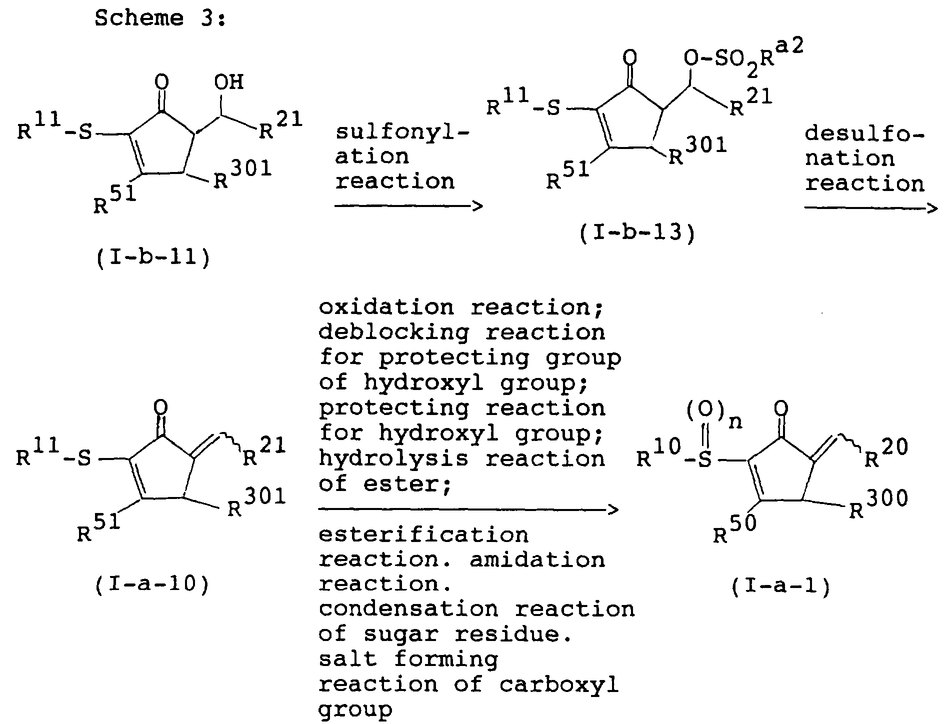 wherein R¹⁰, R¹¹, R⁰, R¹, R³⁰⁰, R³⁰¹, R⁵⁰, R⁵¹ and n are each as defined above;
represents that the substituent attached to the double bond is in an E-configuration or a Z-configuration or a mixture thereof in any proportion; and
R^{a2} stands for an alkyl group which may be substituted with a halogen atom, a substituted or unsubstituted phenyl group or a substituted or unsubstituted phenyl (C₁ - C₂) alkyl group.

Specific examples of the R^{a2} in the form of -O-SO₂R^{a2} include the groups referred to in the specific examples of the alkylsulfonyloxy group having 1 to 4 carbon atoms which may be substituted with a halogen atom, the substituted or unsubstituted phenylsulfonyloxy group or the substituted or unsubstituted phenyl (C₁ - C₂) alkylsulfonyloxy group in the A described above in connection with the above-described formula (I).

Among the 2-substituted-2-cyclopentenone compounds represented by the above-described formula (I), a 2-substituted-2-cyclopentenone compound represented by the formula (I-b-1-3) and a 2-substituted-2-cyclopentenone compound represented by the formula (I-a-1) can be produced through the following scheme 4 by reacting a 2,3-epoxycyclopentanone compound represented by the formula (IV-a-1) with a thiol compound represented by the formula (V) in the presence of a basic compound to prepare 2-substituted-2-cyclopentenone compounds respectively represented by the formula (I-b-14) and (I-a-10) and then subjecting the 2-substituted-2-cyclopentenone compounds to the following optional reactions: an oxidation reaction; a deblocking reaction for a protecting group of a hydroxyl group; a protecting reaction for a hydroxyl group; a hydrolysis reaction of an ester; and an esterification reaction, an amidation reaction, a condensation reaction of a sugar residue and/or a salt forming reaction of a carboxyl group. 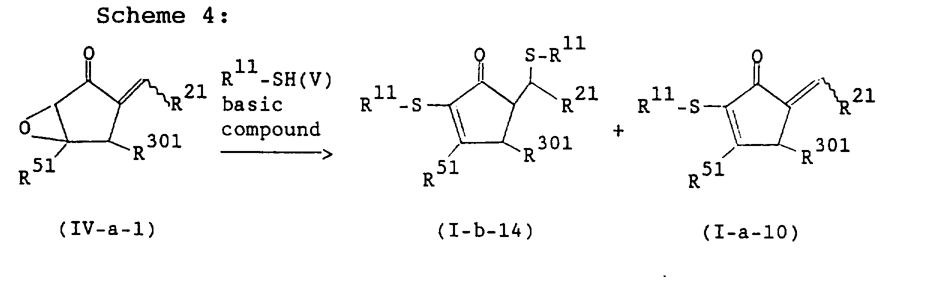 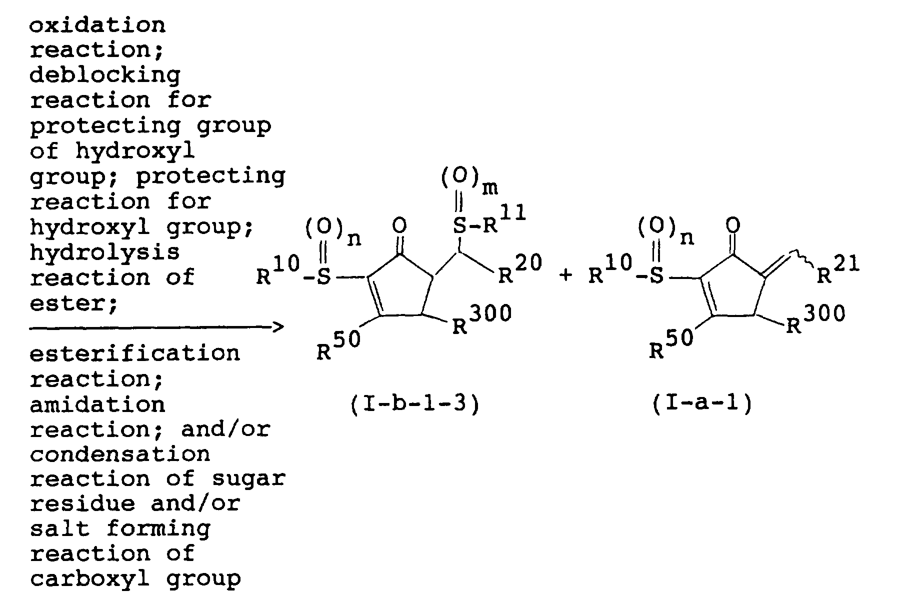 wherein R¹⁰, R¹¹, R⁰, R¹, R³⁰⁰, R³⁰¹, R⁵⁰, R⁵¹ and n are each as defined above; and
m is 0, 1 or 2.

The starting compound represented by the formula (IV-a-1) can be prepared, for example, through the following scheme in the same manner as that described in Japanese Unexamined Patent Publication (Kokai) Nos. 61-47437.

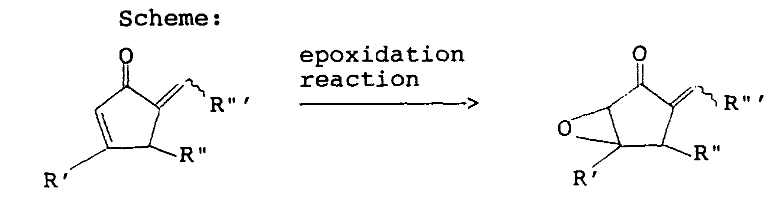

Among the 2-substituted-2-cyclopentenone compounds represented by the above-described formula (I), a 2-substituted-2-cyclopentenone compound represented by the formula (I-c-1) can be produced through the following scheme 5 by subjecting a 2-cyclopentenone compound represented by the formula (III-a) to an epoxidation reaction to prepare a 2,3-epoxycyclopentanone compound represented by the formula (IV-a-2), reacting the 2,3-epoxycyclopentanone compound with a thiol compound in the presence of a basic compound represented by the formula (V) to prepare a 2-substituted-2-cyclopentenone compound represented by the formula (I-c-2) and then subjecting the 2-substituted-2-cyclopentenone compound to the following optional reactions: an oxidation reaction; a deblocking reaction for a protecting group of a hydroxyl group; a protecting reaction for a hydroxyl group; a hydrolysis reaction of an ester; and an esterification reaction, an amidation reaction, a condensation reaction of a sugar residue and/or a salt forming reaction of a carboxyl group. 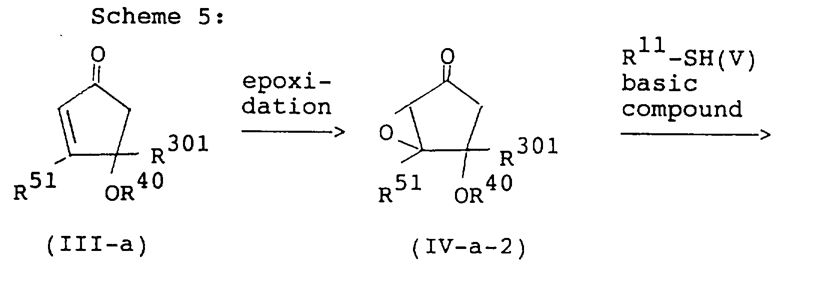 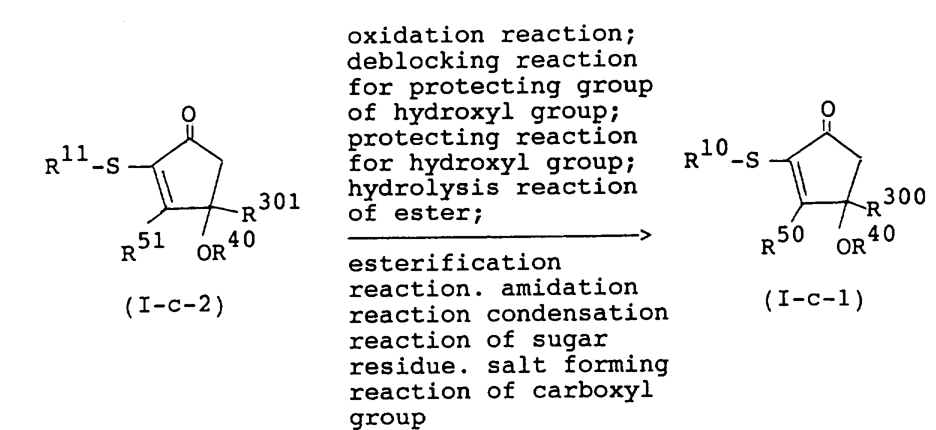 wherein R¹⁰, R¹¹, R³⁰⁰, R³⁰¹, R⁵⁰ and R⁵¹ are each as defined above; and
R⁴⁰ stands for a hydrogen atom, an alkyl group having 1 to 4 carbon atoms, an acyl group having 2 to 7 carbon atoms, an alkoxycarbonyl group having 2 to 5 carbon atoms, a tri(C₁ - C₇) hydrocarbon silyl group or a group capable of forming an acetal bond together with an oxygen atom attached to the R⁴⁰.

Specific examples of the R⁴⁰ include the groups referred to in the specific examples of the R⁴ described above in connection with the above-described formula (I).

The starting compound represented by the formula (III-a) can be prepared, for example, by a process represented by the following scheme.

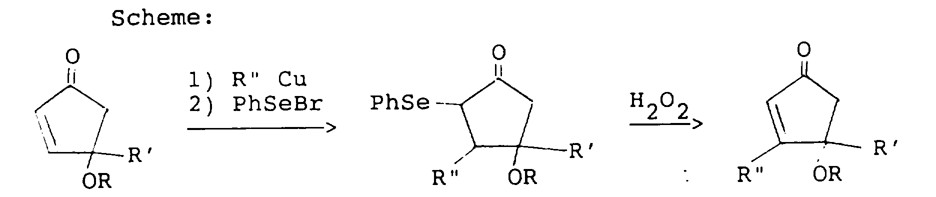

Among the 2-substituted-2-cyclopentenone compounds represented by the above-described formula (I), a 2-substituted-2-cyclopentenone compound represented by the formula (I-b-2-1) can be produced through the following scheme 6 by subjecting a 2-cyclopentenone compound represented by the formula (I-c-21) belonging to the 2-substituted-2-cyclopentenone compound represented by the above-described formula (I-c-2) to an aldol condensation reaction with an aldehyde compound represented by the formula (II-a) in the presence of a lithium amide compound or (a tertiary amine compound and a dialkylborontrifluoromethanesulfonic acid) to prepare a 2-substituted-2-cyclopentenone compound represented by the formula (I-b-21) and then subjecting the 2-substituted-2-cyclopentenone compound to the following optional reactions: an oxidation reaction; a deblocking reaction for a protecting group of a hydroxyl group; a protecting reaction for a hydroxyl group; a hydrolysis reaction of an ester; and an esterification reaction, an amidation reaction, a condensation reaction of a sugar residue and/or a salt forming reaction of a carboxyl group. 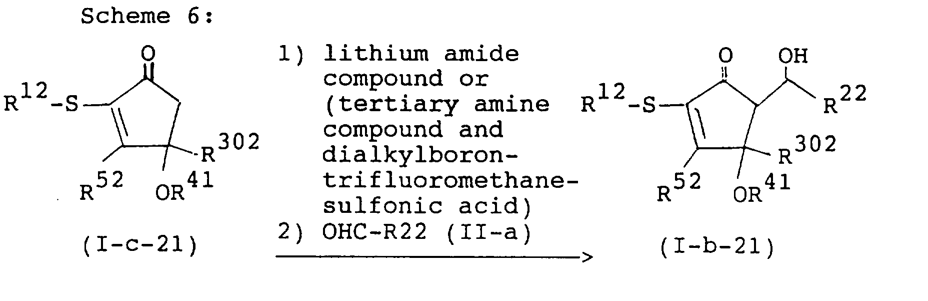 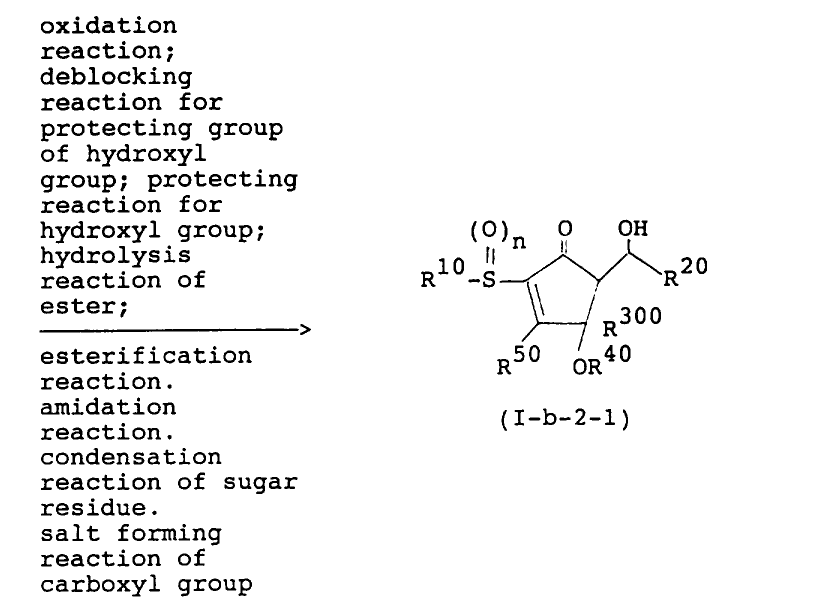
wherein R¹⁰, R¹, R³⁰⁰, R⁴⁰, R⁵⁰ and n are each as defined above;
R¹ stands for a substituted or unsubstituted aliphatic hydrocarbon group having 1 to 10 carbon atoms, alicyclic hydrocarbon group having 4 to 10 carbon atoms, aromatic hydrocarbon group having 6 to 10 carbon atoms or heterocyclic group having 1 to 9 carbon atoms;
R stands for a substituted or unsubstituted aliphatic hydrocarbon group having 1 to 10 carbon atoms, alicyclic hydrocarbon group having 4 to 10 carbon atoms, aromatic hydrocarbon group having 6 to 10 carbon atoms or heterocyclic group having 1 to 9 carbon atoms;
R³⁰ stands for a hydrogen atom or a substituted or unsubstituted aliphatic hydrocarbon group having 1 to 10 carbon atoms, alicyclic hydrocarbon group having 4 to 10 carbon atoms or aromatic hydrocarbon group having 6 to 10 carbon atoms;
R⁴¹ stands for an alkyl group having 1 to 4 carbon atoms, an acyl group having 2 to 7 carbon atoms, an alkoxycarbonyl group having 2 to 5 carbon atoms, a tri(C₁ - C₇) hydrocarbon silyl group or a group capable of forming an acetal bond together with an oxygen atom attached to the R⁴¹; and
R⁵ stands for a hydrogen atom or a substituted or unsubstituted aliphatic hydrocarbon group having 1 to 10 carbon atoms or alicyclic hydrocarbon group having 4 to 10 carbon atoms.

Specific examples of the R¹ include the groups referred to in the specific examples of the R¹ described above in connection with the above-described formula (I).

Specific examples of the R include the groups referred to in the specific examples of the R described above in connection with the above-described formula (I).

Specific examples of the R³⁰ include the groups referred to in the specific examples of the R³ described above in connection with the above-described formula (I).

Specific examples of the R⁵ include the groups referred to in the specific examples of the R⁵ described above in connection with the above-described formula (I).

Preferred substituents of the R¹, R, R³⁰ and R⁵ are respectively the groups described as the substituents of the R¹, R, R³ and R⁵ exclusive of the groups having a carboxylic acid, a salt of a carboxylic acid and a hydroxyl group.

Among the 2-substituted-2-cyclopentenone compounds represented by the above-described formula (I), a 2-substituted-2-cyclopentenone compound represented by the formula (I-b-2-2) can be produced through the following scheme 7 by subjecting a 2-cyclopentenone compound represented by the formula (I-b-21) to an acylation reaction or an alkoxycarbonylation reaction to prepare a 2-substituted-2-cyclopentenone compound represented by the formula (I-b-22) and then subjecting the 2-substituted-2-cyclopentenone compound to the following optional reactions: an oxidation reaction; a deblocking reaction for a protecting group of a hydroxyl group; a protecting reaction for a hydroxyl group; a hydrolysis reaction of an ester; and an esterification reaction, an amidation reaction, a condensation reaction of a sugar residue and/or a salt forming reaction of a carboxyl group. 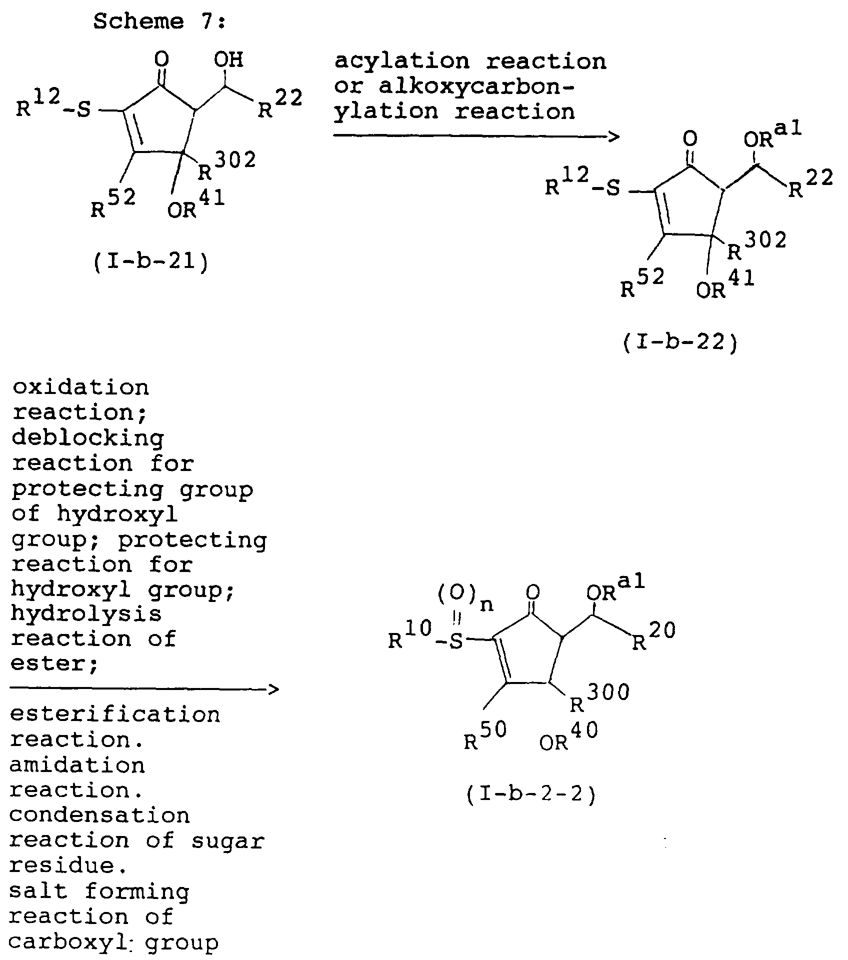 wherein R¹⁰, R¹, R⁰, R, R³⁰⁰, R³⁰, R⁴⁰, R⁴¹, R⁵⁰, R⁵, R^{a1} and n are each as defined above.

Among the 2-substituted-2-cyclopentenone compounds represented by the above-described formula (I), a 2-substituted-2-cyclopentenone compound represented by the formula (I-b-2-3) can be produced through the following scheme 8 by subjecting a 2-substituted-2-cyclopentenone compound represented by the formula (I-c-21) to an enolation with a lithium amide compound, reacting the enolation product with an organoiodide represented by the formula (II-b) in the presence of an organotin compound to prepare a 2-substituted-2-cyclopentenone compound represented by the formula (I-b-23) and then subjecting the 2-substituted-2-cyclopentenone compound to the following optional reactions: an oxidation reaction; a deblocking reaction for a protecting group of a hydroxyl group; a protecting reaction for a hydroxyl group; a hydrolysis reaction of an ester; and an esterification reaction, an amidation reaction, a condensation reaction of a sugar residue and/or a salt forming reaction of a carboxyl group. 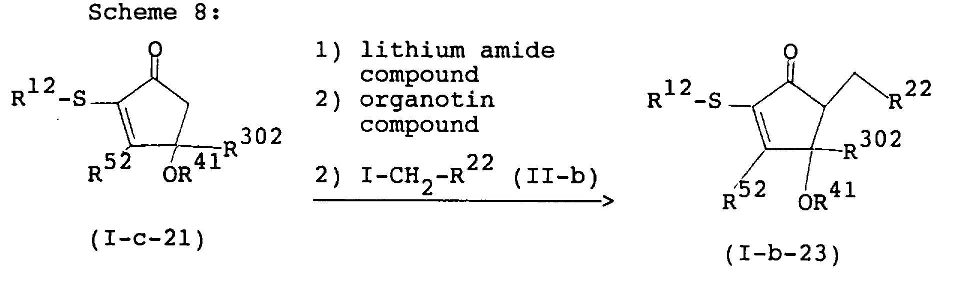 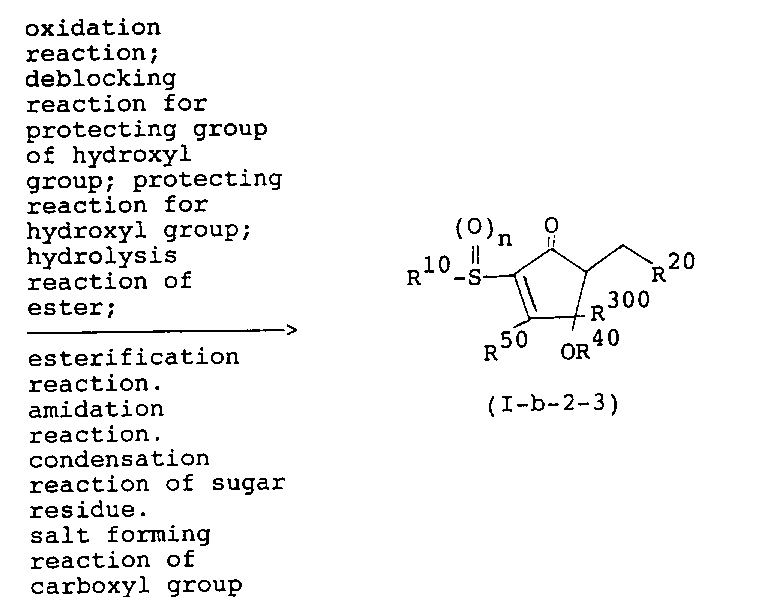 wherein R¹⁰, R¹, R⁰, R, R³⁰⁰, R³⁰, R⁴⁰, R⁴¹, R⁵⁰, R⁵, and n are each as defined above.

Among the 2-substituted-2-cyclopentenone compounds represented by the above-described formula (I), a 2-substituted-2-cyclopentenone compound represented by the formula (I-a-2) can be produced through the following scheme 9 by subjecting a 2-substituted-2-cyclopentenone compound represented by the formula (I-b-21) to a sulfonylation reaction to prepare a 2-substituted-2-cyclopentenone compound represented by the formula (I-b-24), subjecting the 2-substituted-2-cyclopentenone compound to a desulfonation reaction to prepare a 2-substituted-2-cyclopentenone compound represented by the formula (I-a-20) and then subjecting the 2-substituted-2-cyclopentenone compound to the following optional reactions: an oxidation reaction; a deblocking reaction for a protecting group of a hydroxyl group; a protecting reaction for a hydroxyl group; a hydrolysis reaction of an ester; and an esterification reaction, an amidation reaction, a condensation reaction of a sugar residue and/or a salt forming reaction of a carboxyl group.

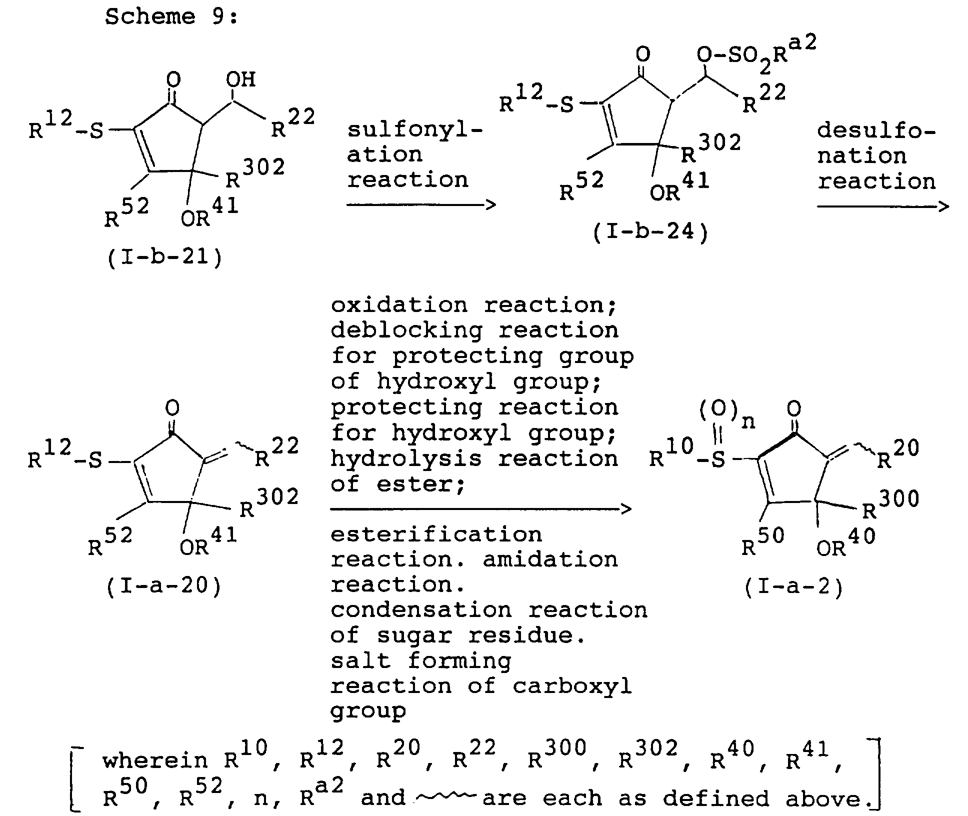

Among the 2-substituted-2-cyclopentenone compounds represented by the above-described formula (I), a 2-substituted-2-cyclopentenone compound represented by the formula (I-a-3) can be produced through the following scheme 10 by subjecting a 2-substituted-2-cyclopentenone compound represented by the formula (I-a-21) belonging to the 2-substituted-2-cyclopentenone compounds represented by the above-described formula (I-a-2) to an elimination reaction under an acidic condition to prepare a 2-substituted-2-cyclopentenone compound represented by the formula (I-a-22) and then subjecting the 2-substituted-2-cyclopentenone compound to the following optional reactions: an oxidation reaction; a deblocking reaction for a protecting group of a hydroxyl group; a protecting reaction for a hydroxyl group, a hydrolysis reaction of an ester; and an esterification reaction, an amidation reaction, a condensation reaction of a sugar residue and/or a salt forming reaction of a carboxyl group. 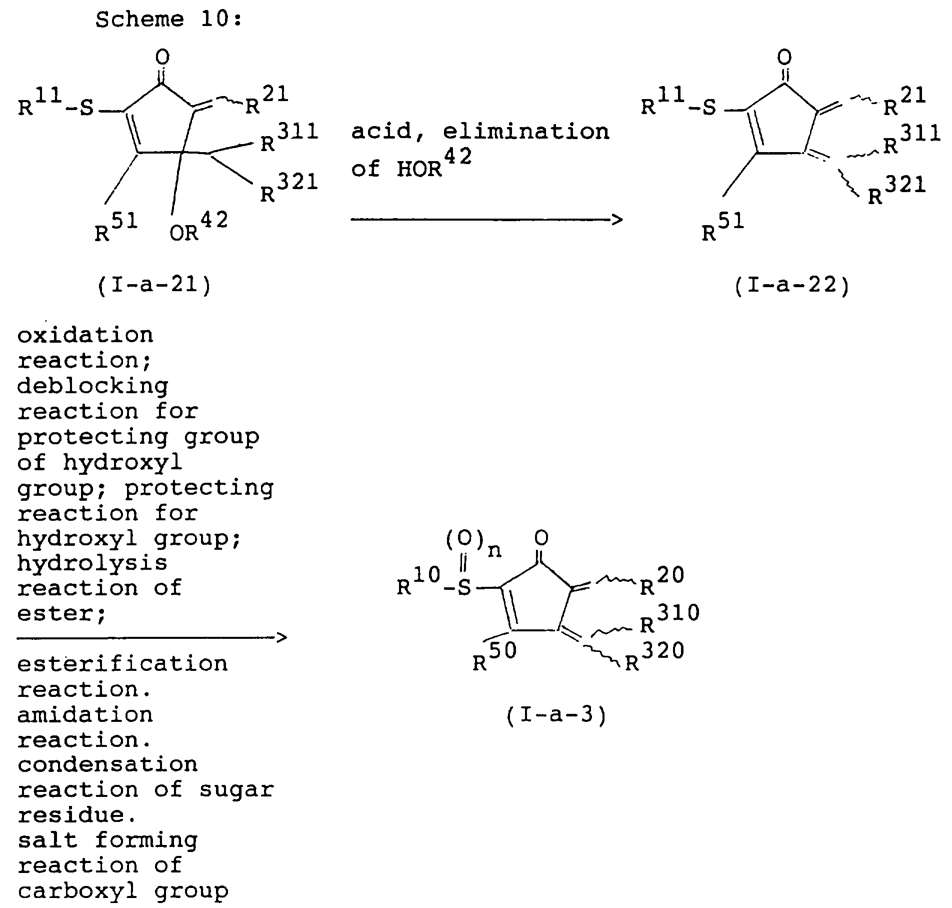
wherein R¹⁰, R¹¹, R⁰, R¹, R⁵⁰, R⁵¹, and 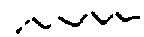 are each as defined above;
R³¹⁰ and R³⁰, or R³¹¹ and R³¹ which may be the same or different from each other stand for a hydrogen atom, a substituted or unsubstituted aliphatic hydrocarbon group having 1 to 9 carbon atoms, alicyclic hydrocarbon group having 4 to 10 carbon atoms or aromatic hydrocarbon group having 6 to 10 carbon atoms or a group wherein R³¹⁰ and R³⁰, or R³¹¹ and R³¹ are combined with each other to form an alicyclic hydrocarbon group having a four to ten-membered ring; and
R⁴ stands for a hydrogen atom or a tri(C₁ -C₇) hydrocarbon silyloxy group.

Specific examples of the R³¹⁰ and R³⁰, or R³¹¹ and R³¹ include the groups referred to in the specific examples of the R³¹ and R³ described above in connection with the above-described formula (I).

Specific examples of the R⁴ include the groups referred to in the specific examples of the R⁴ described above in connection with the above-described formula (I).

Preferred substituents of the R³¹⁰ and R³⁰ are respectively the same substituents as those of the R³¹ and R³, and preferred substituents of the R³¹¹ and R³¹ are respectively the groups described as the substituents of the R³¹ and R³ exclusive of the salt of a carboxylic acid.

The compounds of the present invention are administered to patients by methods such as oral administration, suppository administration, dermal administration, nasal administration, subcutaneous administration, intramuscular administration, intravenous injection and intra-arterial injection.

In the case of the oral administration, the compounds of the present invention may be in the form of a solid preparation or a liquid preparation. Examples of the dosage form include tablets, pills, powders, granules, solutions, suspensions and capsules.

Pharmaceutical preparations in the form of a tablet are prepared by a conventional procedure through the use of additives, for example, excipients such as lactose, starch, calcium carbonate, crystalline cellulose and silicic acid; binders such as carboxymethyl cellulose, methyl cellulose, calcium phosphate and polyvinyl pyrrolidone; disintegrators such as sodium alginate, sodium bicarbonate, sodium laurylsulfate and monoglyceride stearate; lubricants such as glycerin; absorbers such as kaolin and colloidal silica; and lubricants such as talc and granular boric acid.

Pharmaceutical preparations in the form of a pill, powder or granule also may be prepared by a conventional procedure through the use of the same additives as those described above.

Liquid preparations, such as a solution and a suspension, also may be prepared by a conventional procedure. Examples of the carrier used include glycerol esters such as tricaprylin, triacetin and iodided poppy seed oil fatty acid esters; water; alcohols such as ethanol; and oleaginous bases such as liquid paraffin, coconut oil, soybean oil, sesame oil and corn oil.

The above-described powders, granules and liquid preparations may be encapsulated in a gelatin or the like.

In the present invention, the pharmaceutically acceptable carrier includes, besides the above-described carriers, auxiliary substances, perfuming agents, stabilizers and preservatives commonly used in the art, according to need.

Examples of the dosage form in the case of the dermal administration include ointments, creams, lotions and solutions.

Examples of the base for the ointment include fatty oils such as castor oil, olive oil, sesame oil and safflower oil; lanolin; white, yellow or hydrophilic petrolatum; wax; higher alcohols such as oleyl alcohol, isostearyl alcohol, octyldodecanol and hexyldecanol; and glycols such as glycerin, diglycerin, ethylene glycol, propylene glycol, sorbitol and 1,3-butanediol. Ethanol, dimethylsulfoxide, polyethylene glycol, etc. may be used as a solubilizing agent for the compound of the present invention. If necessary, it is also possible to use preservatives such as p-oxybenzoates, sodium benzoate, salicylic acid, sorbic acid and boric acid; and antioxidants such as butylhydroxyanisole and dibutylhydroxytoluene.

Absorbefacients, such as diisopropyl adipate, diethyl sebacate, ethyl caproate and ethyl laurate, may be added to thereby promote the percutaneous absorption. Further, to enhance the stabilization, the compounds of the present invention can be used in the form of a compound included in an α, β or γ-cyclodextrin.

The ointment can be prepared by a conventional procedure. The cream is preferably in an oil-in-water cream form from the viewpoint of stabilizing the compounds of the present invention. The above-described fatty oils, higher alcohols and glycols are used as the base, and use is made of emulsifiers such as diethylene glycol, propylene glycol, sorbitan monofatty acid ester, polysorbate 80 and sodium laurylsulfate. Further, if necessary, the above-described preservatives, antioxidants, etc. may be added. As with the ointment, in the case of the cream, the compound of the present invention may be used in the form of a compound included in a cyclodextrin or a methylated cyclodextrin. The cream can be prepared by a conventional procedure.

Examples of the lotion include lotions in the form of a suspension, an emulsion and a solution. The lotion in the form of a suspension is prepared through the use of a suspending agent, such as sodium alginate, tragacanth or sodium carboxymethylcellulose, and antioxidants, preservatives, etc. are added thereto according to need.

The lotion in the form of an emulsion is prepared through the use of an emulsifier, such as sorbitan monofatty acid ester, polysorbate 80 or sodium laurylsulfate, by a conventional procedure.

The lotion in the form of a solution is preferably an alcoholic lotion, and the alcoholic lotion is prepared through the use of an alcohol, such as ethanol, by a conventional procedure. Examples of the preparation in the form of a solution include that prepared by dissolving the compound of the present invention in ethanol, and optionally, adding an antioxidantor or a preservative, etc. to the solution.

Examples of other dosage forms include dermatologic pastes, cataplasms and aerosols. These preparations can be prepared by a conventional procedure.

The preparation for nasal administration is provided in the form of a liquid or powdery composition. Water, a saline solution, a phosphate buffer and an acetate buffer are used as a base for the liquid formulation, and the liquid formulation may contain surfactants, antioxidants, stabilizers, preservatives and tackifiers. Water absorbing bases are preferred as a base for the powder formulation, and examples thereof include bases easily soluble in water, for example, polyacrylates such as sodium polyacrylate, potassium polyacrylate and ammonium polyacrylate, cellulose lower alkyl ethers, such as methyl cellulose, hydroxyethyl cellulose, hydroxypropyl cellulose and sodium carboxymethyl cellulose, polyethylene glycol polyvinyl pyrrolidone, amylose and pullulan, and bases hardly soluble in water, for example, celluloses such as crystalline cellulose, α-cellulose and crosslinked carboxymethyl cellulose, starches such as hydroxypropyl starch, carboxymethyl starch, crosslinked starch, amylose, amylopectin and pectin, proteins such as gelatin, casein, sodium casein, gums such as gum arabic, tragacanth gum and glucomannan, and crosslinked vinyl polymers such as polyvinyl polypyrrolidone, crosslinked polyacrylic acid and its salts, crosslinked polyvinyl alcohol and polyhydroxyethyl methacrylate, which may be used in the form of a mixture thereof. Further, the powder formulation may contain antioxidants, colorants, preservatives, antiseptics, and corrigents, etc. The above-described liquid and powder formulations may be administered by, for example, a spray.

The preparation for injection administration is provided in the form of an aseptic aqueous or non-aqueous solution, suspension or emulsion. In the non-aqueous solution or suspension, propylene glycol, polyethylene glycol, vegetable oils such as olive oil and injectable organic esters such as ethyl oleate and iodided poppy seed oil fatty acid ester are used as a pharmaceutically acceptable carrier. These preparations may contain auxiliary agents such as antiseptics, wetting agents, emulsifiers, dispersants and stabilizers, and may be in a sustained release form. The above-described solutions, suspensions and emulsions can be made aseptic through a proper filtration whereby they are passed through a bacteria retaining filter, incorporation of a germicide, or treatments such as irradiation. Further, an aseptic solid preparation may be prepared and dissolved in an aseptic water or an aseptic solvent for injection immediately before use.

Further, it is also possible to use the compound of the present invention in the form of a compound included in an α, β or γ-cyclodextrin or a methylated cyclodextrin. Further, the compound of the present invention may be used in the form of an injection wherein a fat is bonded to the compound.

Although the effective dose of the compound of the present invention varies depending upon the administration method, age, sex and condition of patients, it is generally 1 to 10⁵ µg/kg/day, preferably about 10 to 10⁴ µg/kg/day.

### Industrial Applicability

The 2-substituted-2-cyclopentenone compound of the present invention exhibits, at a low concentration thereof, a strong effect of inhibiting the growth of L1210 leukemia cells and therefore, can be considered for use as an anticancer agent. Moreover, this compound enhances the alkaline phosphatase activity of human osteoblasts, and further, enhances the content of calcium and phosphorus in the human osteoblasts. Therefore, the compound of the present invention is useful as a bone formation accelerator and is effective for the treatment or prevention of osteoporosis and osteomalalacia. Further, the compound of the present invention can be expected to have an antiviral activity and an antimicrobial activity, which renders the compound of the present invention very useful as a pharmaceutical.

### Examples

The present invention will now be described in more detail by way of the following Examples, but is not limited to these Examples.

### Reference Example 1

First, 10 g of a starting 2-cyclopentenone compound as given in the following Table 1 was dissolved in 10 ml of dichloromethane, 5.71 ml of pyridine was added to the solution and 10.8 g of phenylselenenyl chloride was then added and the mixture stirred for 5 hr. The stirred mixture was poured in an aqueous potassium hydrogen sulfate solution and extracted with ethyl acetate, and the resultant organic phase was washed with an aqueous sodium bicarbonate and a saline solution, and then dried over magnesium sulfate. The dried organic phase was filtered, concentrated, and then subjected to silica gel chromatography to obtain a 2-cyclopentenone compound as given in Table 1. Spectral data (¹H-NMR (δ, CDCl₃)) are also given in Table 1.

### Reference Example 2

First, 1.56 g of 1-pentynyl copper was weighed and purged with nitrogen, 5.4 ml of hexamethylphosphoric triamide was added thereto, the mixture was stirred for 20 min, 15 ml of tetrahydrofuran and 40 ml of an ether were added thereto, and the mixture was cooled to -70°C. Then, 7.86 ml of a 1.52 M pentane solution of a starting organolithium reagent as given in the following Table 2 was added thereto, the mixture was stirred for 20 min, a solution of 4.0 g of a starting 2-cyclopentenone compound as given in Table 2 in 20 ml of tetrahydrofuran was added thereto, and the mixture was again stirred at -70 to -50°C for 2 hr. The stirred mixture was poured in an acetate buffer having a pH value of 4, and hexane was added thereto for extraction. The resultant organic phase was washed with a saline solution, dried over magnesium sulfate, and filtered and concentrated to obtain an oily residue. The oily residue was dissolved in 50 ml of dichloromethane, 1 ml of pyridine was added to the solution, then 5 ml of a 30% aqueous hydrogen peroxide was added thereto with stirring and cooling on ice, and the mixture was stirred for 30 min. The stirred mixture was poured in an aqueous potassium hydrogensulfate and extracted with hexane. The resultant organic phase was washed with a saline solution, dried over magnesium sulfate, filtered, concentrated, and then subjected to silica gel chromatography to obtain a 2-cyclopentenone compound (an intended product) as given in the following Table 2.

### Reference Example 3

First, 157 mg of 1-pentynyl copper was weighed and purged with nitrogen, 545 µl of hexamethylphosphoric triamide was added thereto, and the mixture was stirred for 20 min. Then 5 ml of tetrahydrofuran was added thereto, and the mixture was cooled to -70°C and added to 1.2 mmol of a starting organolithium compound as given in the following Table 2. The mixture was stirred at -70°C for 20 min, a solution of 318 mg of a starting 2-cyclopentenone compound given in Table 2 in 10 ml of tetrahydrofuran was added thereto, and the mixture was again stirred for 2 hr. A solution of 283 mg of phenylselenenyl bromide in 10 ml of tetrahydrofuran was added thereto, and the mixture was stirred for 1 hr, and the stirred mixture was poured in an aqueous ammonium chloride and extracted with ethyl acetate. The resultant organic phases were combined with each other, washed with a saline solution, and dried over magnesium sulfate. The dried organic phase was filtered, concentrated, and then subjected to silica gel chromatography to obtain an intermediate. The intermediate was dissolved in 20 ml of dichloromethane, 500 µl of pyridine was added thereto, 2 ml of a 30% aqueous hydrogen peroxide was added thereto, and the mixture was stirred for 1.5 hr. The stirred mixture was poured in potassium hydrogensulfate and extracted with ethyl acetate. The resultant organic phases were combined with each other, washed with an aqueous sodium carbonate solution and a saline solution, in that order, and then dried over magnesium sulfate. The dried organic phase was filtered, concentrated, and then subjected to silica gel chromatography to obtain a 2-cyclopentenone compound as given in Table 2.

### Reference Examples 4 and 5

The 2-cyclopentenone compounds (intended products) as given in the following Table 2 were prepared in the same manner as that of Reference Example 3.

### Reference Example 6

First, 61 mg of copper (II) chloride dihydrate and 30 mg of lithium chloride were weighed and heat-dried under a reduced pressure, 5 ml of tetrahydrofuran was added thereto, and the mixture was cooled to -70°C. Then a solution of 955 mg of a starting 2-cyclopentenone as given in the following Table 3 in 10 ml of tetrahydrofuran was added thereto, 3.6 mmol of a Grignard's reagent as given in Table 3 was added thereto, and the mixture was stirred at -70 to -30°C for 2.5 hr. Then a solution of 944 mg of phenylselenenyl bromide in 10 ml of tetrahydrofuran was added thereto, the mixture was stirred at -30°C for 1 hr, an aqueous ammonium chloride solution was added thereto, and the mixture was extracted with ethyl acetate. The resultant organic phase was washed with a saline solution, the washed organic layer was concentrated, the resultant oil residue containing an intermediate was dissolved in 40 ml of dichloromethane, 1 ml of pyridine was added thereto, and then 4 ml of a 30% aqueous hydrogen peroxide was added thereto with stirring and cooling on ice, and the mixture was stirred for 1 hr. An aqueous potassium hydrogensulfate solution was added thereto, and the mixture was extracted with ethyl acetate. The resultant organic layer was washed with an aqueous sodium bicarbonate solution and a saline solution and dried over magnesium sulfate. The dried organic phase was filtered, concentrated and then subjected to silica gel chromatography to obtain a 2-cyclopentenone compound (an intended product) given in Table 3.

### Reference Examples 7 to 13

The 2-cyclopentenone compounds as given in the following Table 3 were prepared in the same manner as that of Reference Example 6.

### Reference Example 14

First, 1.3 ml of hexamethylphosphorous triamide was added to 313 mg of 1-pentynyl copper, the mixture was stirred for 20 min, 5 ml of an ether was added thereto, and the mixture was cooled to -70°C and added to 2.5 mmol of a starting organolithium compound as given in the following Table 4. Then the mixture was stirred at -70°C for 15 min, a solution of 453 mg of a starting 2-cyclopentenone compound given in Table 4 in 10 ml of an ether was added thereto, 300 µl of a boron trifluoride-ether complex was further added thereto, and the mixture was stirred at -70 to -30°C for 1 hr. Then a solution of 375 mg of an aldehyde compound given in Table 1 in 10 ml of an ether was added thereto, the mixture was stirred at -30°C for 1 hr, an aqueous ammonium chloride was added thereto, and the mixture was extracted with ethyl acetate. The resultant organic phase was washed with a saline solution and dried over magnesium sulfate, the dried organic phase was filtered and concentrated, the resultant oily residue was dissolved in 40 ml of dichloromethane, 400 µl of 1,8-diazabicyclo[5.4.0.]-7-undecene was added thereto, and the mixture was stirred for 5 hr. Then an aqueous potassium hydrogensulfate solution was added thereto, and the mixture was extracted with ethyl acetate. The resultant organic phase was washed with a saline solution and then dried over magnesium sulfate. The dried organic phase was filtered, concentrated and then subjected to silica gel chromatography to obtain a 2-cyclopentenone compound as given in Table 4.

### Reference Examples 15 to 17

The 2-cyclopentenone compounds as given in the following Table 4 were prepared in the same manner as that of Reference Example 14.

### Reference Example 18

First, 3.3 g of a starting 2-cyclopentenone compound as given in the following Table 5 was dissolved in 50 ml of methanol, 5.0 ml of a 30% aqueous hydrogen peroxide was added thereto with stirring and cooling on ice, and 500 µl of a 1 N aqueous sodium hydroxide solution was added thereto. Then the mixture was stirred for 3.5 hr, an aqueous ammonium chloride solution was added thereto, and the mixture was extracted with ethyl acetate. The resultant organic phase was washed with a saline solution and dried over magnesium sulfate, and the dried organic phase was filtered, concentrated and then subjected to silica gel chromatography to obtain a 2,3-epoxycyclopentanone compound as given in Table 5.

### Reference Examples 19 to 56

The 2,3-cyclopentanone compounds as given in the following Table 5 were prepared in the same manner as that of Reference Example 18.

### Example 1

First, 9.2 mg of sodium thiomethoxide was dissolved in 2 ml of methanol, 11.2 µl of acetic acid was added thereto with stirring and cooling on ice, 36.4 µl of triethylamine was added thereto, 19 mg of a 2,3-epoxycyclopentanone compound as given in the following Table 6 was added, and the mixture was stirred for 5 hr. Then an aqueous ammonium chloride solution was added thereto, and the mixture was extracted with ethyl acetate, and the resultant organic phase was washed with a saline solution and dried over magnesium sulfate, and the dried organic phase was filtered, concentrated and then subjected to silica gel chromatography to obtain a 2-substituted-2-cyclopentenone compound as given in Table 6.

### Example 2

A 2-substituted-2-cyclopentenone compound as given in the following Table 6 was prepared in the same manner as that of Example 1.

### Reference Examples 61 to 63

The 2-Substituted-2-cyclopentenone compounds as given in the following Table 7 were prepared in the same manner as that of Example 1.

### Reference Example 57

First, 1.5 g of sodium thiomethoxide was dissolved in 80 ml of methanol, 1.8 ml of acetic acid was added thereto with cooling on ice, the mixture was stirred for 5 min, 4.8 ml of triethylamine was added thereto, and a solution of 1.38 g of a starting 2,3-epoxycyclopentanone compound as given in the following Table 6 in 20 ml of methanol was added thereto. Then the mixture was stirred for 4 hr, water was added thereto, and the mixture was extracted with ethyl acetate. The resultant organic phases were combined with each other, washed with a saline solution and dried over magnesium sulfate. The dried organic phase was filtered and concentrated, the resultant oily residue was dissolved in 15 ml of dimethylformamide, 1.15 g of imidazole and 1.04 ml of chlorotrimethylsilane were added thereto with stirring and cooling on ice, and the mixture was stirred at 0°C for 3 hr. Water and hexane were added thereto for extraction, and the resultant organic phase was washed with a saline solution. The organic phase was dried over sodium sulfate, filtered, concentrated and then subjected to a silica gel chromatography to obtain a 2-substituted-2-cyclopentenone compound as given in Table 6.

### Reference Examples 58 to 60

The 2-Substituted-2-cyclopentenone compounds as given in the following Table 6 were prepared in the same manner as that of Reference Example 57.

### Reference Examples 64 to 66

The 2-Substituted-2-cyclopentenone compounds as given in the following Table 7 were prepared in the same manner as that of Reference Example 57.

### Example 3

First, 5 g of a starting 2,3-epoxycyclopentanone compound as given in the following Table 8 was dissolved in 100 ml of methanol, 10 ml of triethylamine was added thereto, 1.2 g of a thiol compound was added thereto, and the mixture was stirred for 2 hr. The reaction mixture was poured in an aqueous potassium hydrogensulfate solution and extracted with ethyl acetate, and the extract was washed with a saline solution and dried over magnesium sulfate, and the dried extract was filtered, concentrated and then subjected to silica gel chromatography to obtain a 2-substituted-2-cyclopentenone compound as given in Table 8.

### Examples 4 to 29 and Reference Example 67.

The 2-Substituted-2-cyclopentenone compounds as given in the following Table 8 were prepared in the same manner as that of Example 3.

### Reference Examples 68 to 76

The 2-Substituted-2-cyclopentenone compounds as given in the following Table 9 were prepared in the same manner as that of Example 3.

### Reference Examples 114 to 121

The 2-Substituted-2-cyclopentenone compounds as given in the following Table 11 were prepared in the same manner as that of Reference Example 77.

### Reference Example 77

First, 1.8 g of a starting 2,3-epoxycyclopentanone compound as given in the following Table 10 was dissolved in 15 ml of methanol, 1.0 ml of triethylamine was added thereto, 790 mg of a thiol compound was added thereto, and the mixture was stirred for 1.5 hr. The reaction mixture was poured in an aqueous potassium hydrogensulfate solution and extracted with ethyl acetate, and the resultant organic phase was washed with a saline solution and dried over magnesium sulfate. The dried organic phase was filtered and concentrated, the resultant crude oily residue was dissolved in 20 ml of dimethylformamide, and 1.5 g of imidazole was added thereto with stirring and cooling on ice. Then 1.4 g of chlorotrimethylsilane was added thereto, the mixture was stirred at 0°C for 5 hr, and water and hexane were added thereto for extraction. The resultant organic phase was washed with a saline solution, dried over sodium sulfate, filtered, concentrated and then subjected to silica gel chromatography to obtain a 2-substituted-2-cyclopentenone compound as given in Table 10.

### Reference Examples 78 to 113

The 2-Substituted-2-cyclopentenone compounds as given in the following Table 10 were prepared in the same manner as that of Reference Example 77.

### Example 30

First, 216 mg of a starting 2-substituted-2-cyclopentenone compound as given in the following Table 13 was weighed, 2.5 ml of an ether and 2.5 ml of hexane were added thereto, 157 µl of diisopropylethylamine was added thereto, and the mixture was cooled to -70°C. Then, 750 µl of a 1 M dichloromethane solution of dibutylboron trifluoromethanesulfonate was added thereto, the mixture was stirred for 1 hr, a solution of 373 mg of a starting aldehyde compound in 10 ml of an ether was cooled to -70°C and added thereto, and the mixture was stirred at -70 to -25°C for 4 hr. Then the stirred mixture was dried over magnesium sulfate, filtered, concentrated and then subjected to silica gel chromatography to obtain a 2-substituted-2-cyclopentenone compound (an intended product) as given in Table 13.

### Examples 31 to 107

The 2-Substituted-2-cyclopentenone compounds (intended products) as given in the following Table 13 were prepared in the same manner as that of Example 30.

### Reference Examples 122 to 127

The 2-Substituted-2-cyclopentenone compounds (intended products) as given in the following Table 14 were prepared in the same manner as that of Example 30.

### Example 203

First, 861 mg of a starting 2-substituted-2-cyclopentenone compound as given in the following Table 15 was dissolved in 5 ml of tetrahydrofuran, the solution was cooled to -70°C, 1.67 ml of a 1.5 M tetrahydrofuran solution of lithium diisopropylamide was added thereto, and the mixture was stirred for 30 min. Then a solution of 526 mg of a starting aldehyde compound in 5 ml of tetrahydrofuran was added thereto, the mixture was stirred at -70 to -40°C for 3 hr, the reaction mixture was poured in an aqueous ammonium chloride solution, and the mixture was extracted with ethyl acetate. The resultant organic phase was washed with a saline solution and dried over magnesium sulfate, and the dried organic phase was filtered, concentrated and then subjected to silica gel chromatography to obtain a 2-substituted-2-cyclopentenone compound (an intended product) as given in Table 15.

### Examples 204 to 207

The 2-Substituted-2-cyclopentenone compounds as given in the following Table 15 were prepared in the same manner as that of Example 203.

### Example 208

First, 60 mg of a starting 2-substituted-2-cyclopentenone compound as given in the following Table 16 was dissolved in 3 ml of dichloromethane, 200 µl of pyridine was added thereto, 100 µl of acetyl chloride was added thereto, and the mixture was stirred for 16 hr. Then the mixture was poured in an aqueous potassium hydrogensulfate solution, the mixture was extracted with ethyl acetate, and the resultant organic phase was washed with an aqueous sodium bicarbonate solution and a saline solution and then dried over magnesium sulfate. The dried organic phase was filtered, concentrated and then subjected to silica gel chromatography to obtain a 2-substituted-2-cyclopentenone compound (an intended product) as given in Table 16.

### Examples 209 to 219

The 2-Substituted-2-cyclopentenone compounds as given in the following Table 16 were prepared in the same manner as that of Example 208.

### Example 220

First, 60 mg of a starting 2-substituted-2-cyclopentenone compound as given in the following Table 16 was dissolved in 3 ml of dichloromethane, 500 µl of pyridine was added thereto, 300 µl of methoxycarbonyl chloride was added thereto, and the mixture was stirred for 16 hr. Then the mixture was poured in an aqueous potassium hydrogensulfate solution, the mixture was extracted with ethyl acetate, and the resultant organic phases were combined with each other, washed with an aqueous sodium bicarbonate solution and a saline solution and then dried over magnesium sulfate. The dried organic phase was filtered, concentrated and then subjected to silica gel chromatography to obtain a 2-substituted-2-cyclopentenone compound (an intended product) as given in Table 16.

### Examples 221 to 222

The 2-Substituted-2-cyclopentenone compounds as given in the following Table 16 were prepared in the same manner as that of Example 220.

### Example 223

First, 60 mg of a starting 2-substituted-2-cyclopentenone compound as given in the following Table 16 was dissolved in 3 ml of dichloromethane, 200 µl of pyridine was added thereto, 300 µl of isopropoxycarbonyl chloride was added thereto, and the mixture was stirred for 16 hr. Then the mixture was poured in an aqueous potassium hydrogensulfate solution, the mixture was extracted with ethyl acetate and the resultant organic phases were combined with each other, washed with an aqueous sodium bicarbonate solution and a saline solution and then dried over magnesium sulfate. The dried organic phase was filtered, concentrated and then subjected to silica gel chromatography to obtain a 2-substituted-2-cyclopentenone compound (an intended product) as given in Table 16.

### Examples 224 to 231

The 2-Substituted-2-cyclopentenone compounds as given in the following Table 16 were prepared in the same manner as that of Example 223.

### Example 232

First, 182 mg of a starting 2-substituted-2-cyclopentenone compound as given in the following Table 17 was weighed and purged with nitrogen, 15 ml of tetrahydrofuran was added thereto, the mixture was cooled to -70°C, 600 µl of a 1.5 M cyclohexane solution of lithium diisopropylamide was added thereto, and the mixture was stirred at -70°C for 40 min. Then 1 ml of N-methyl pyrrolidone was added thereto, a solution of 251 mg of triphenyltin chloride in 10 ml of tetrahydrofuran was added thereto, the mixture was stirred at -70°C for 1 hr, a solution of 400 mg of a starting organoiodide in 10 ml of tetrahydrofuran was added thereto, the mixture was stirred at -40 to -10°C for 19 hr and poured in an aqueous potassium hydrogensulfate solution, and the mixture was extracted with ethyl acetate. The resultant organic phases were combined with each other, washed with an aqueous sodium bicarbonate solution and a saline solution and then dried over magnesium sulfate, and the dried organic phase was filtered, concentrated and then subjected to silica gel chromatography to obtain a 2-substituted-2-cyclopentenone compound (an intended product) as given in Table 17.

### Examples 233 to 237

The 2-Substituted-2-cyclopentenone compounds as given in the following Table 17 were prepared in the same manner as that of Example 232.

### Example 239

First, Dimethylaminopyridine (1.5 g) was added to a solution of 3.5 g of a starting 2-substituted-2-cyclopentenone compound as given in the following Table 18 in dichloromethane (30 ml), the mixture was cooled to 0°C, 0.6 ml of methanesulfonyl chloride was dropwise added thereto, and the mixture was stirred at room temperature for 13 hr. Then ethyl acetate and an aqueous potassium hydrogensulfate were added to the reaction mixture to extract the product into an organic phase, the extract was washed with an aqueous sodium bicarbonate solution and a saline solution, dried over magnesium sulfate, filtered and concentrated, and the concentrate was subjected to silica gel chromatography to obtain a 2-substituted-2-cyclopentenone compound (an intended product) as given in Table 18.

### Examples 240, 241, 243-270, 276, 281, 284-317 and 320-348

The 2-Substituted-2-cyclopentenone compounds as given in the following Table 18 were prepared in the same manner as that of Example 239.

### Example 350

First, 300 mg of a starting 2-substituted-2-cyclopentenone compound as given in the following Table 19 was dissolved in 15 ml of acetonitrile, 2 ml of pyridine was added thereto, 1 ml of a hydrogen fluoride-pyridine solution was added thereto with stirring and cooling on ice, and the mixture was stirred at 0°C to room temperature for 16 hr. Then the stirred mixture was poured in an aqueous potassium hydrogensulfate solution, the mixture was extracted with ethyl acetate, and the resultant organic phases were combined with each other, washed with a saline solution and then dried over magnesium sulfate. The dried organic phase was filtered, concentrated and then subjected to silica gel chromatography to obtain a 2-substituted-2-cyclopentenone compound (an intended product) as given in Table 19.

### Examples 351-353, 355-369, 371-374, 376-398, 401-435, 440, 445, 448-476 and 479-511

The 2-Substituted-2-cyclopentenone compounds as given in the following Table 19 were prepared in the same manner as that of Example 350.

### Example 513

The 2-substituted-2-cyclopentenone compound as given in the following Table 20 was prepared in the same manner as that of Example 350.

### Example 514

First, 50 mg of a starting 2-substituted-2-cyclopentenone compound as given in the following Table 20 was dissolved in 5 ml of a mixed solvent comprising acetic acid, tetrahydrofuran and water in a ratio of 2 : 1 : 1, and the mixture was stirred for 5 hr. Then the the solution was neutralized with sodium bicarbonate and extracted with ethyl acetate, and the resultant organic phases were combined with each other, washed with a saline solution, dried over magnesium sulfate, filtered, concentrated, and then subjected to silica gel chromatography to obtain a 2-substituted-2-cyclopentenone compound (an intended product) as given in Table 20.

### Examples 515

A 2-substituted-2-cyclopentenone compound as given in the following Table 20 was prepared in the same manner as that of Example 514.

### Example 530

### Measurement of anticancer activity

Tumor cells were grown in an RPMI 1640 medium containing of 10% fetal calf serum.

The compound was dissolved in 99.5% ethanol, adjusted before use so that the final concentration of ethanol was 0.1% or less, and added to the medium.

The control was 0.1% ethanol, and L1210 tumor cells were inoculated in the medium in a concentration of 2.5 x 10⁴ cells/ml and grown for 2 days. The number of surviving cells were measured by trypan blue staining.

The results are given in the following Table 21.

### Example 531

### Measurement of bone formation activity

Human osteoblasts (KK-3, 18PDL) were cultured in an α-MEM containing 10% fetal calf serum. After the growth became stationary, the compound was added in a given concentration in the presence of 2 mM α-glycerophosphate and treated for 14 days. The cell phase was washed with physiological saline, and the alkaline phosphatase activity (ALP) was measured through the absorption of OD₄₁₅. Then, calcium (Ca) and phosphorus (P) were extracted with a 2 N hydrochloric acid and quantitatively determined. The results are given in Tables 22 and 24 to 32.

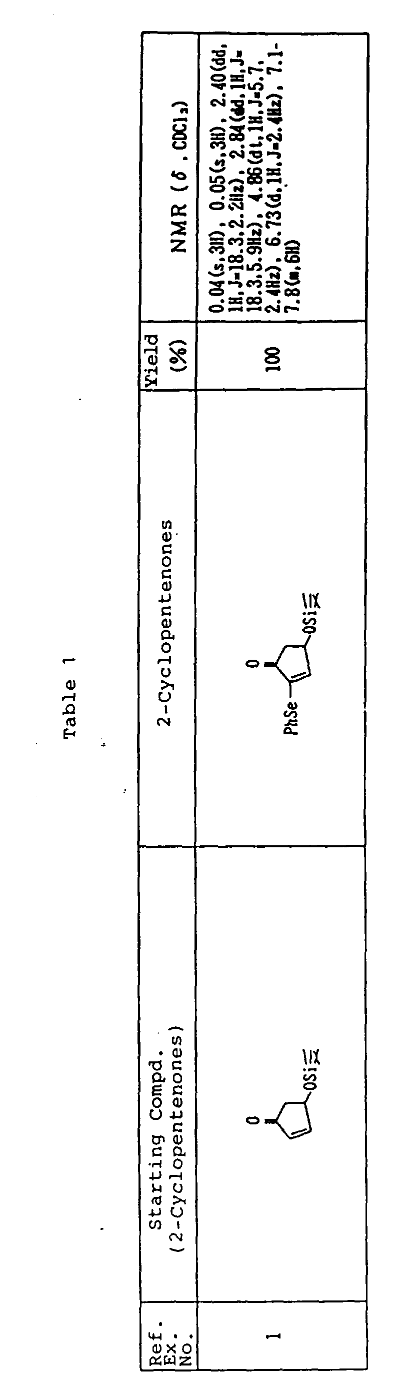

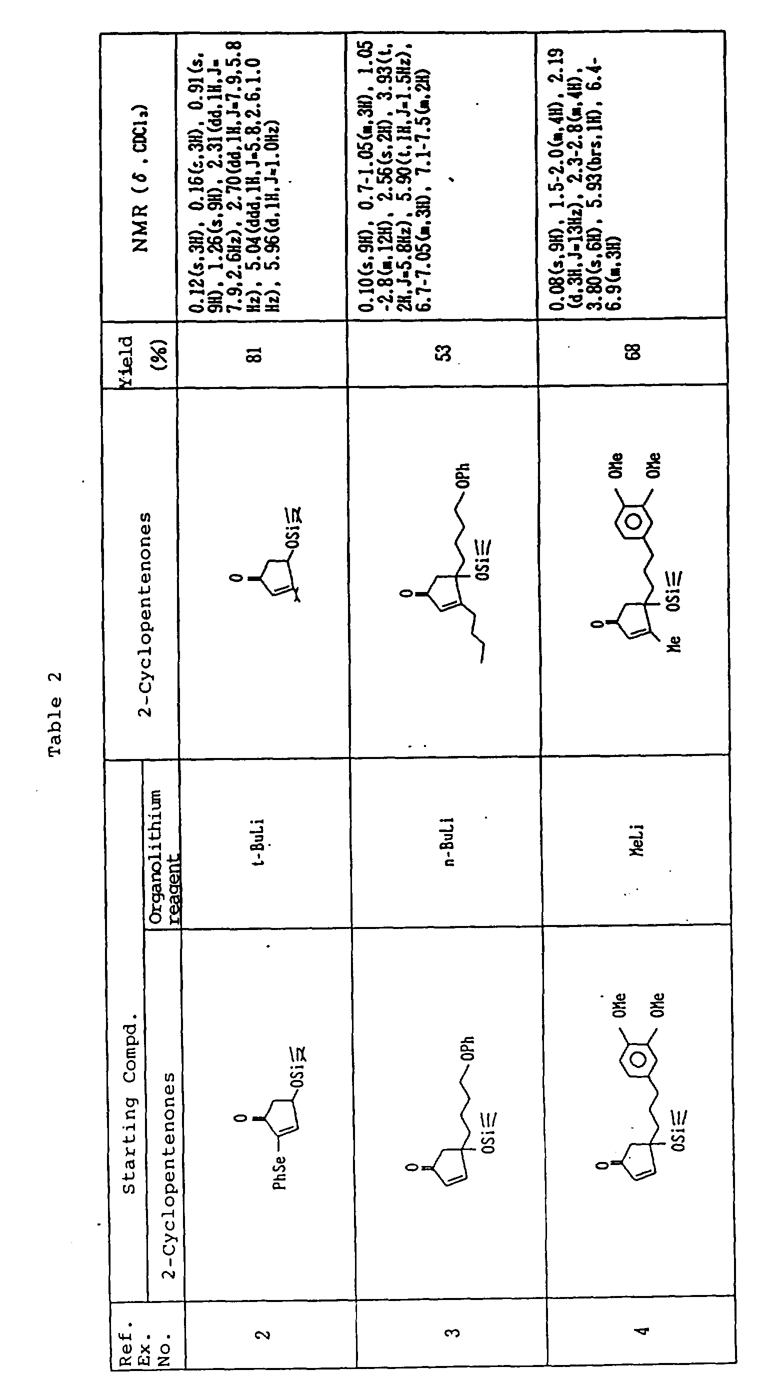

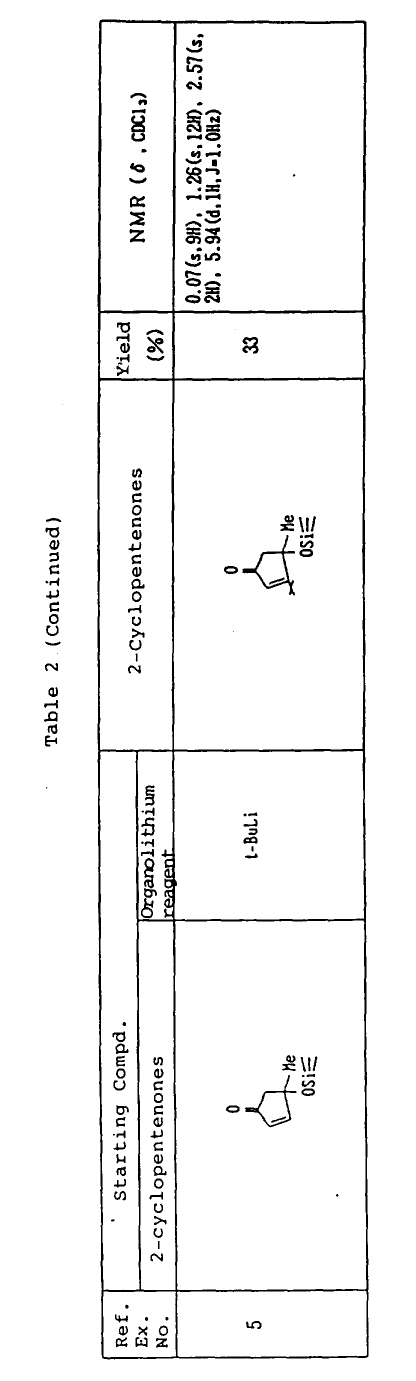

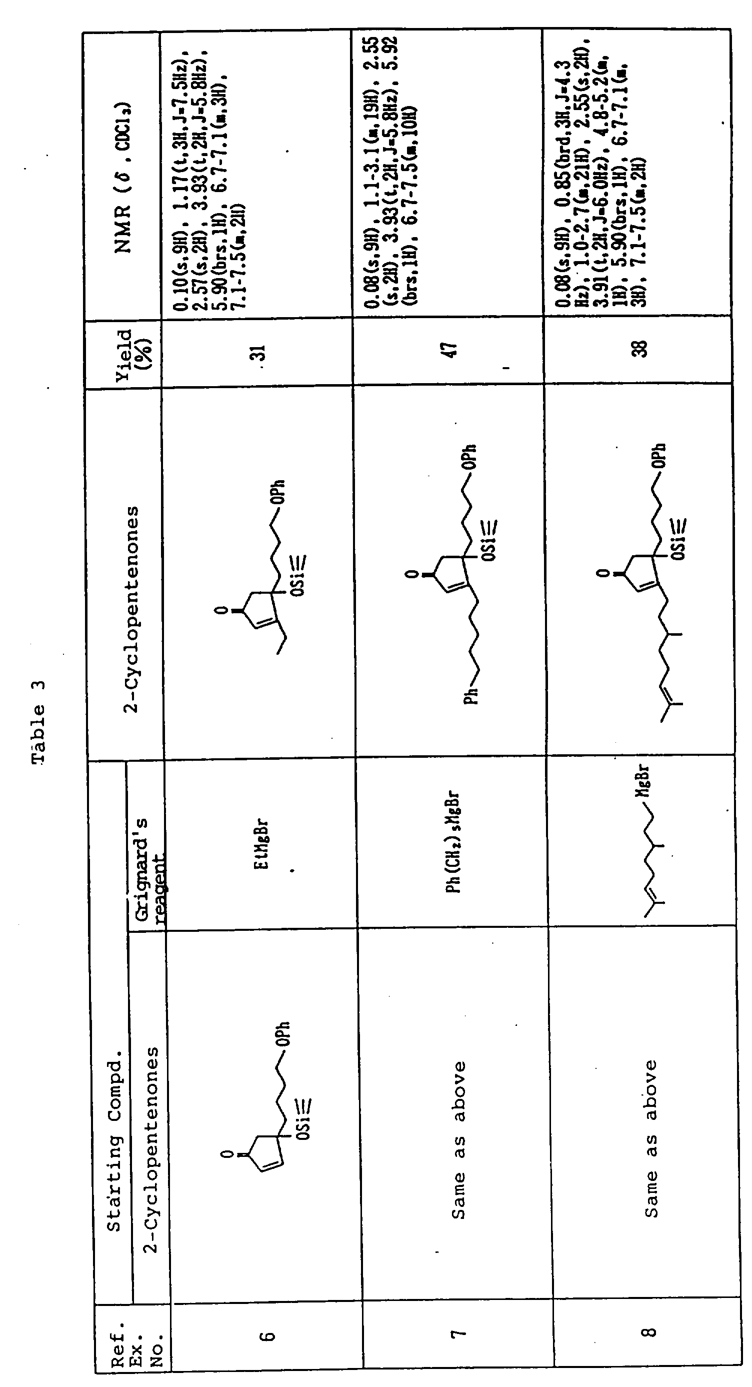

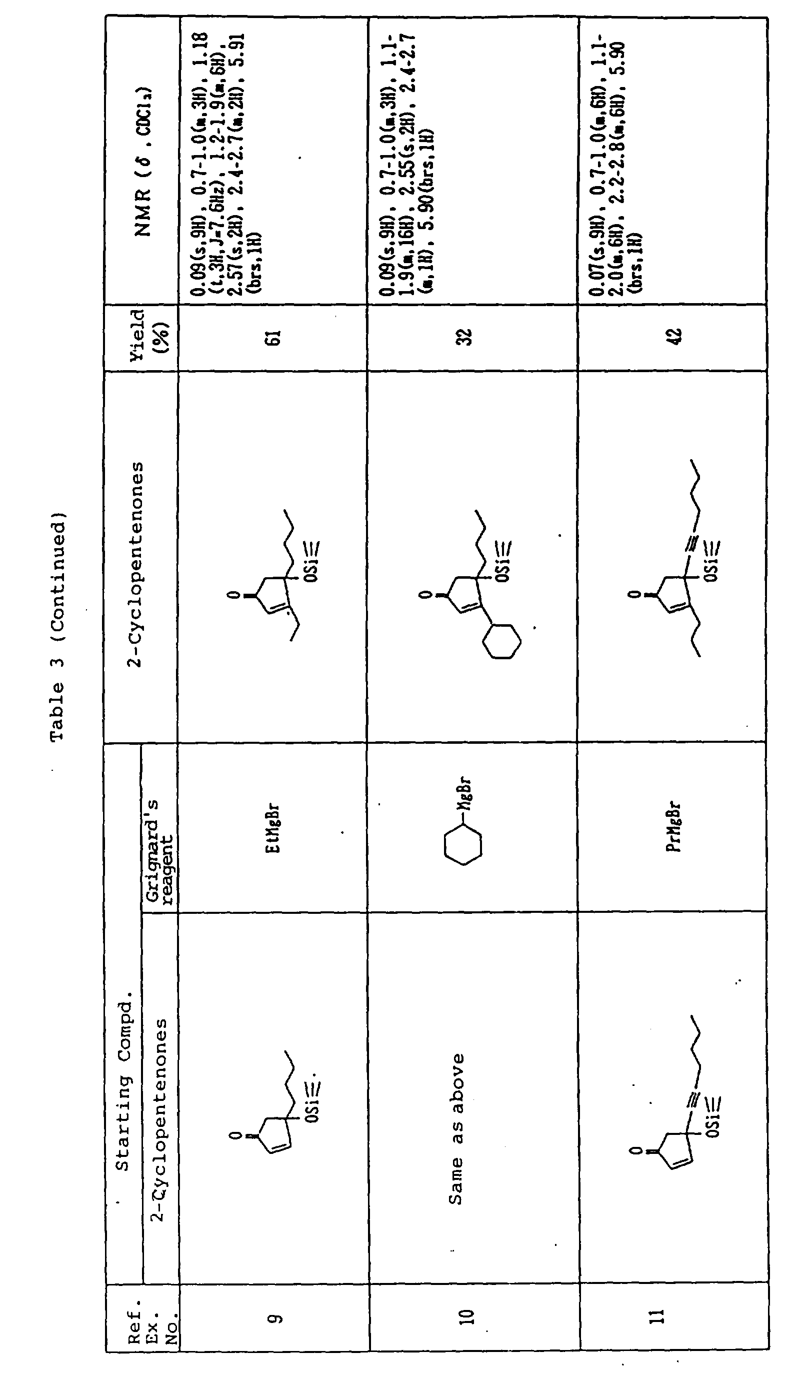

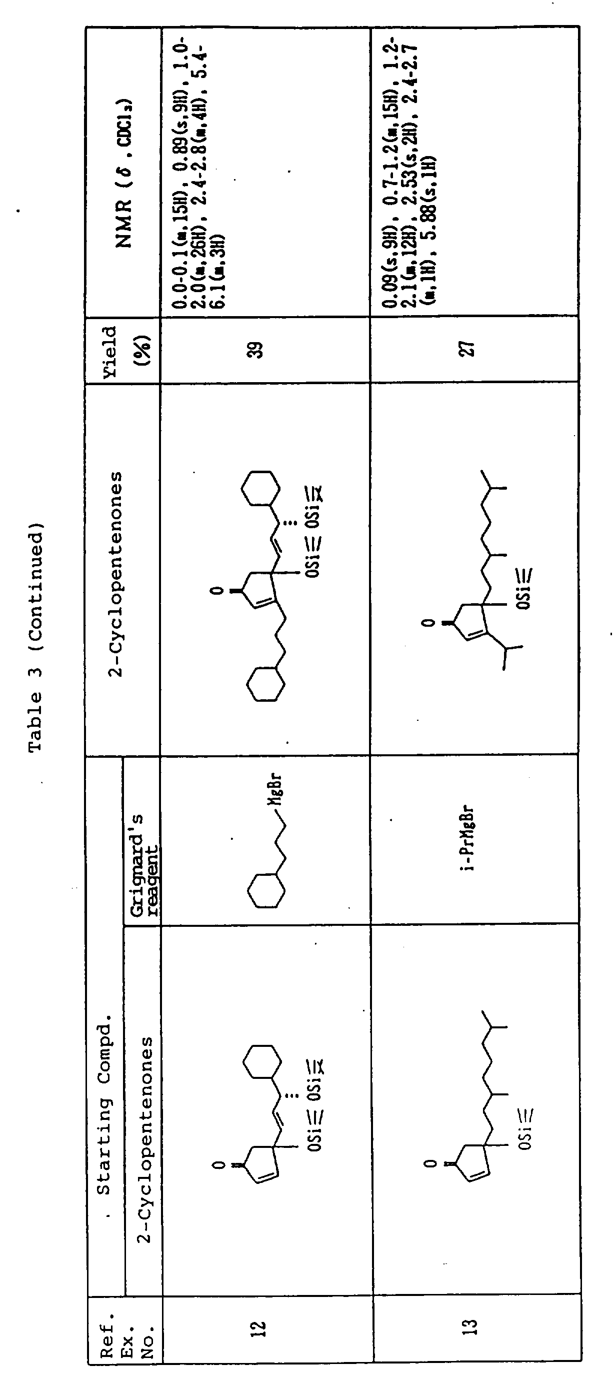

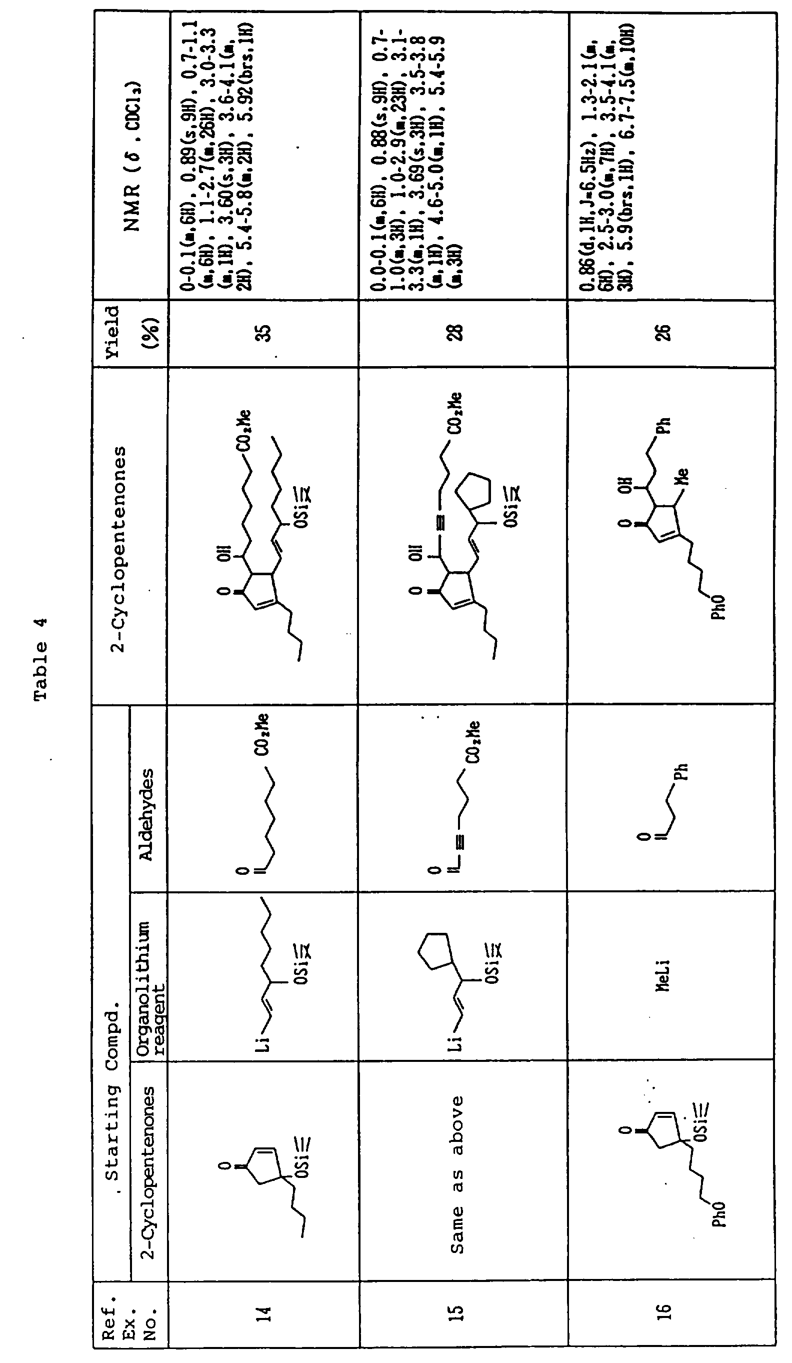

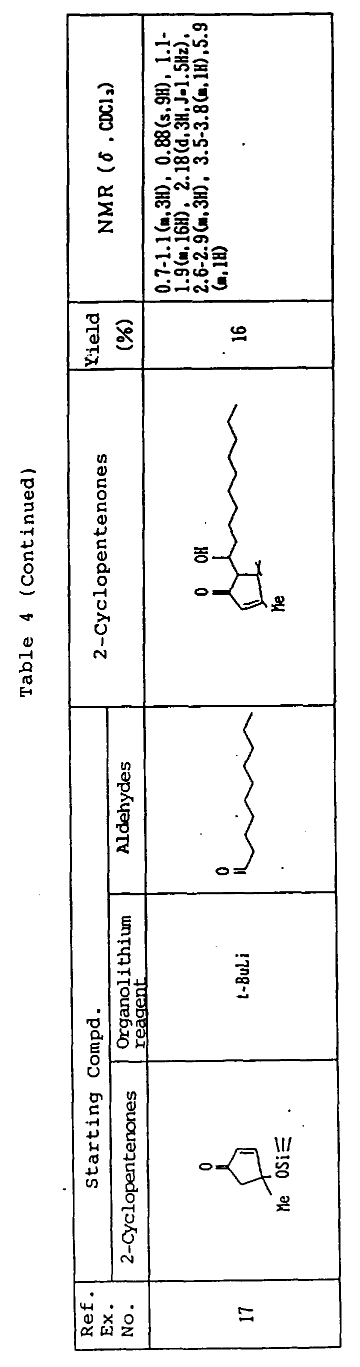

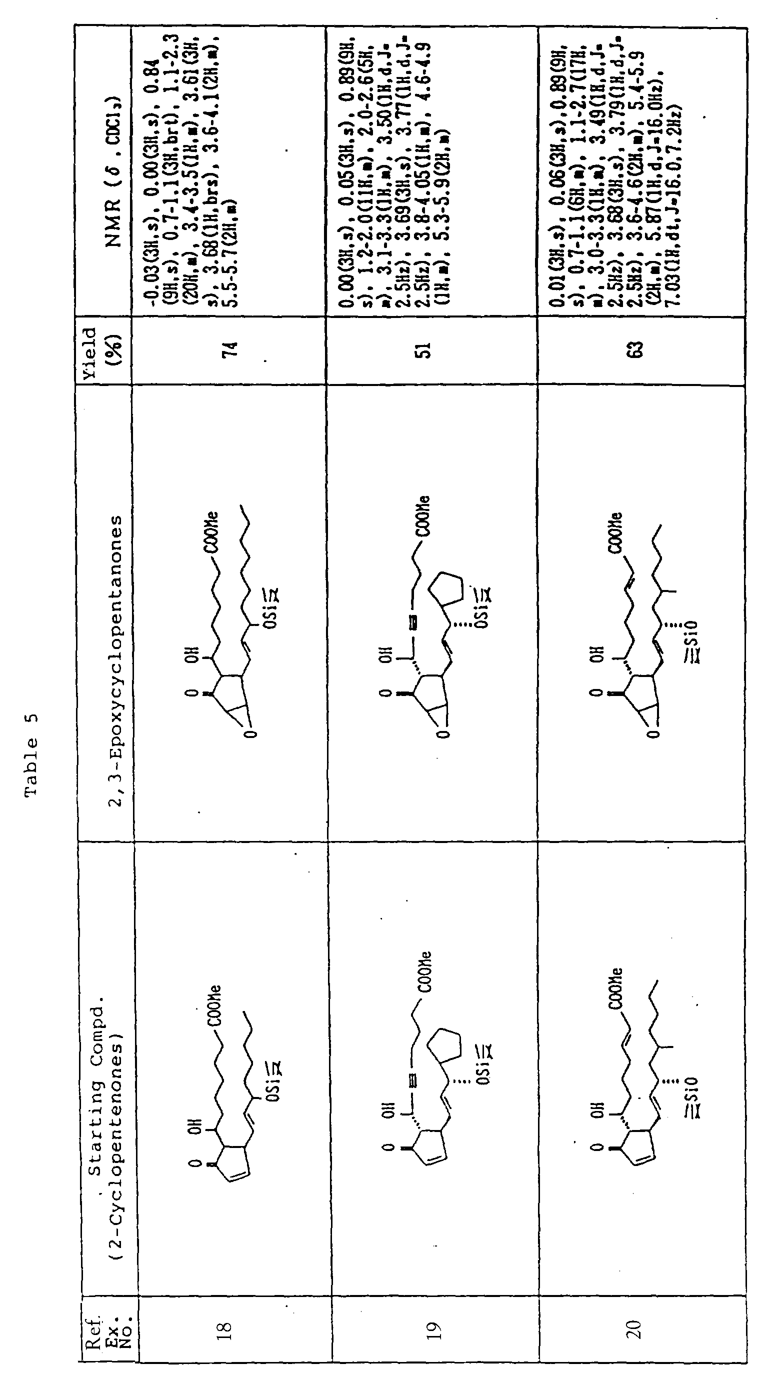

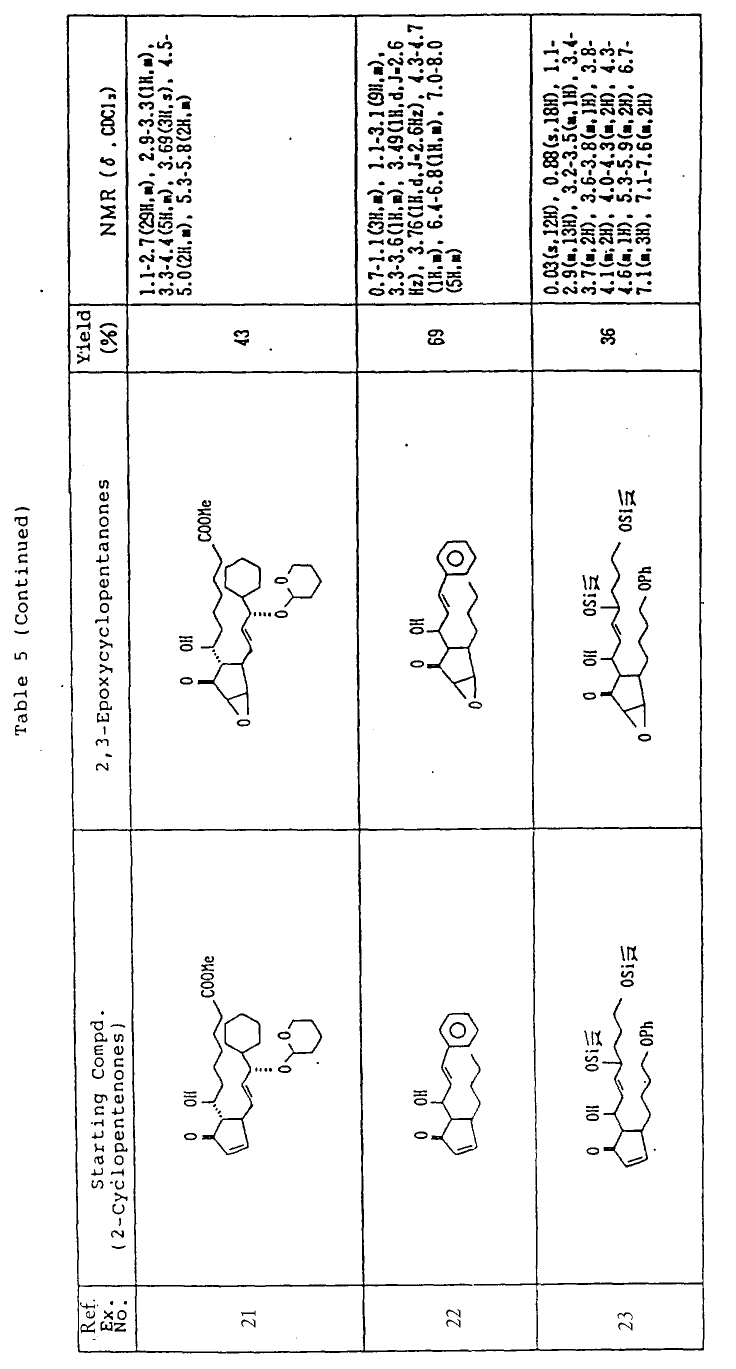

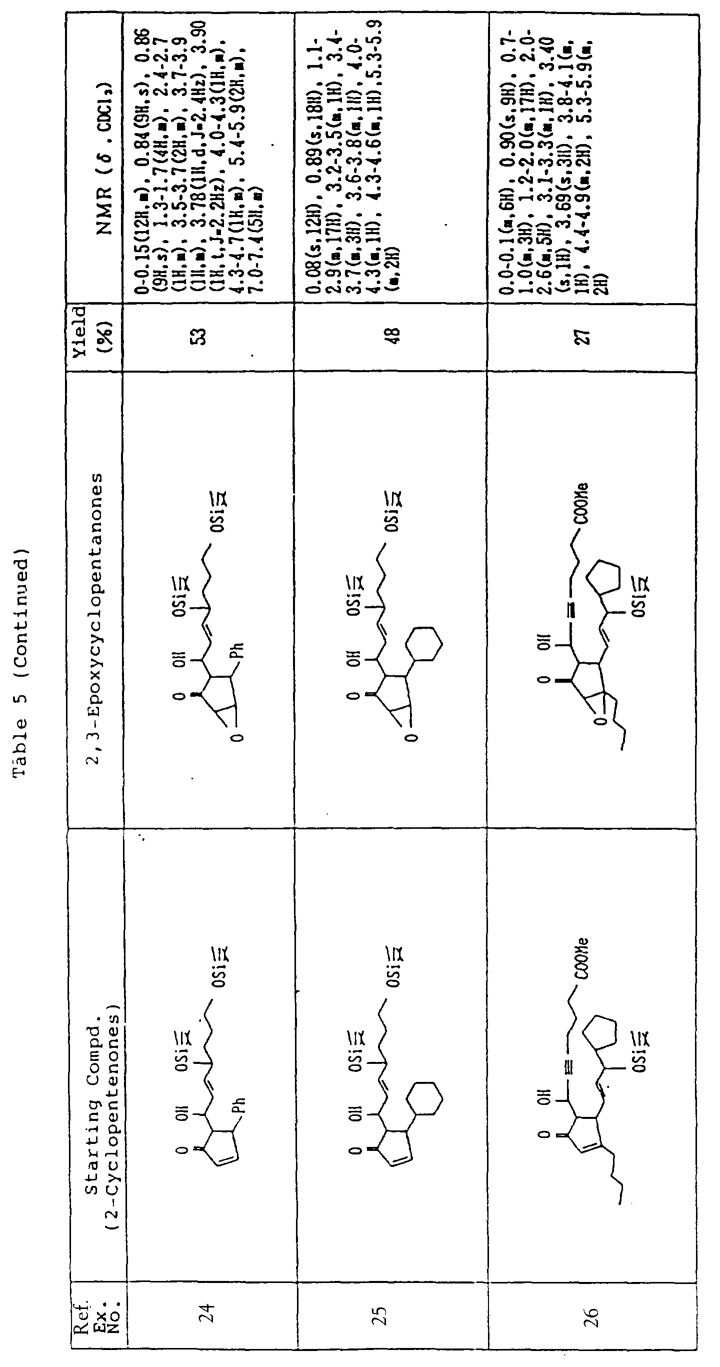

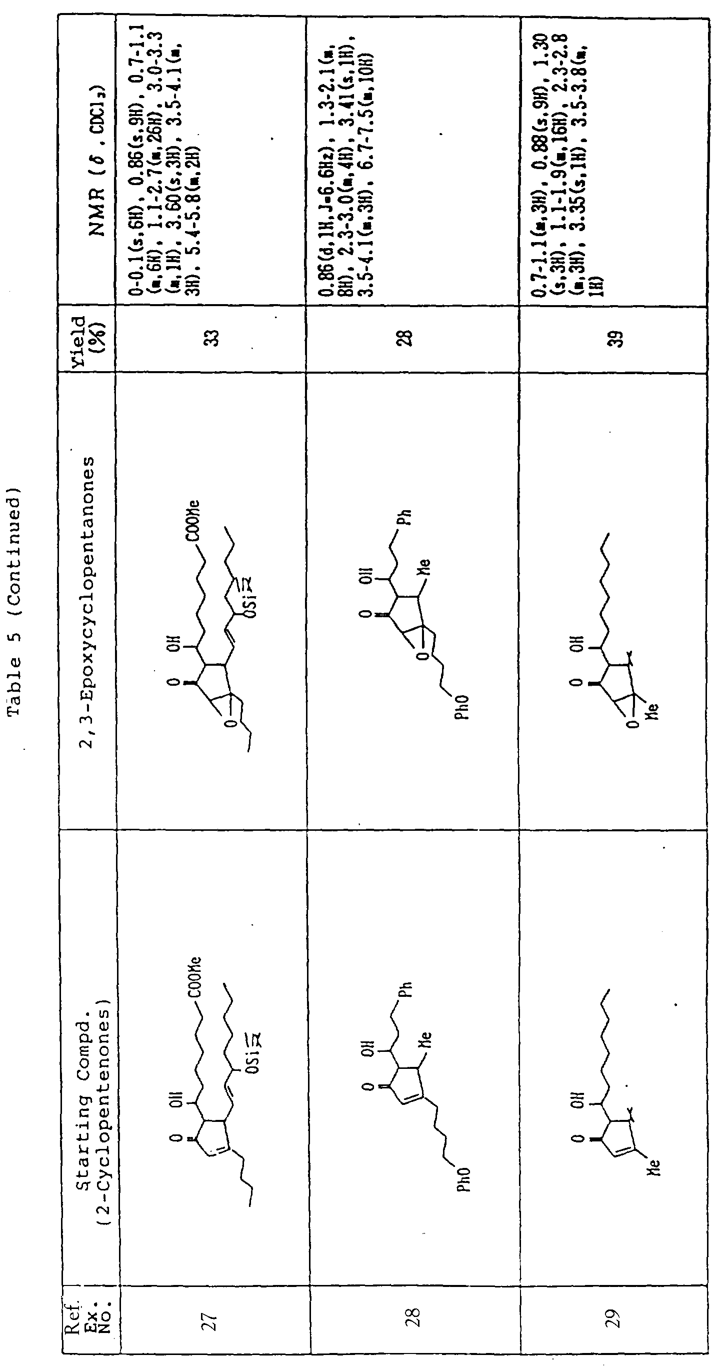

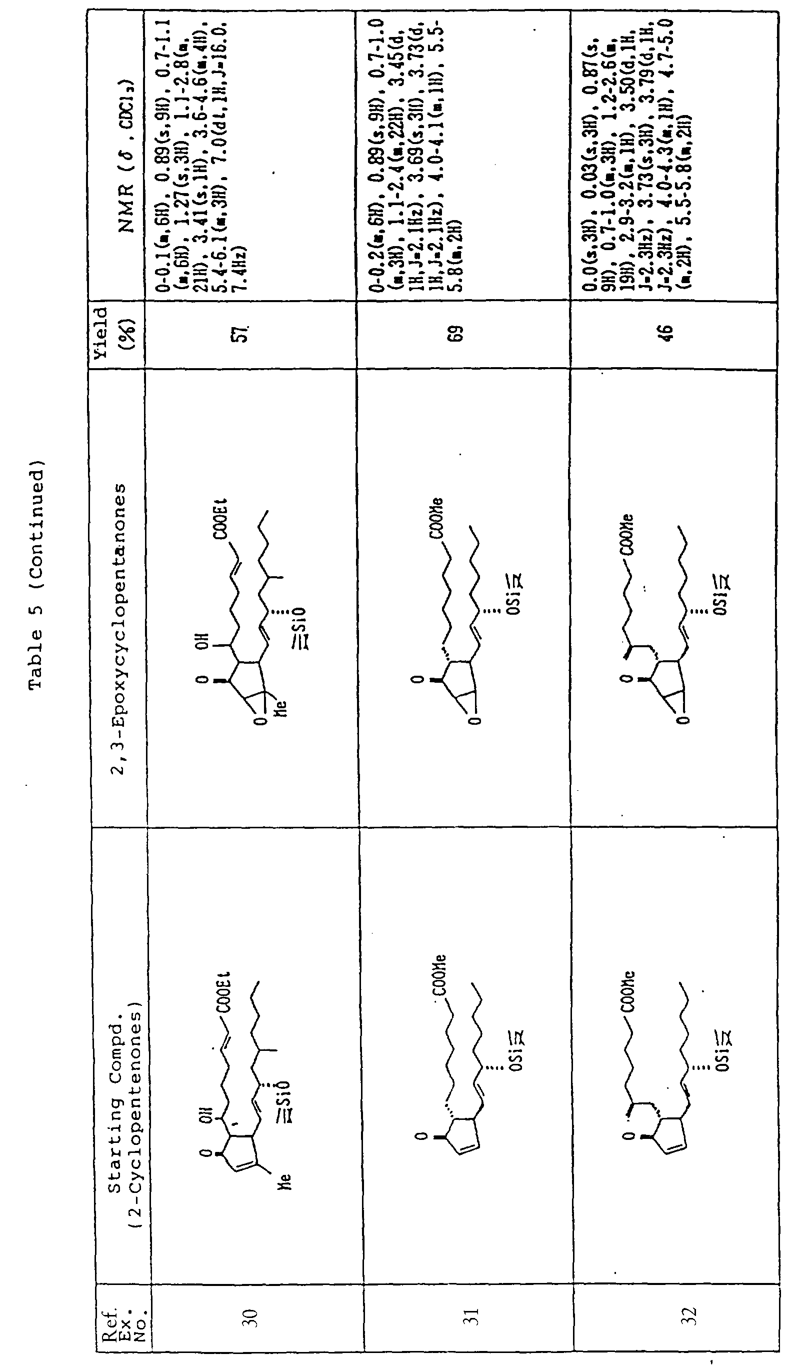

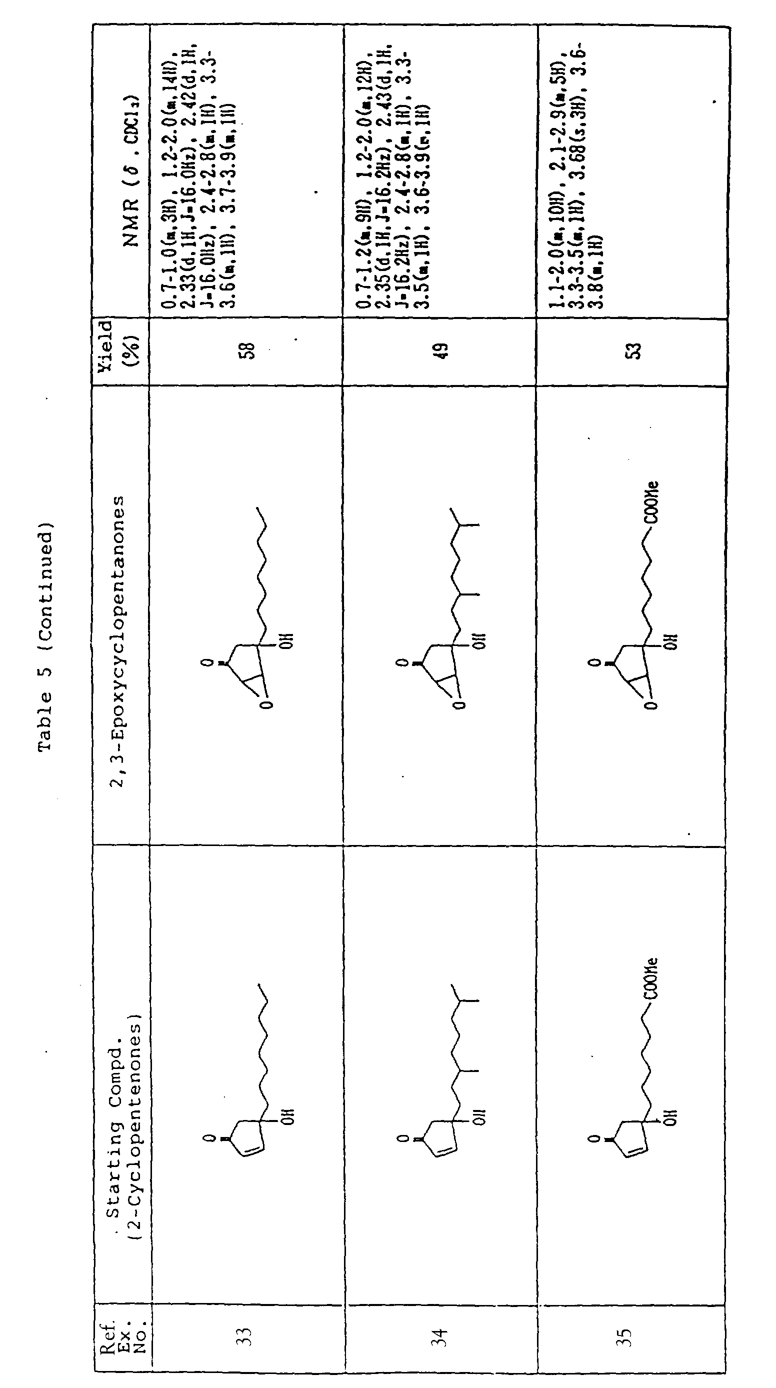

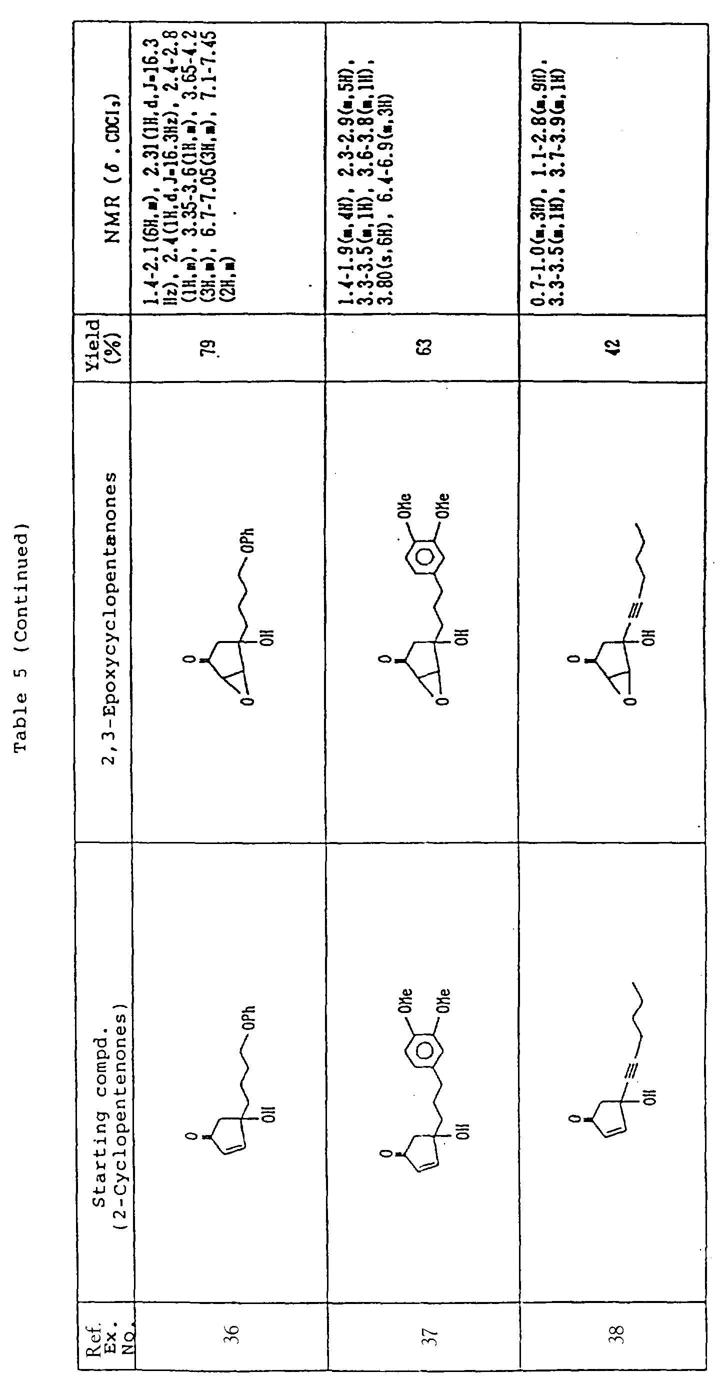

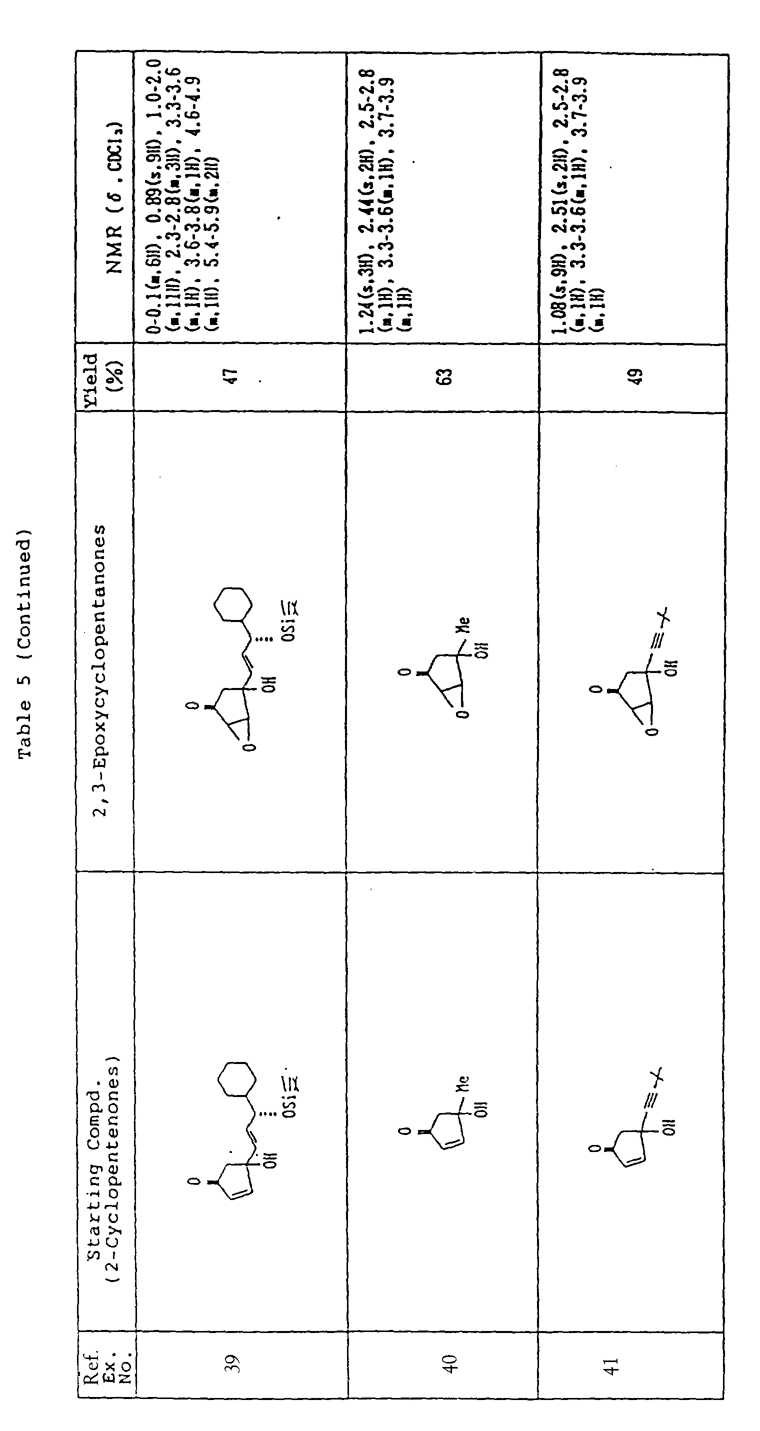

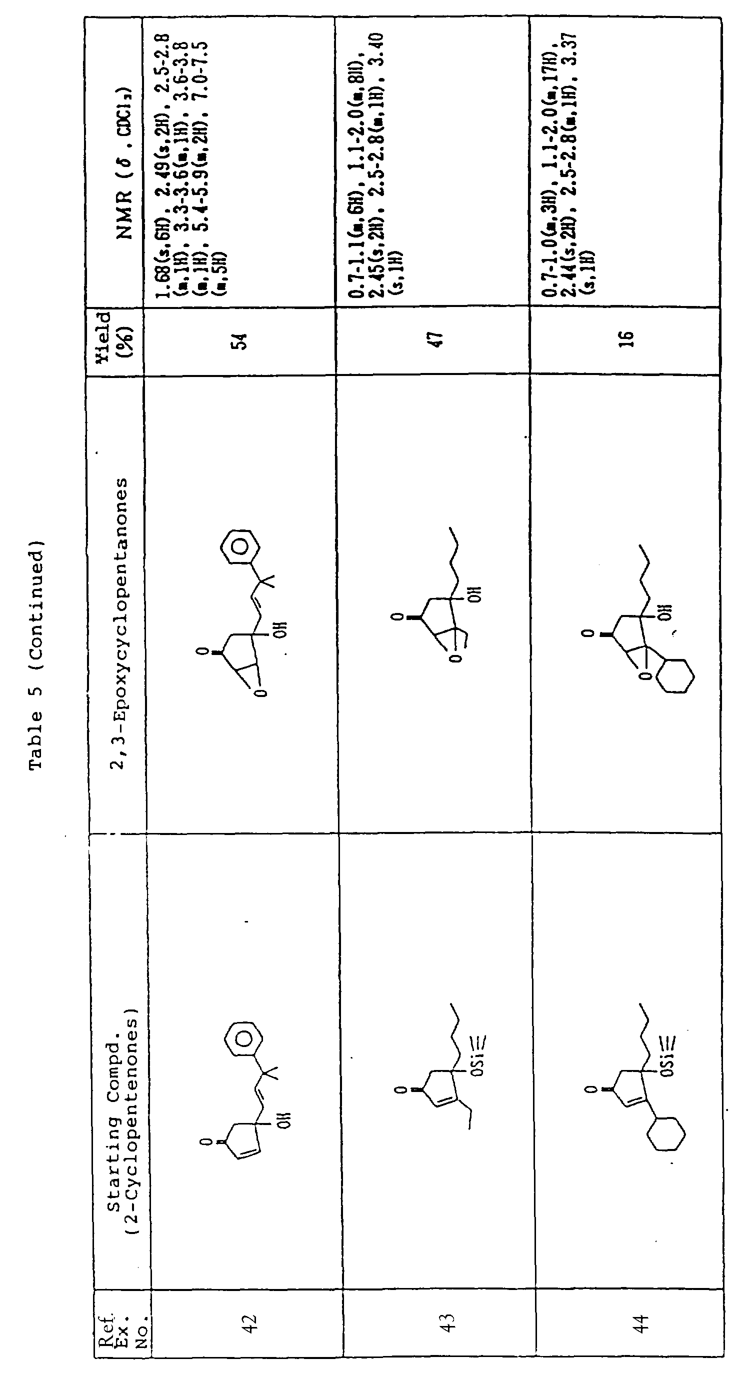

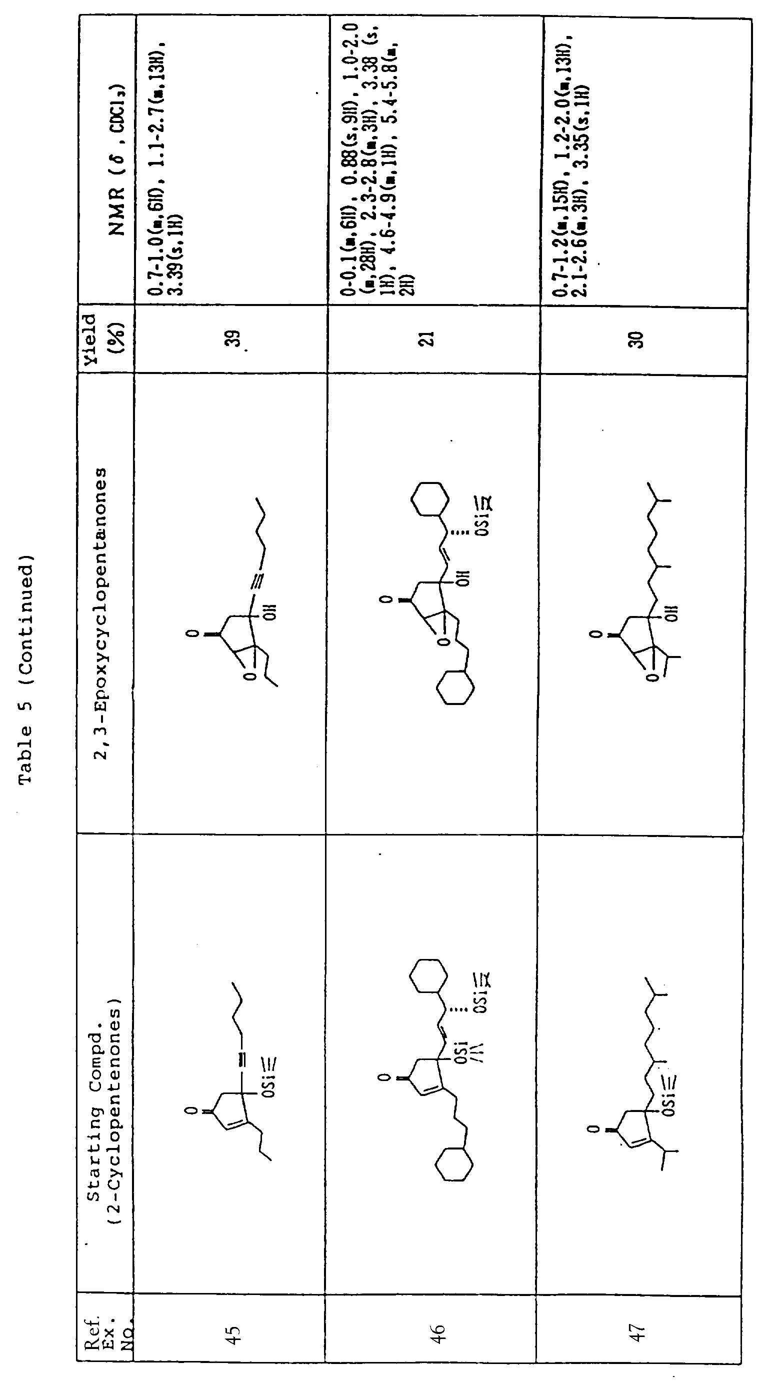

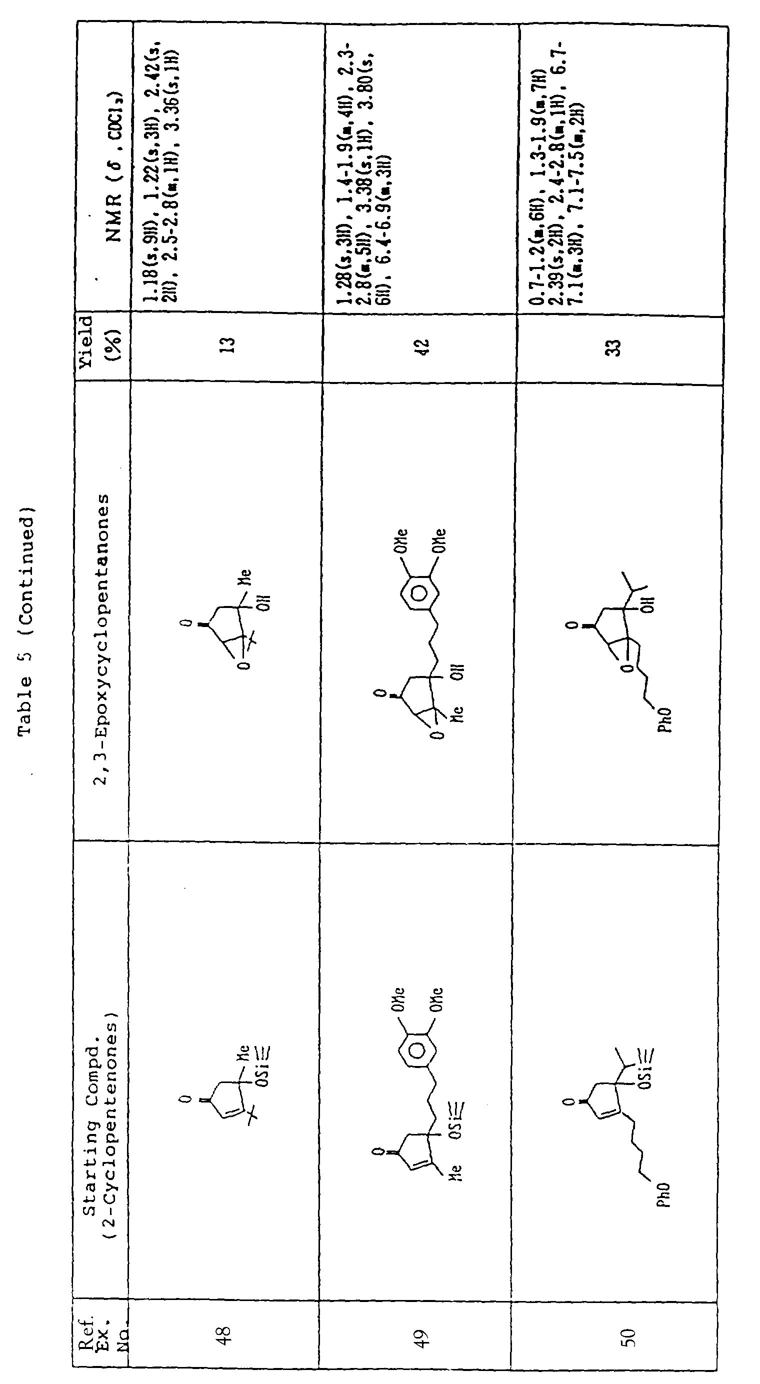

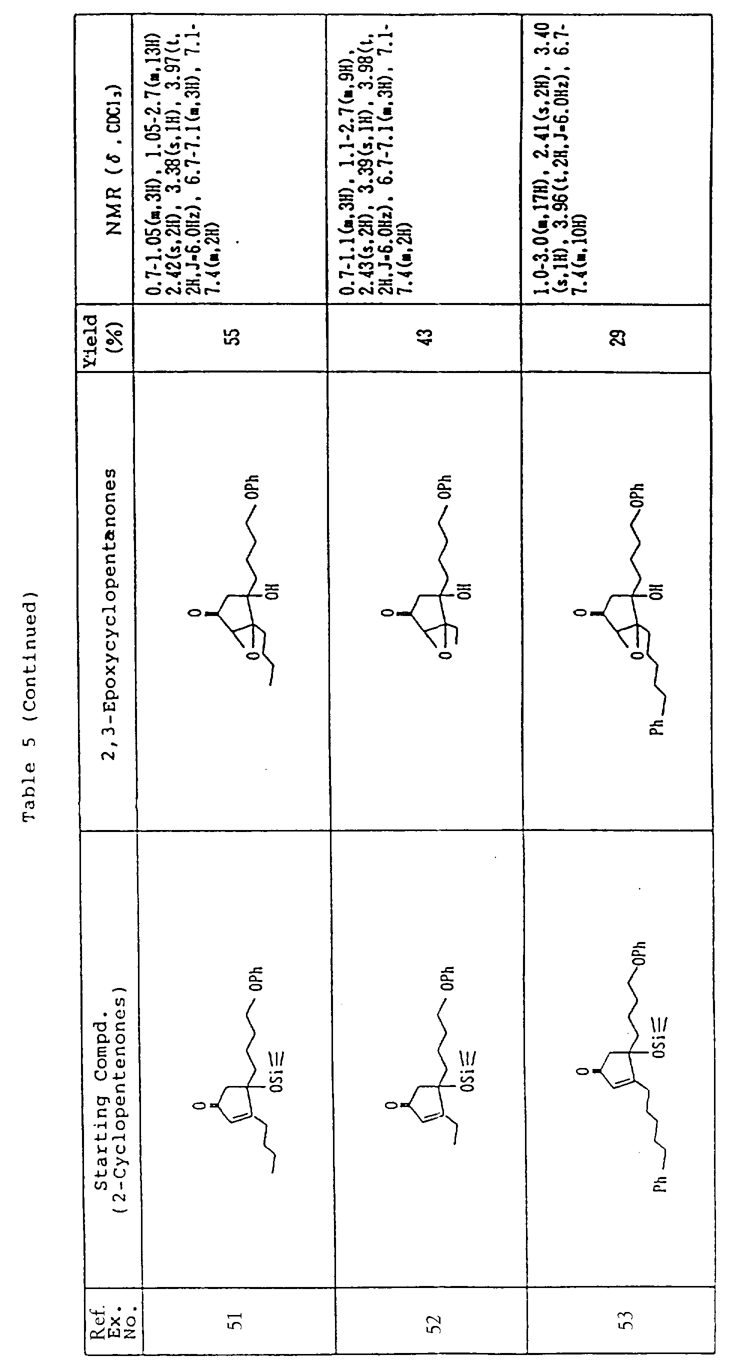

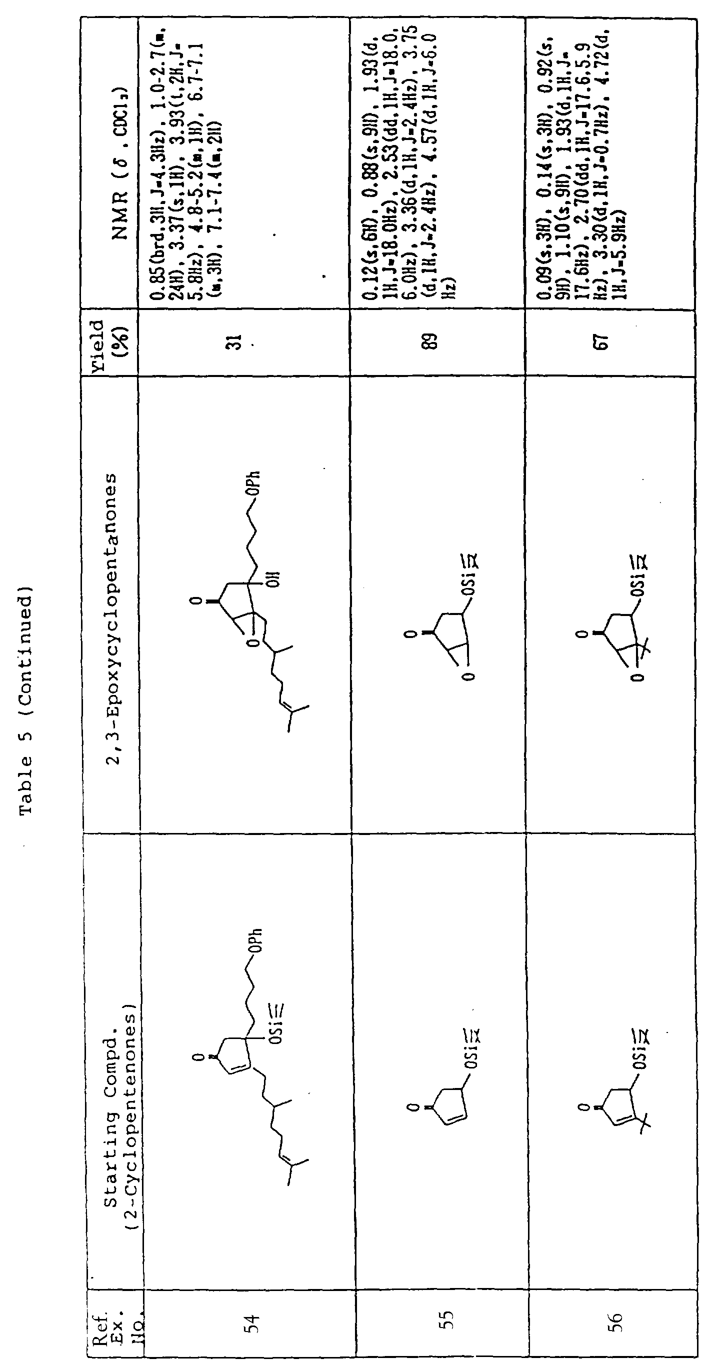

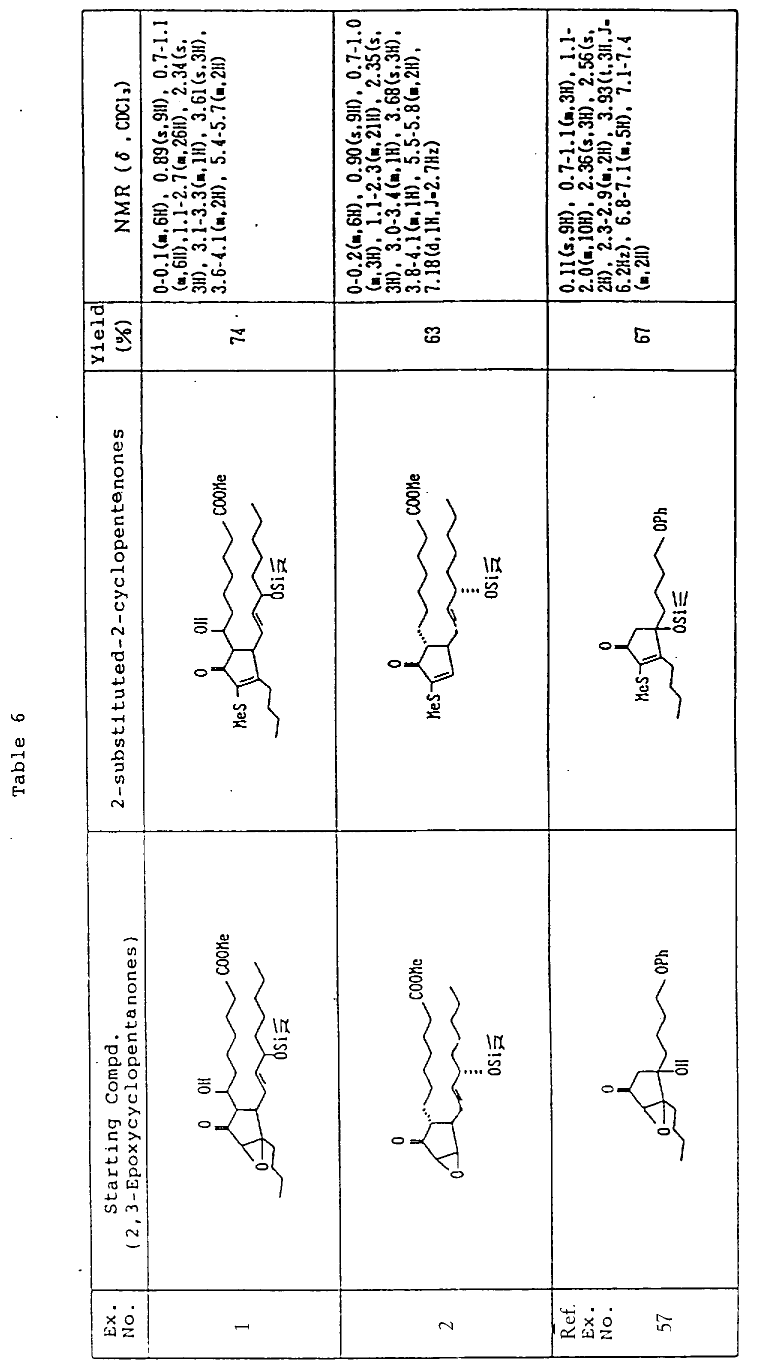

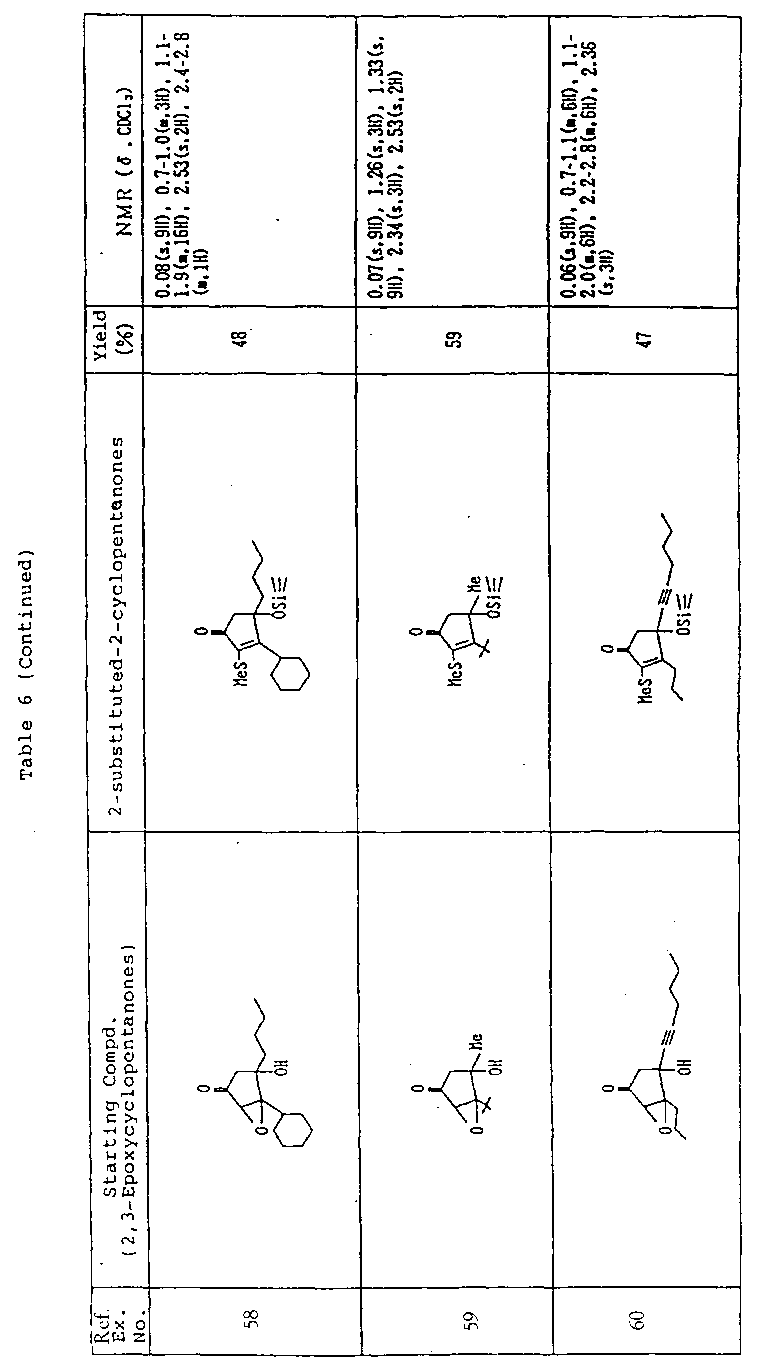

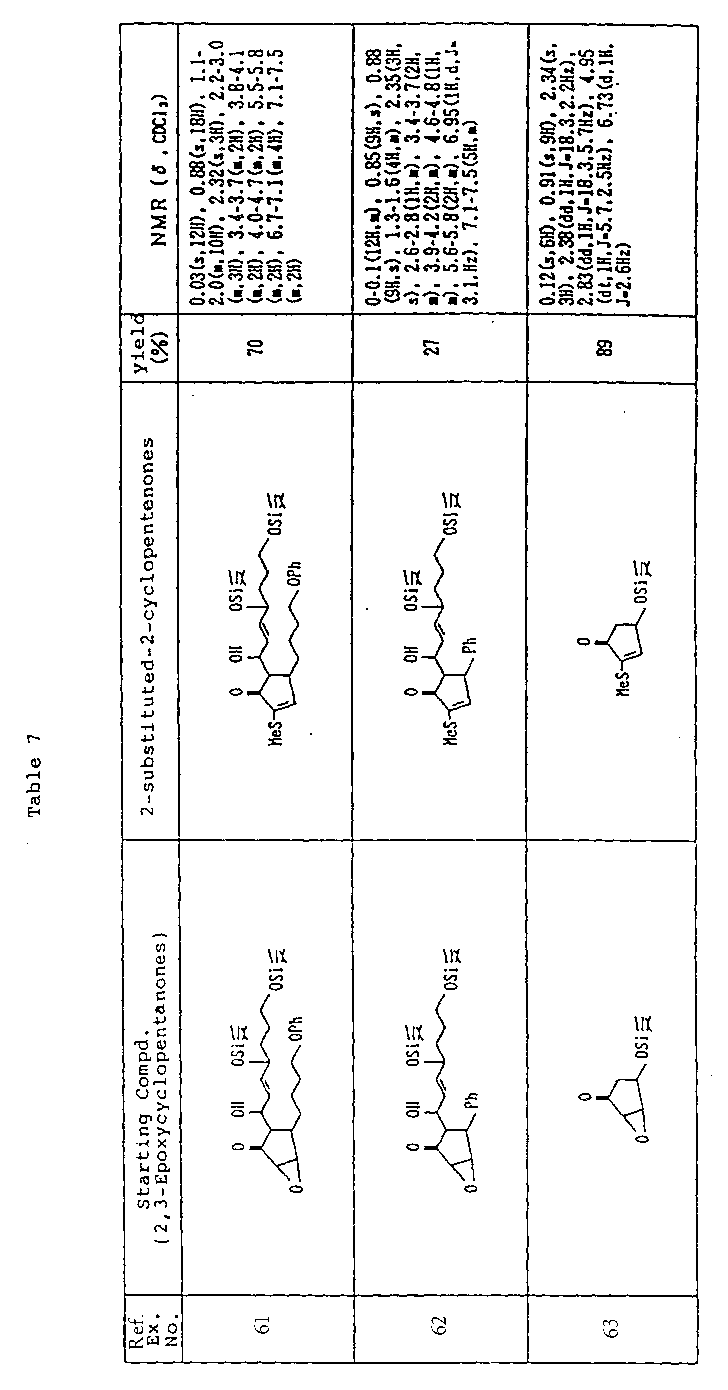

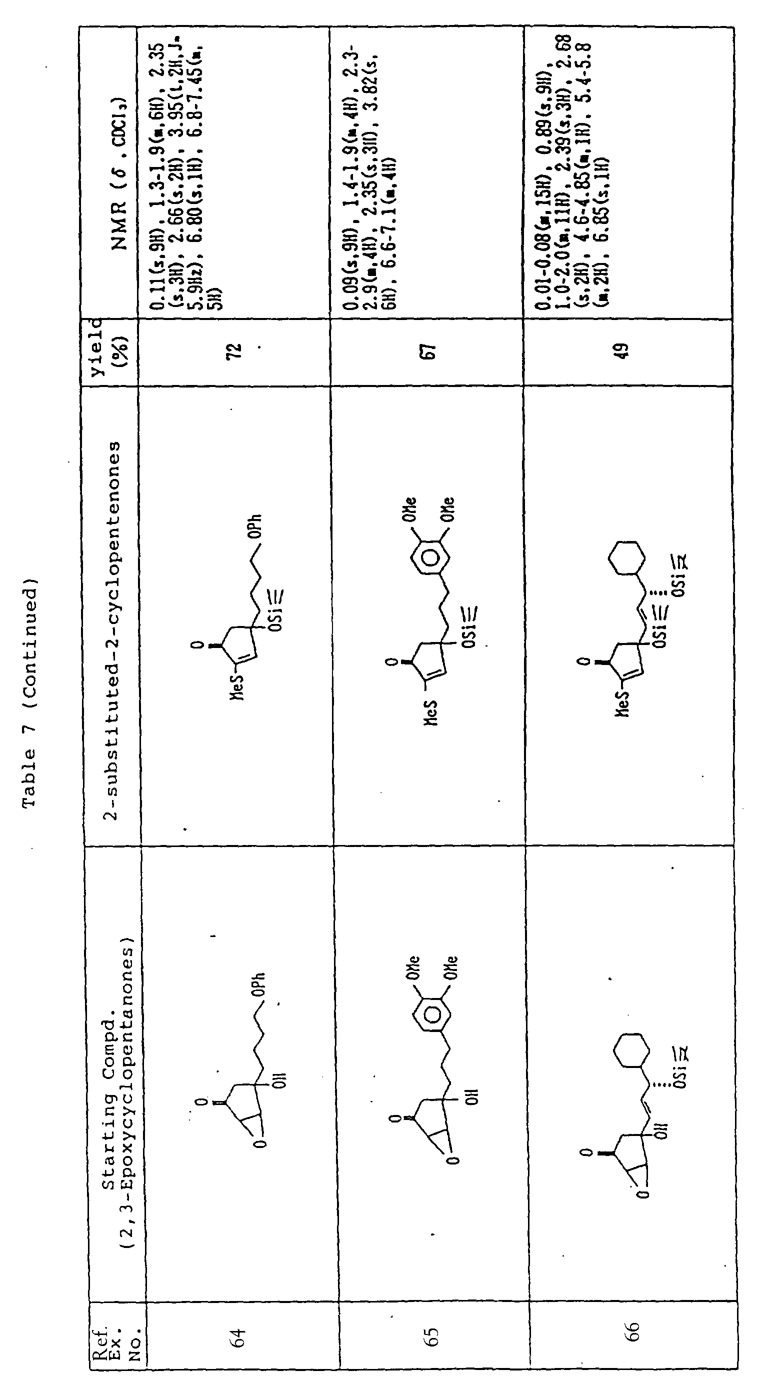

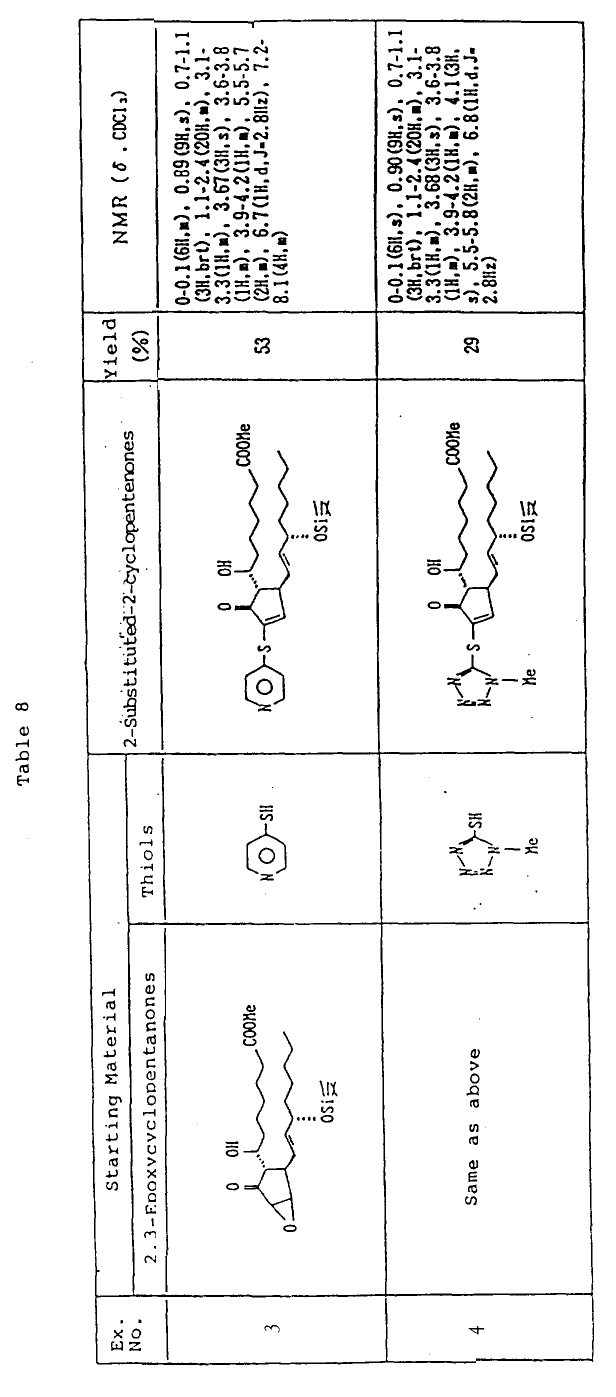

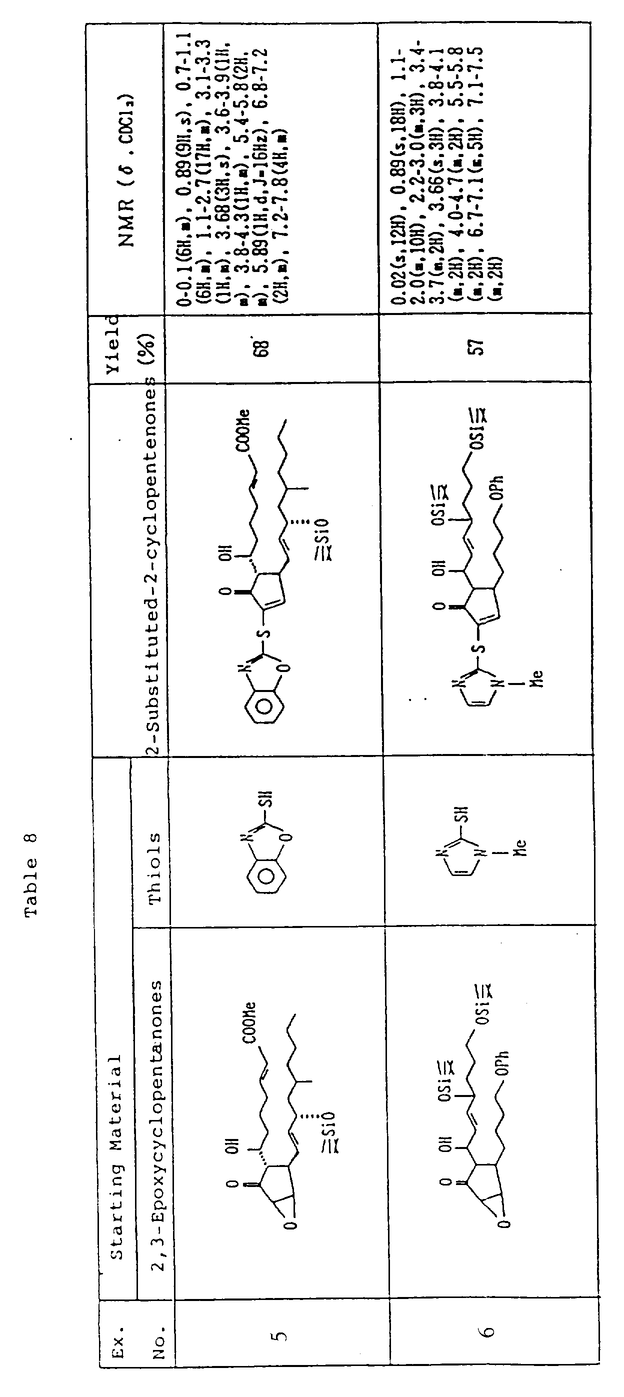

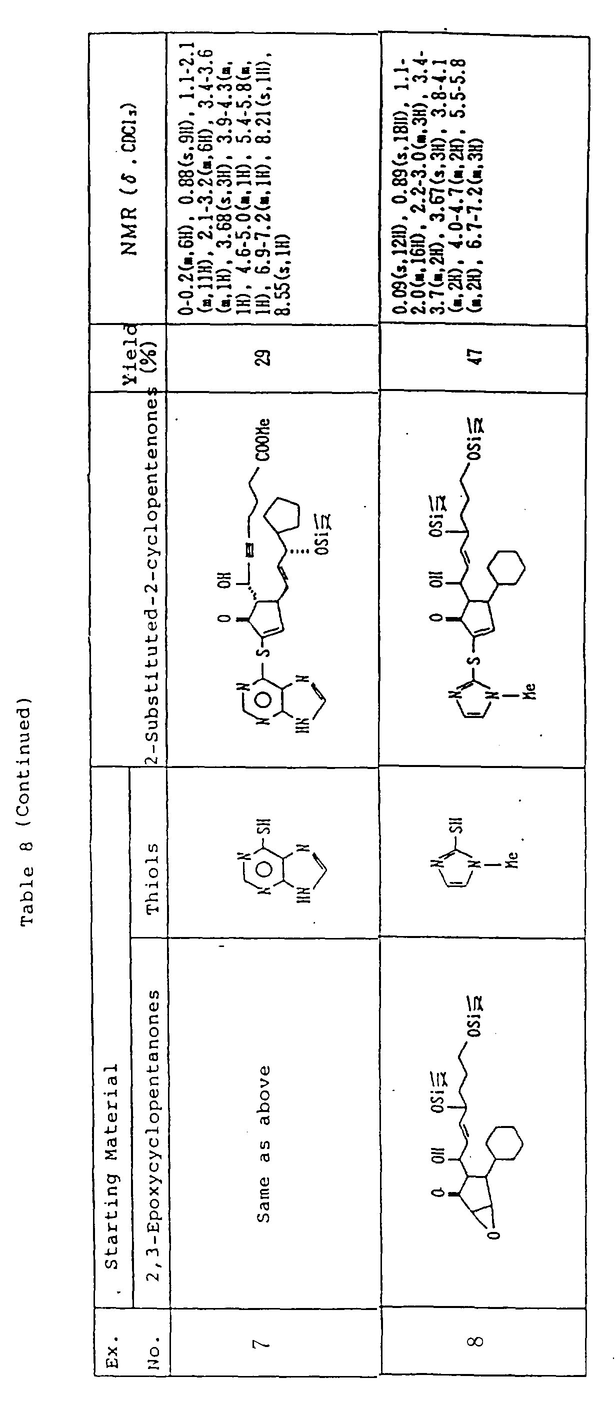

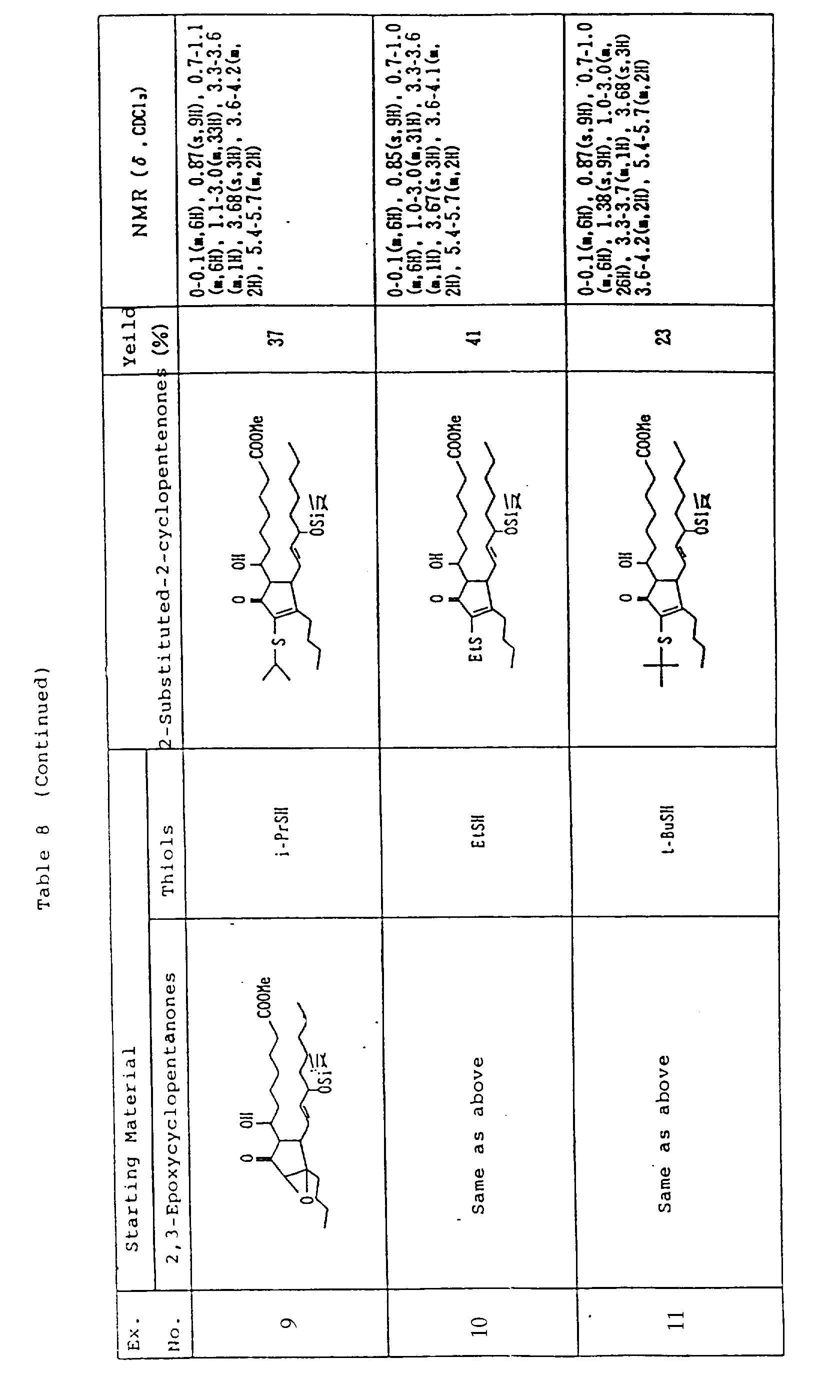

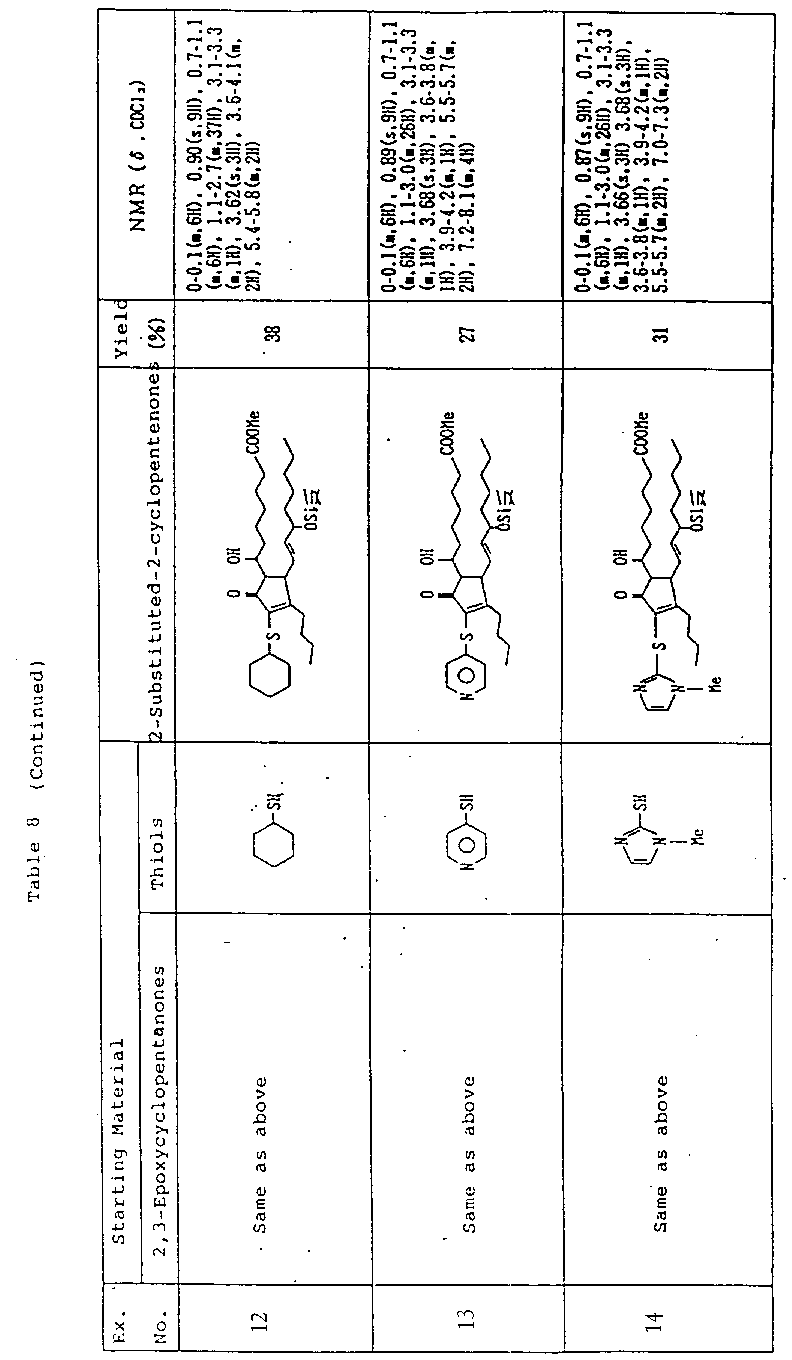

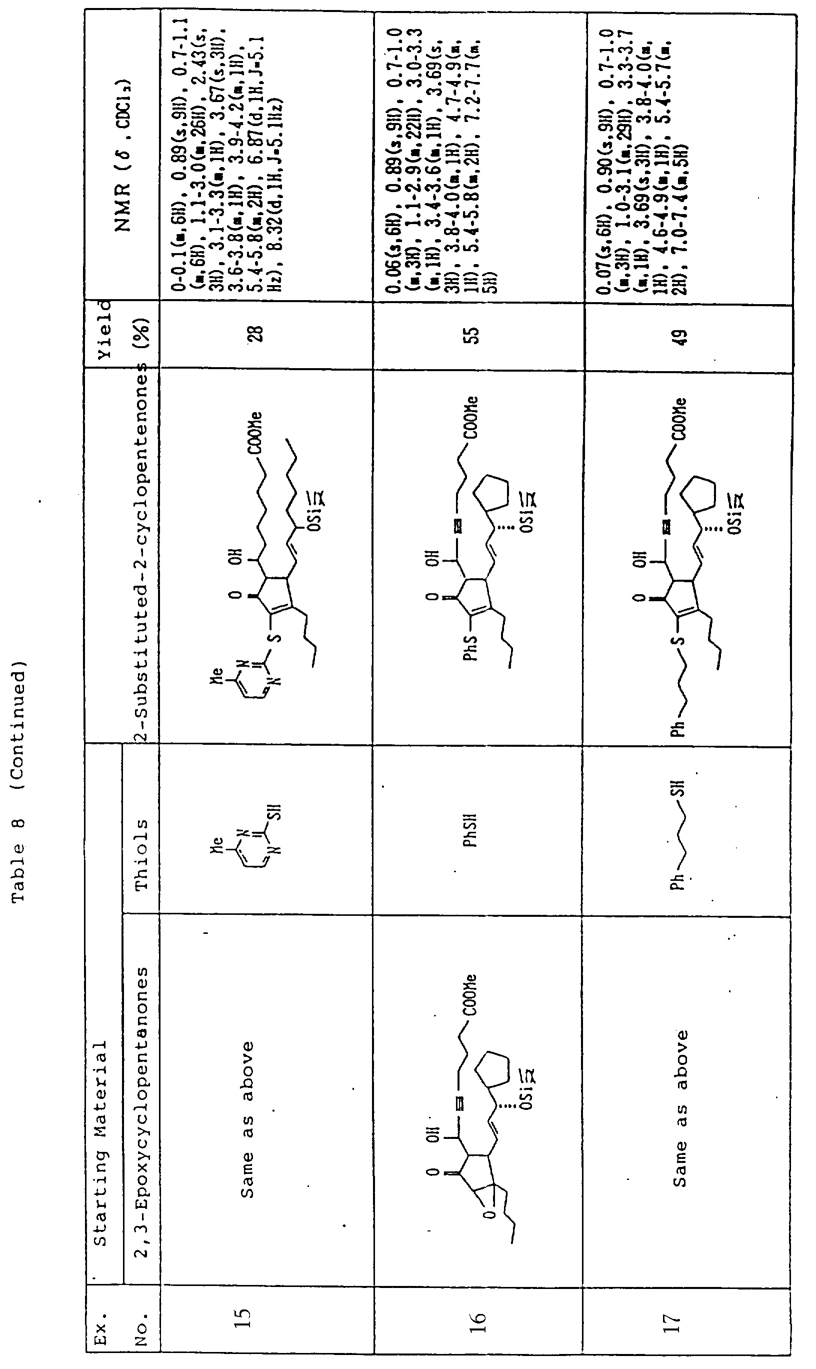

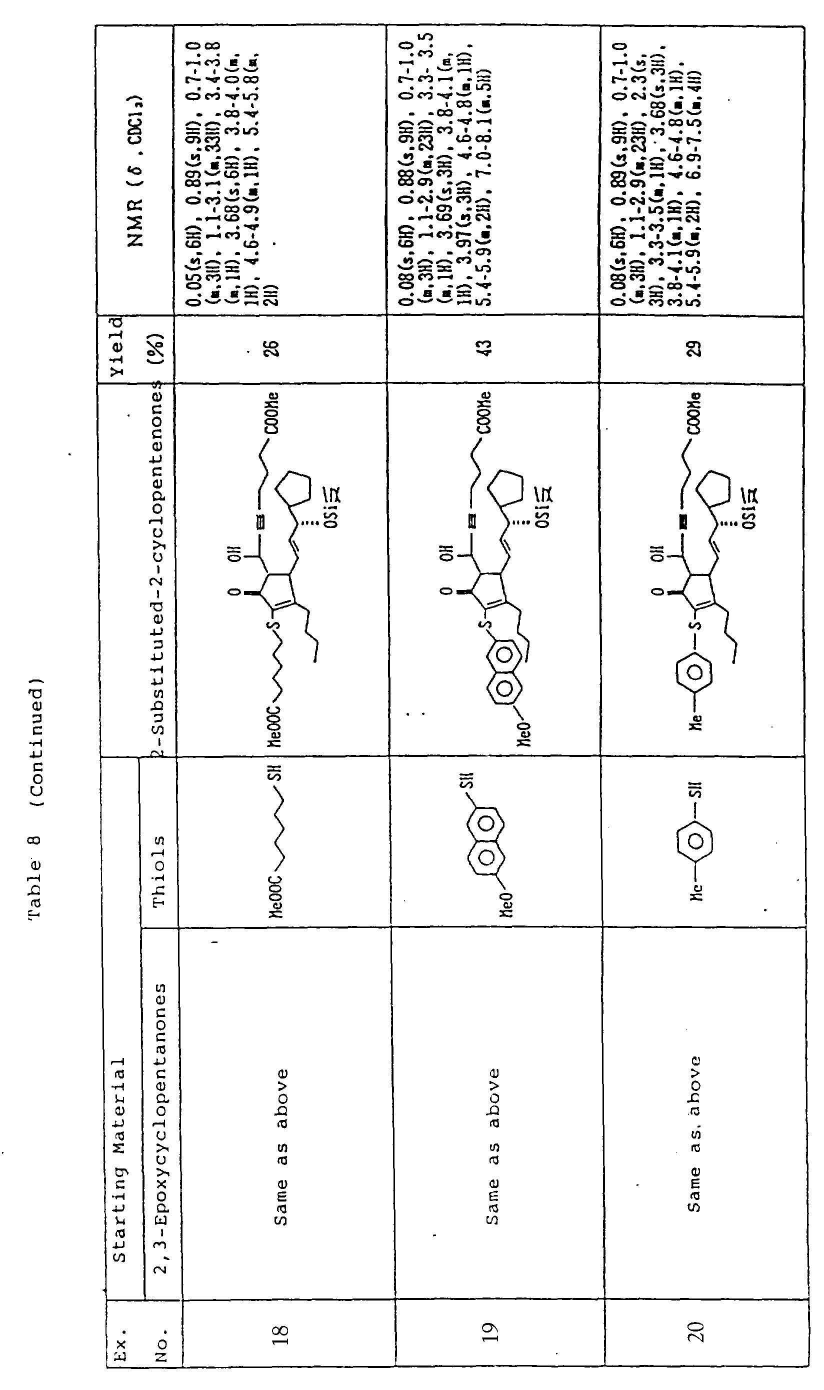

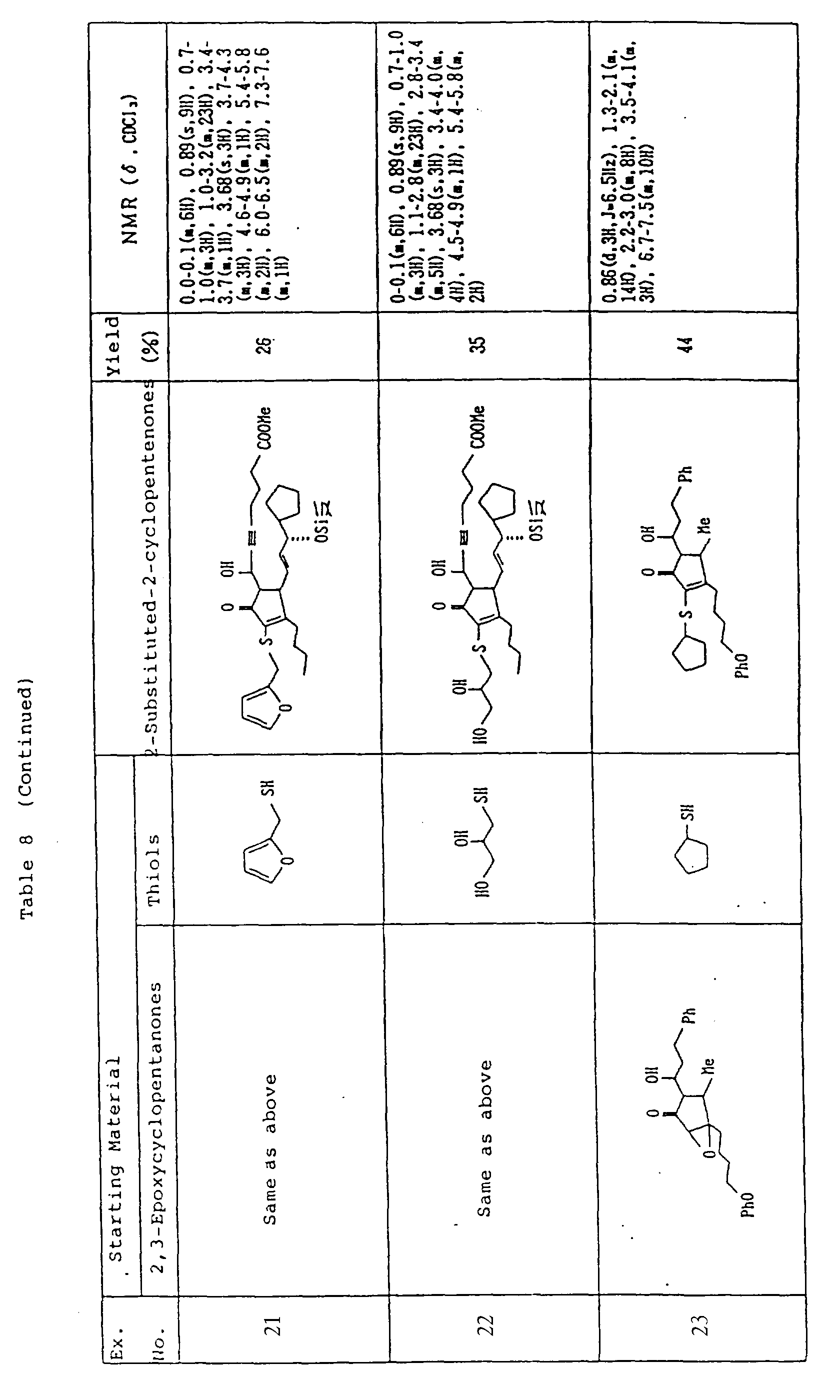

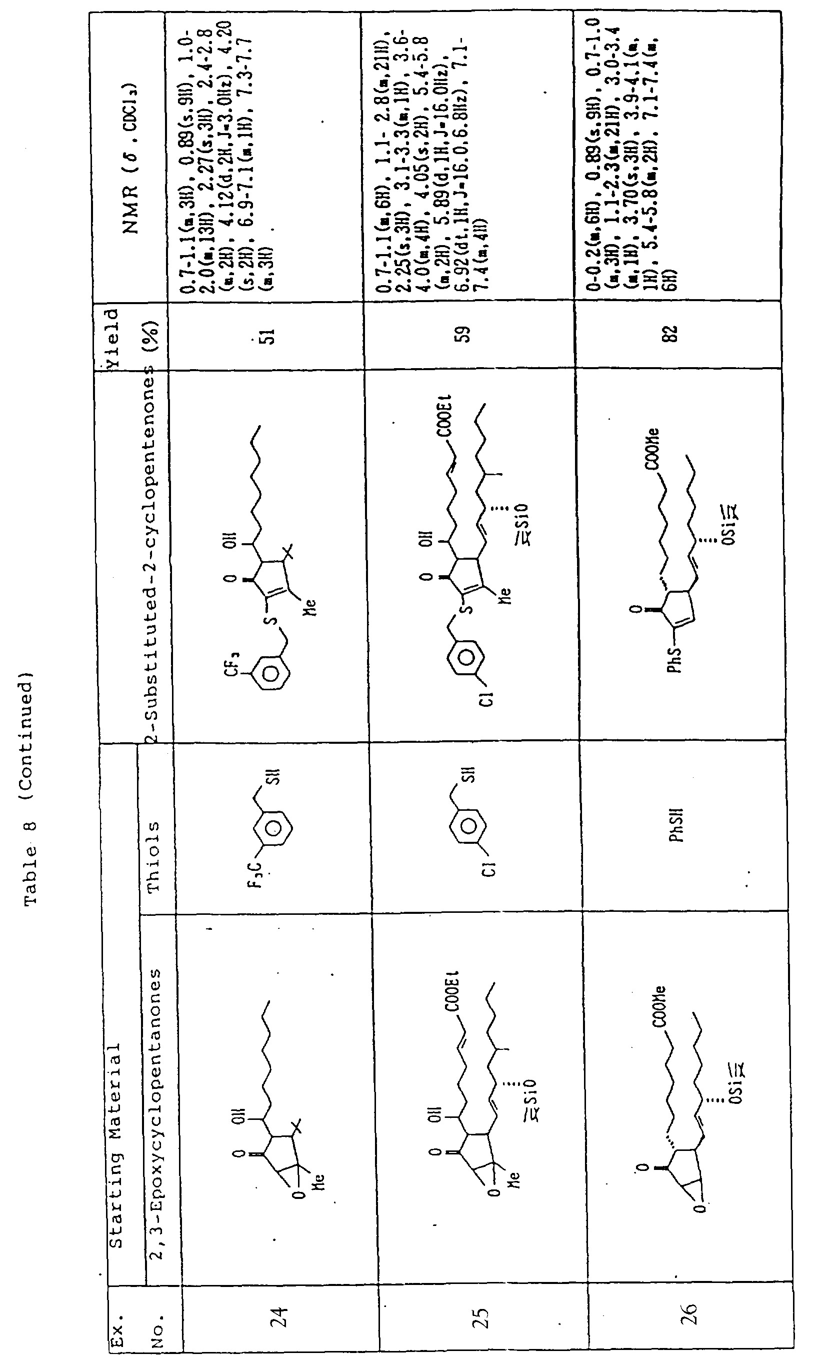

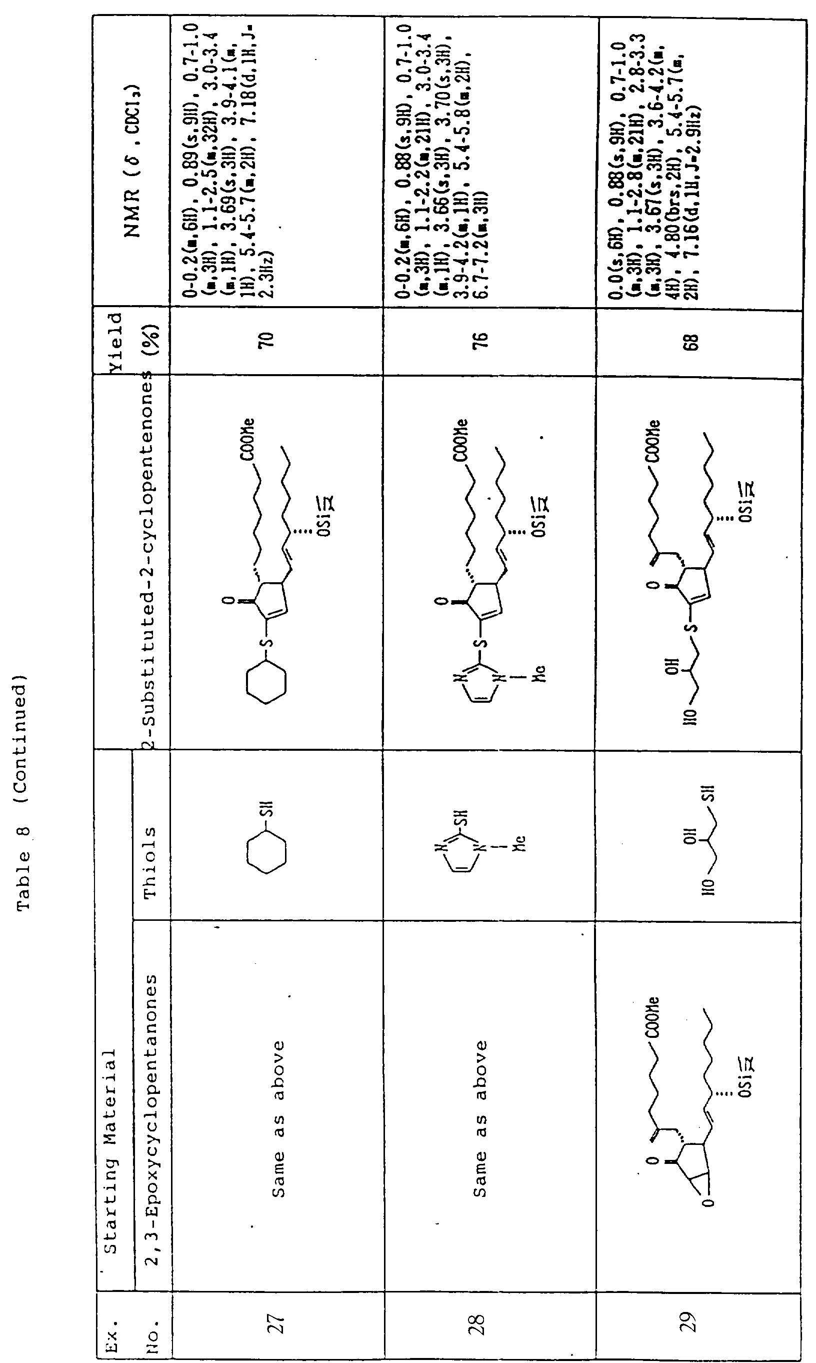

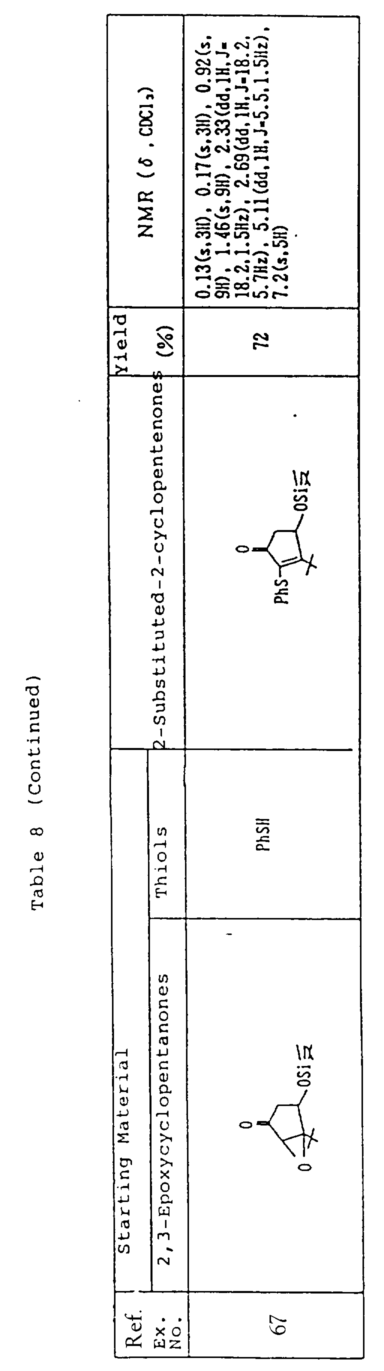

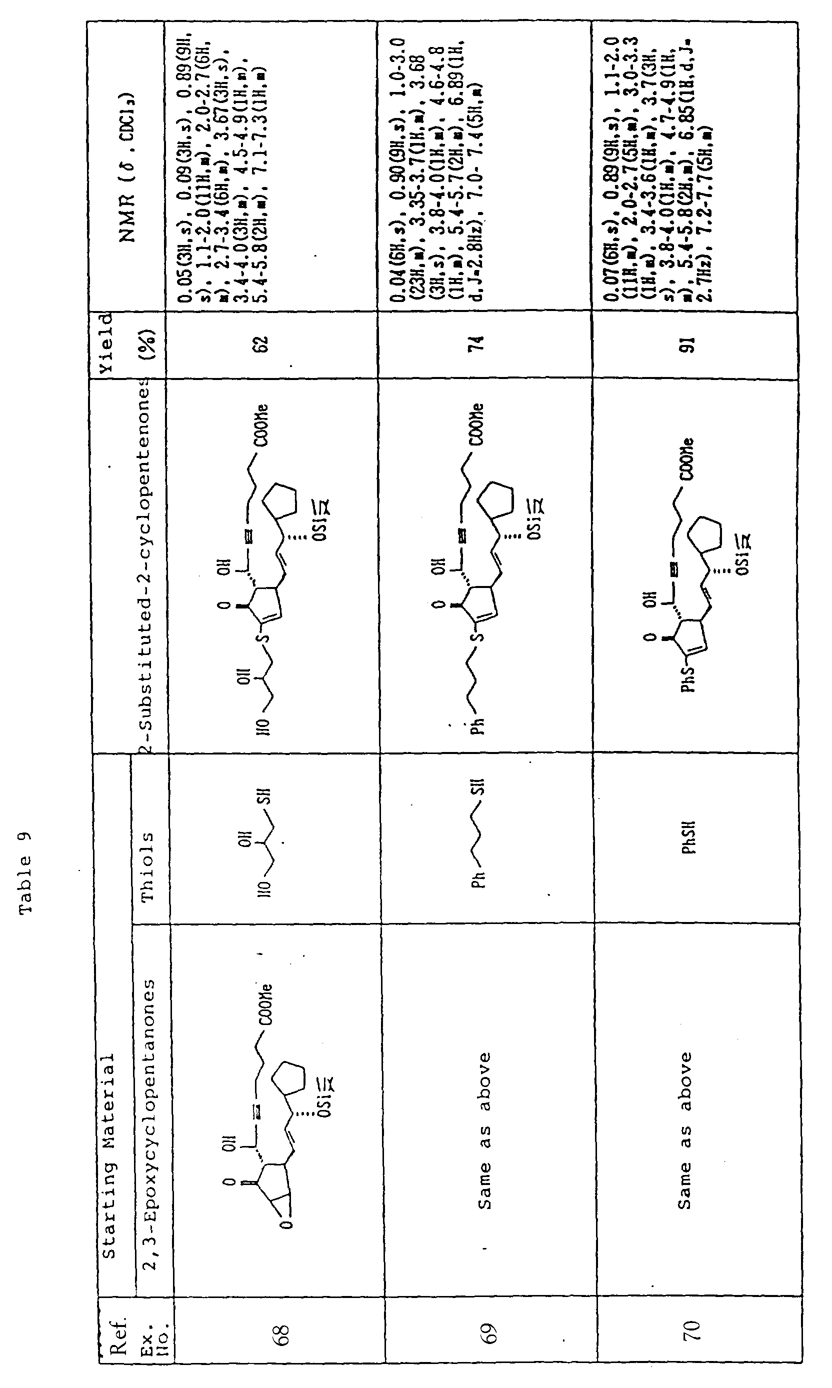

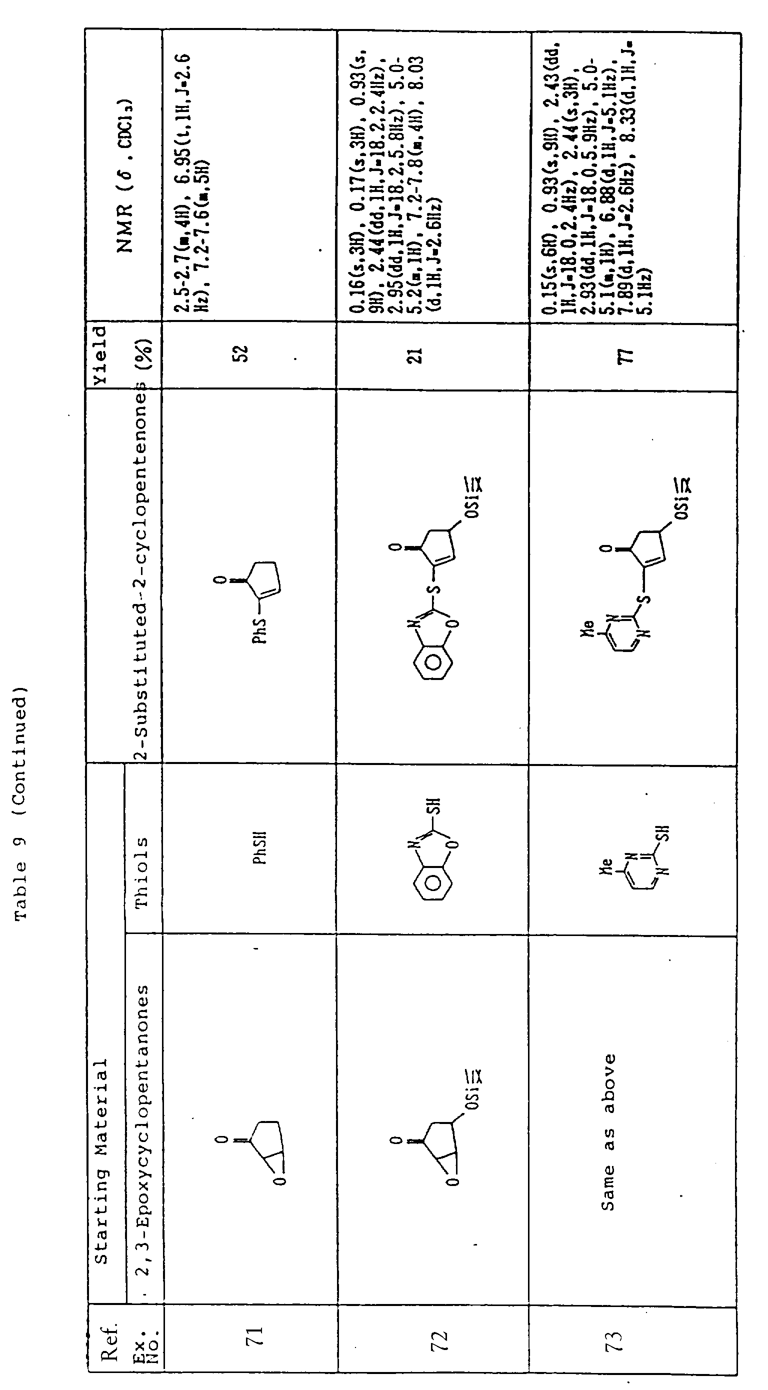

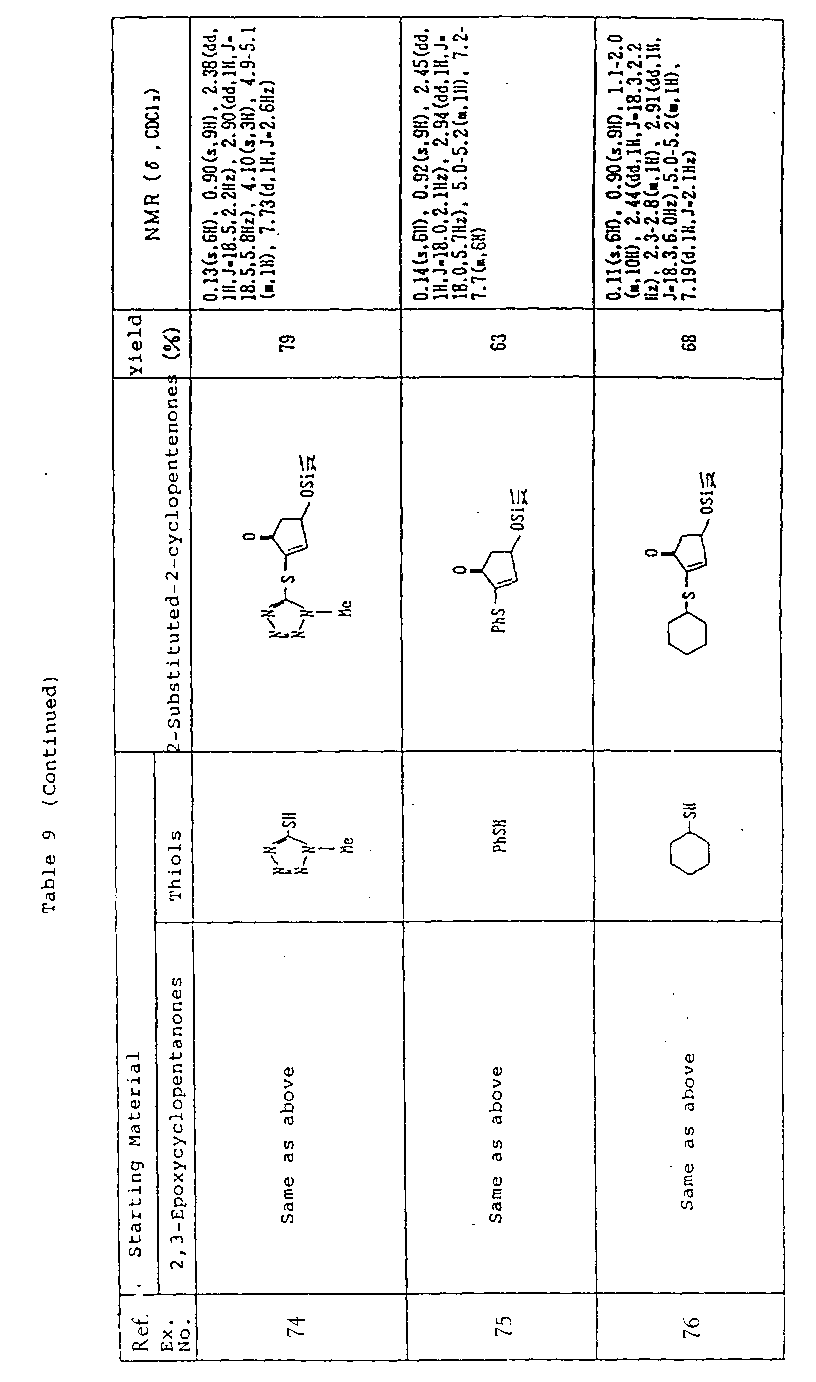

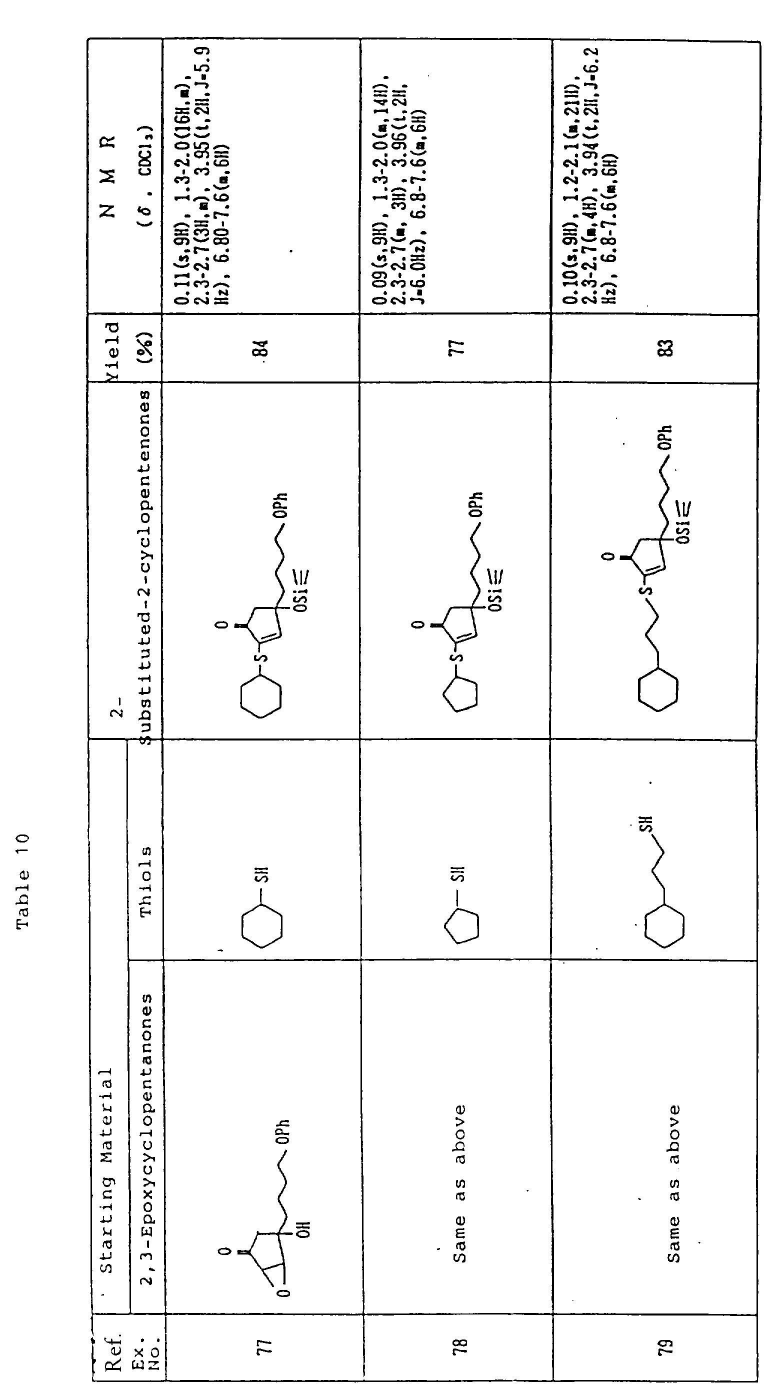

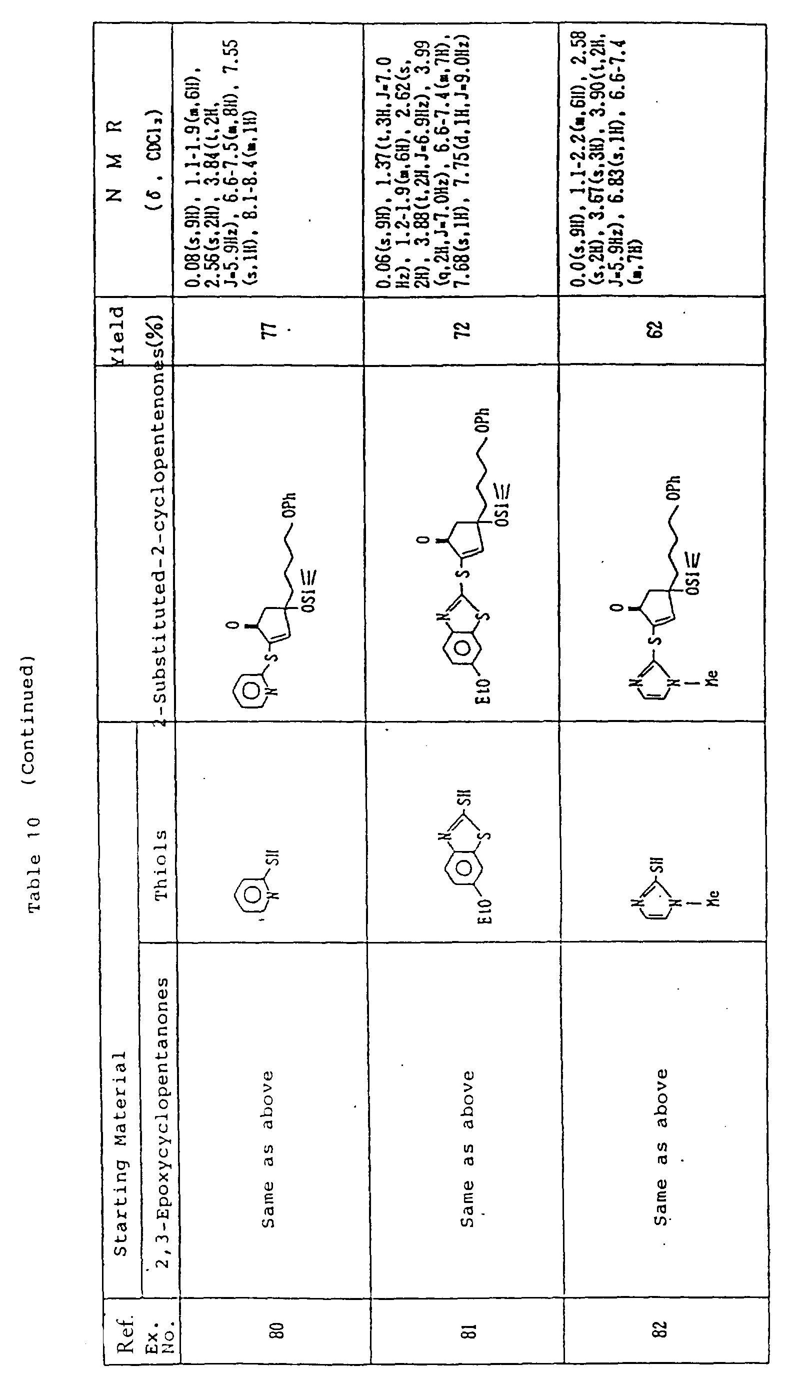

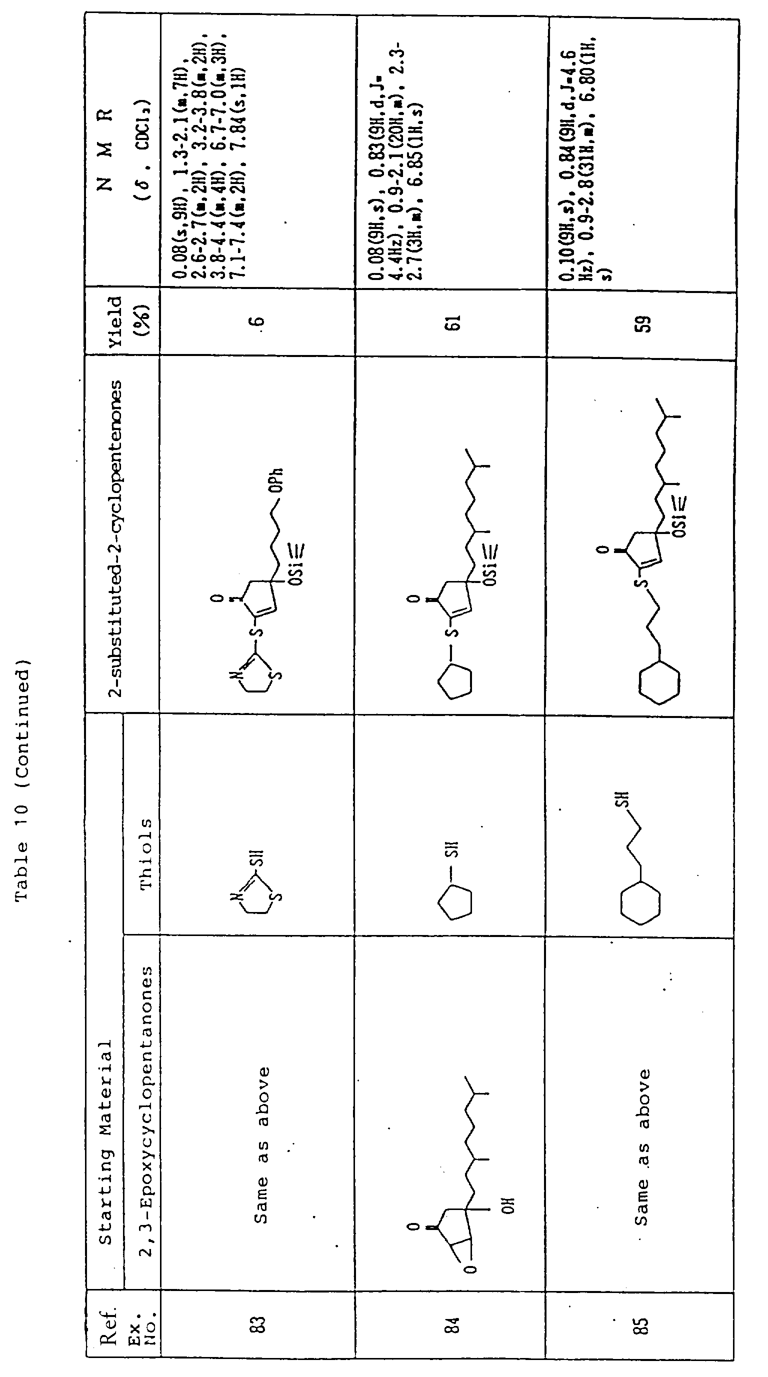

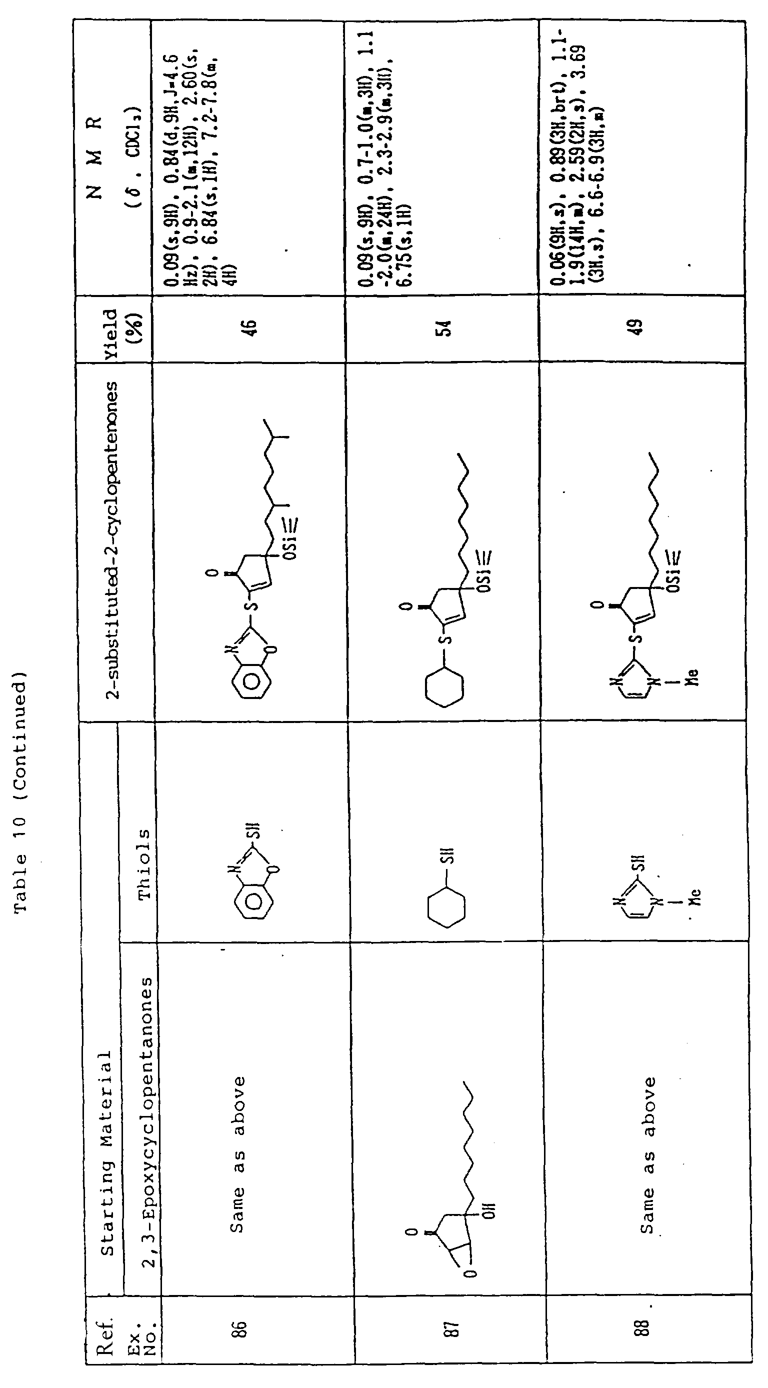

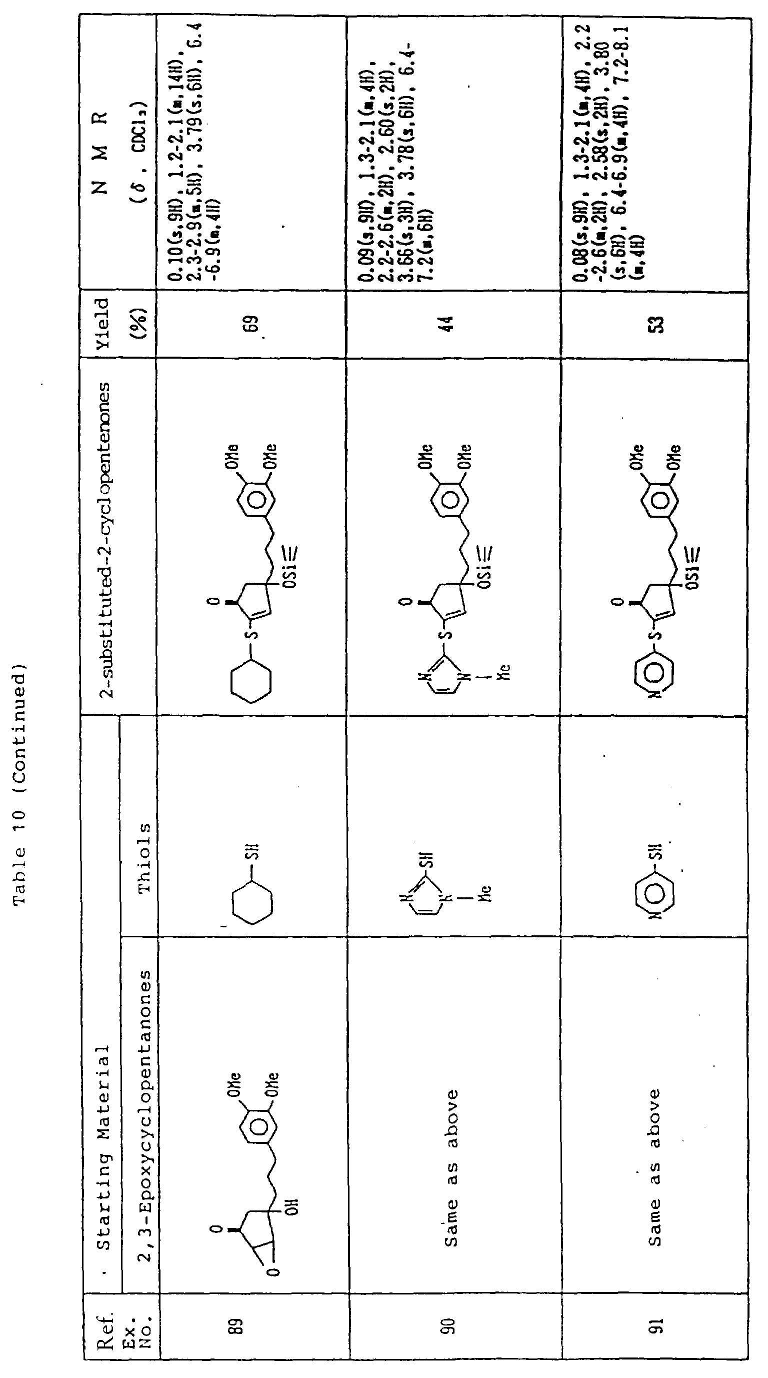

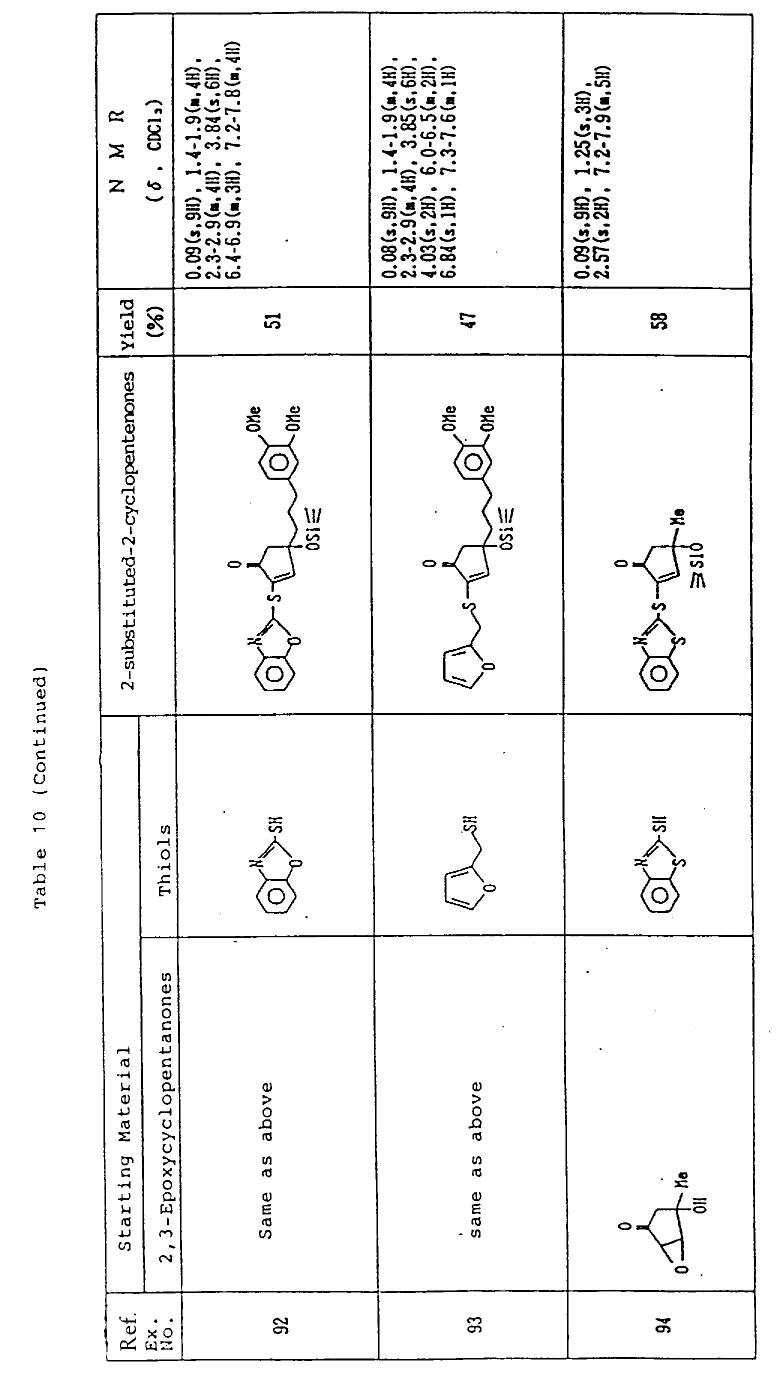

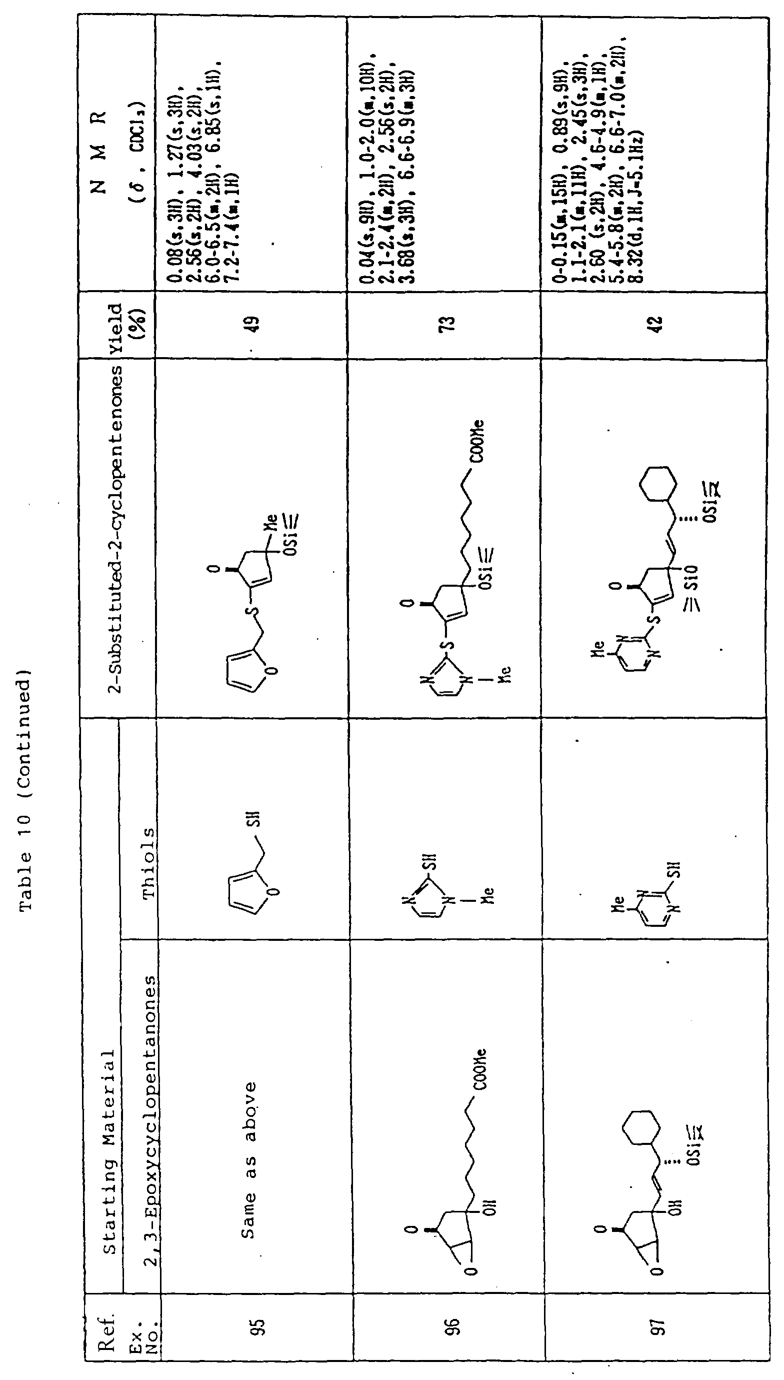

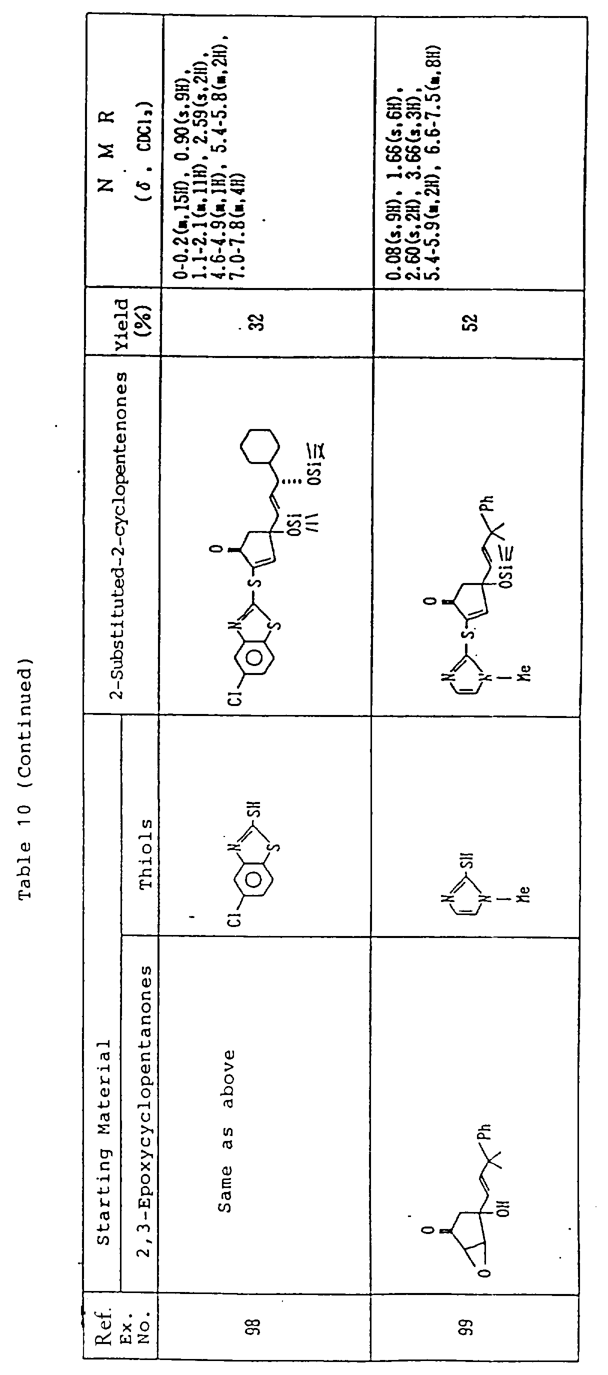

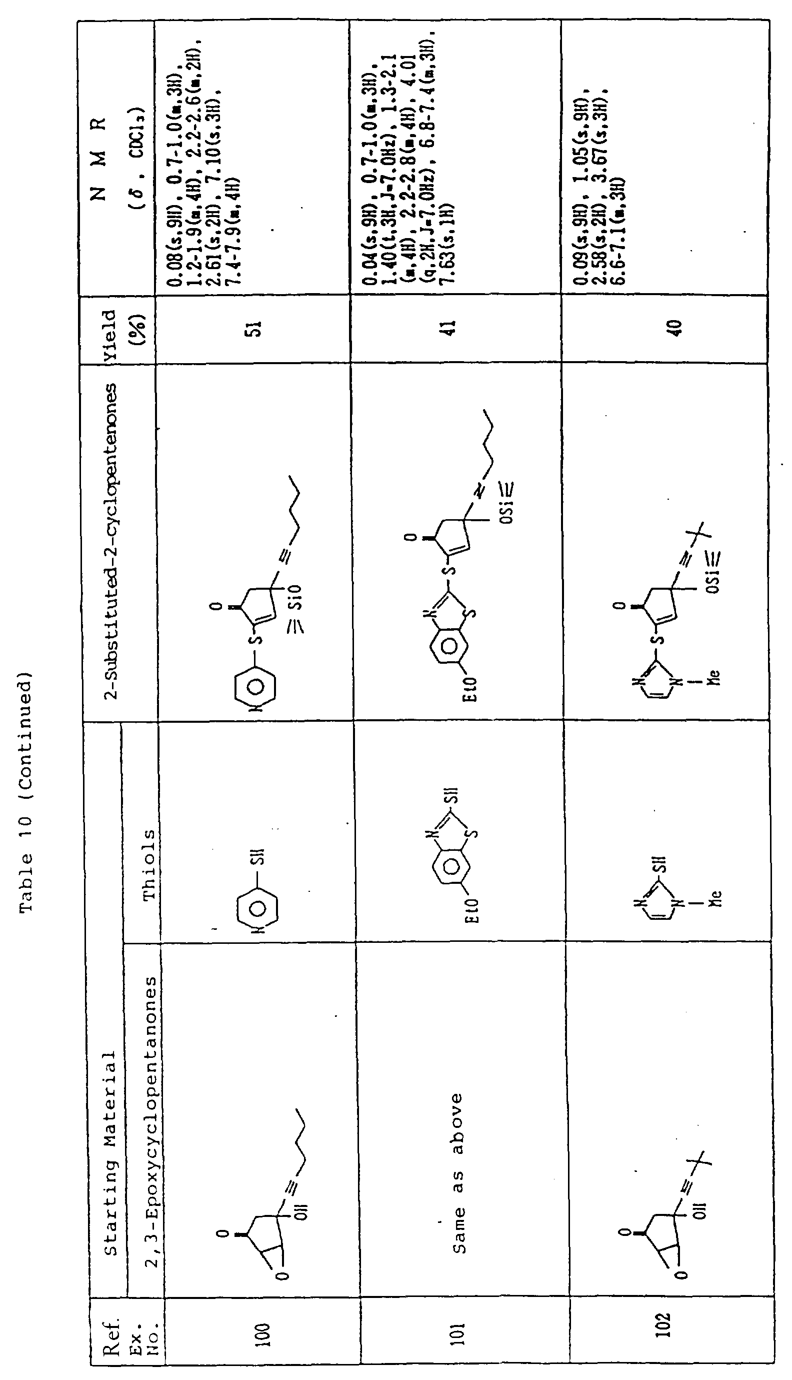

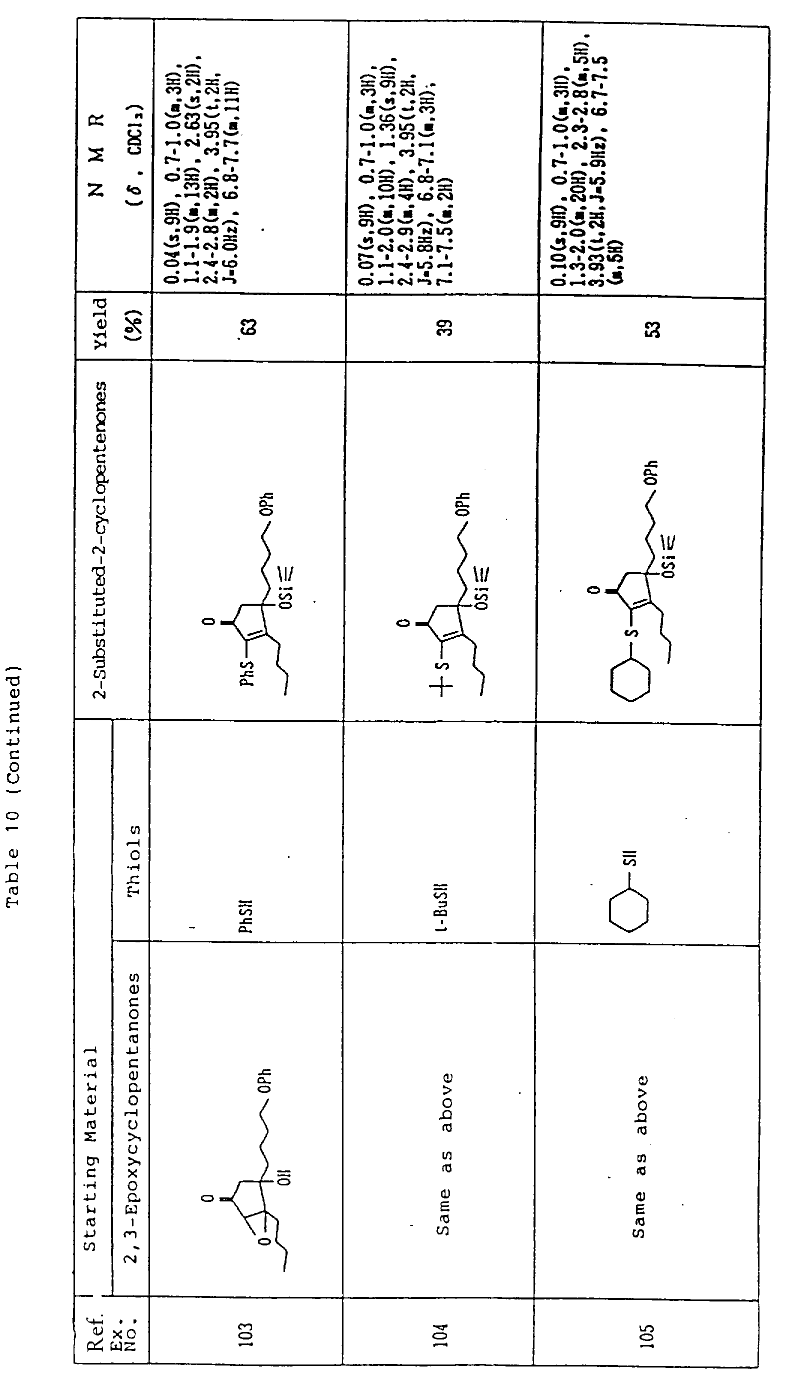

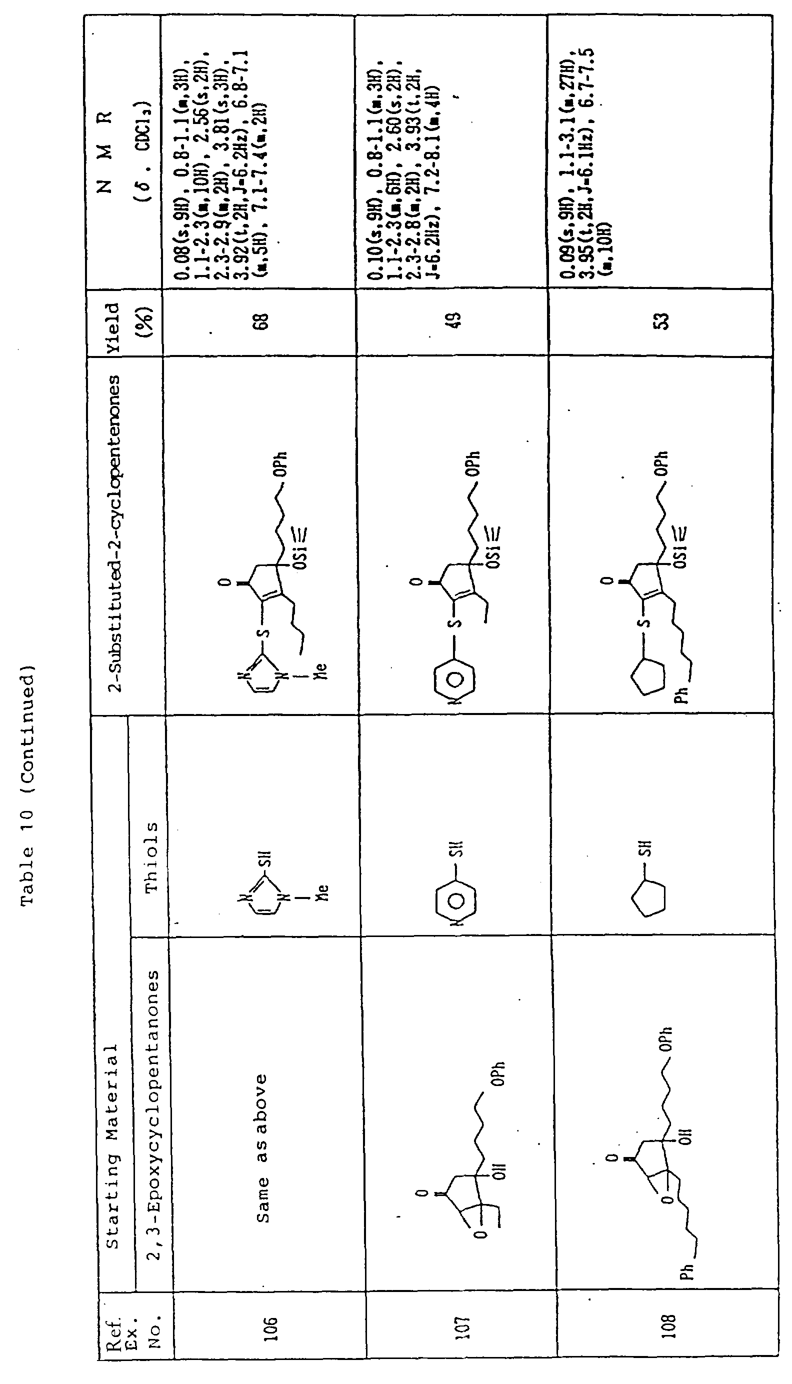

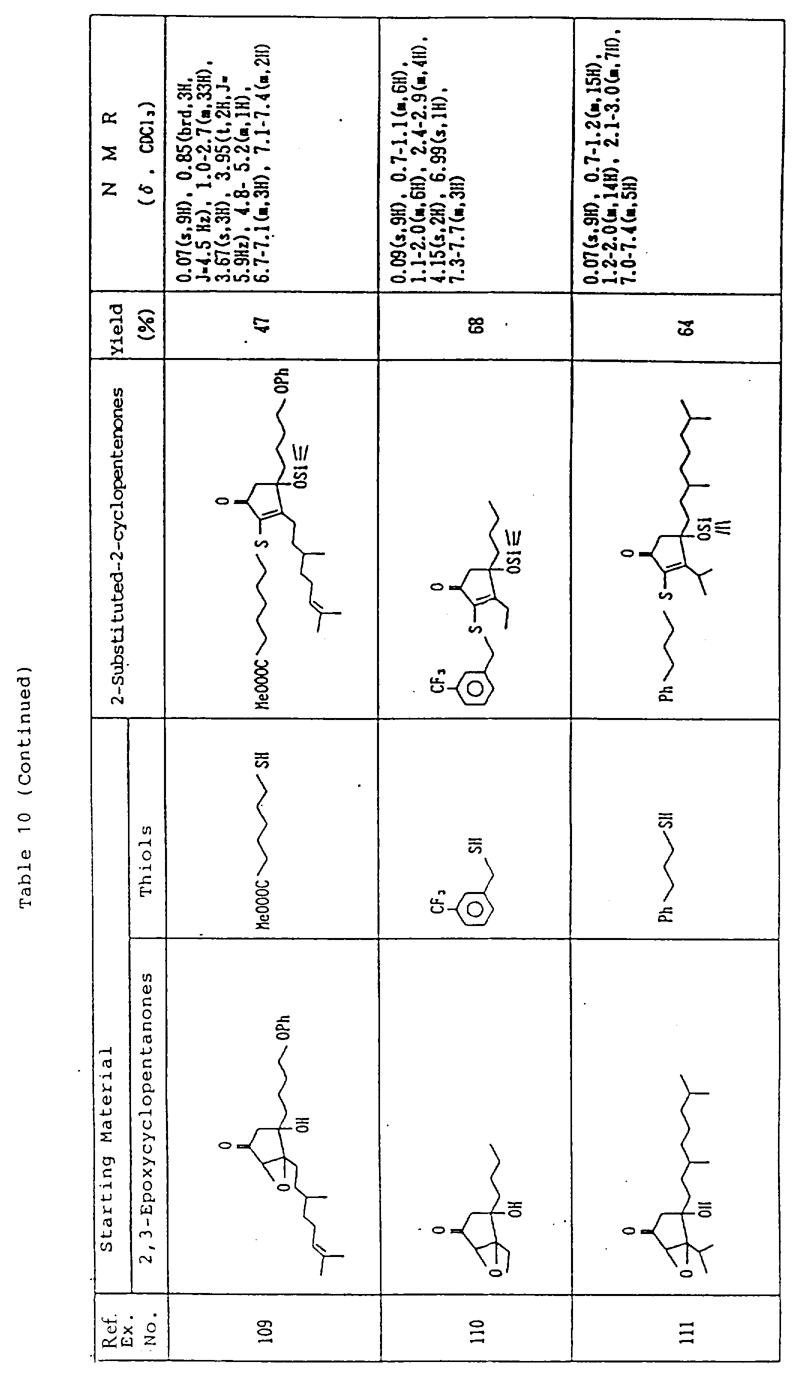

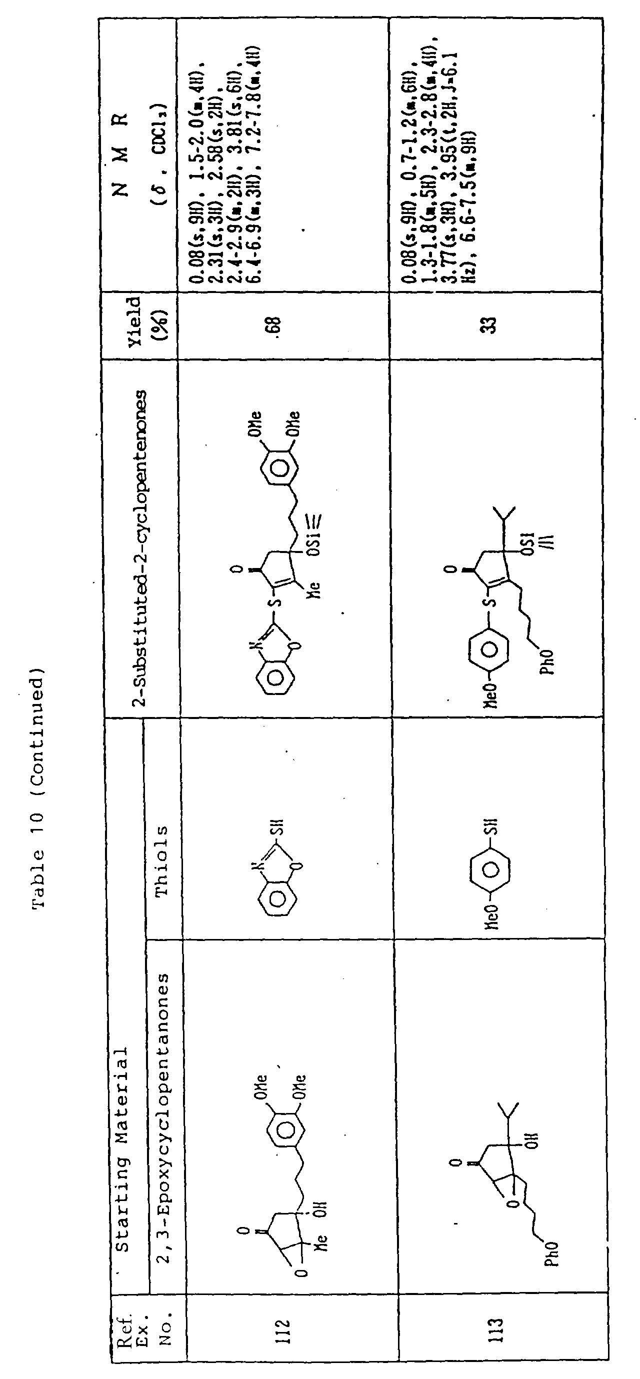

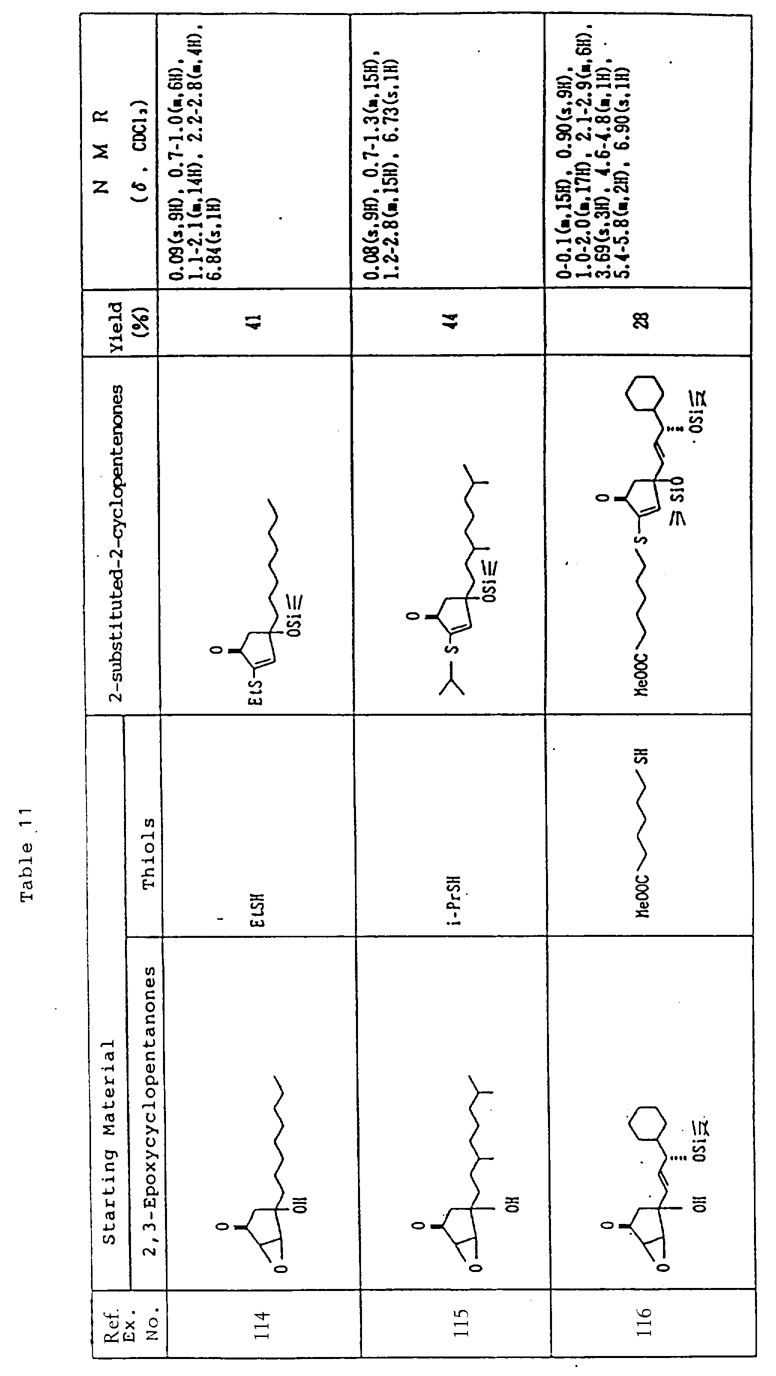

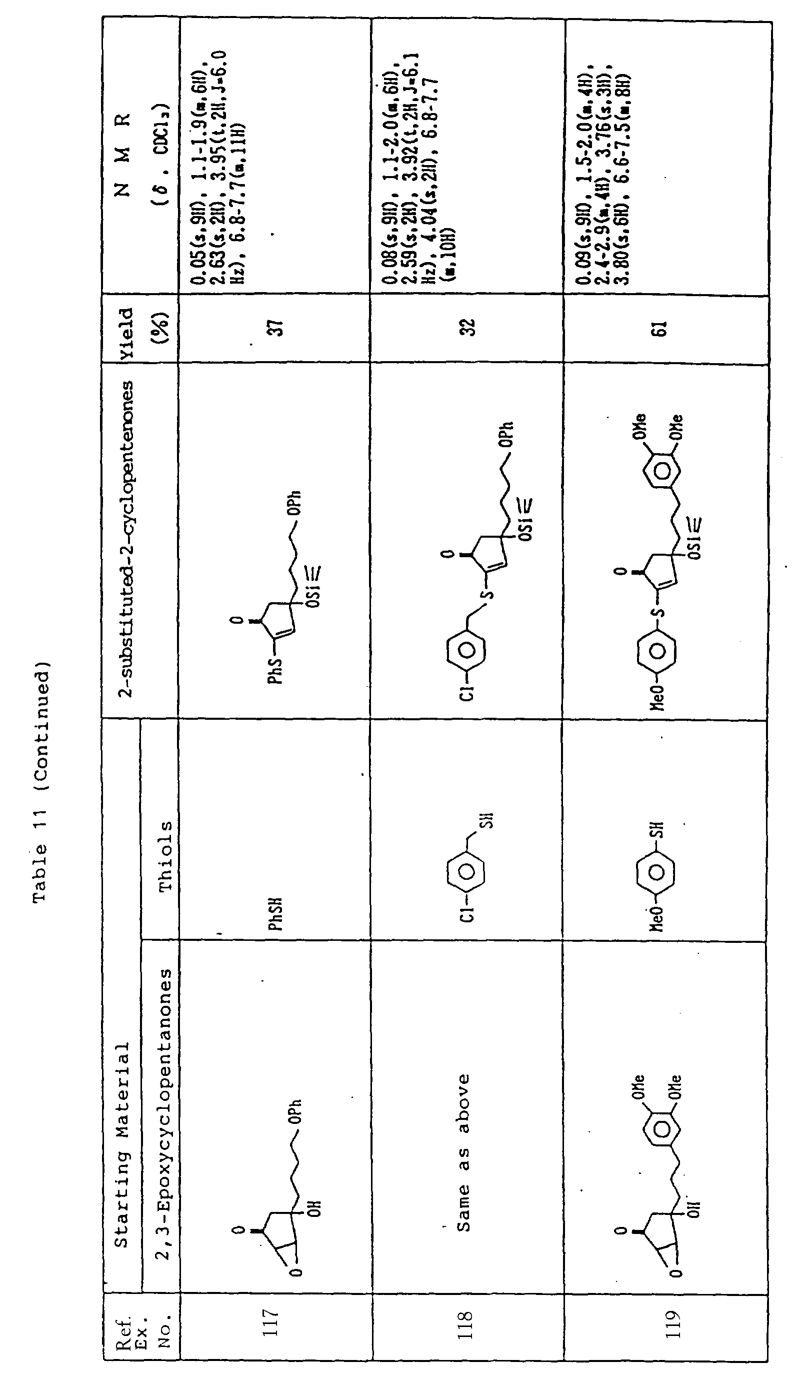

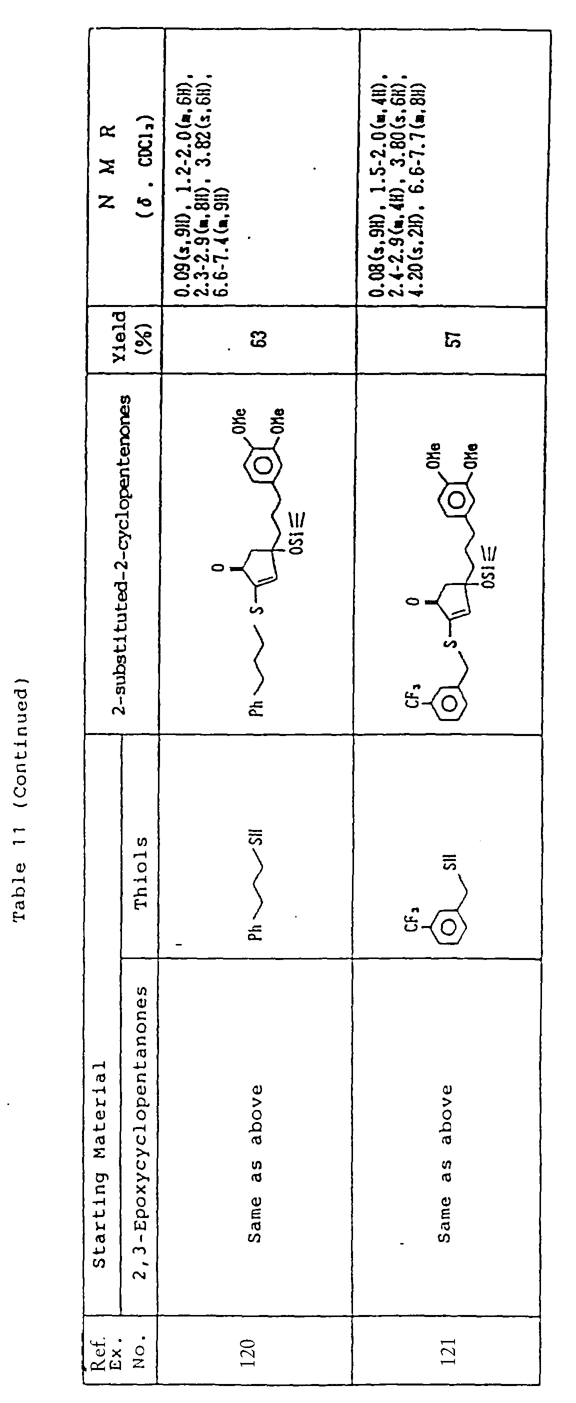

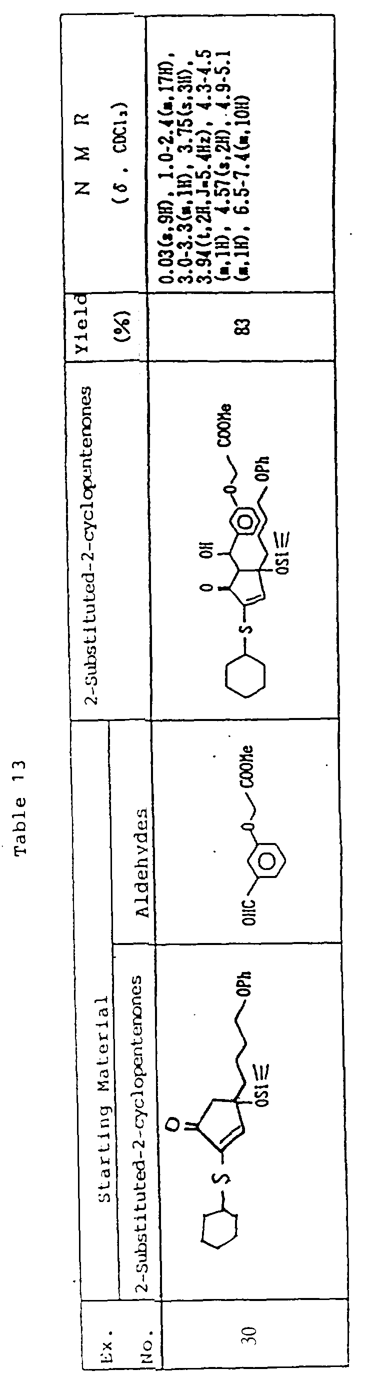

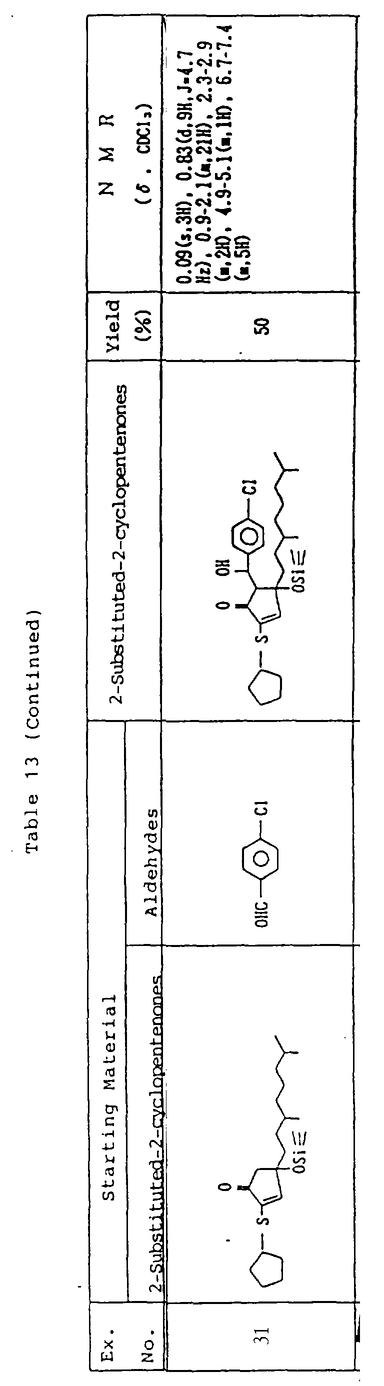

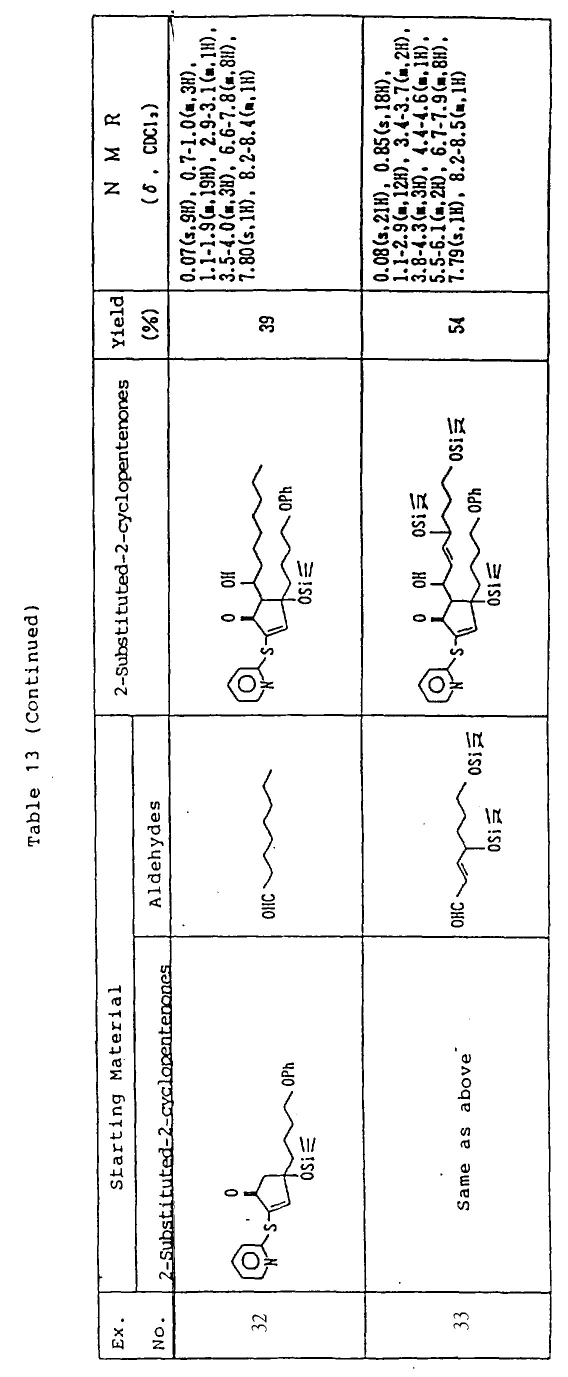

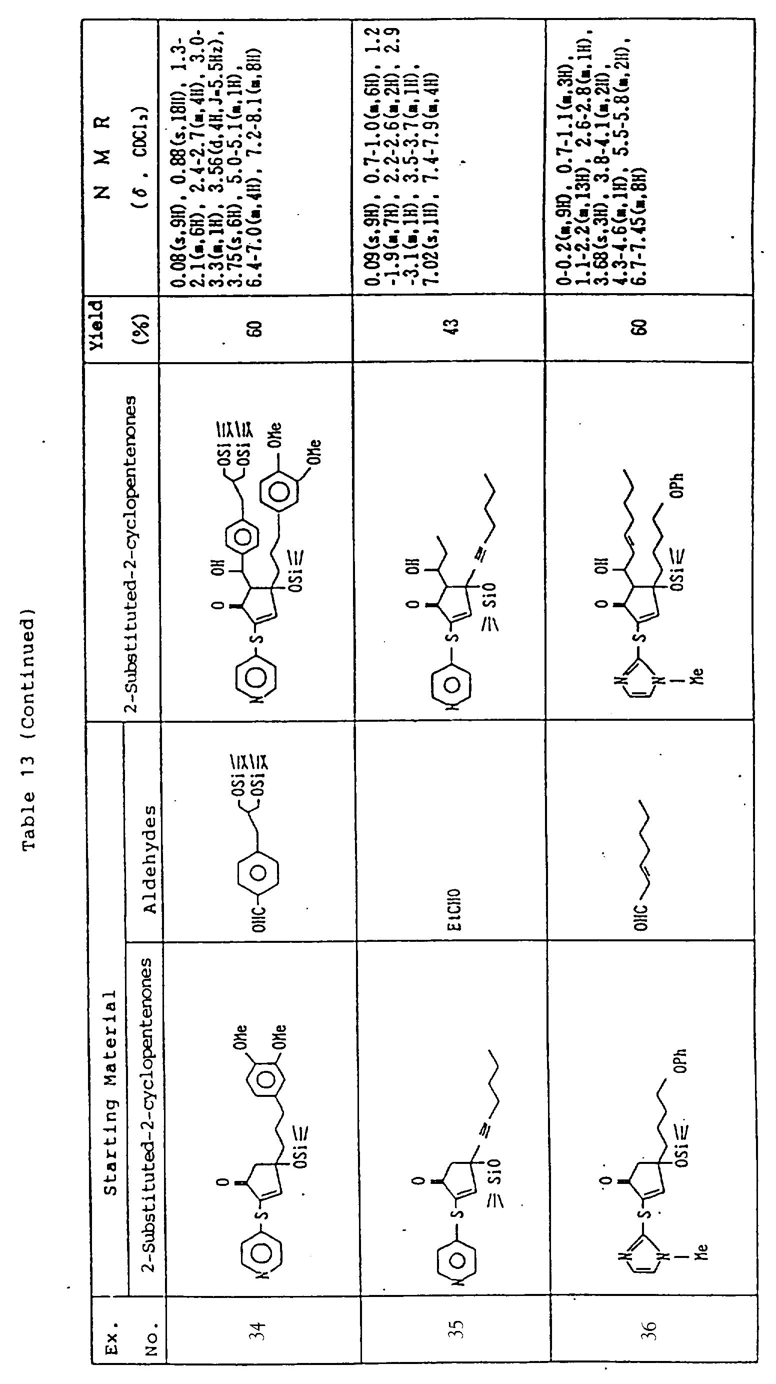

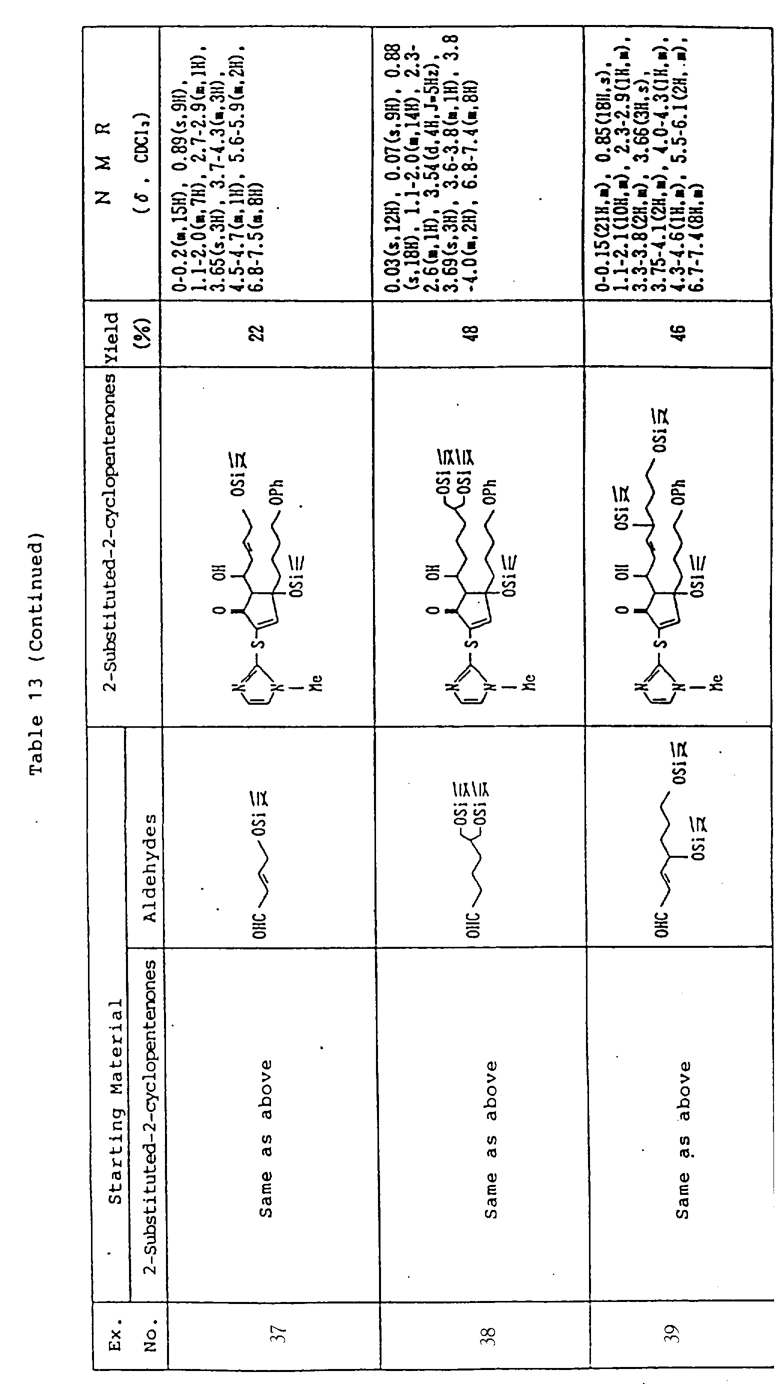

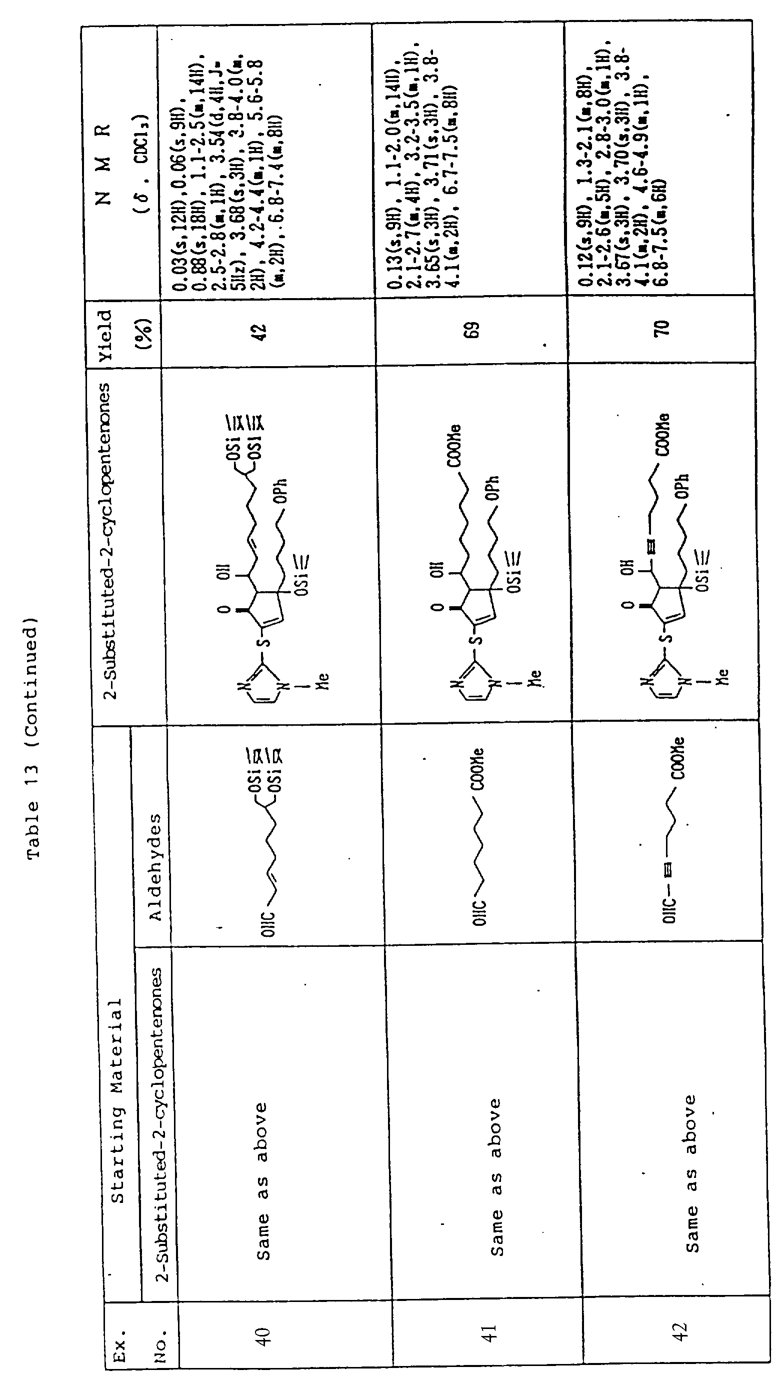

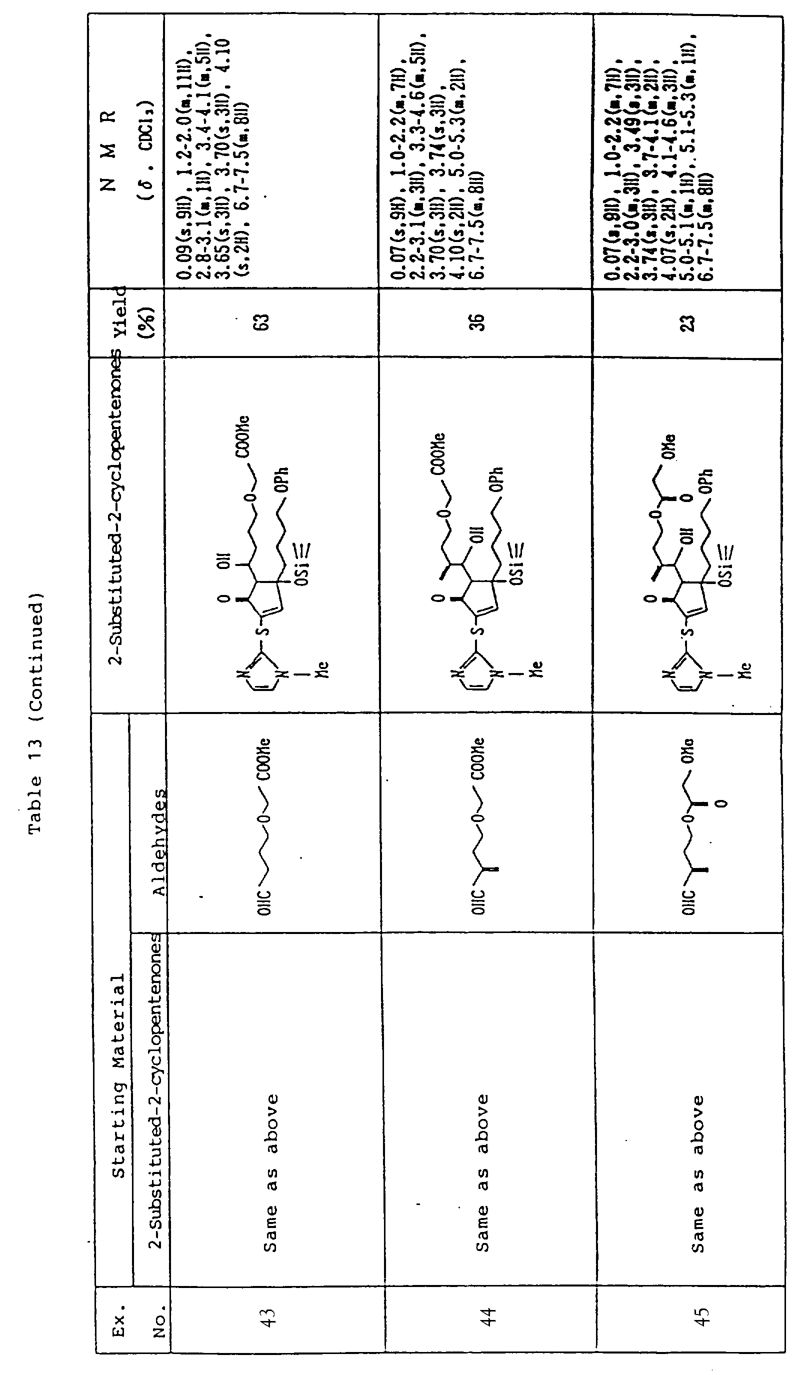

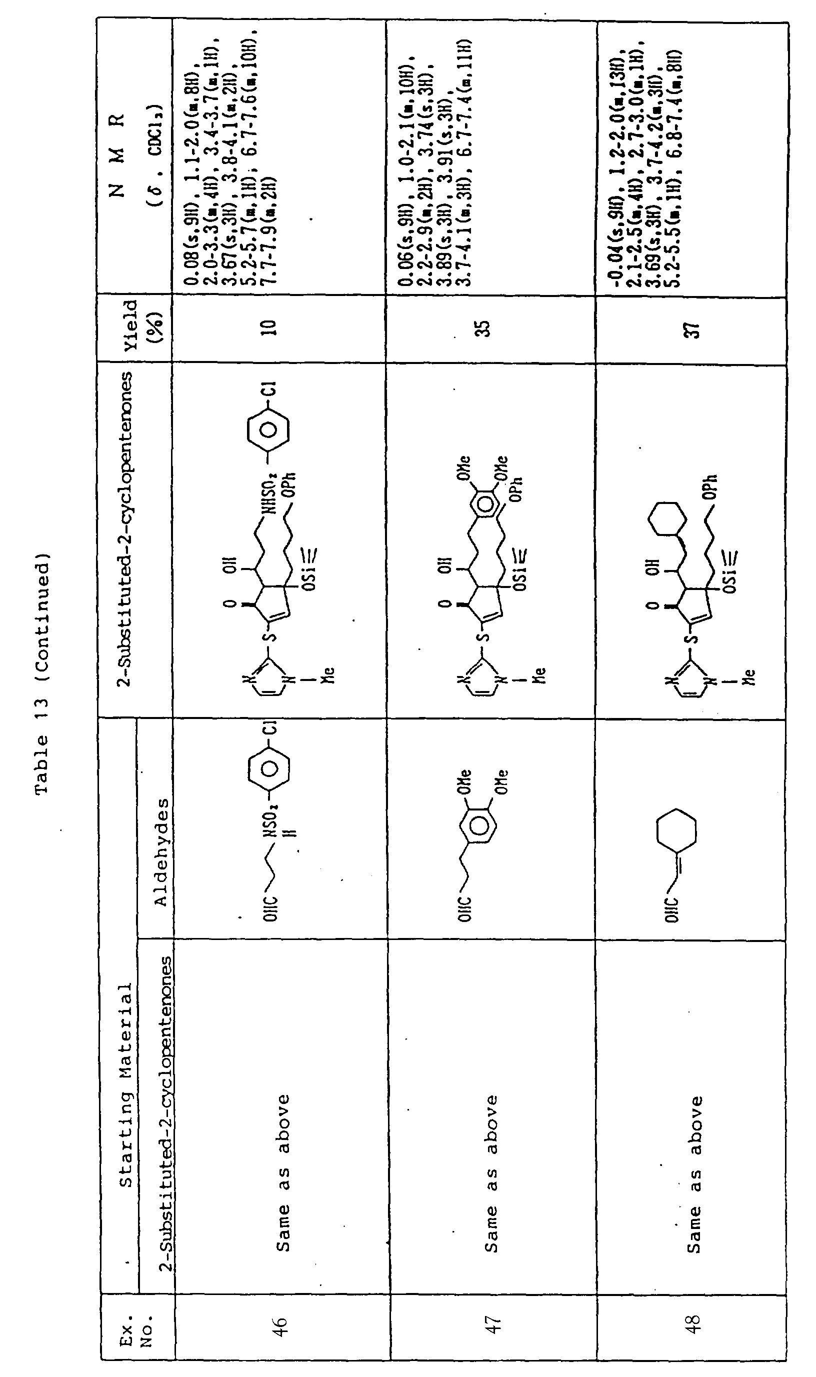

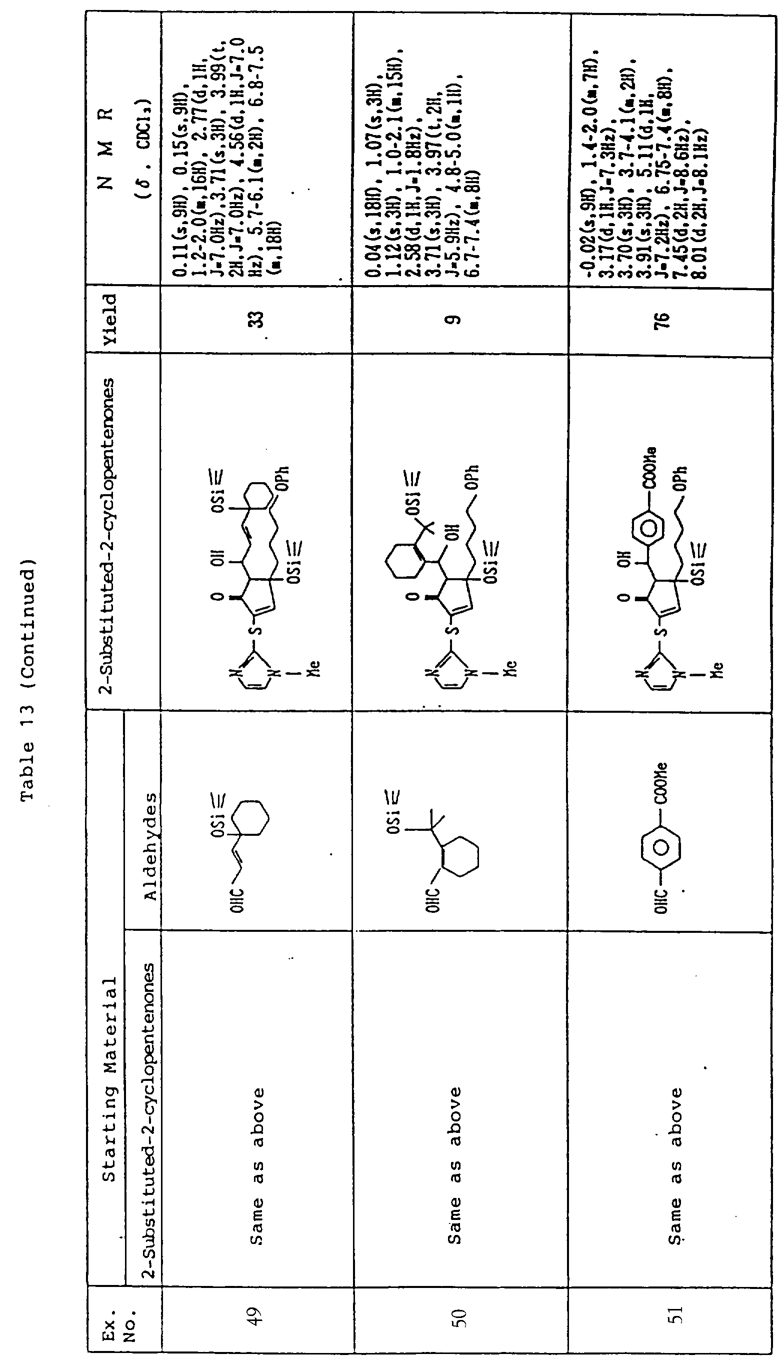

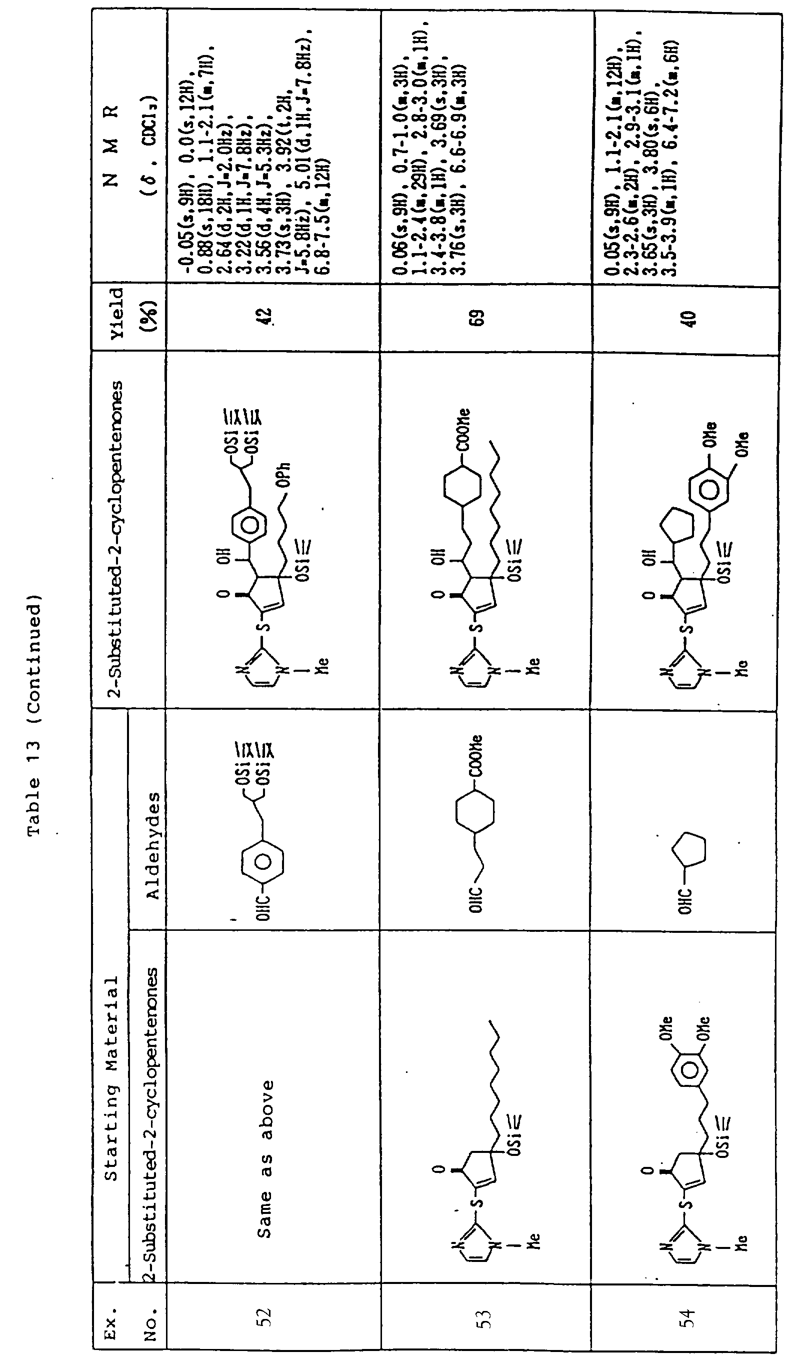

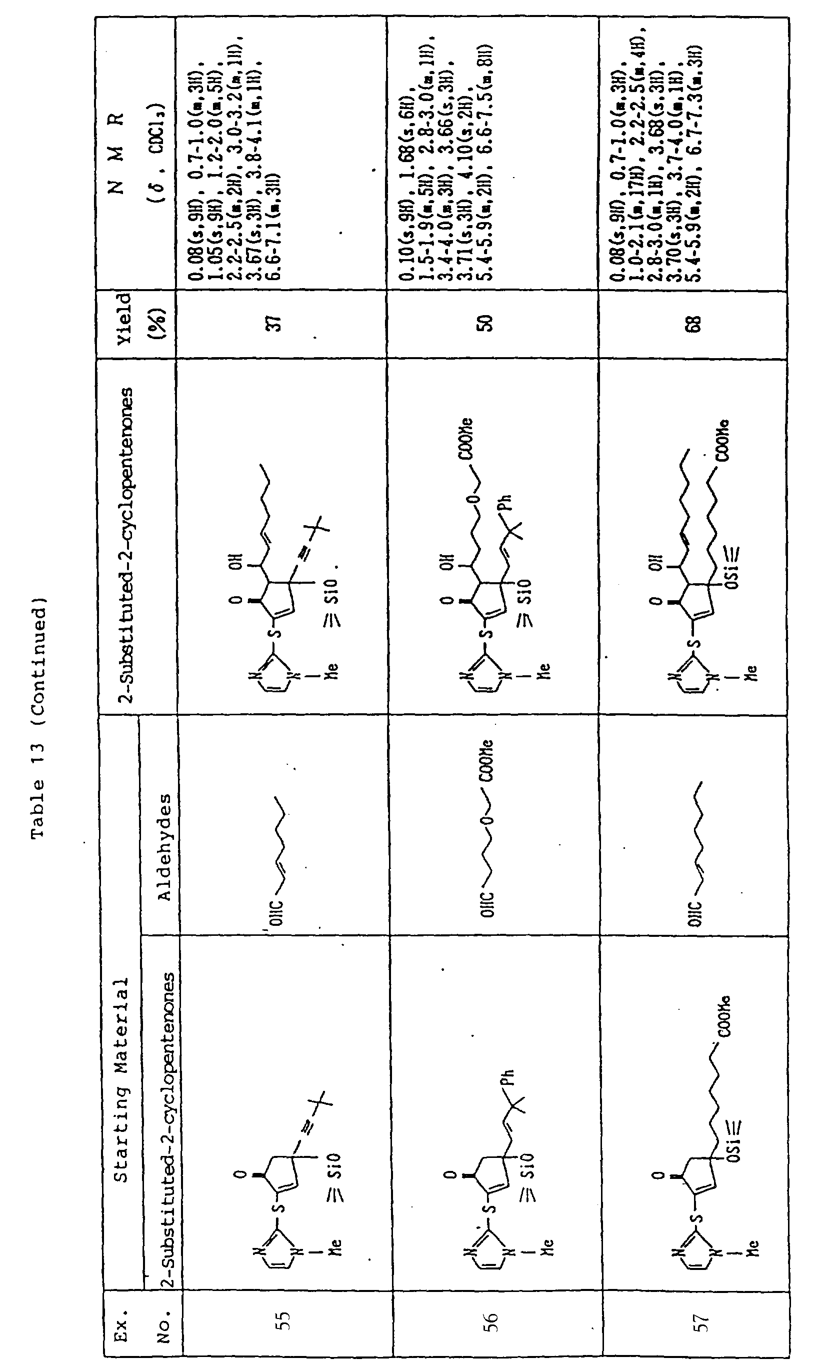

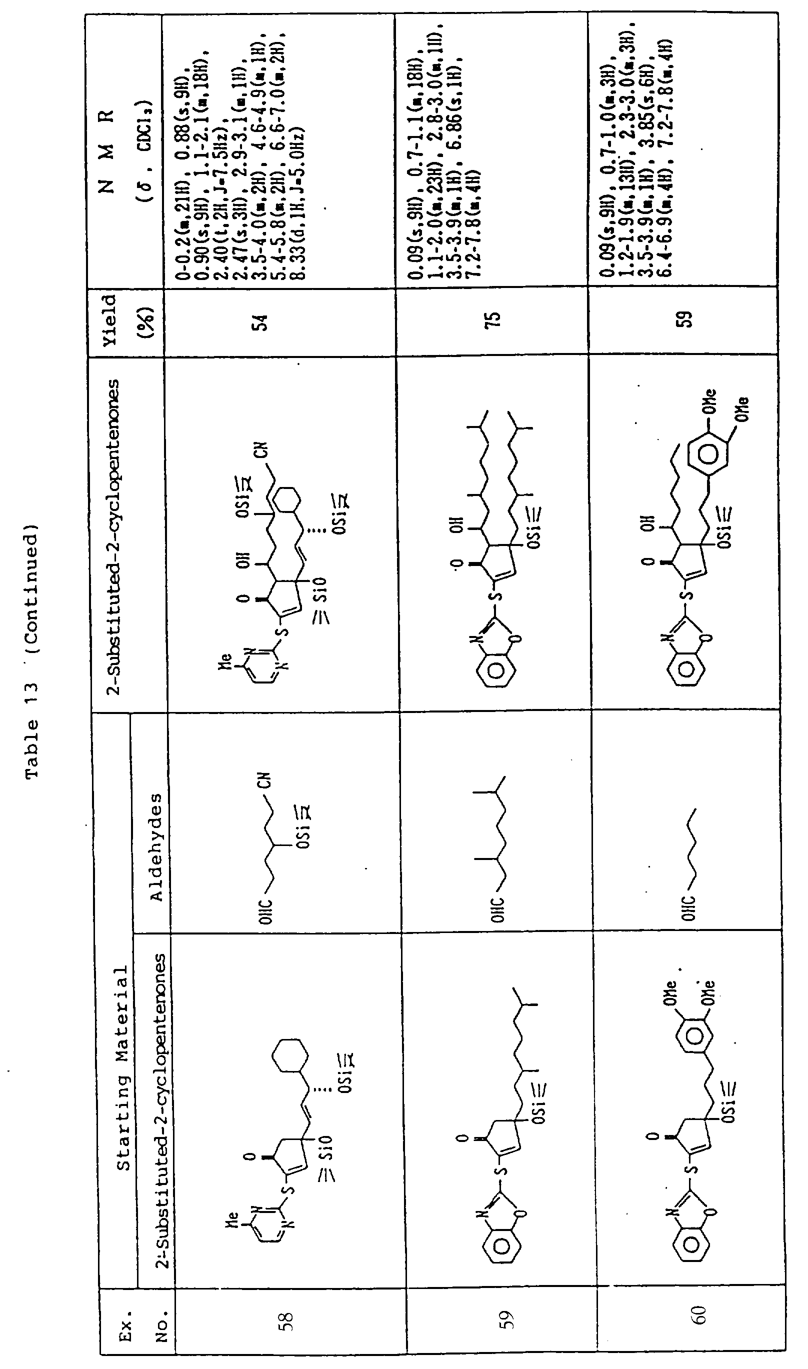

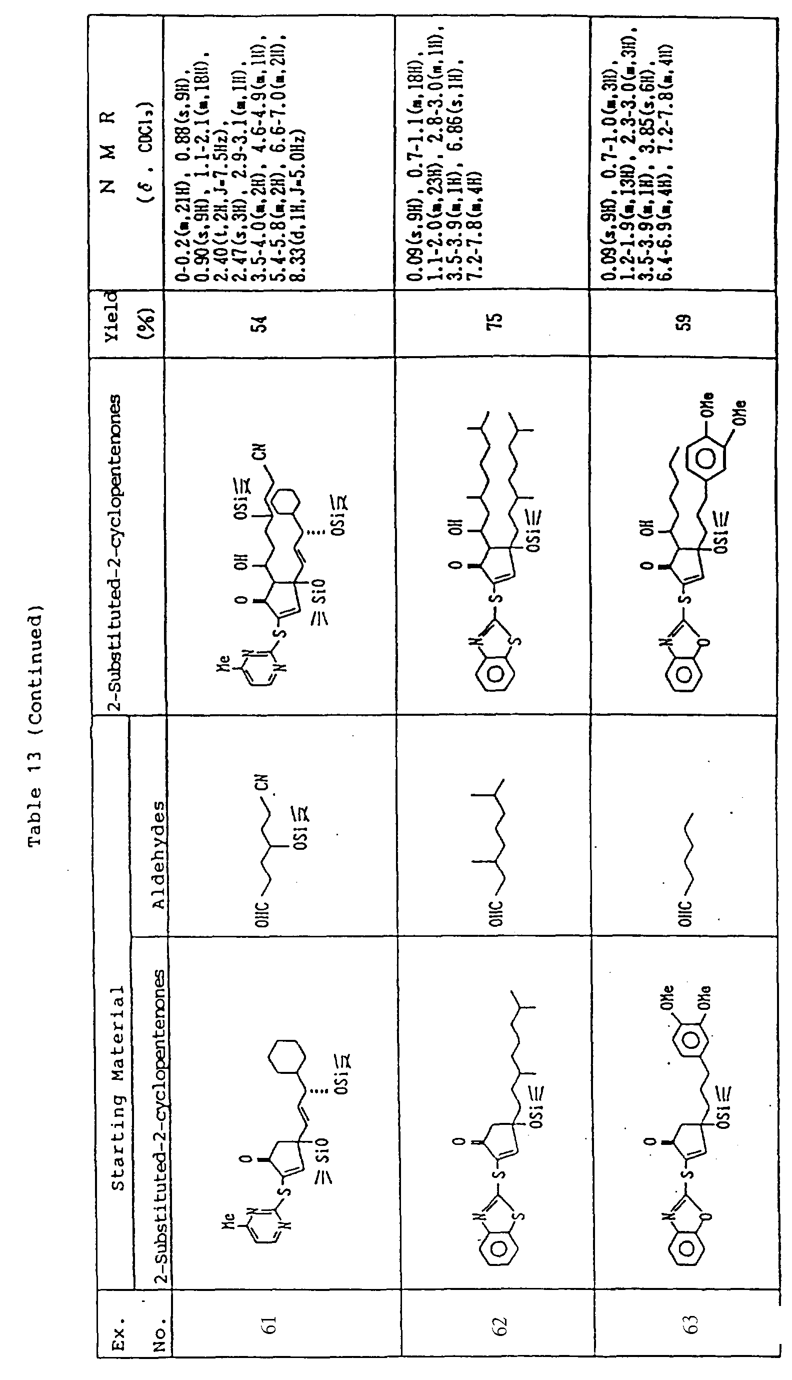

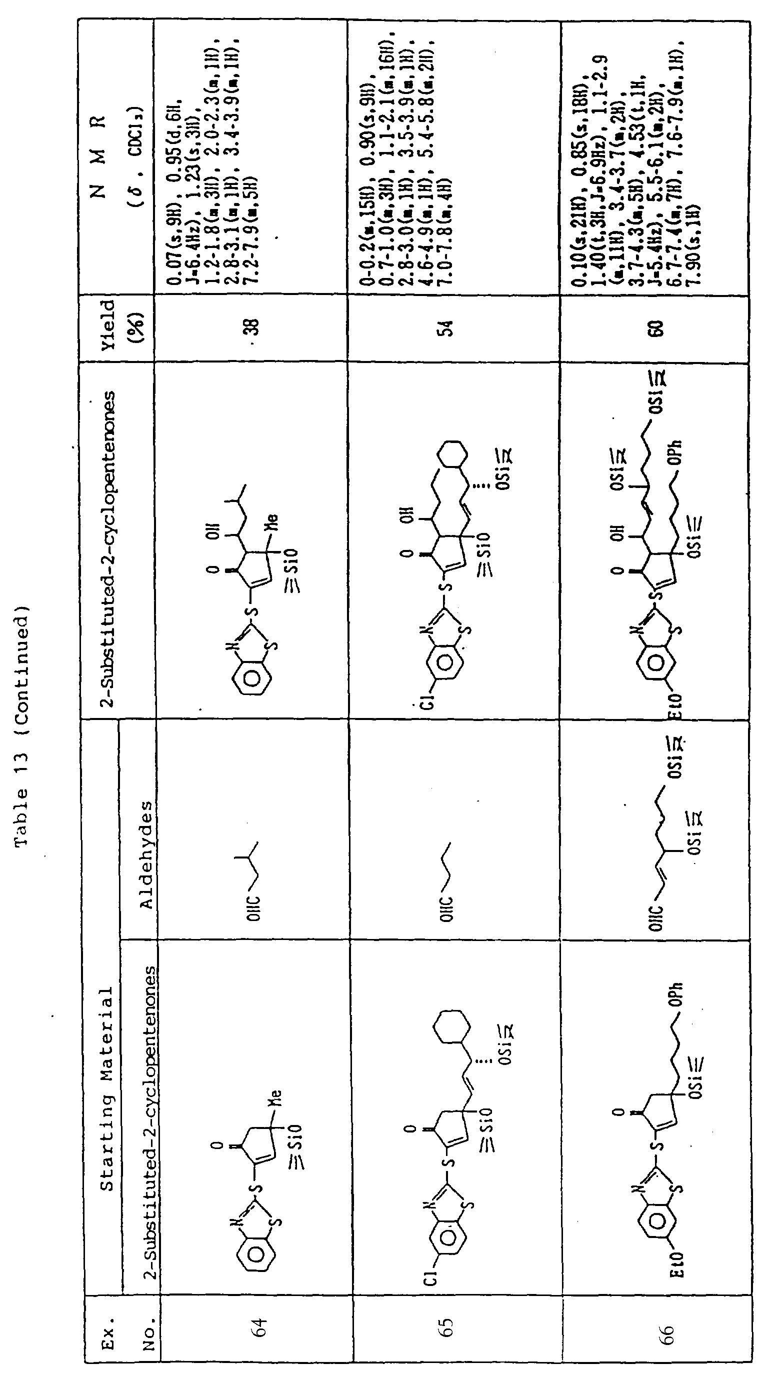

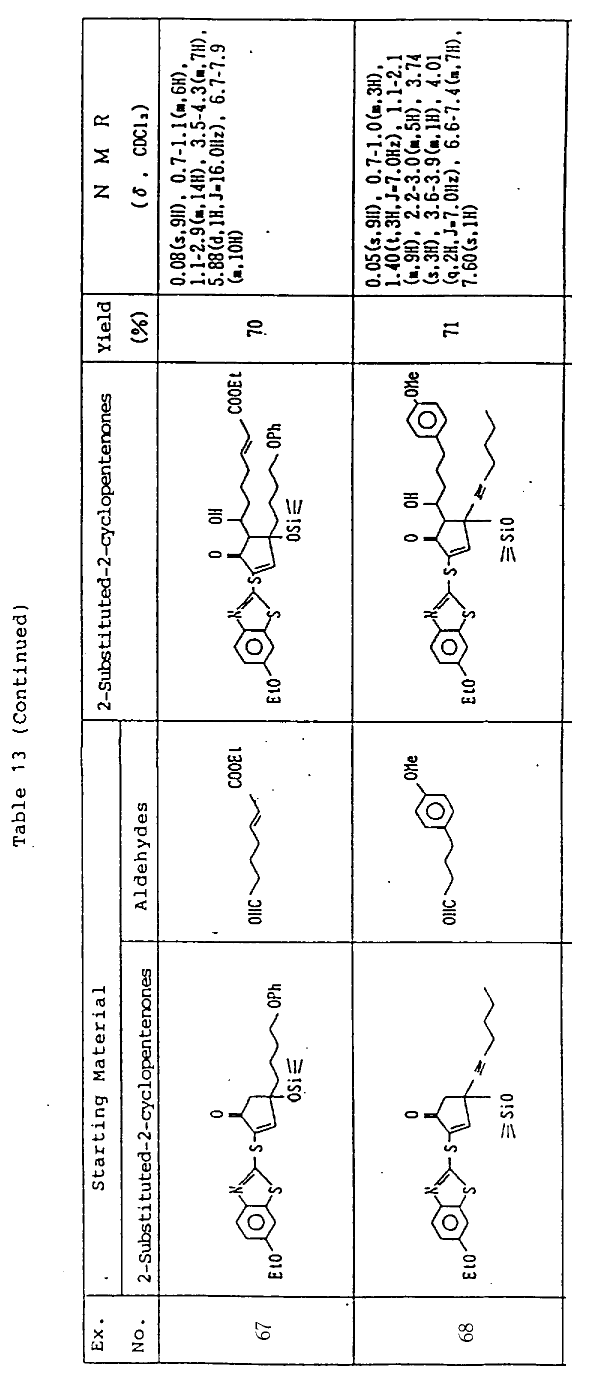

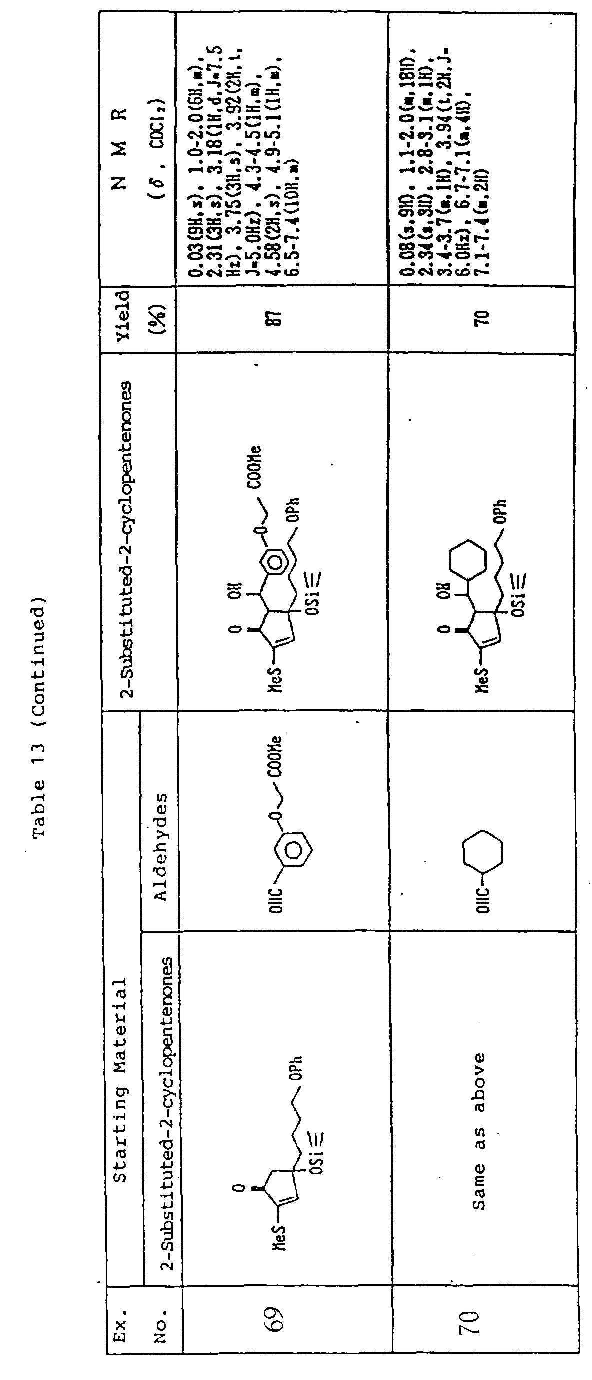

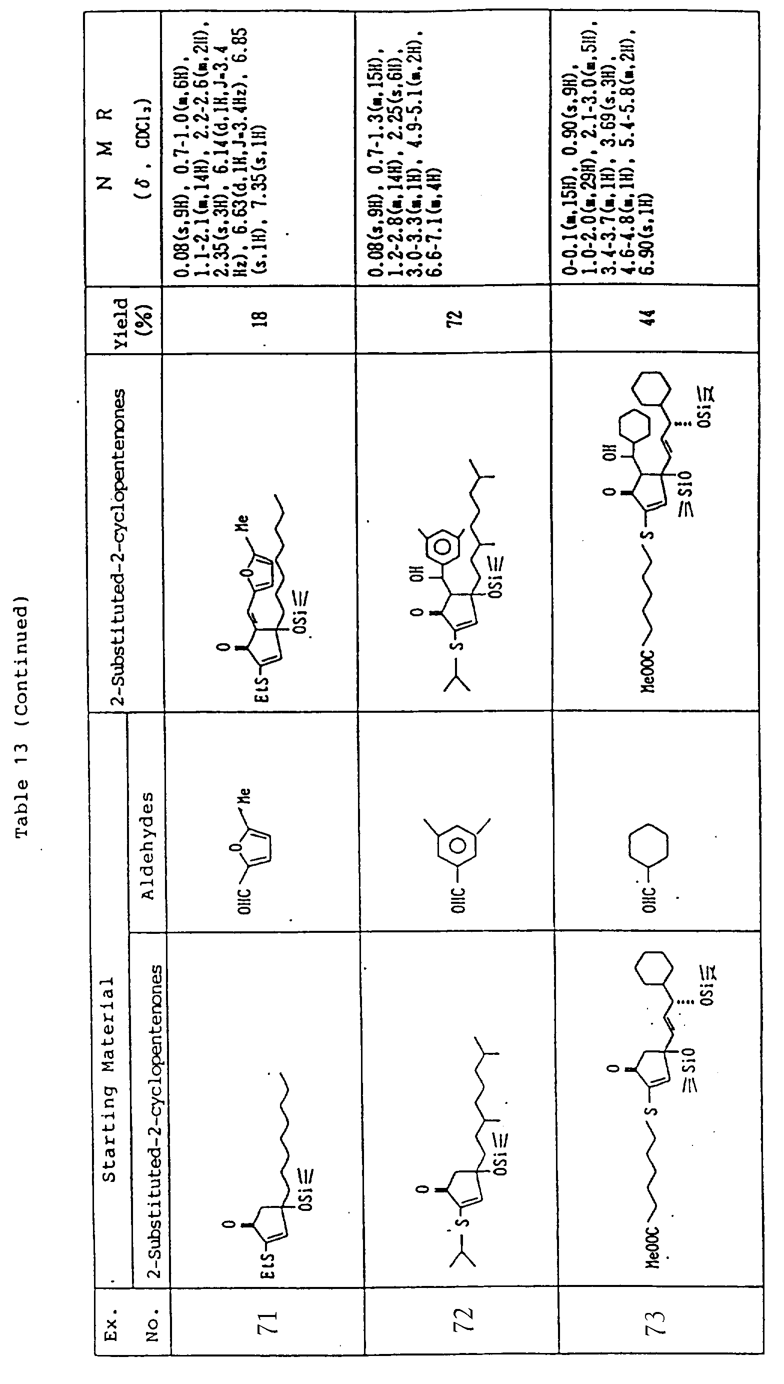

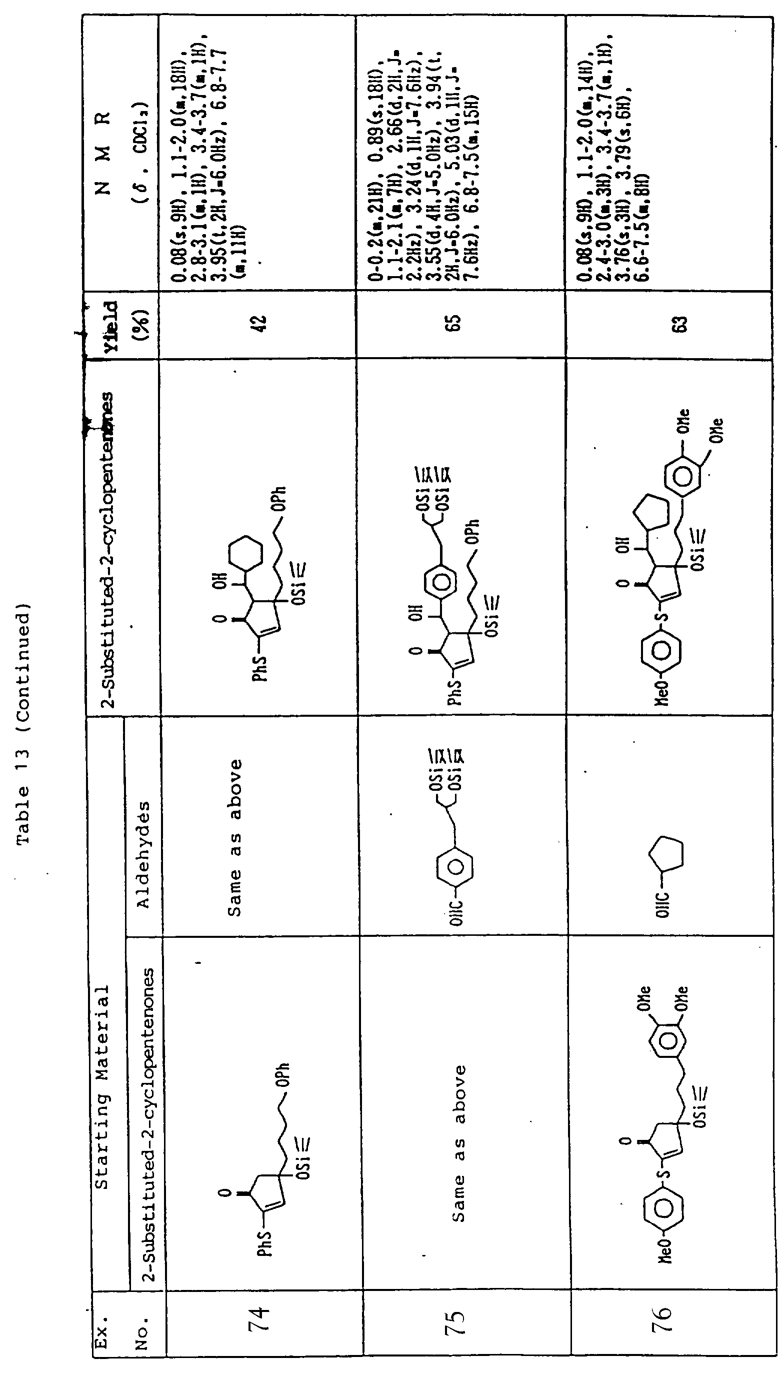

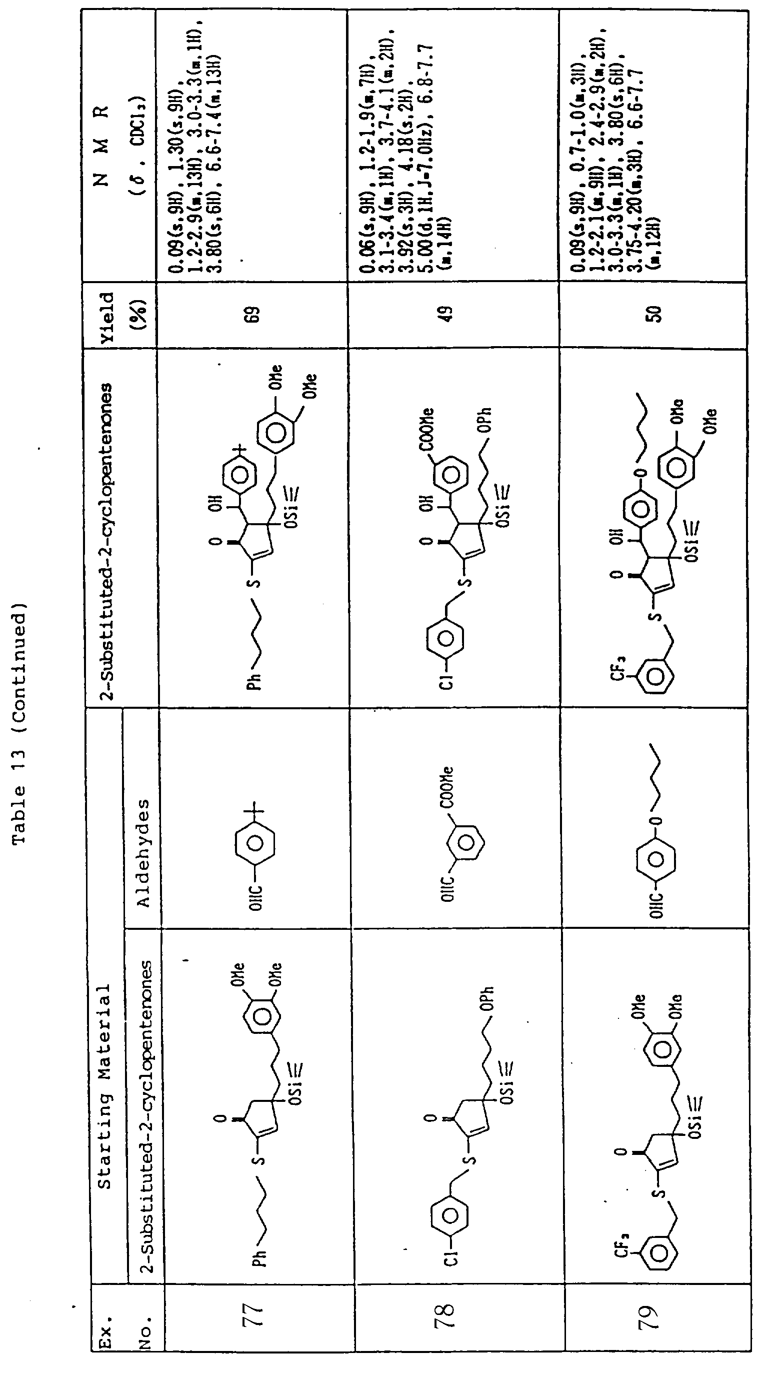

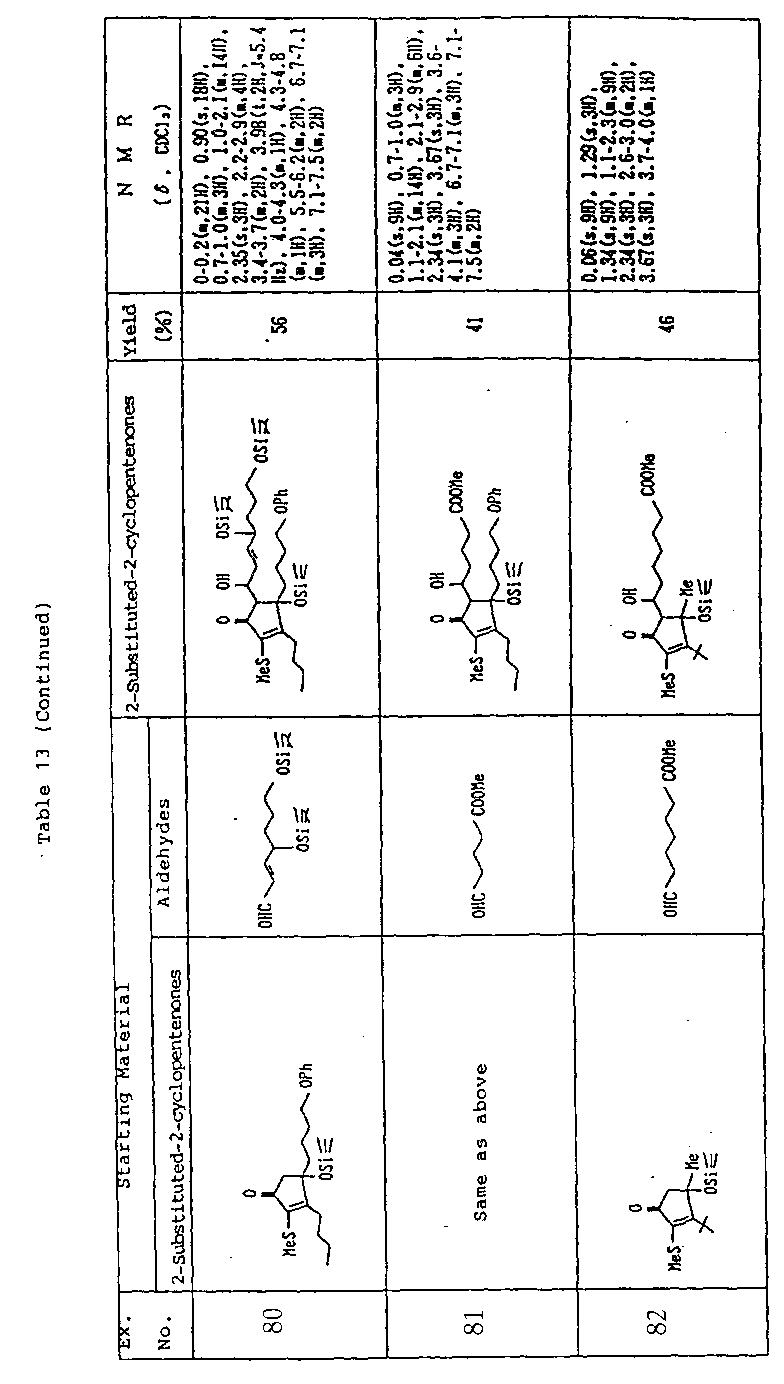

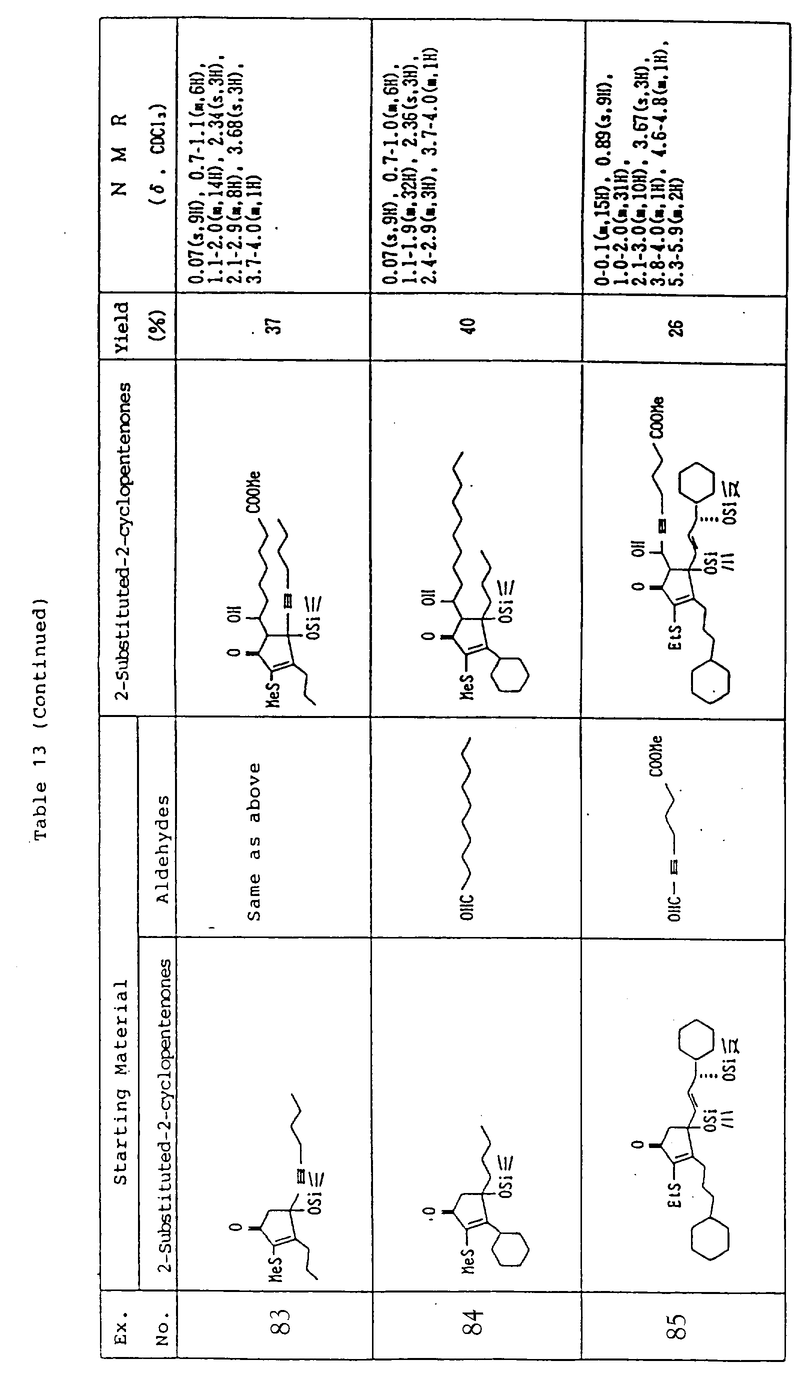

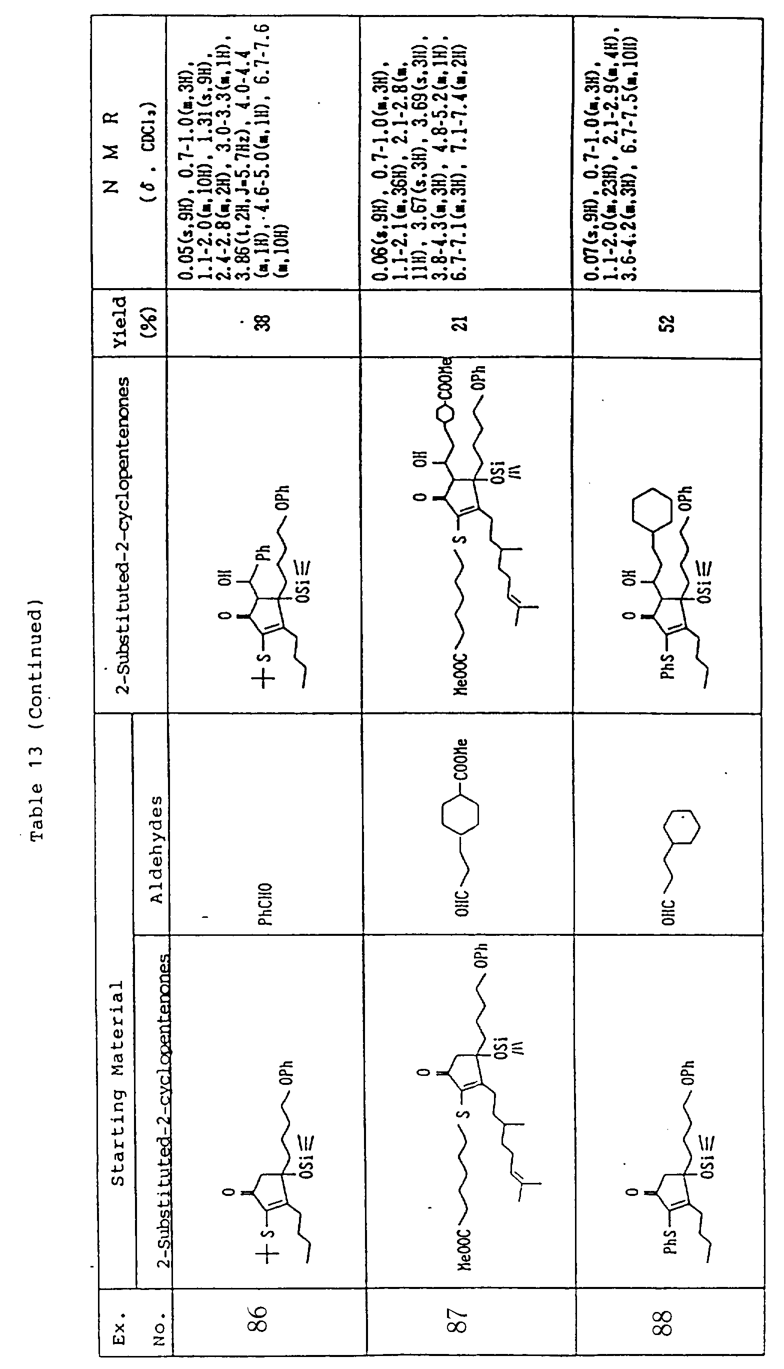

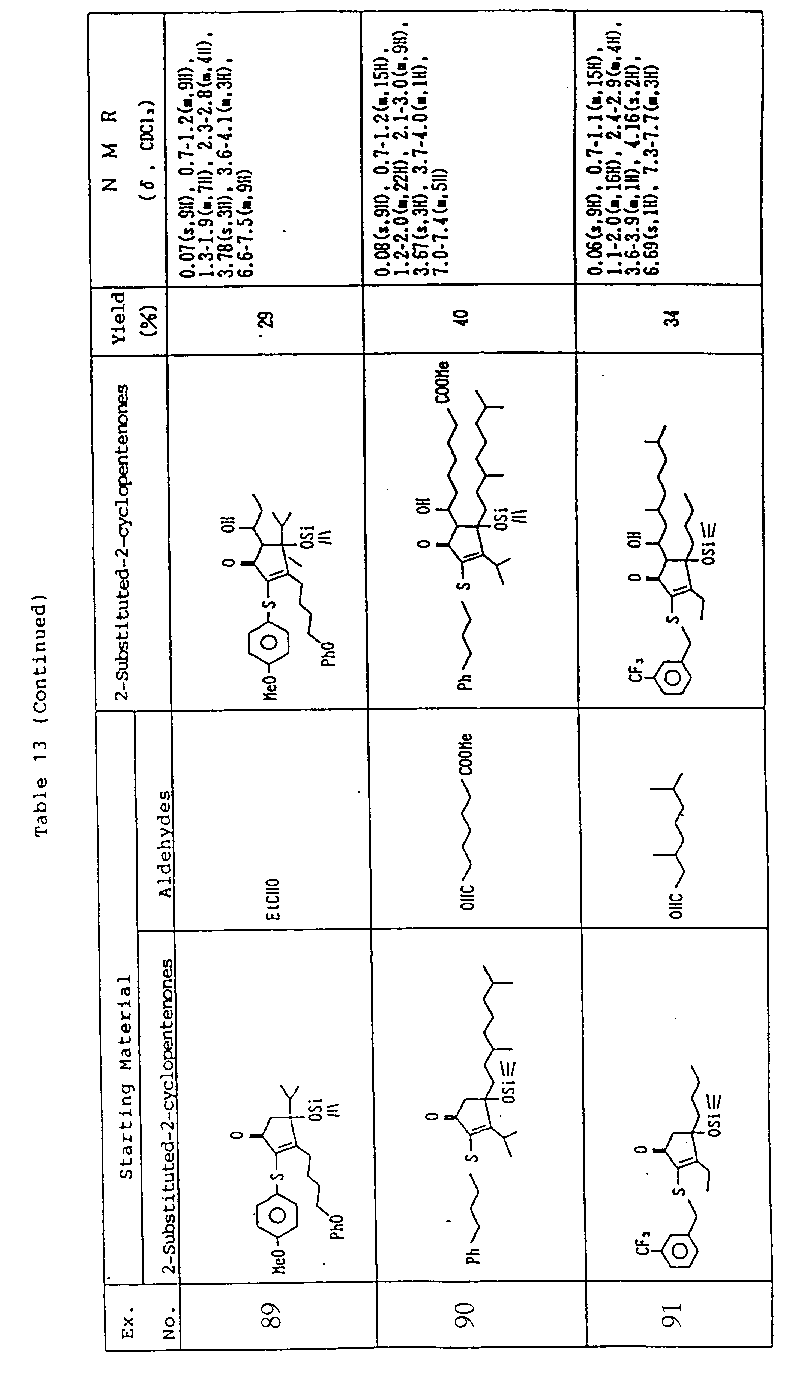

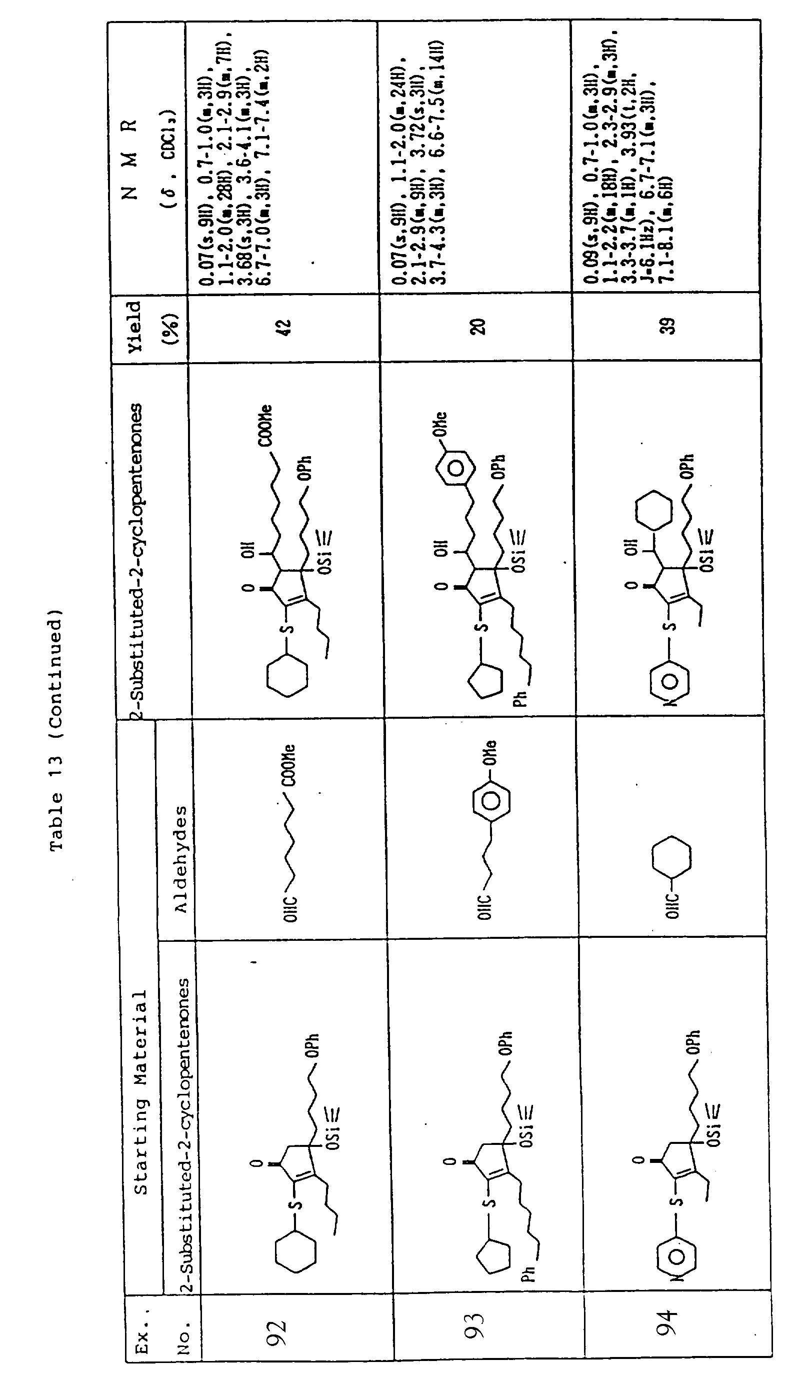

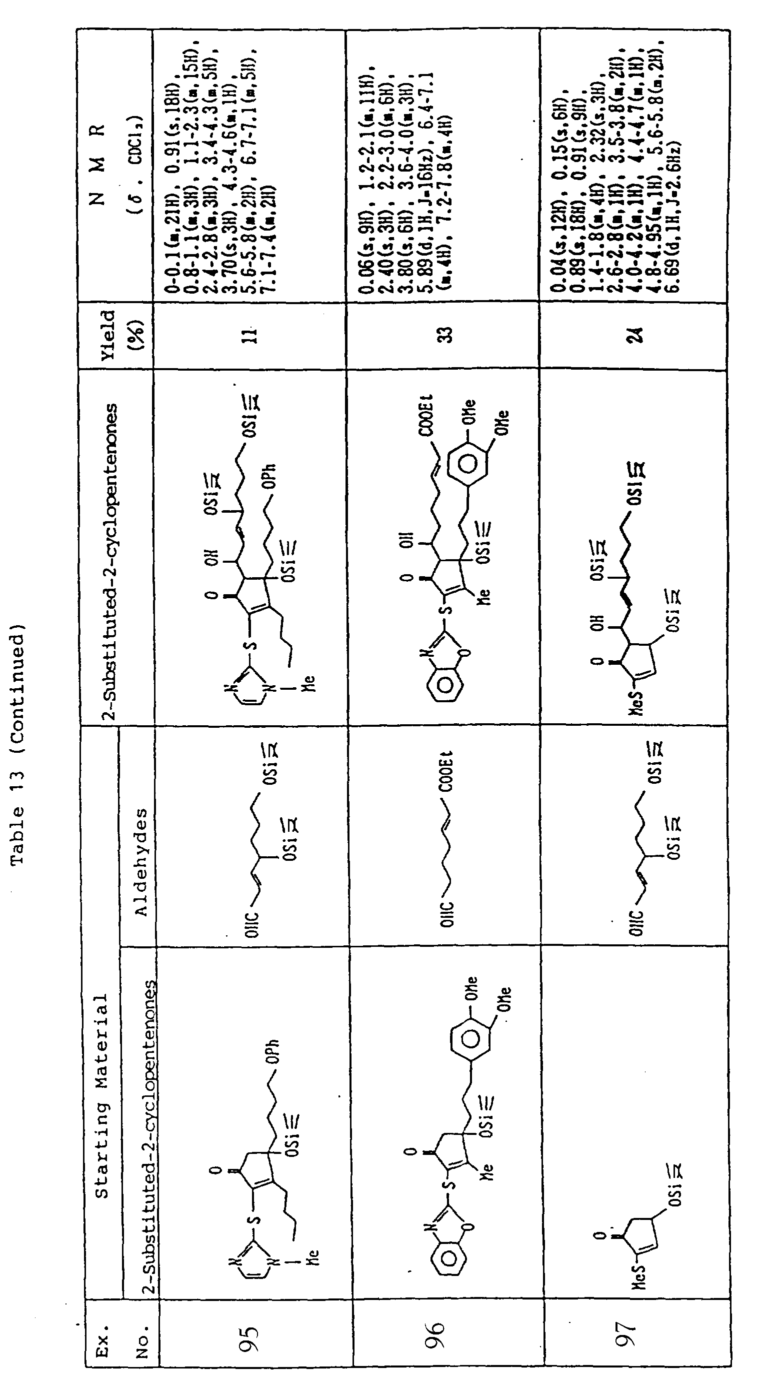

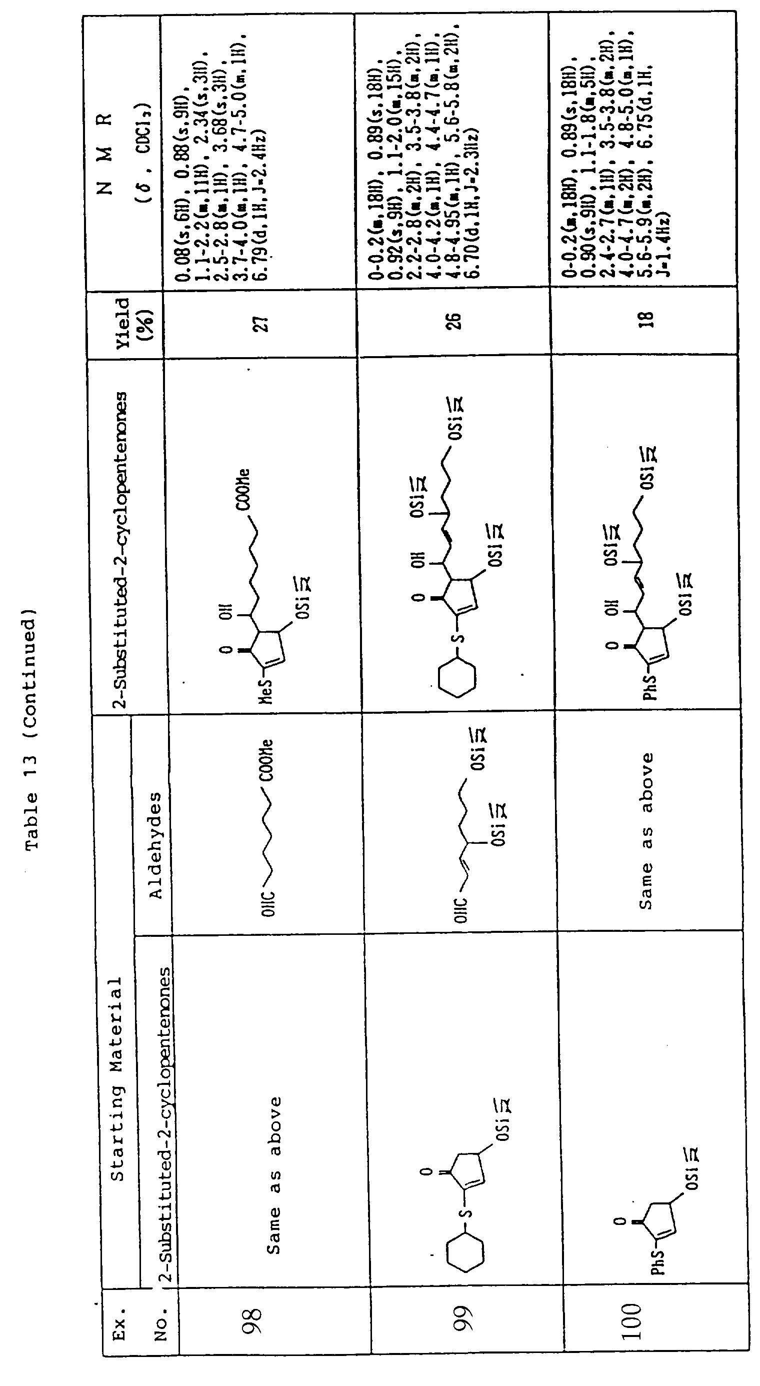

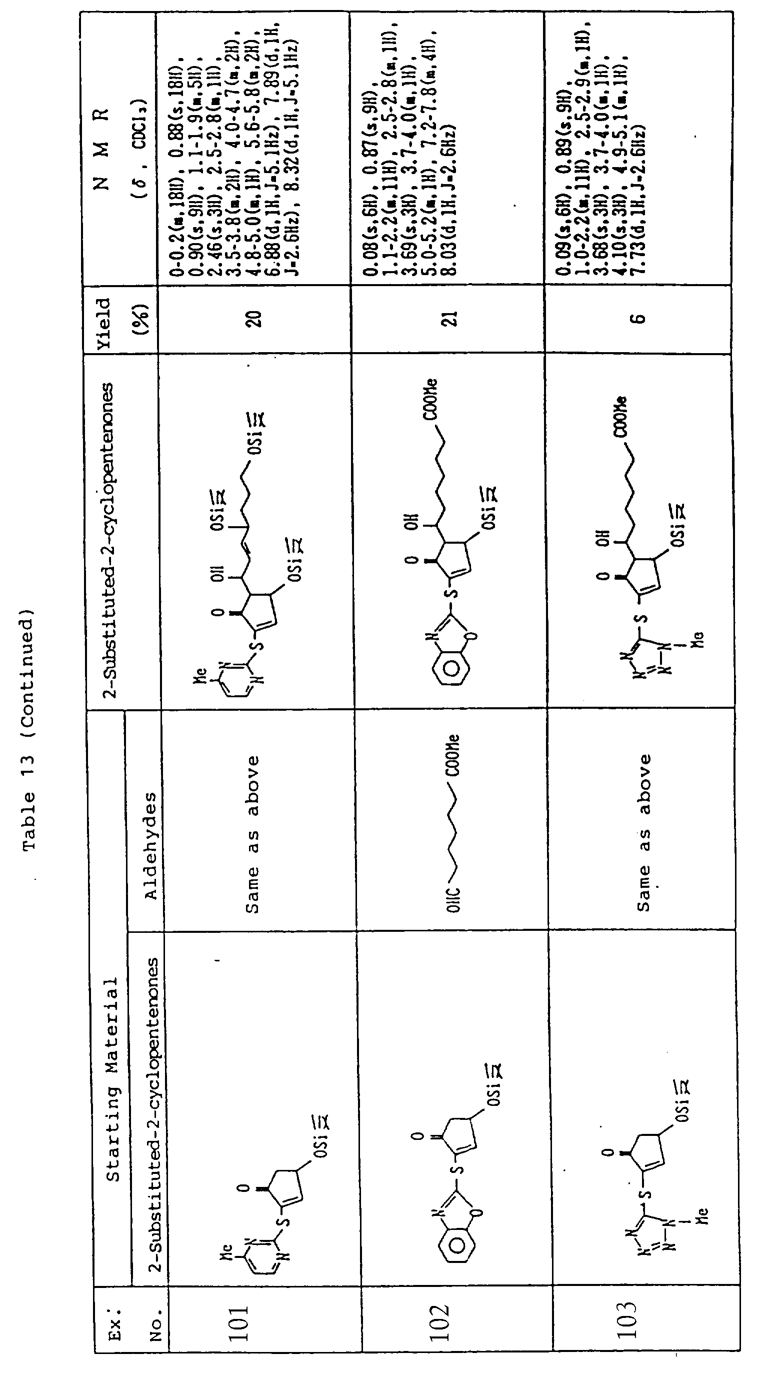

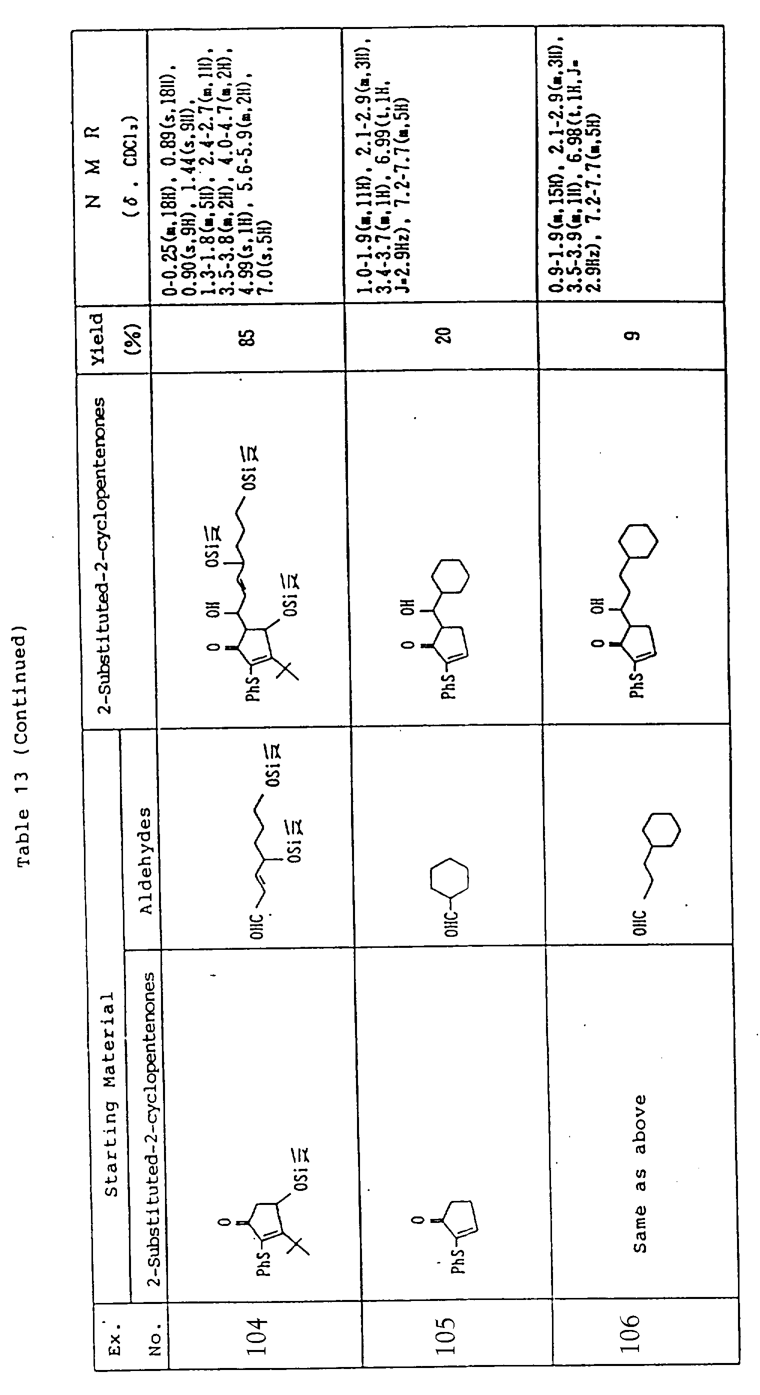

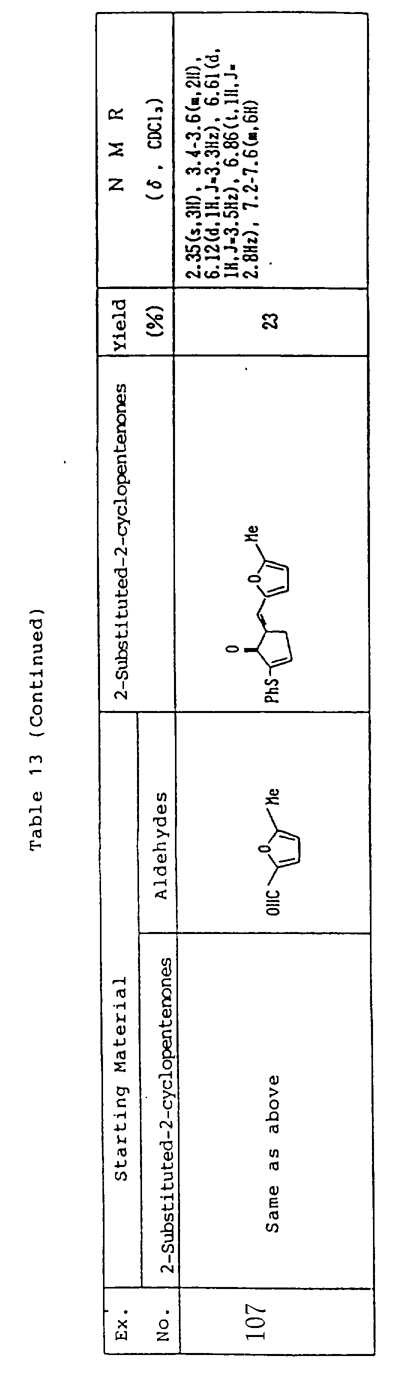

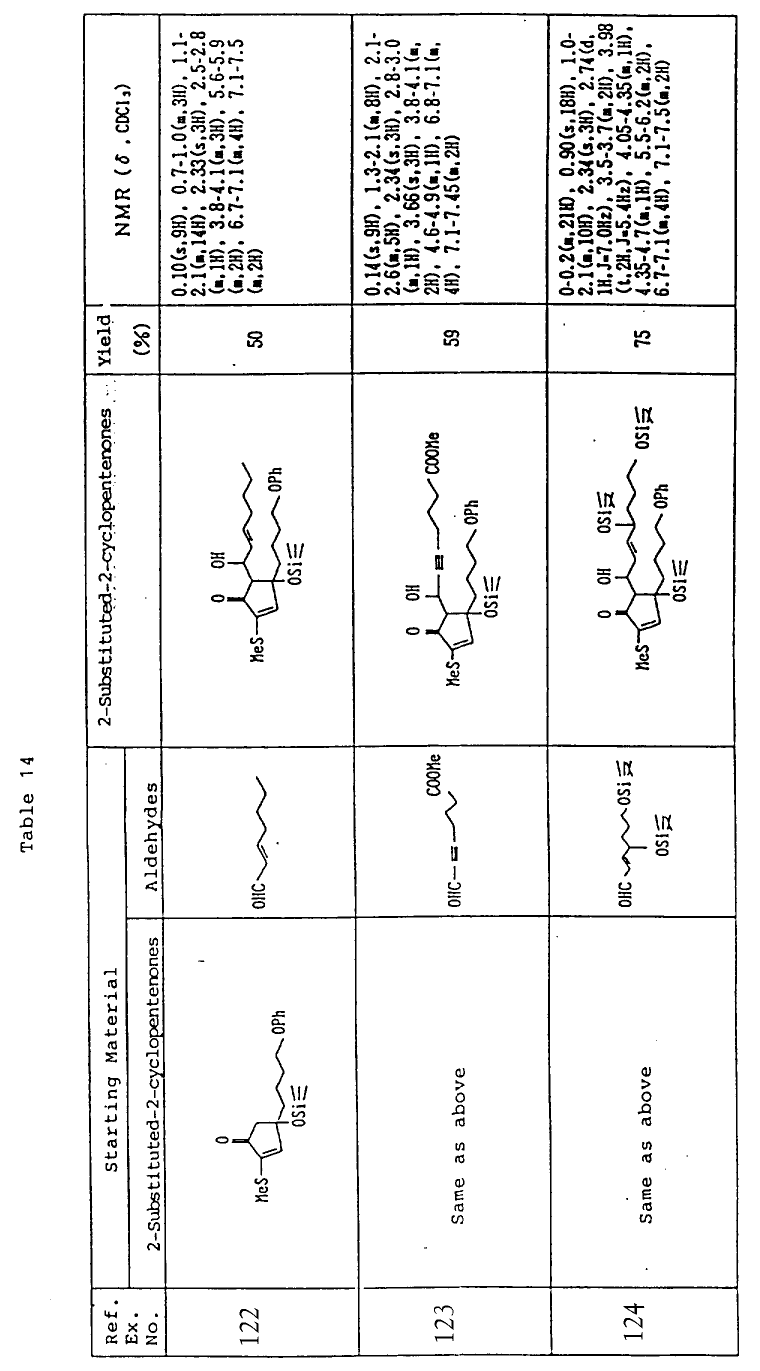

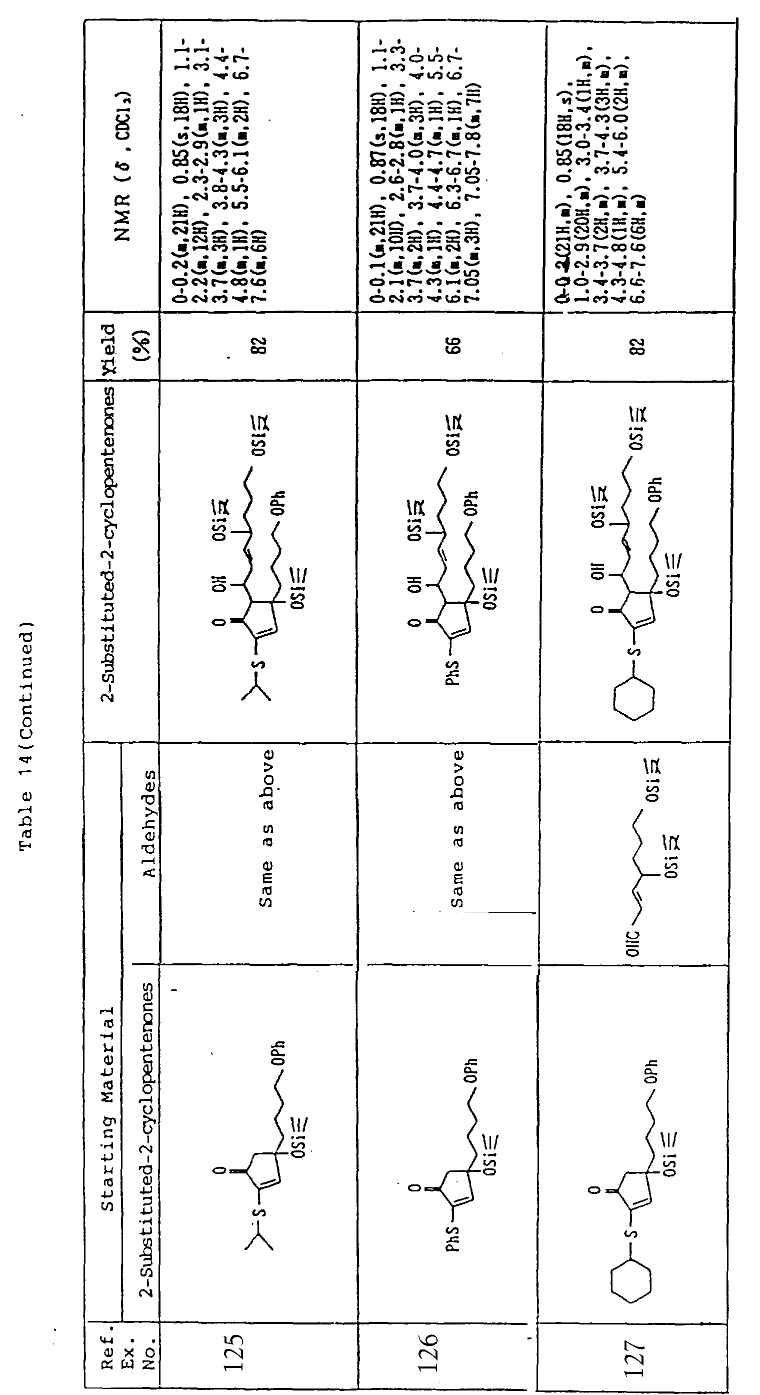

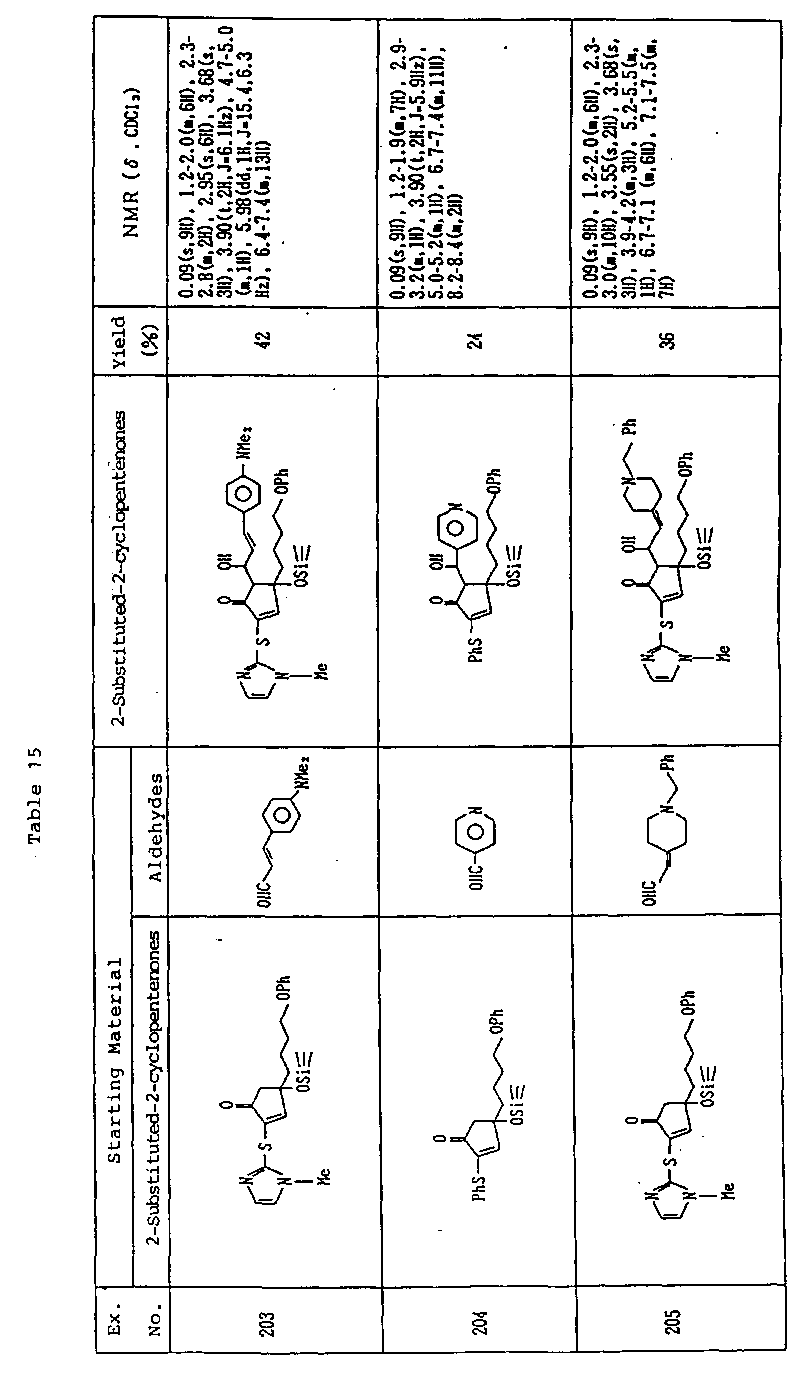

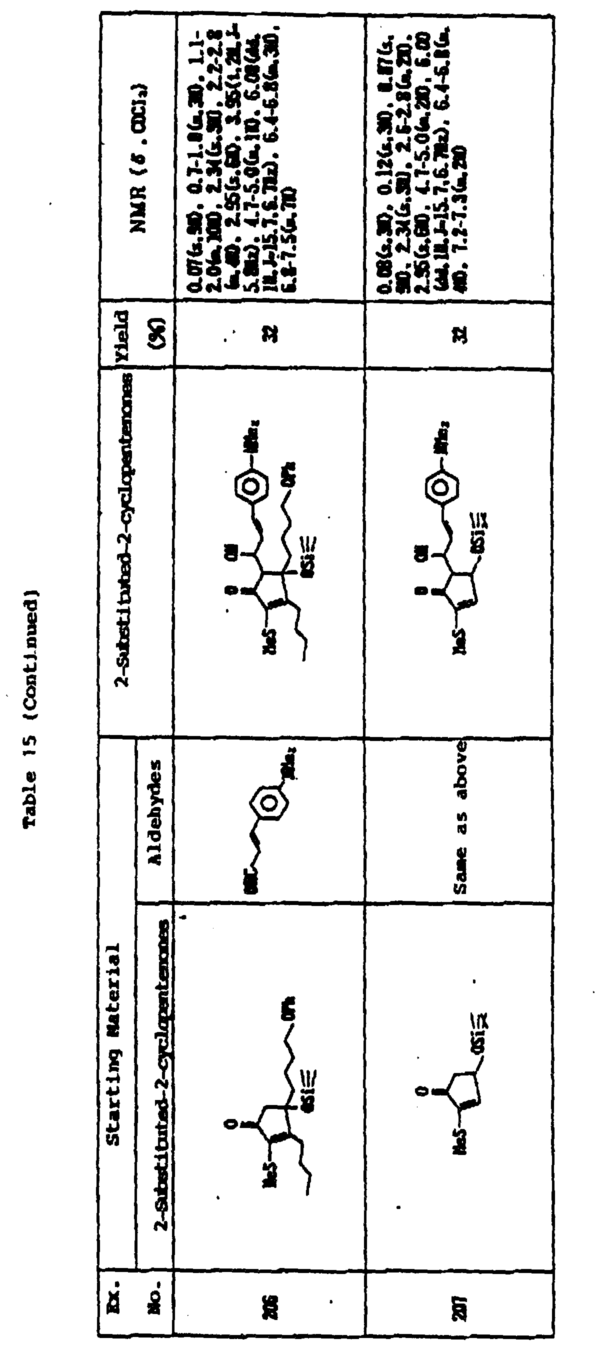

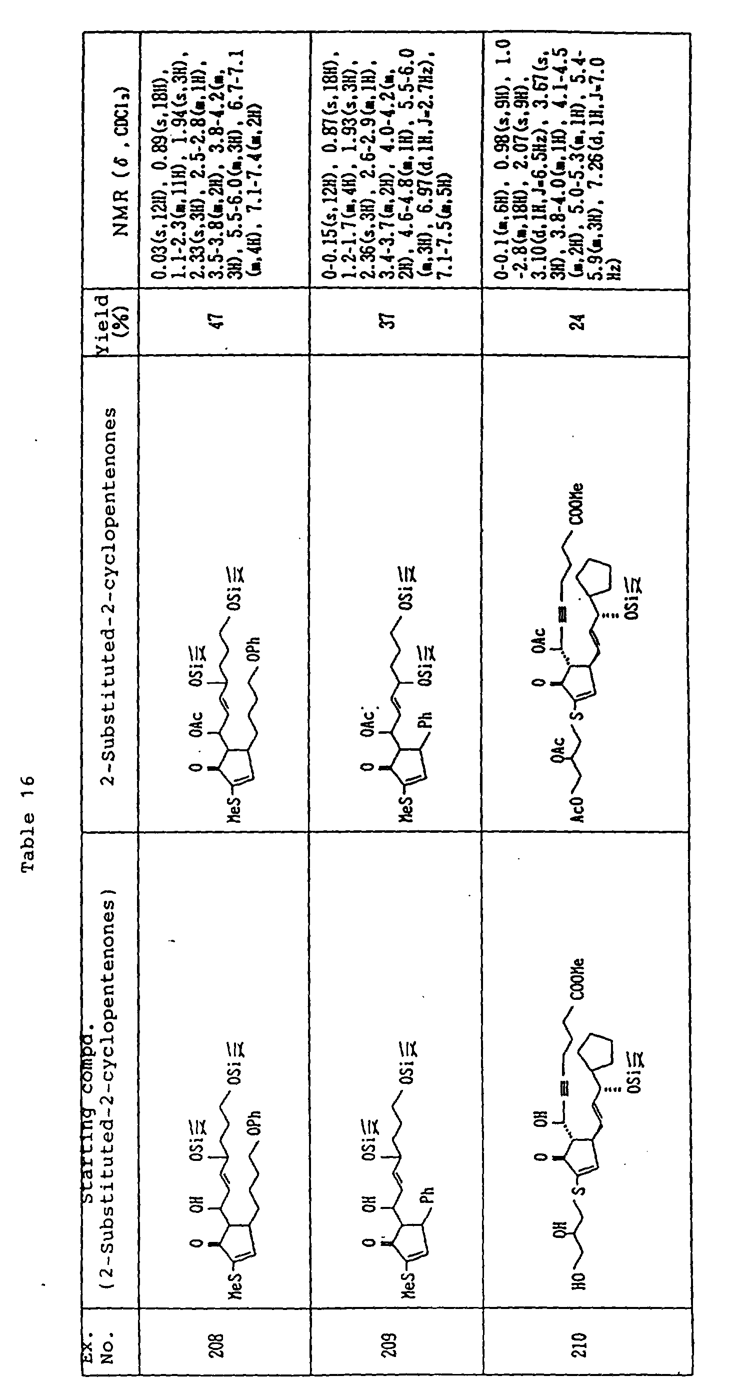

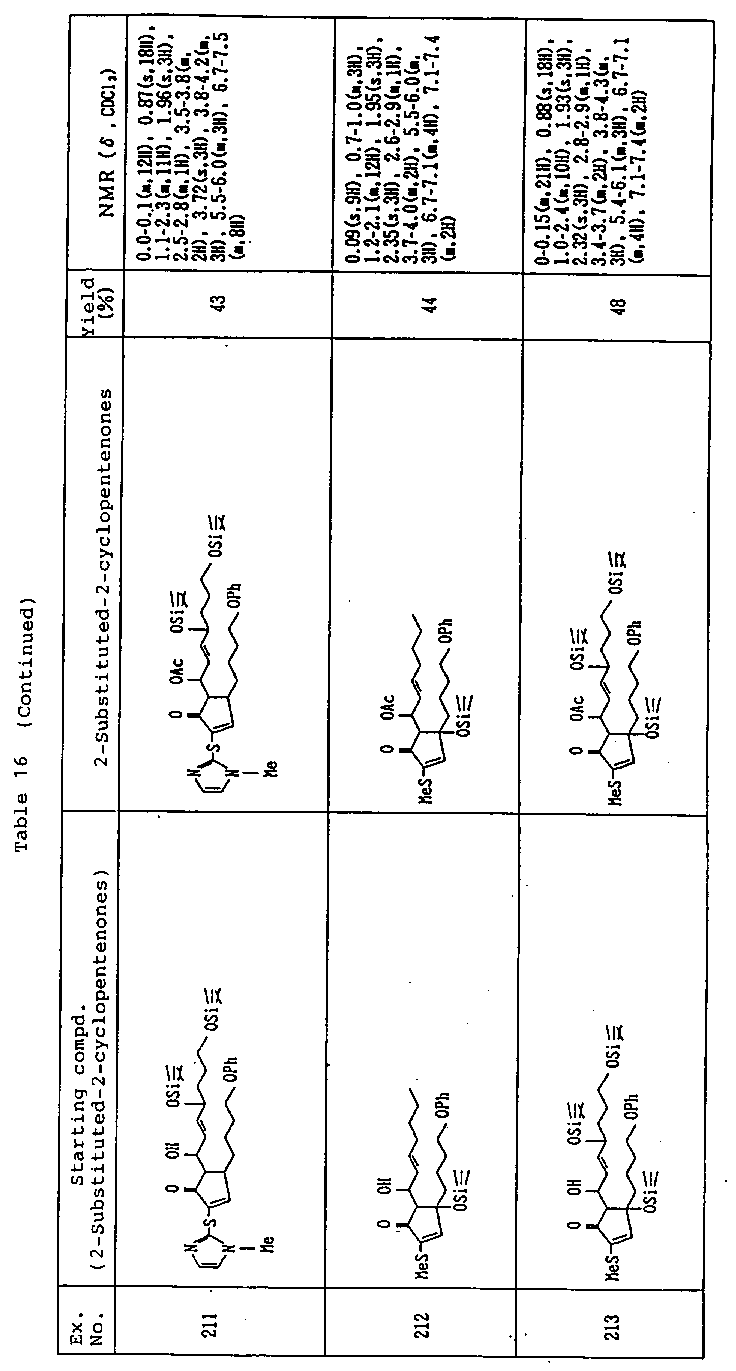

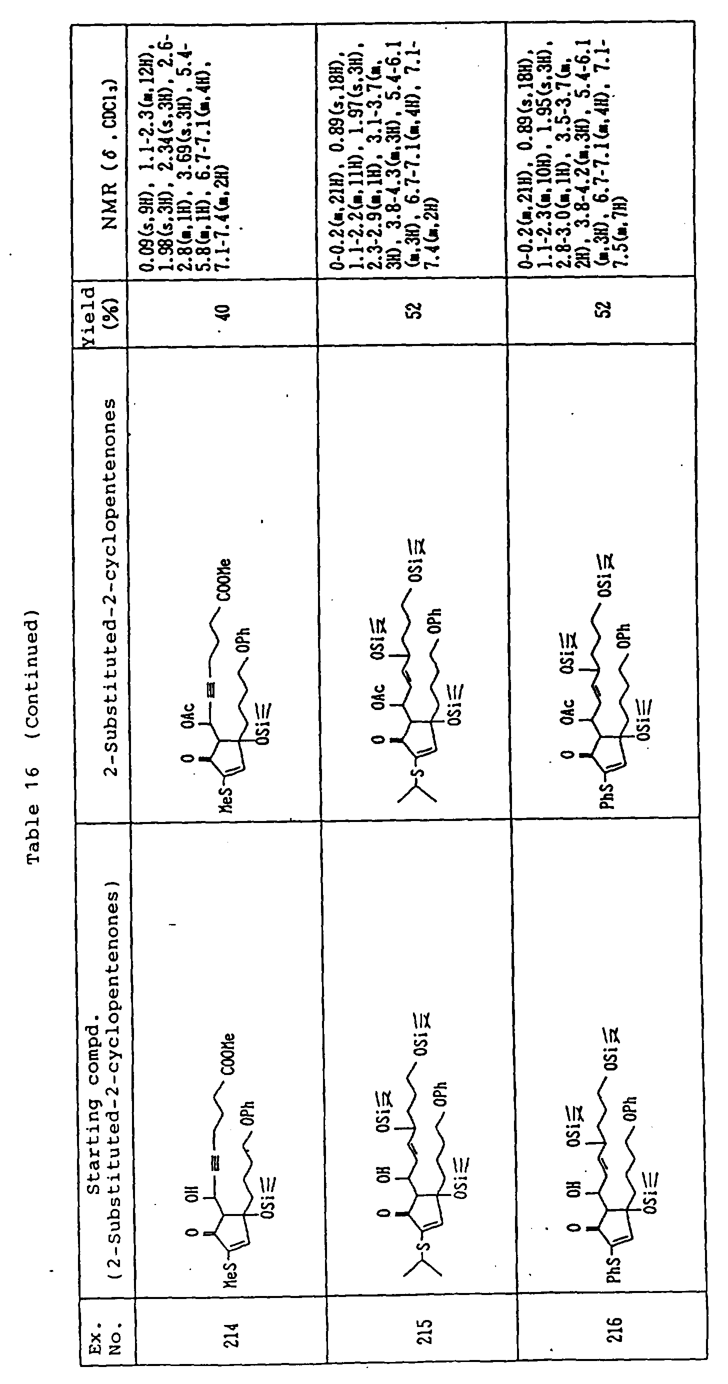

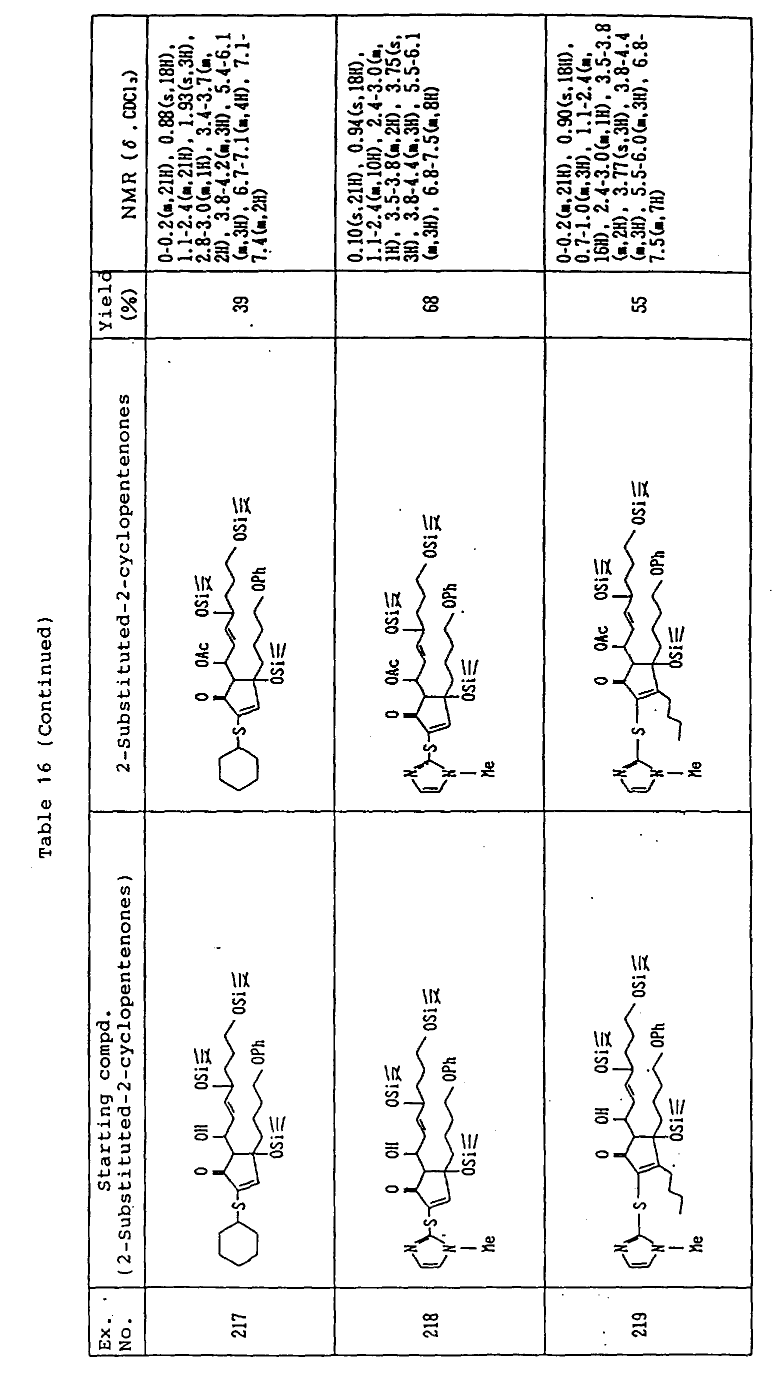

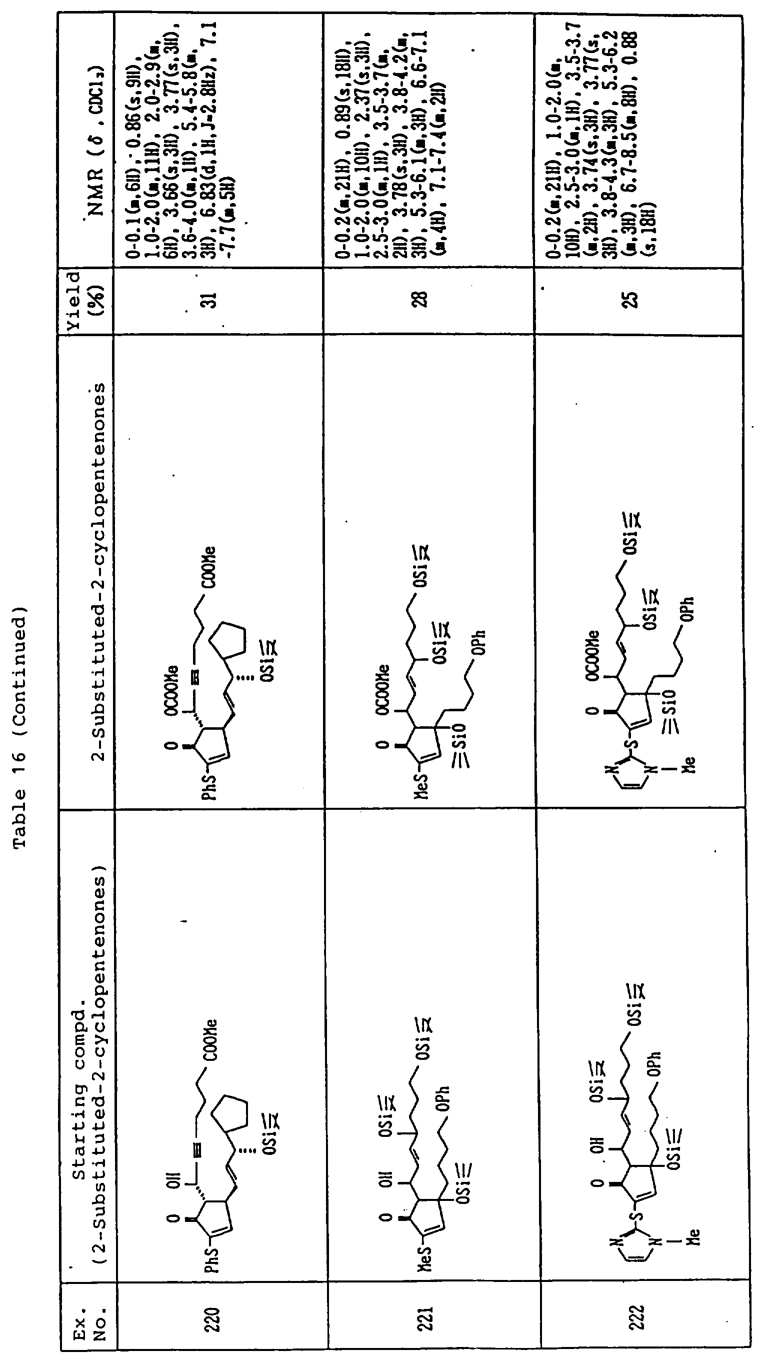

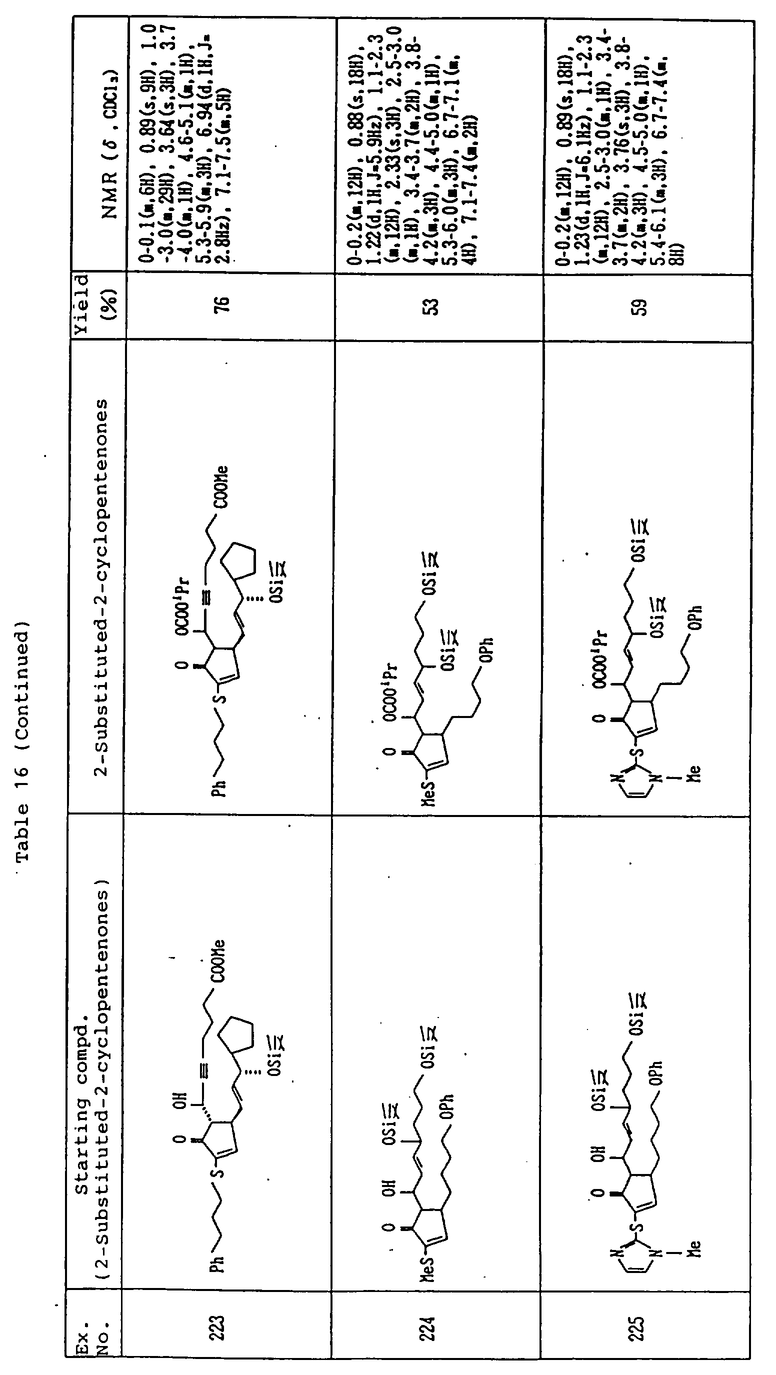

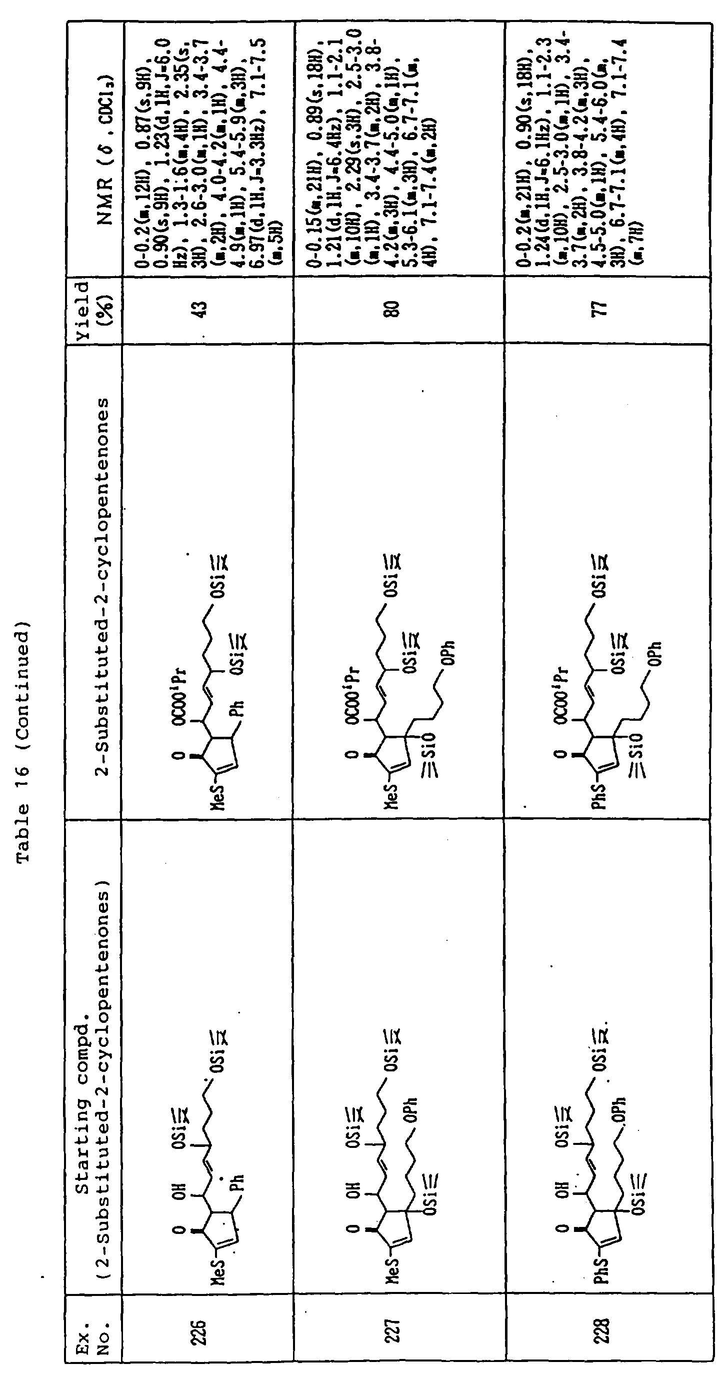

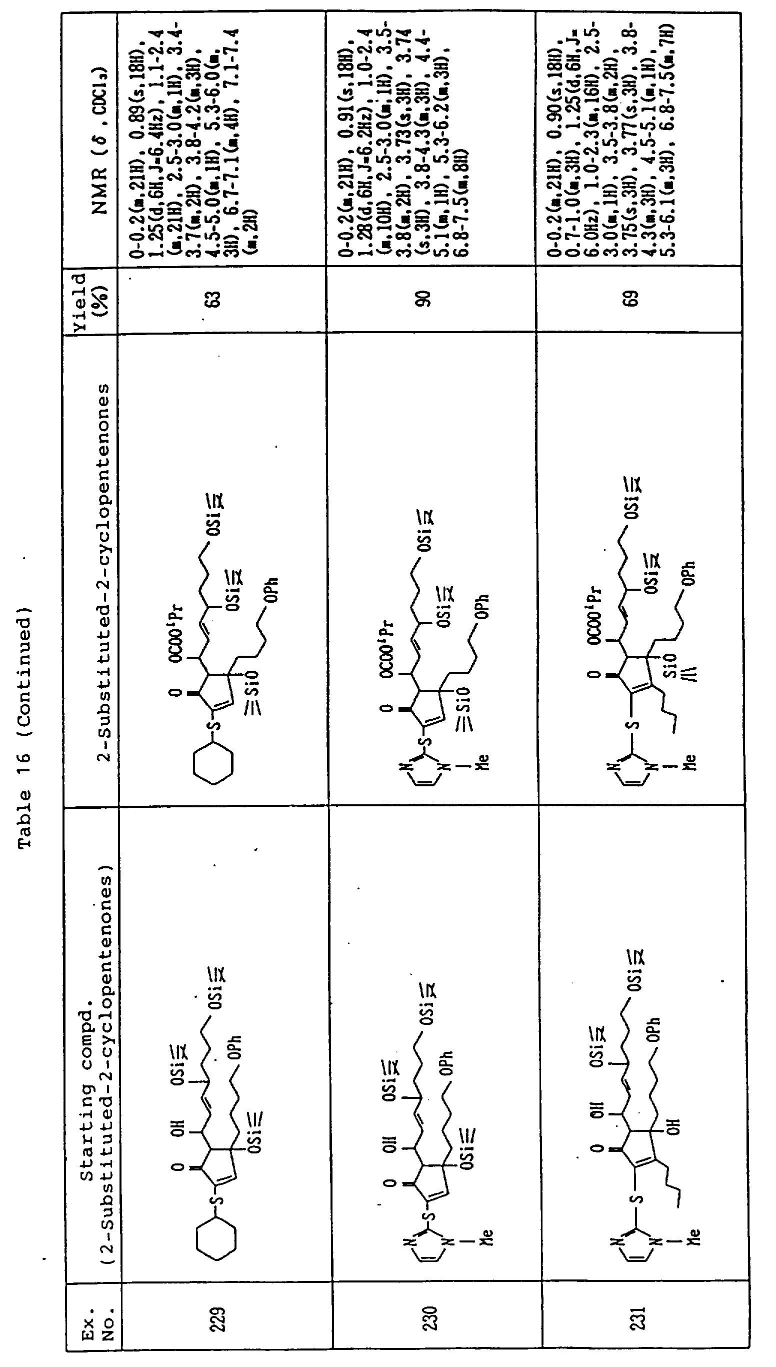

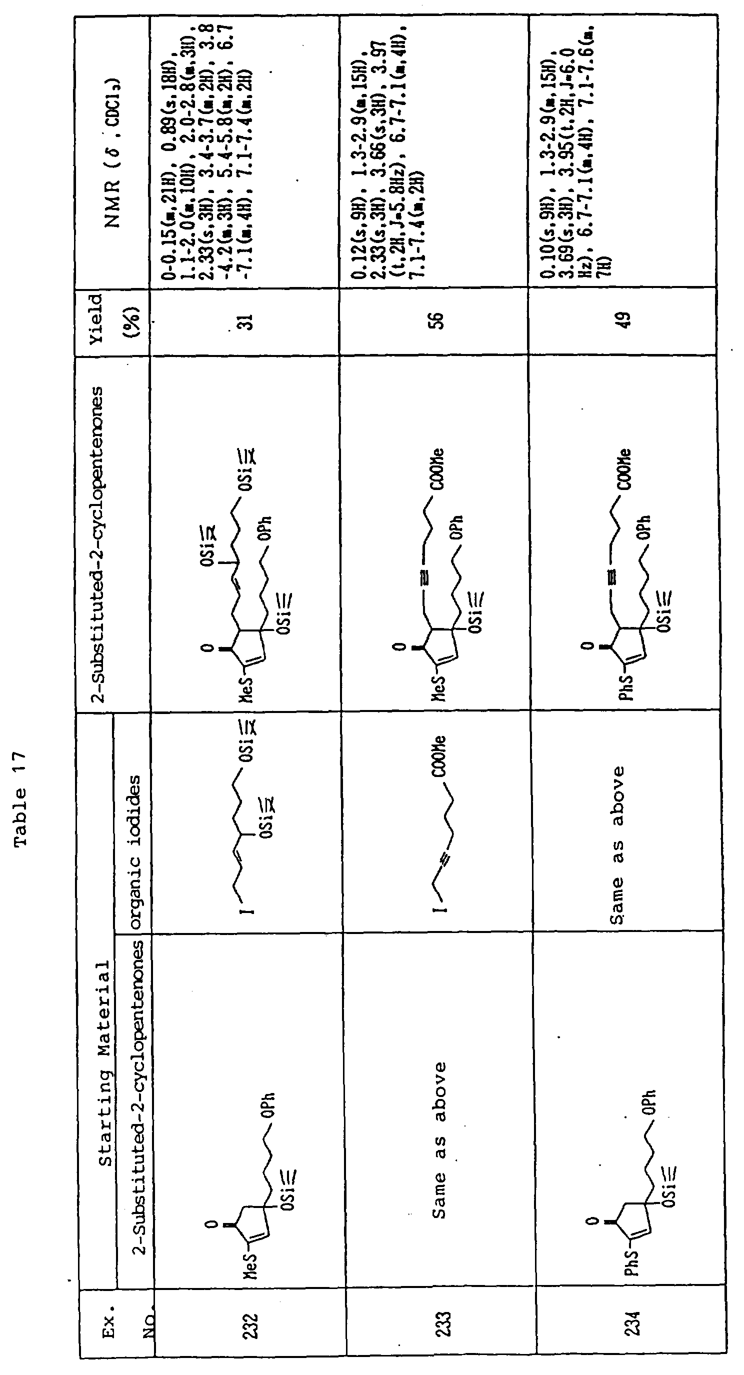

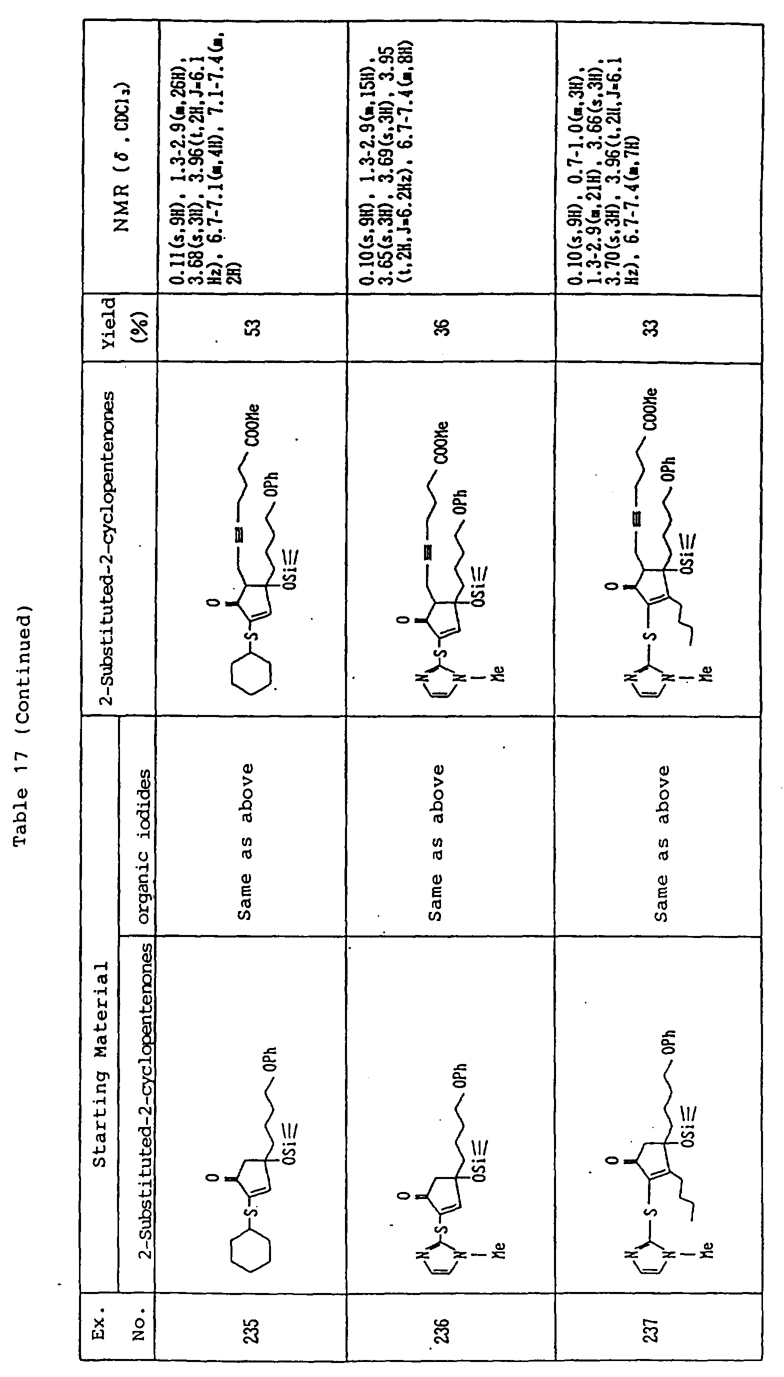

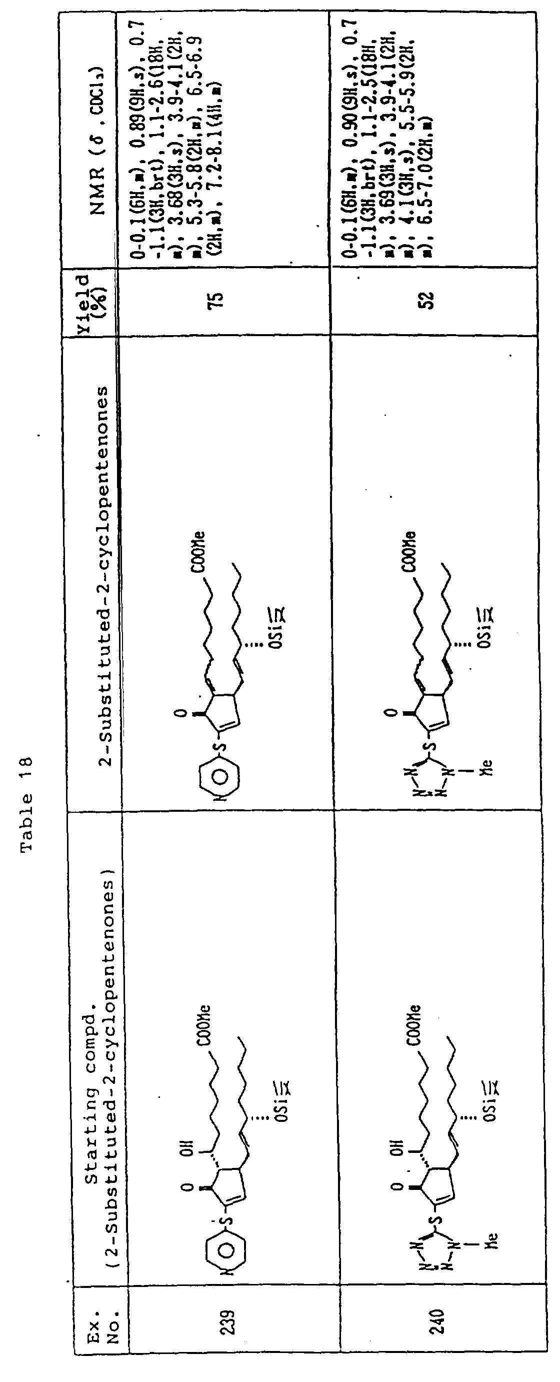

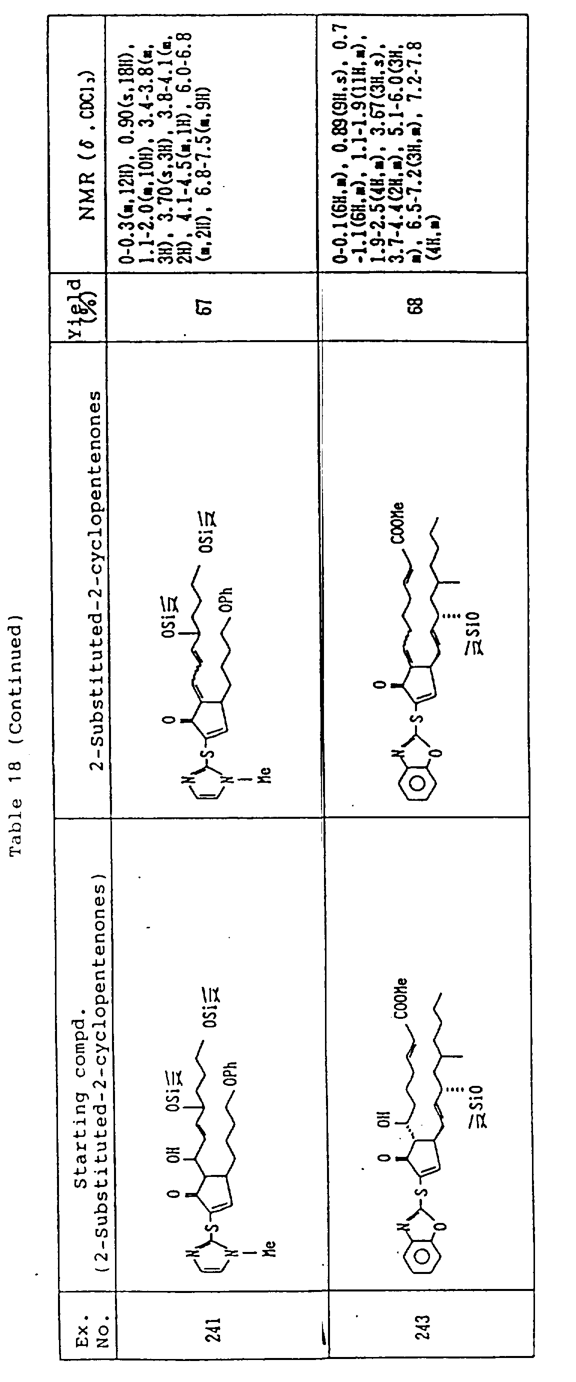

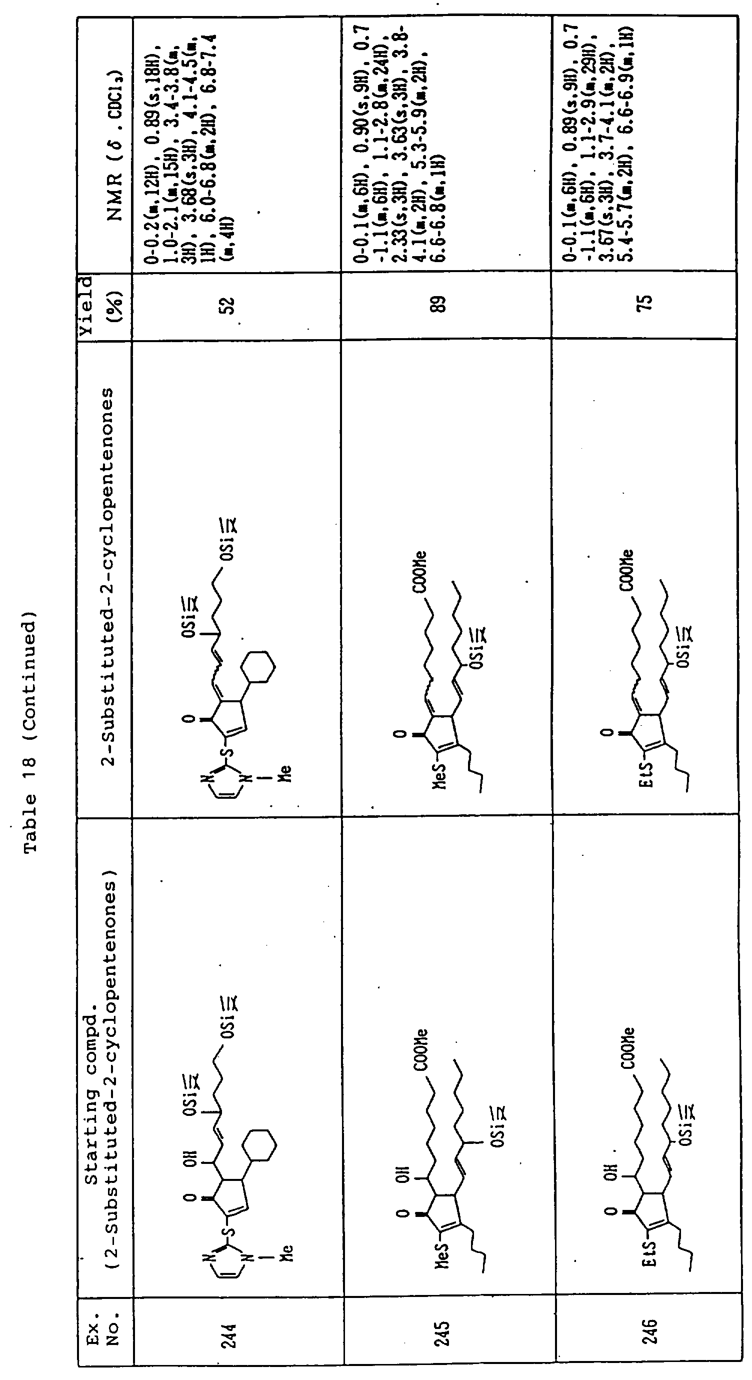

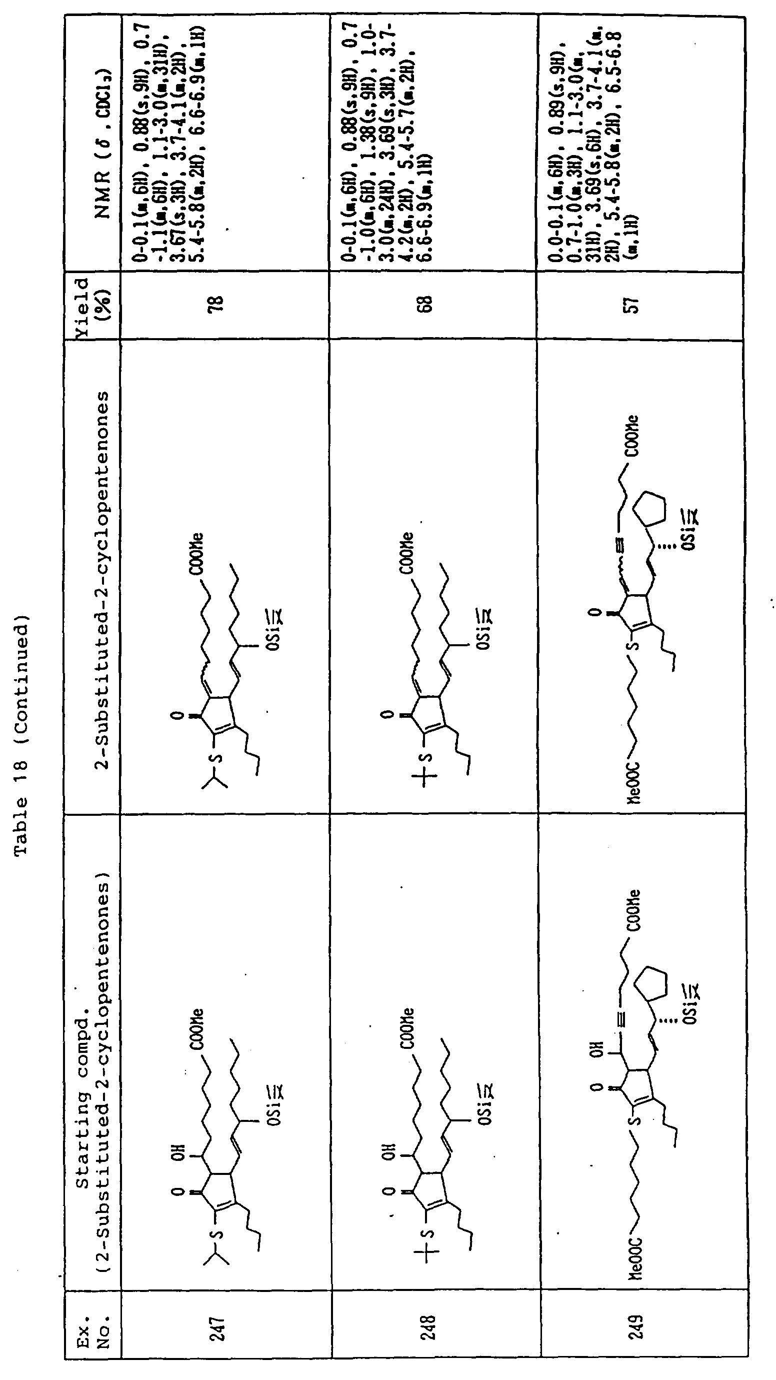

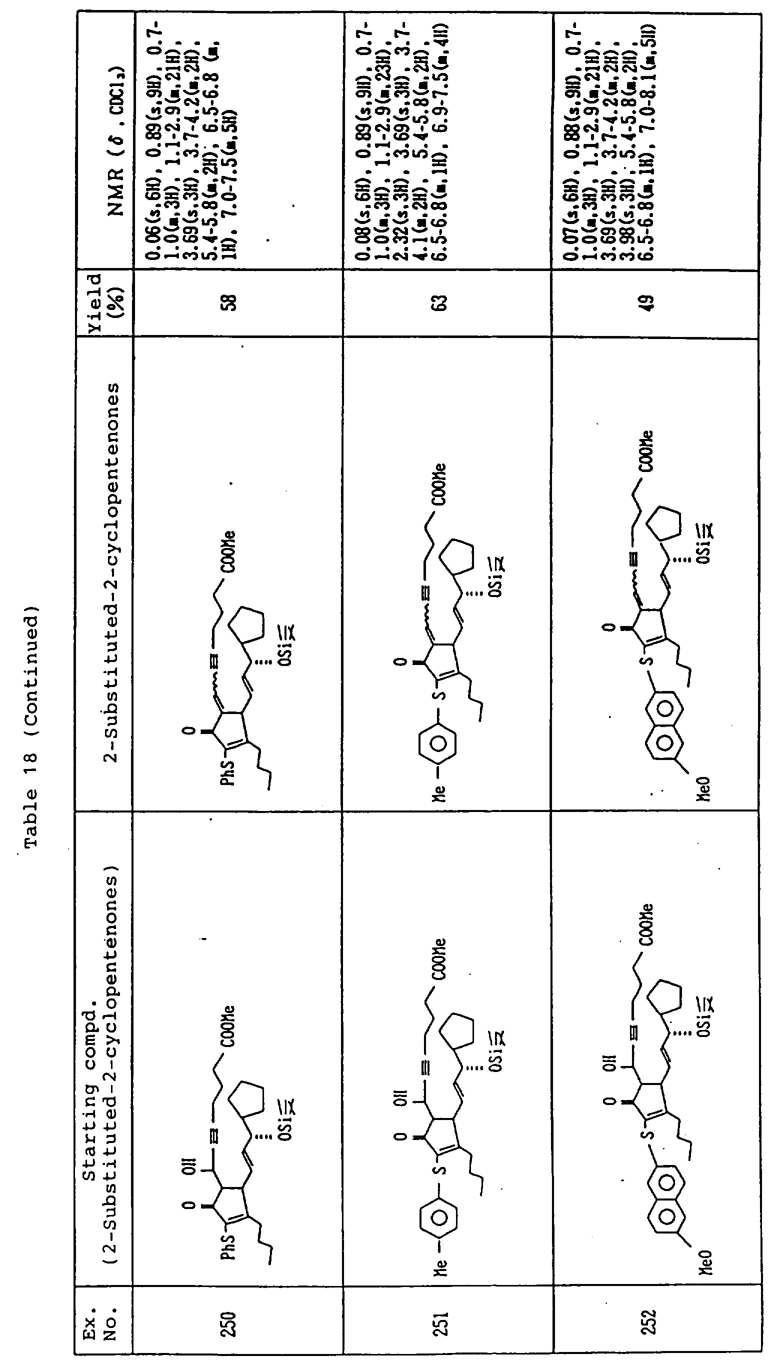

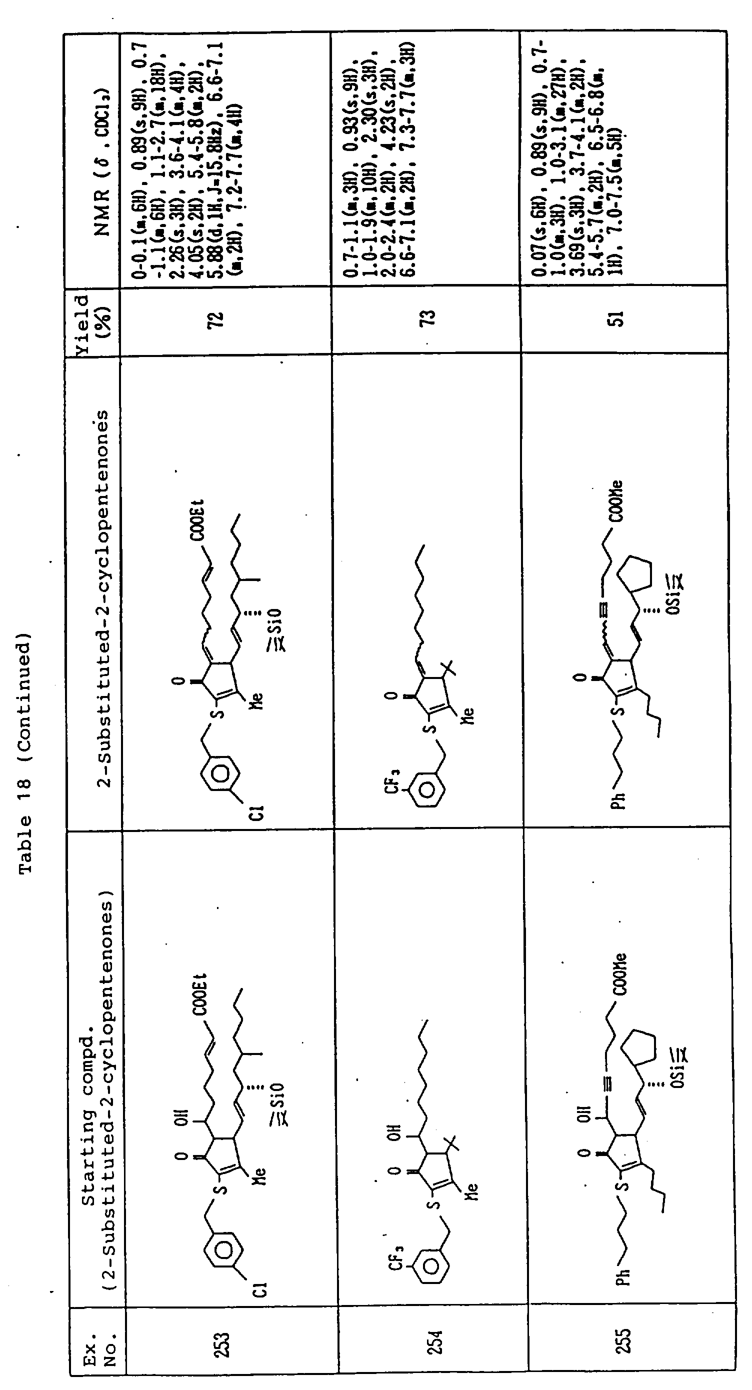

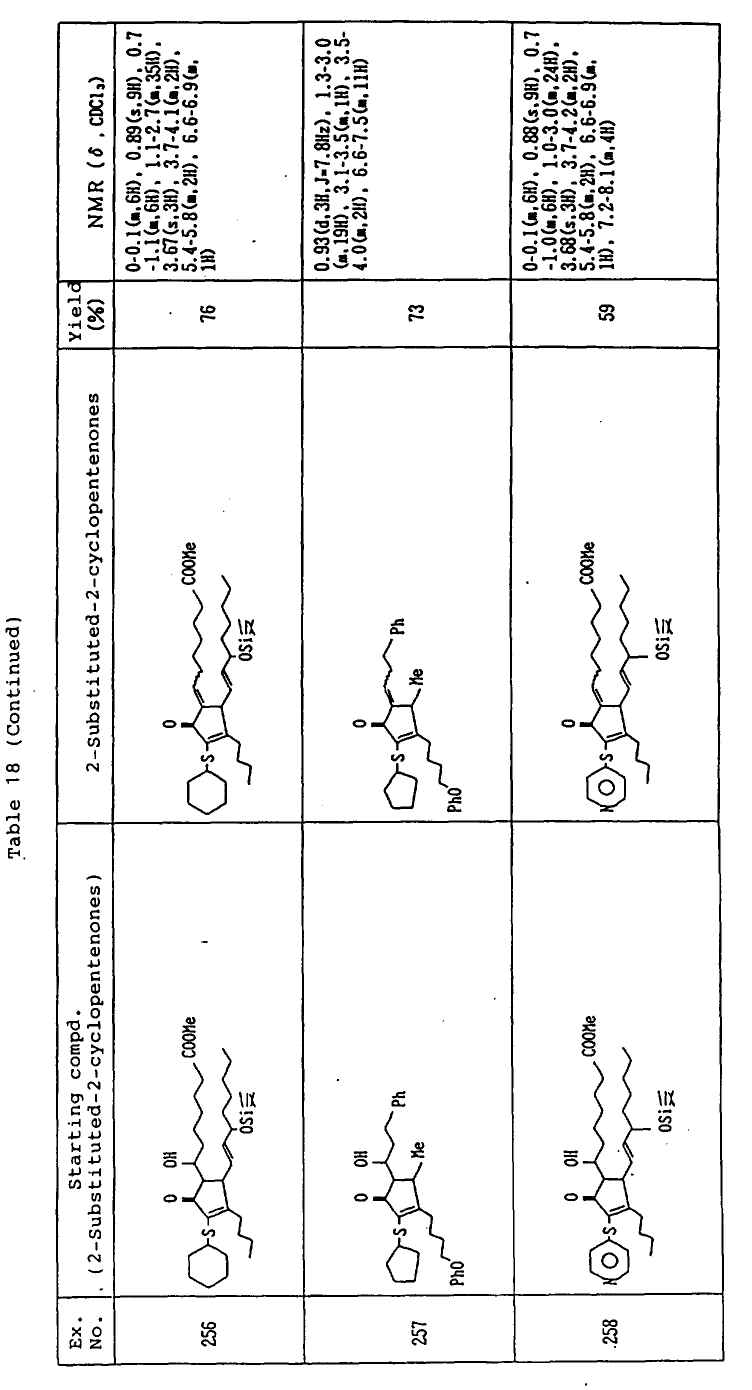

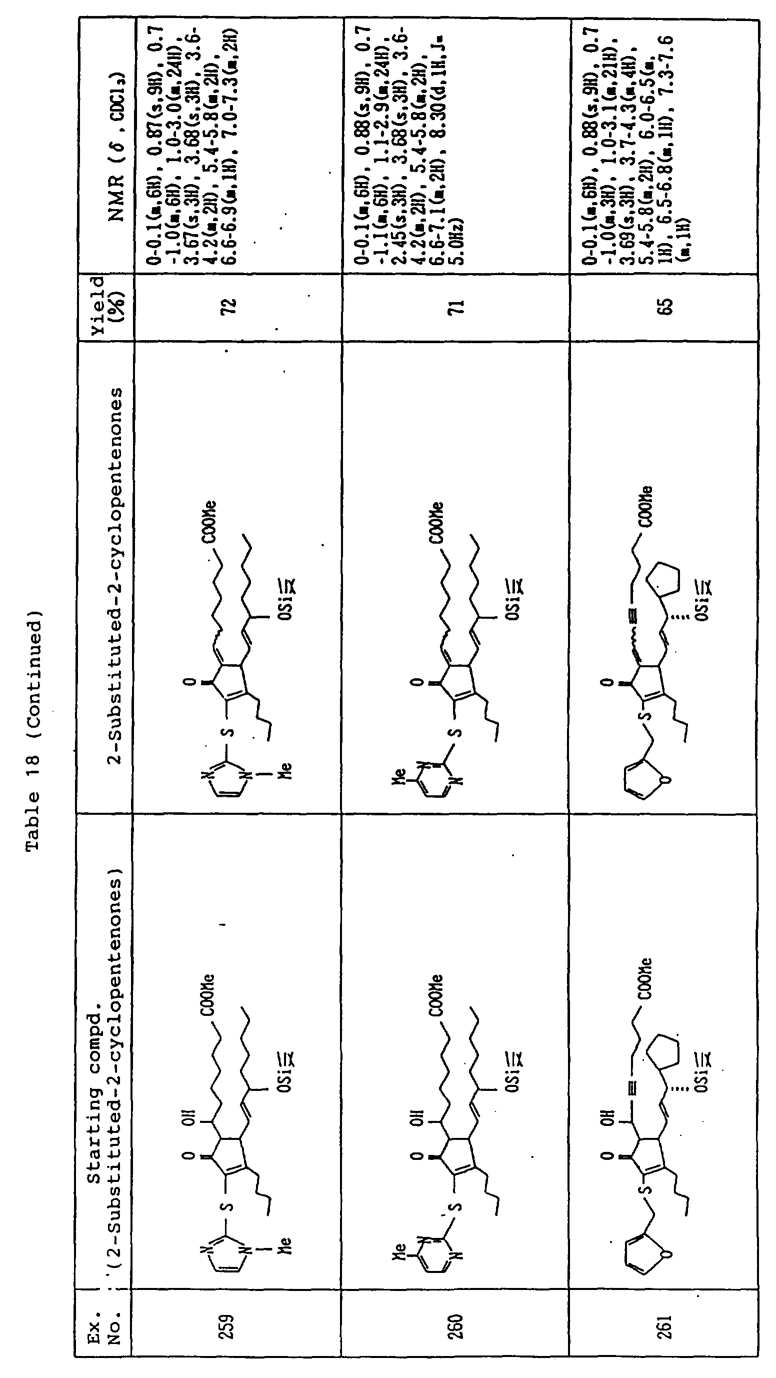

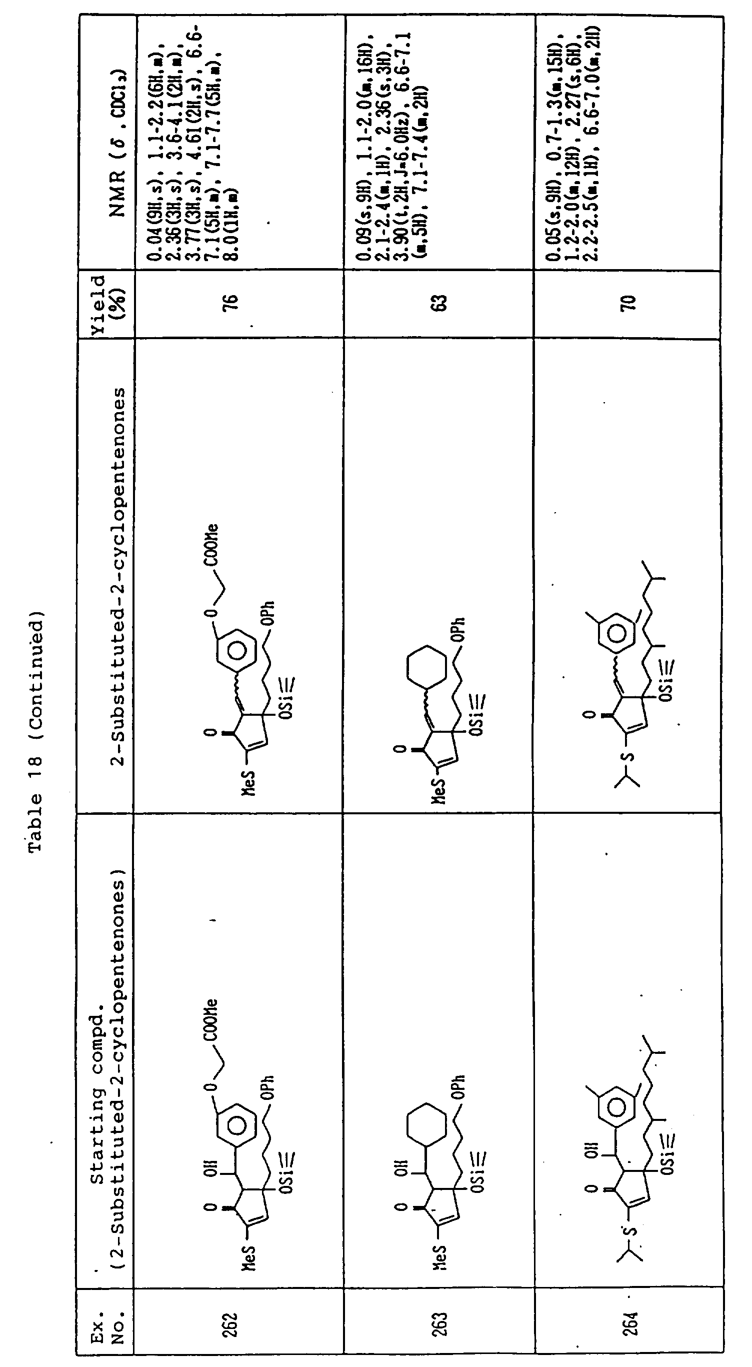

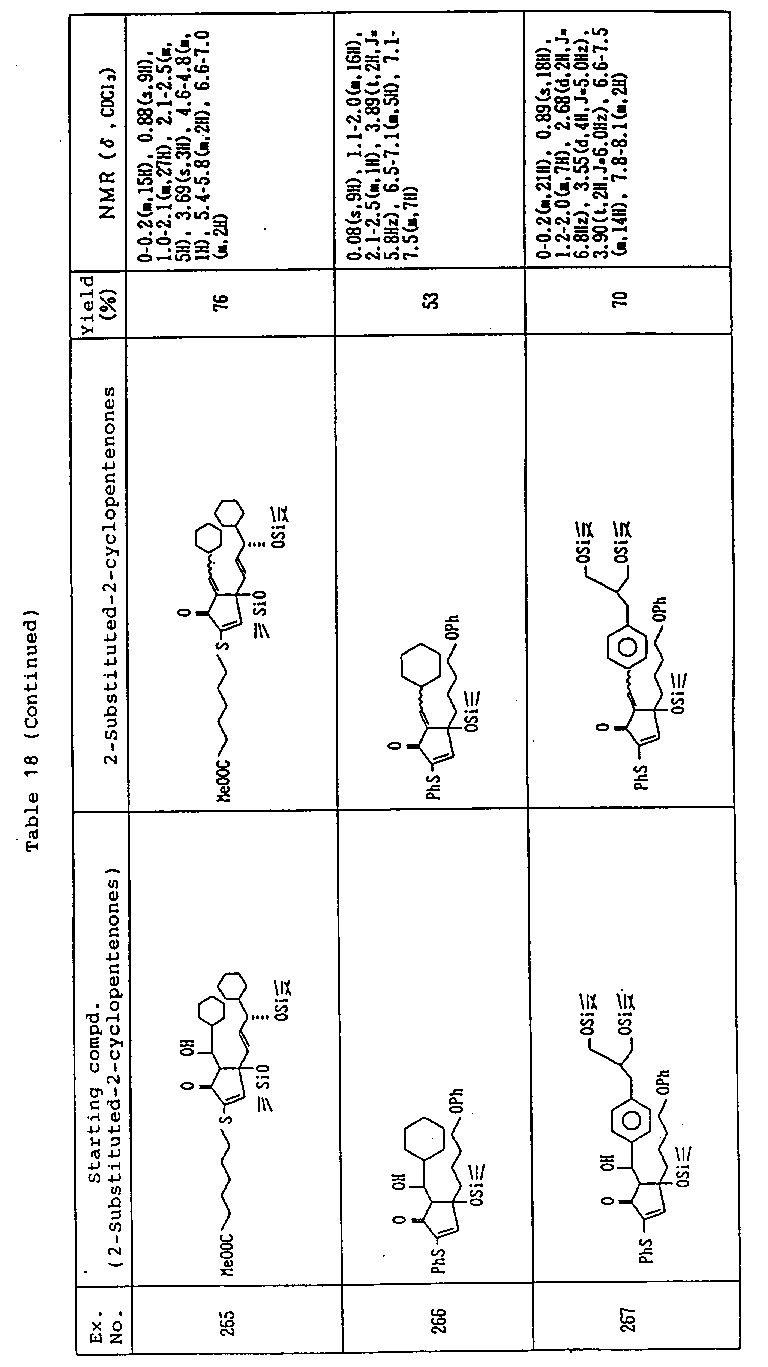

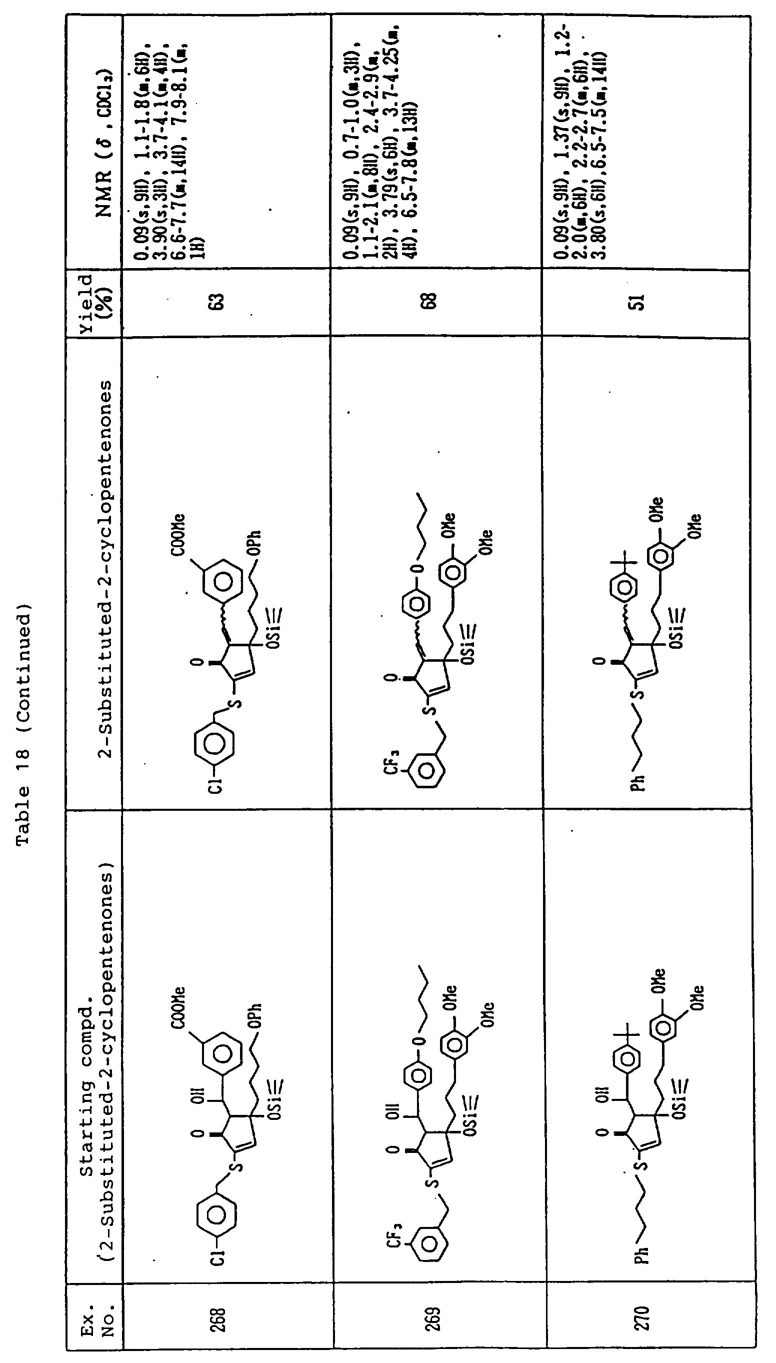

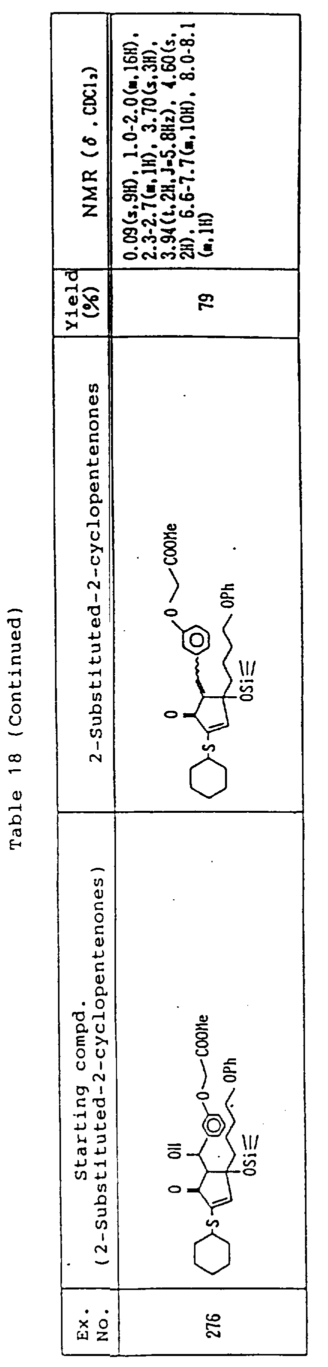

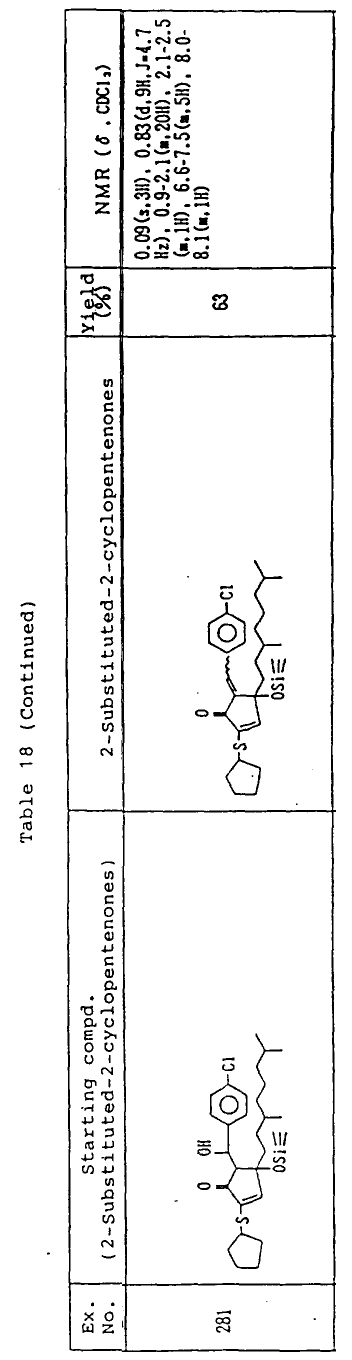

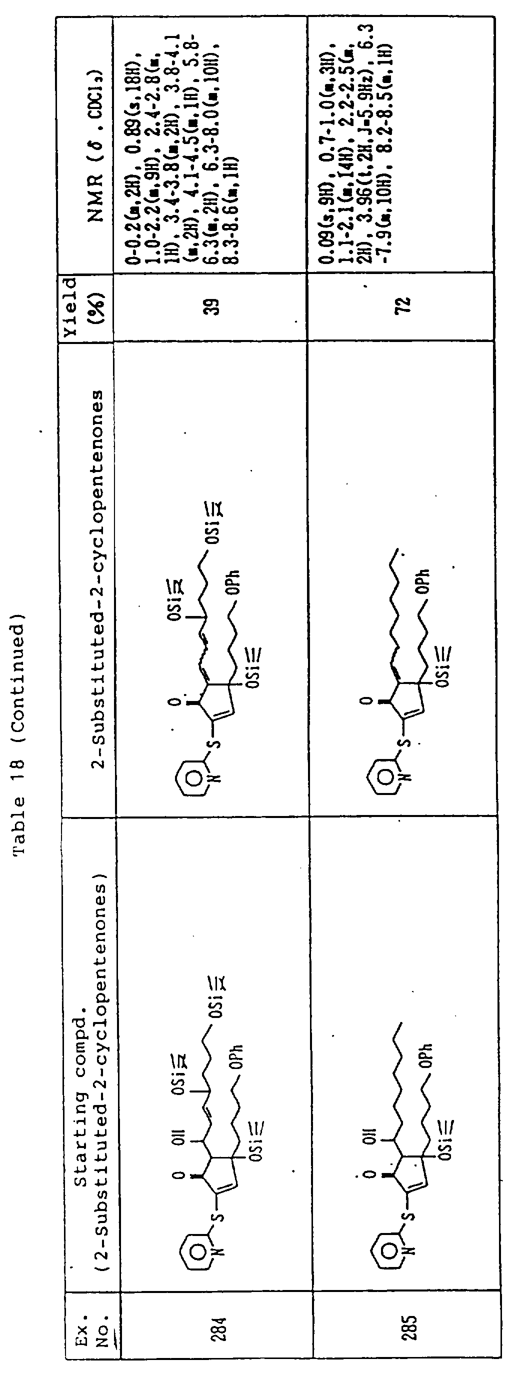

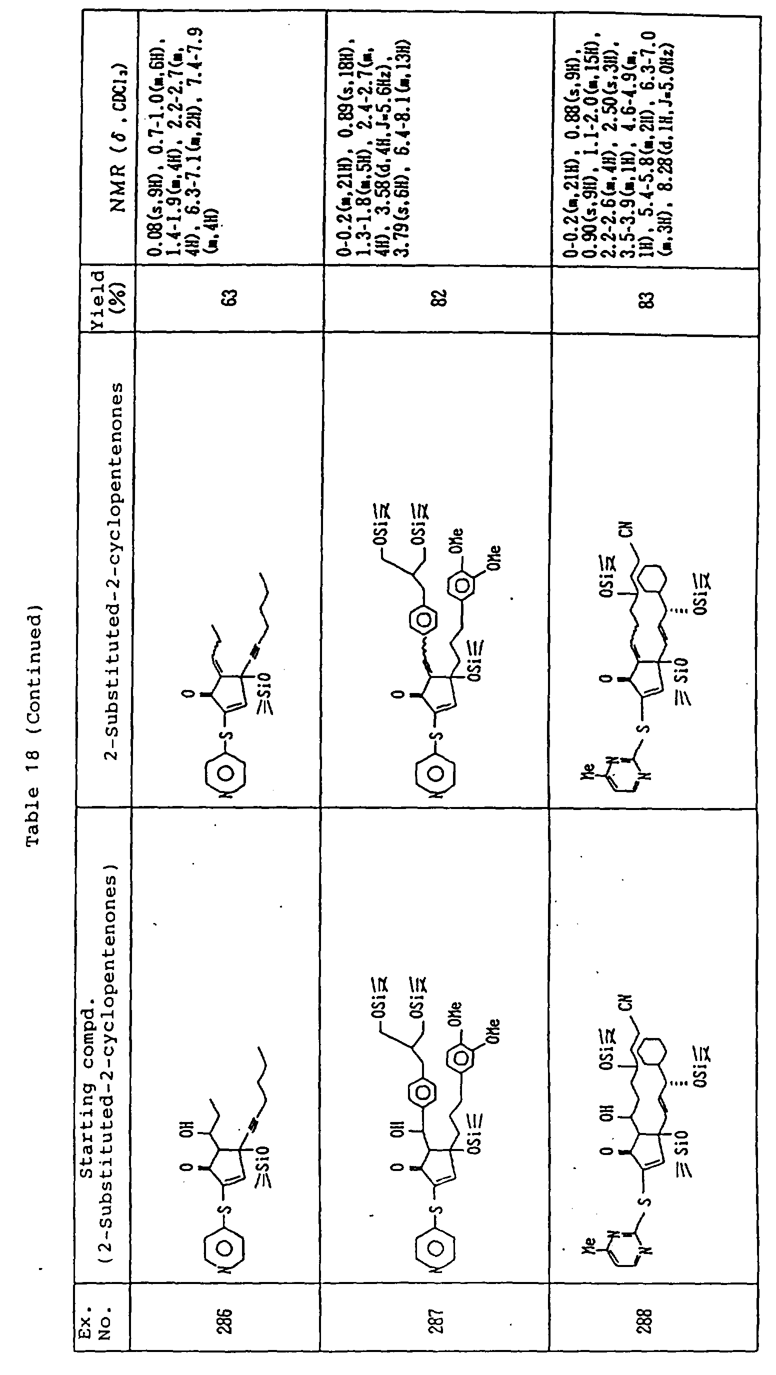

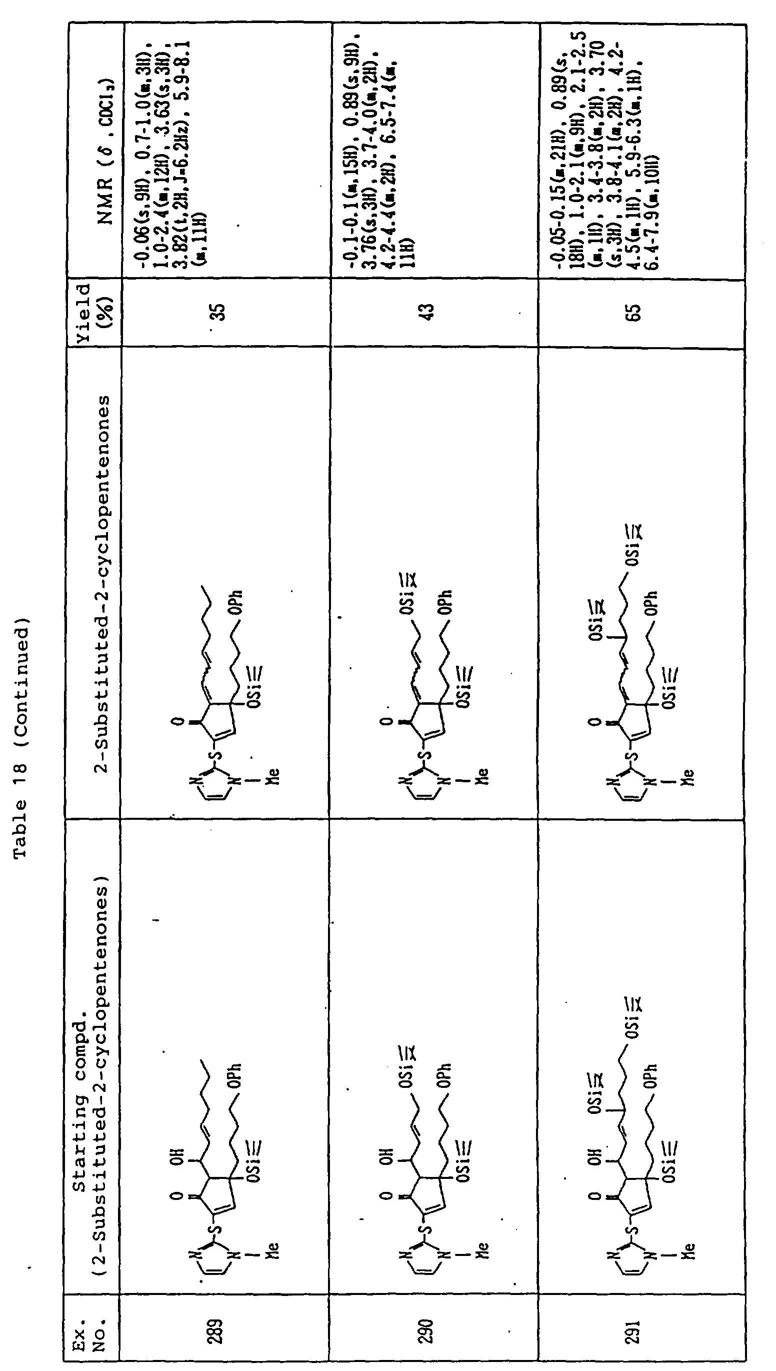

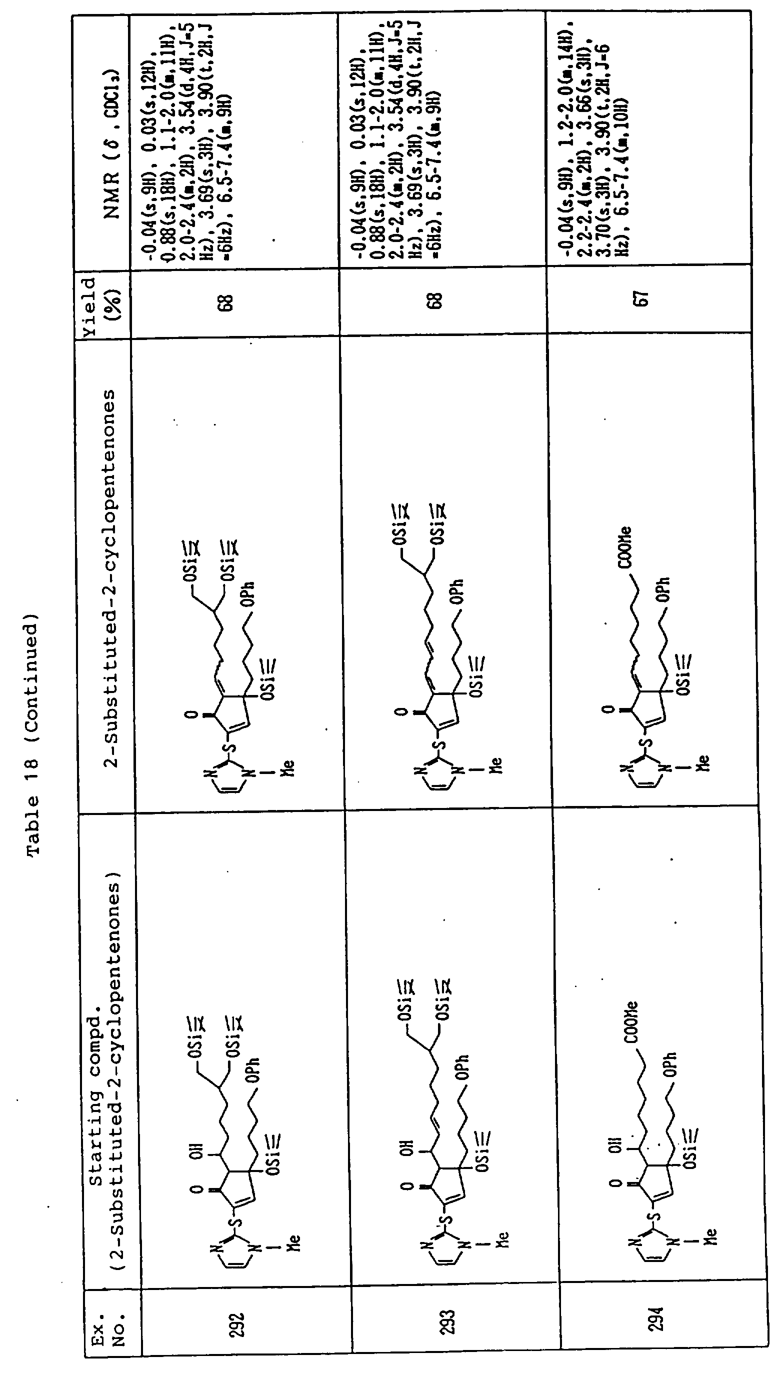

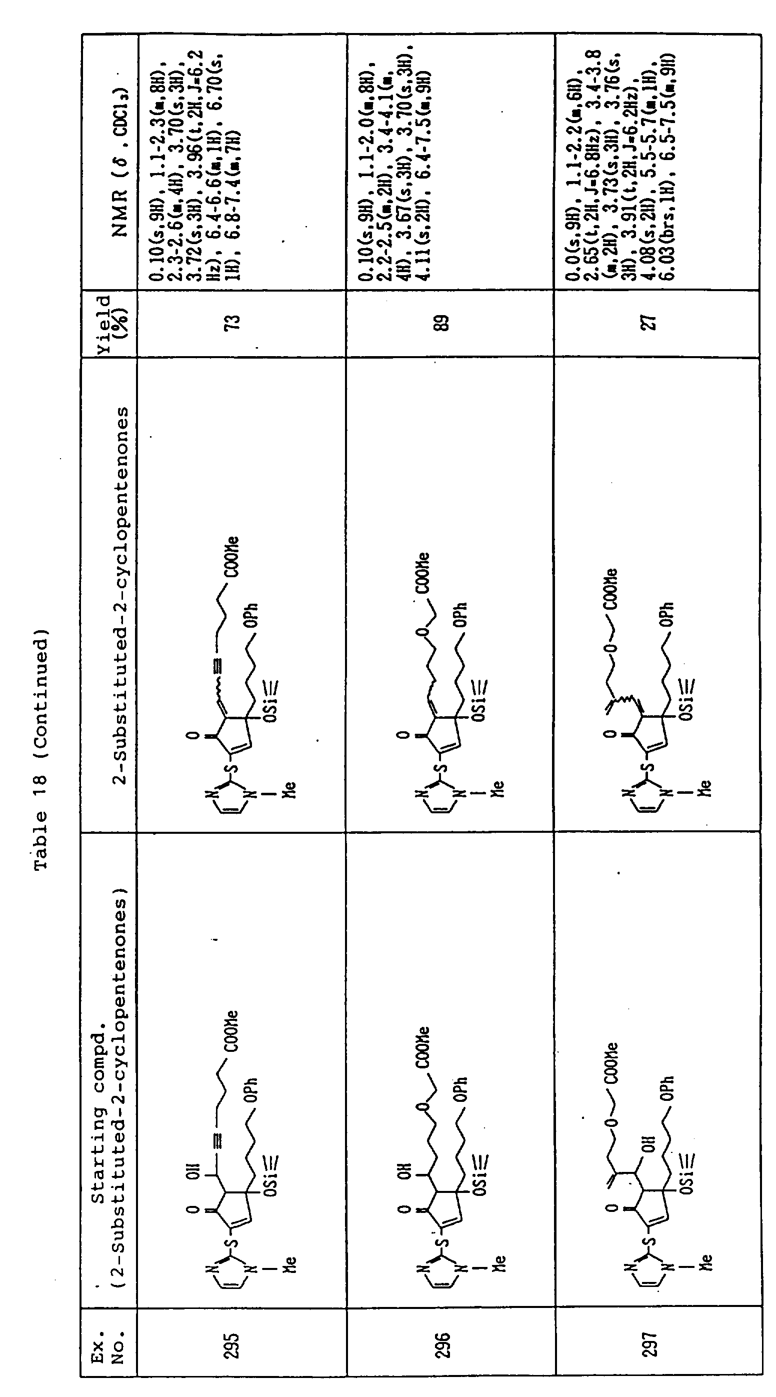

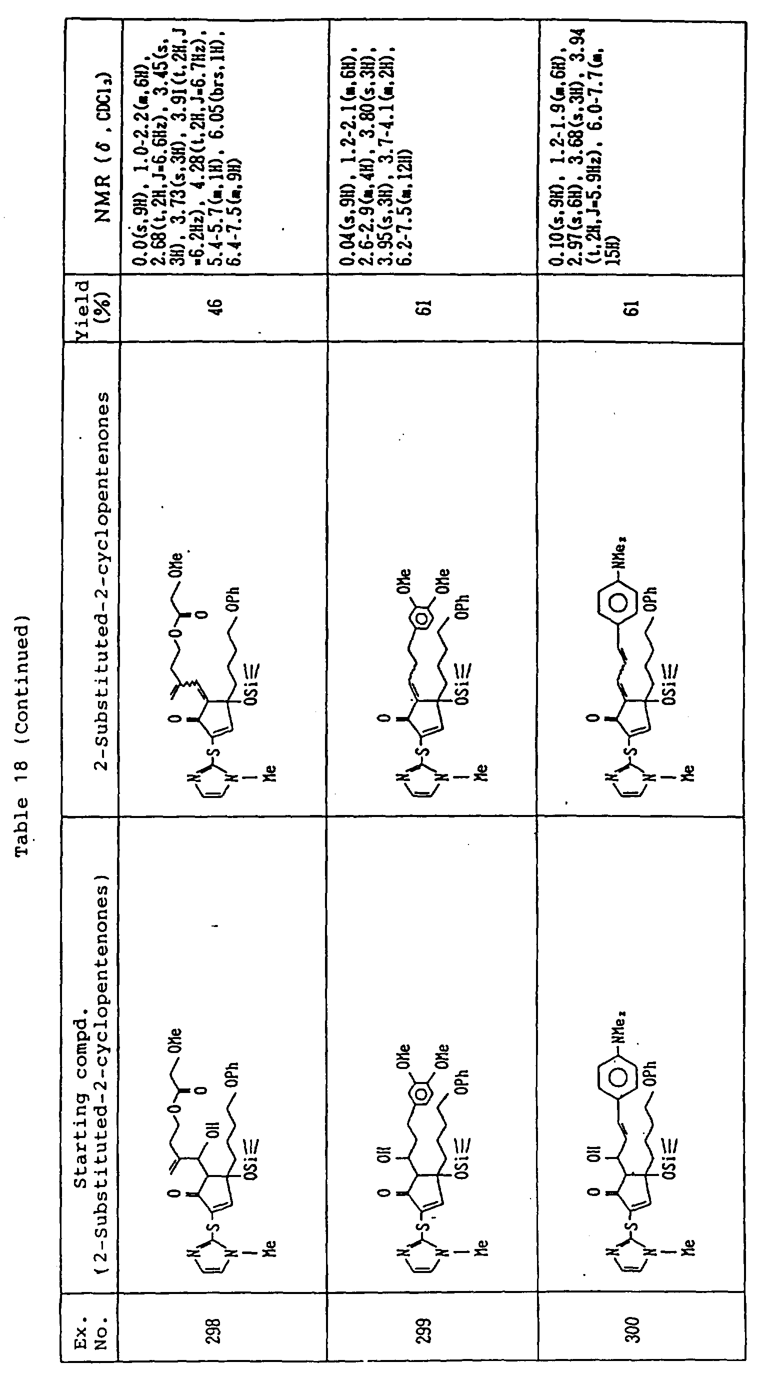

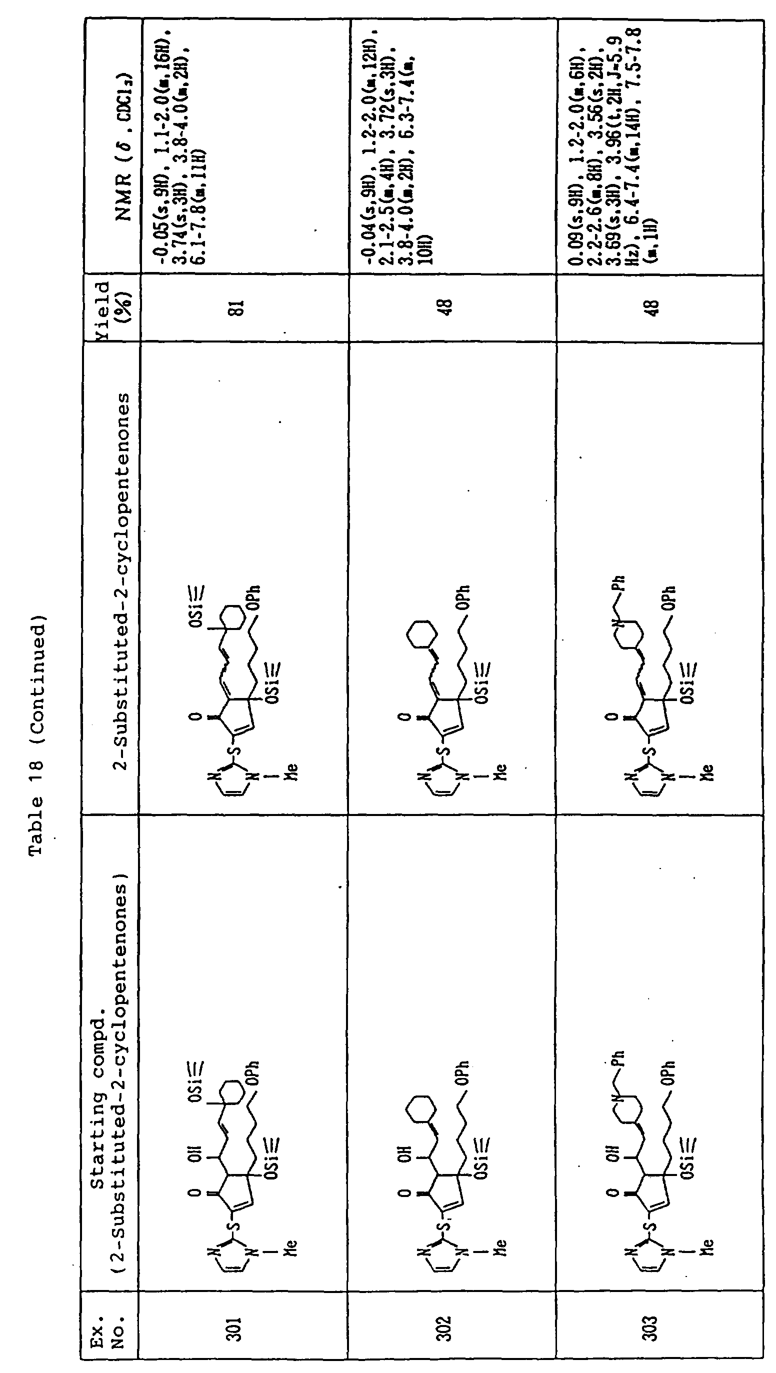

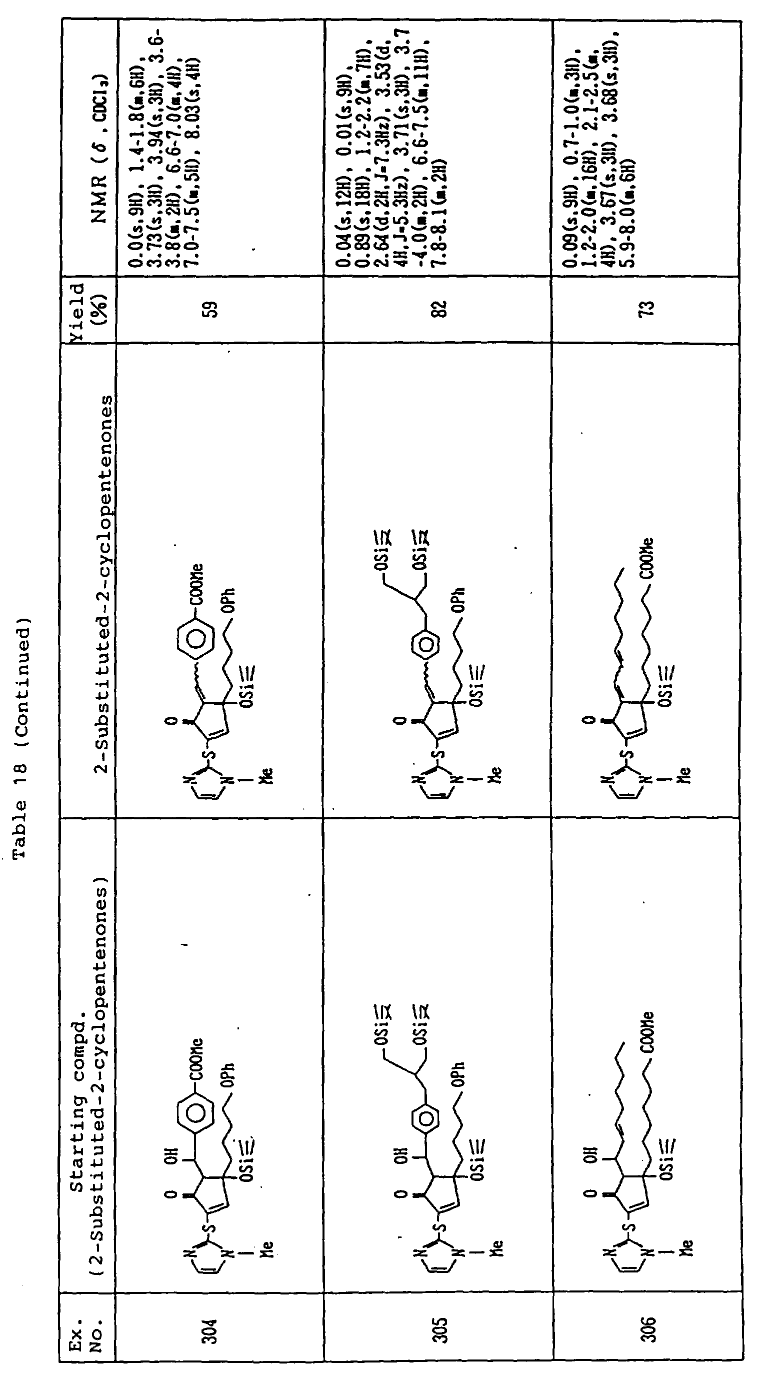

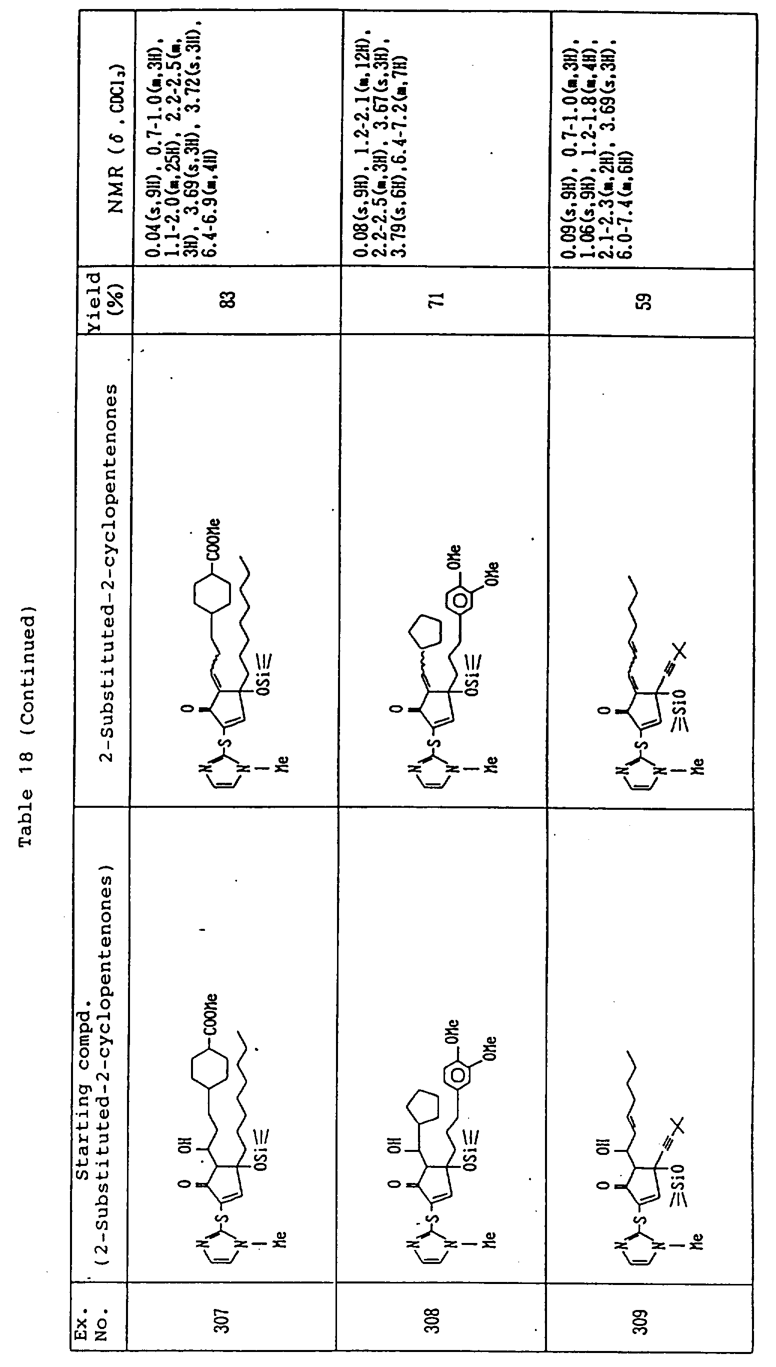

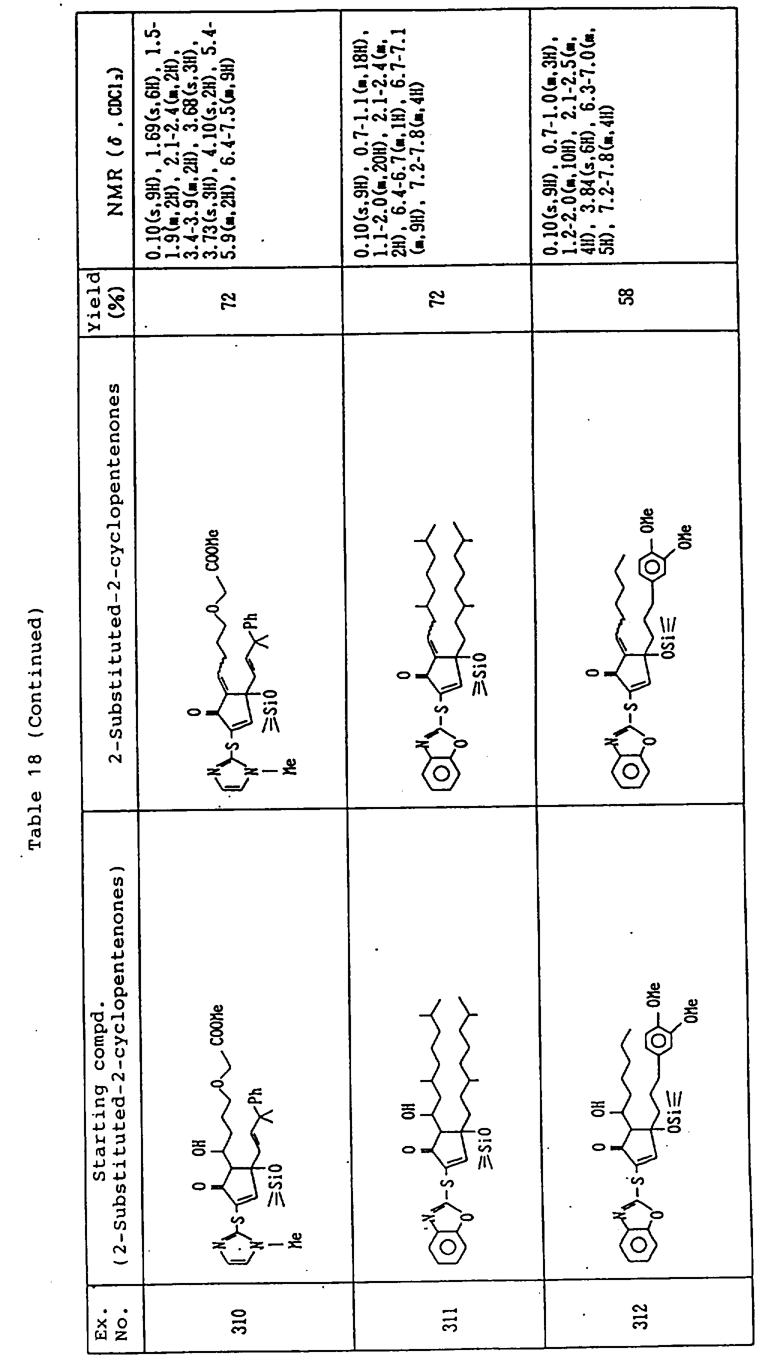

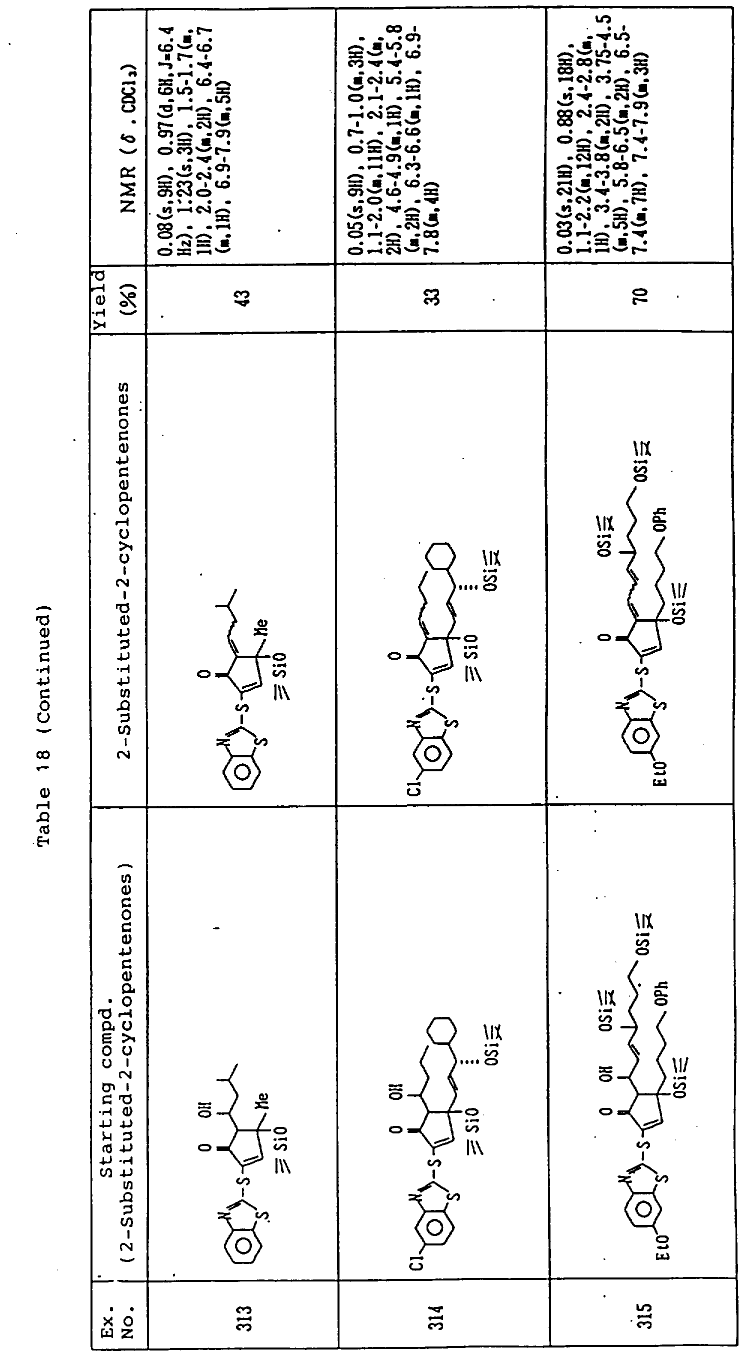

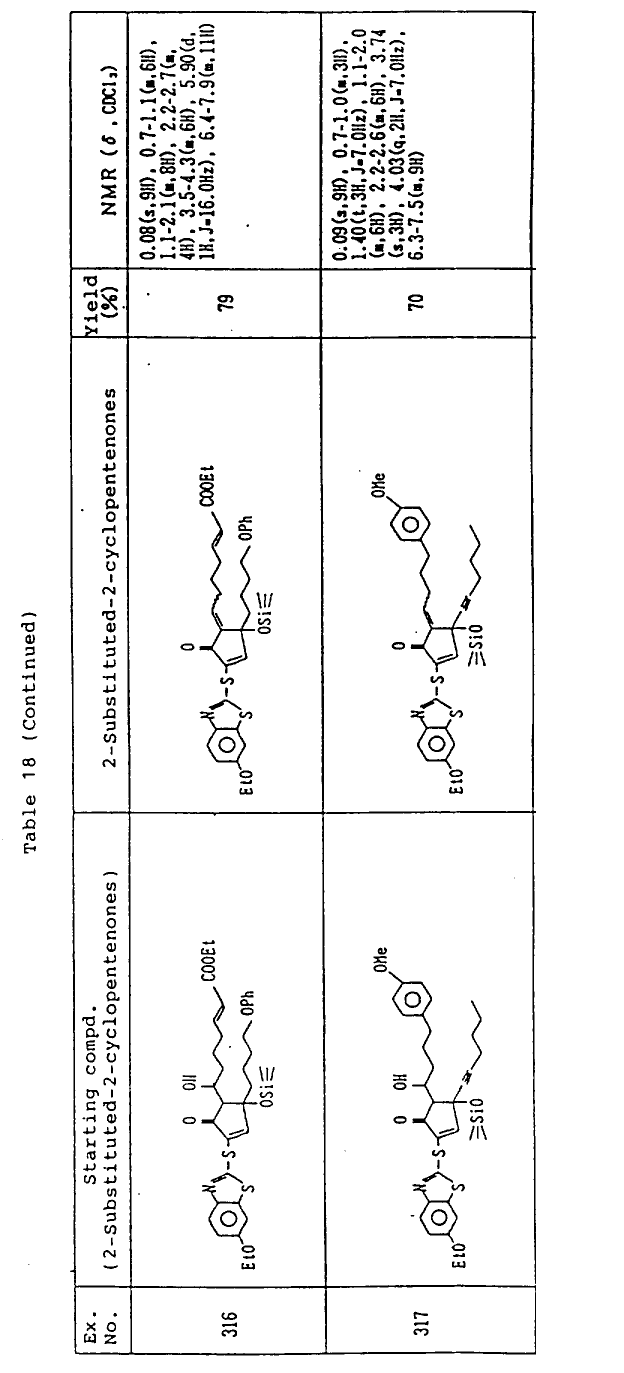

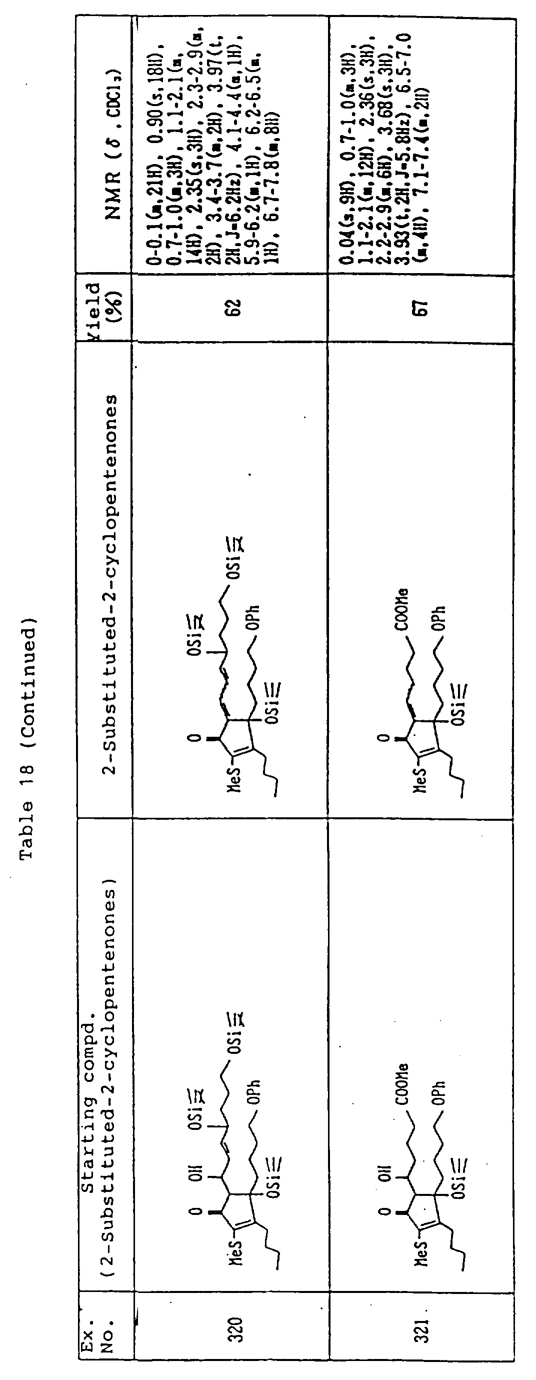

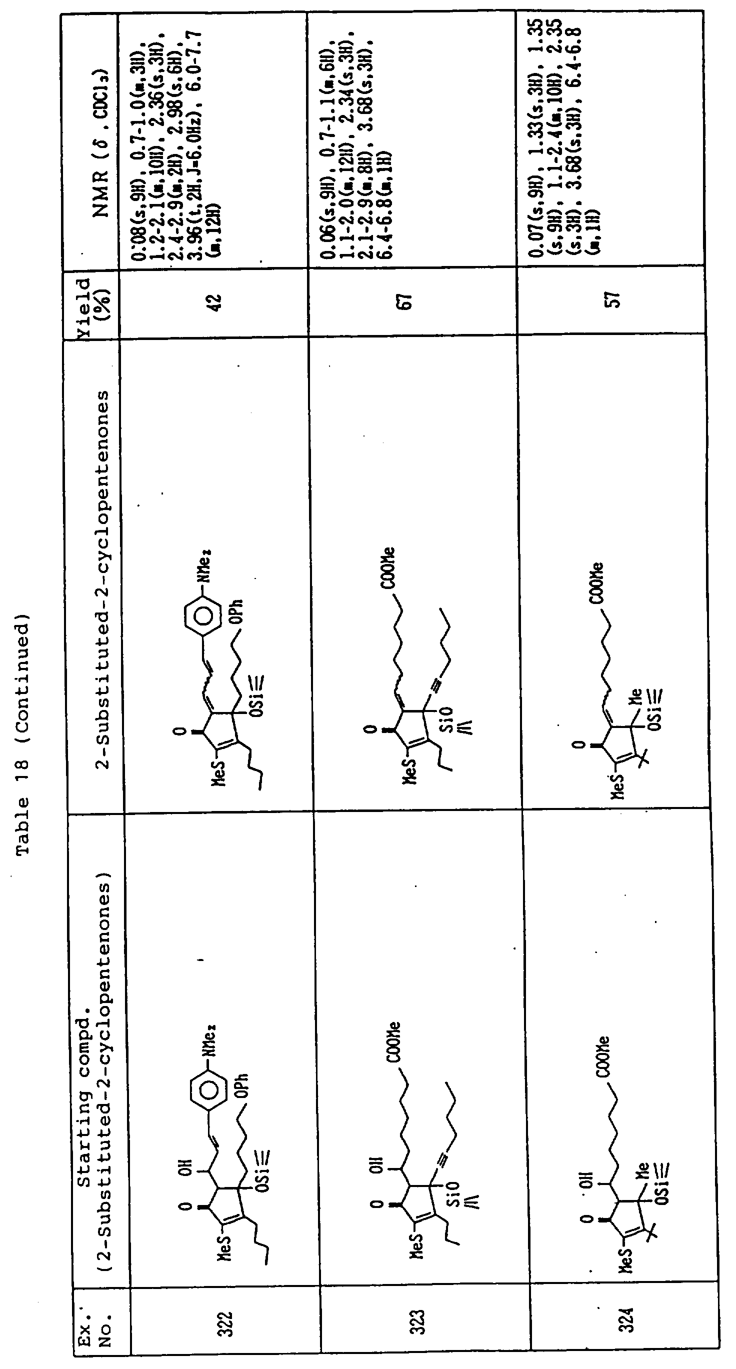

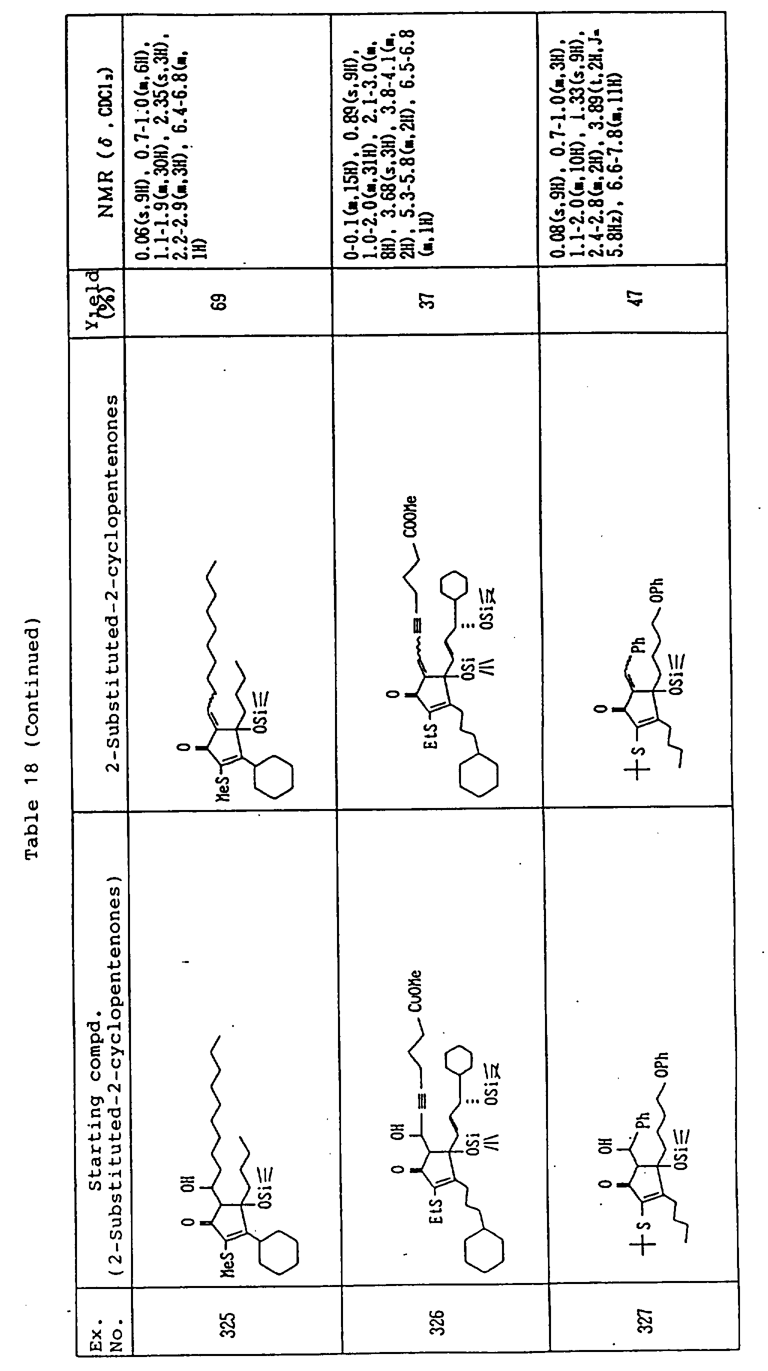

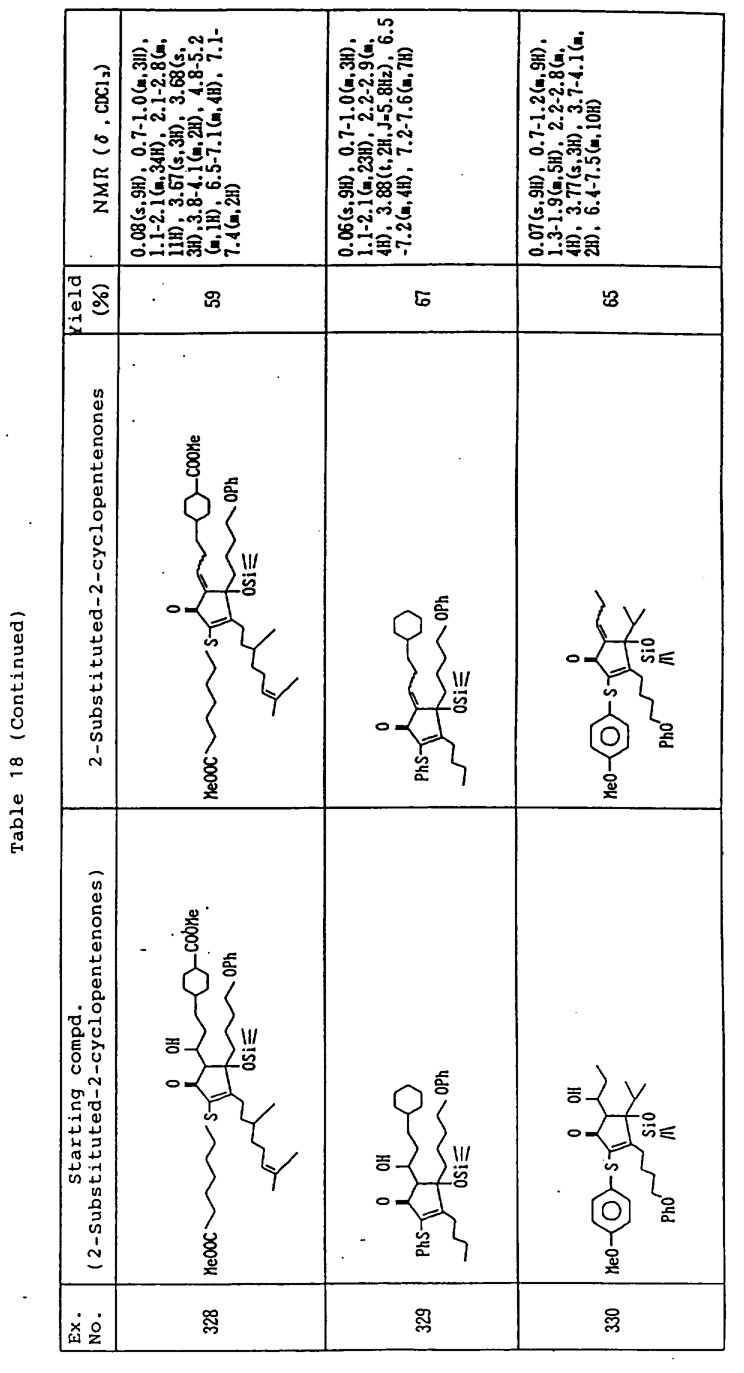

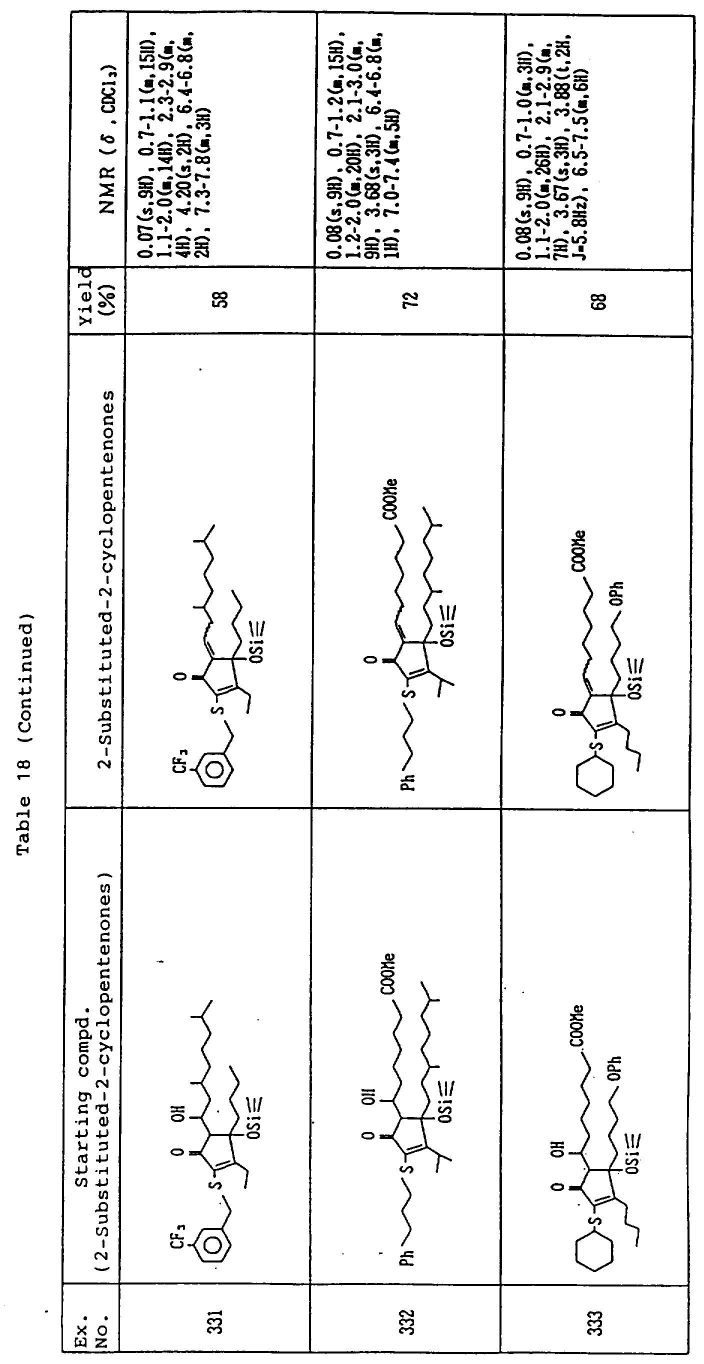

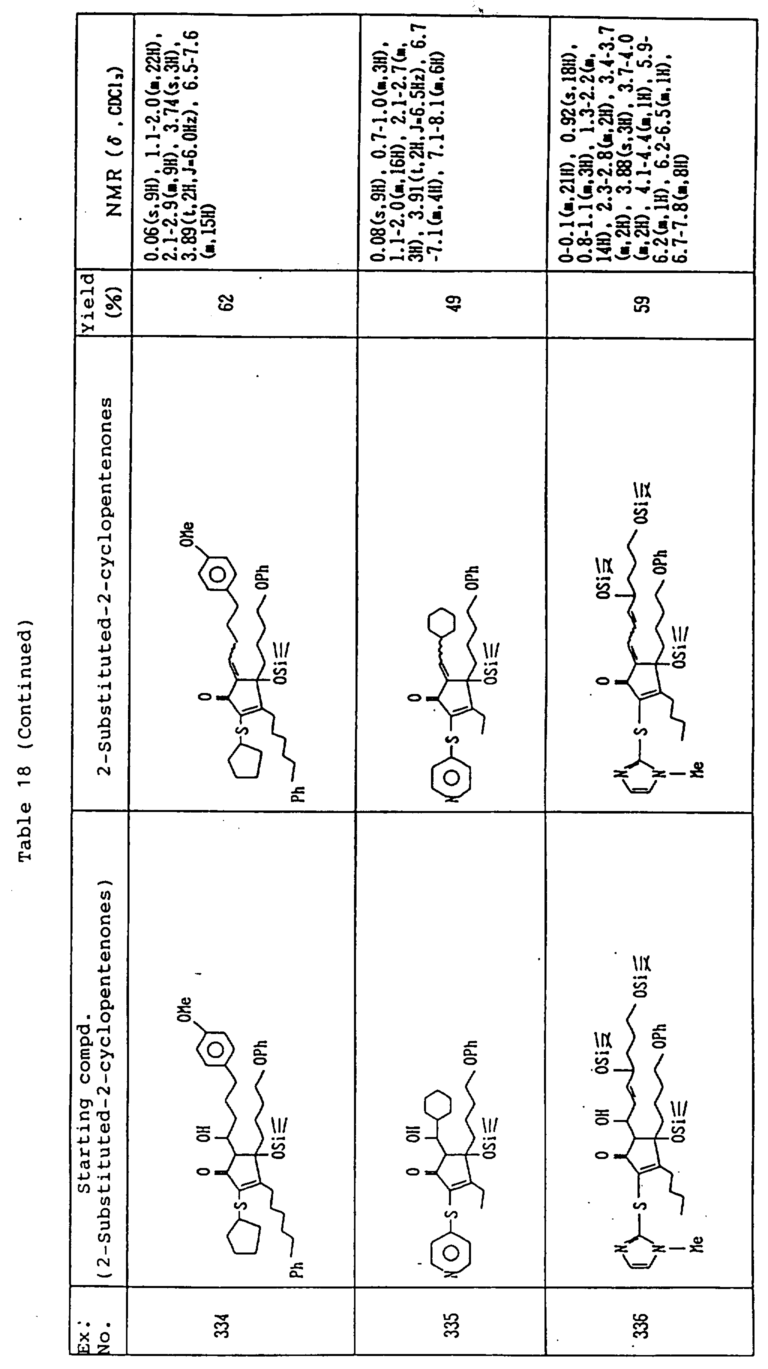

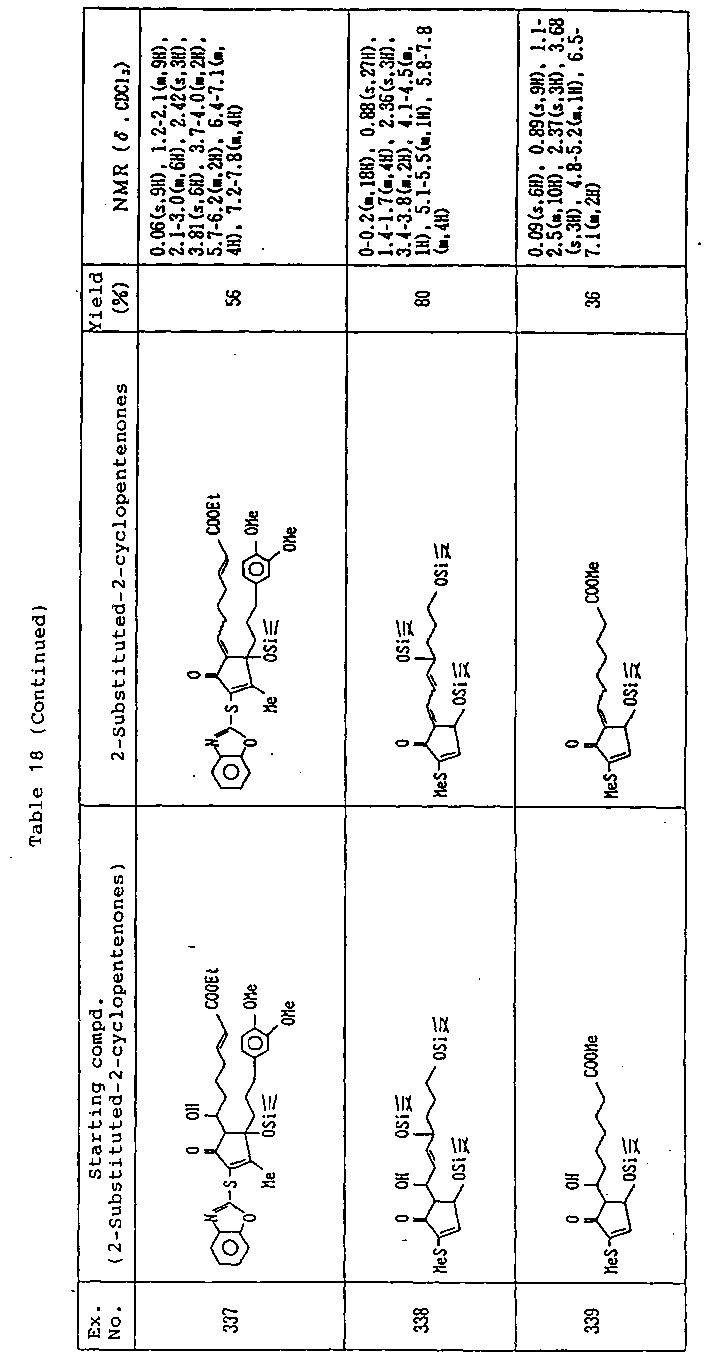

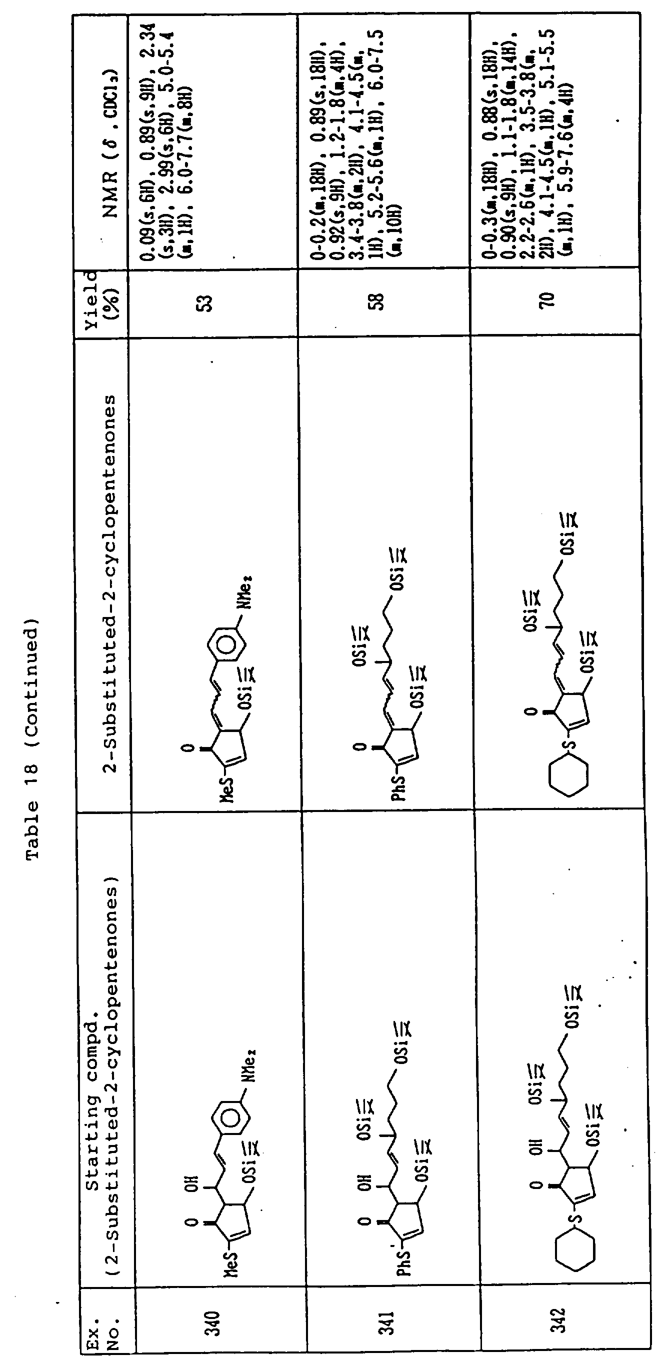

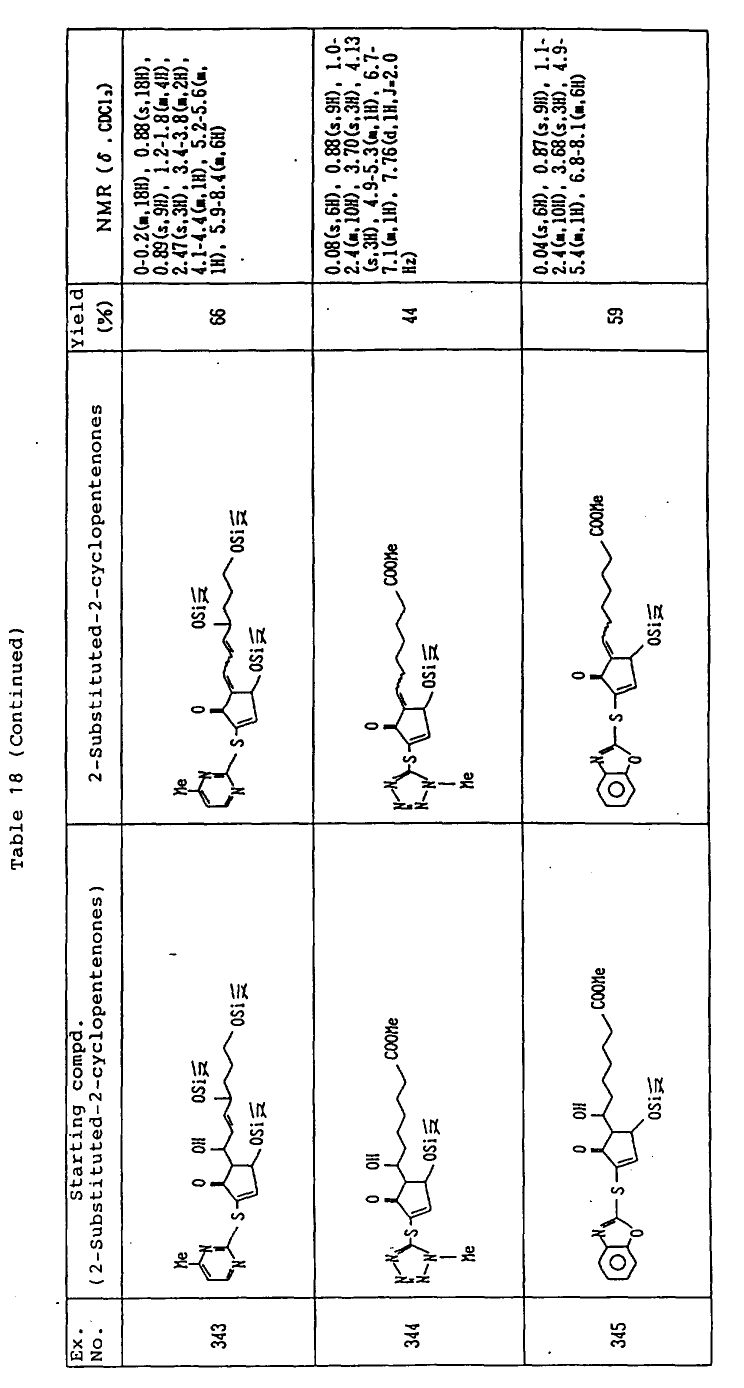

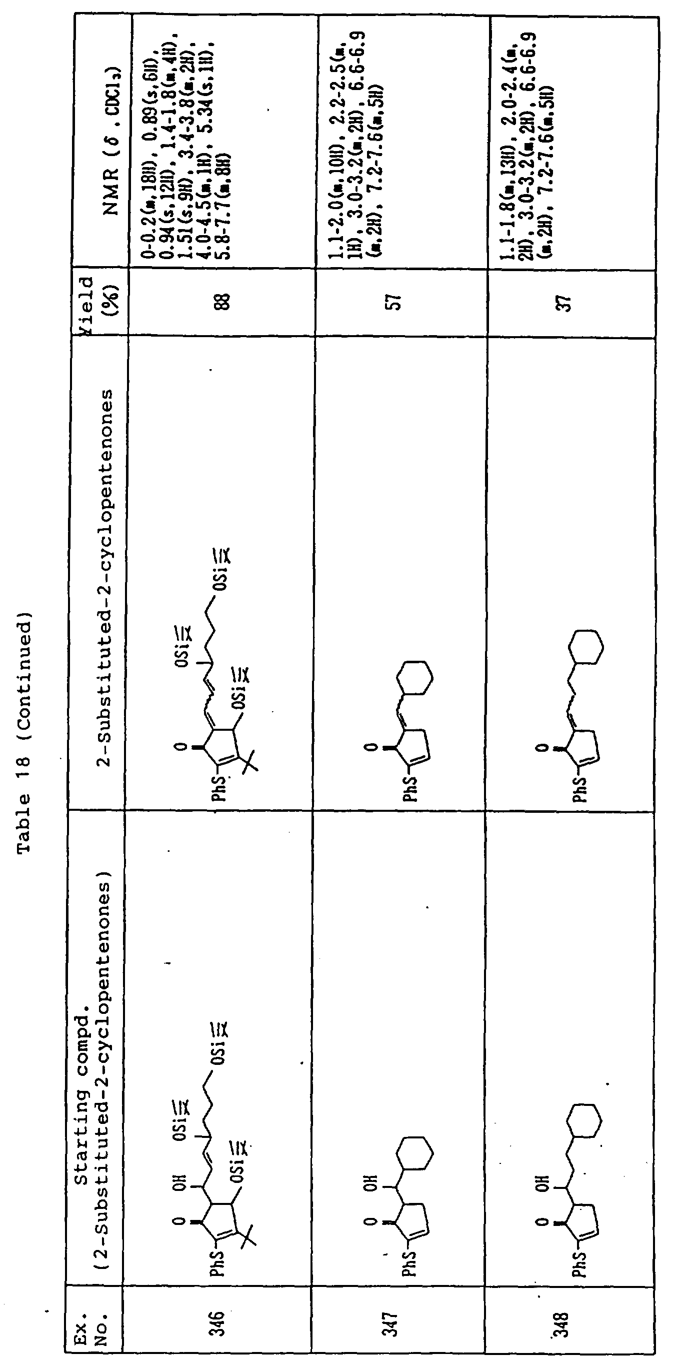

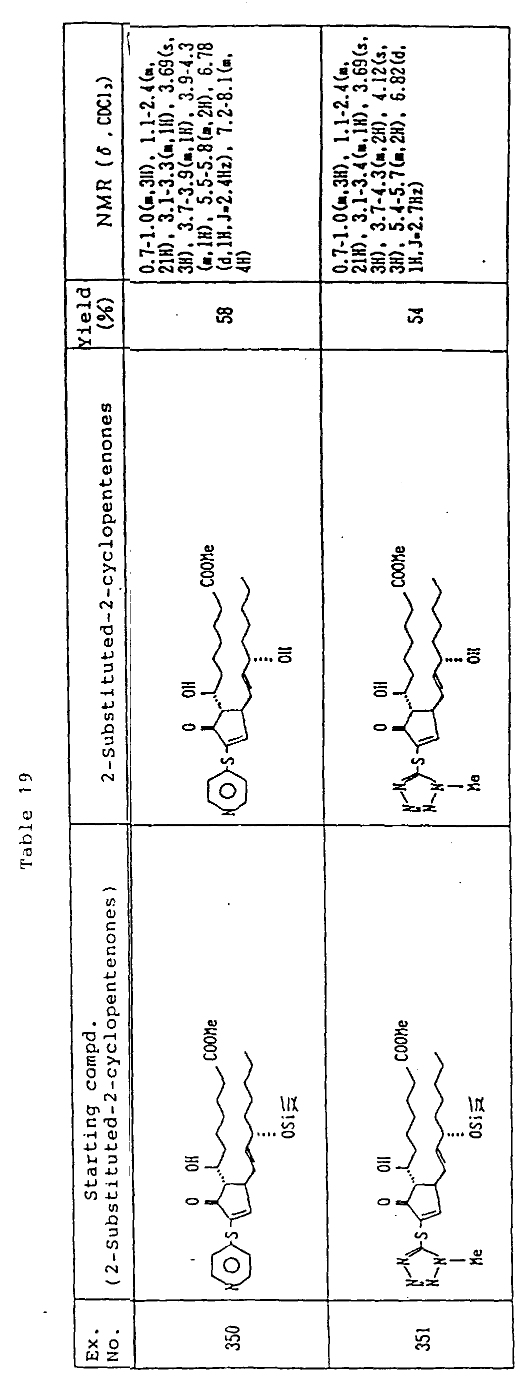

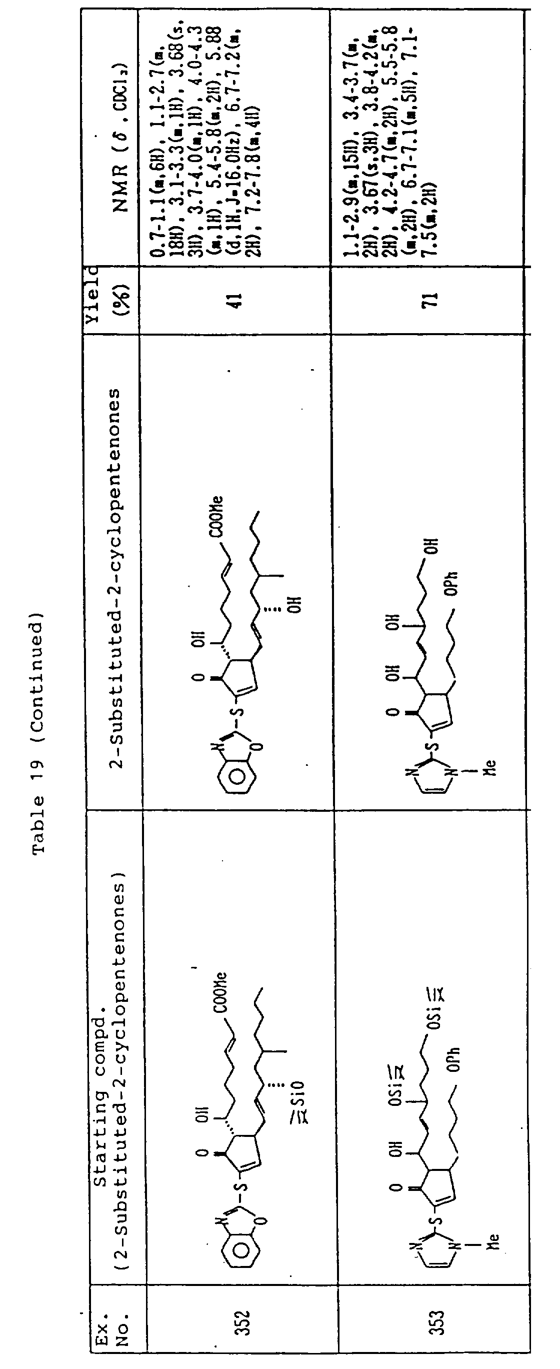

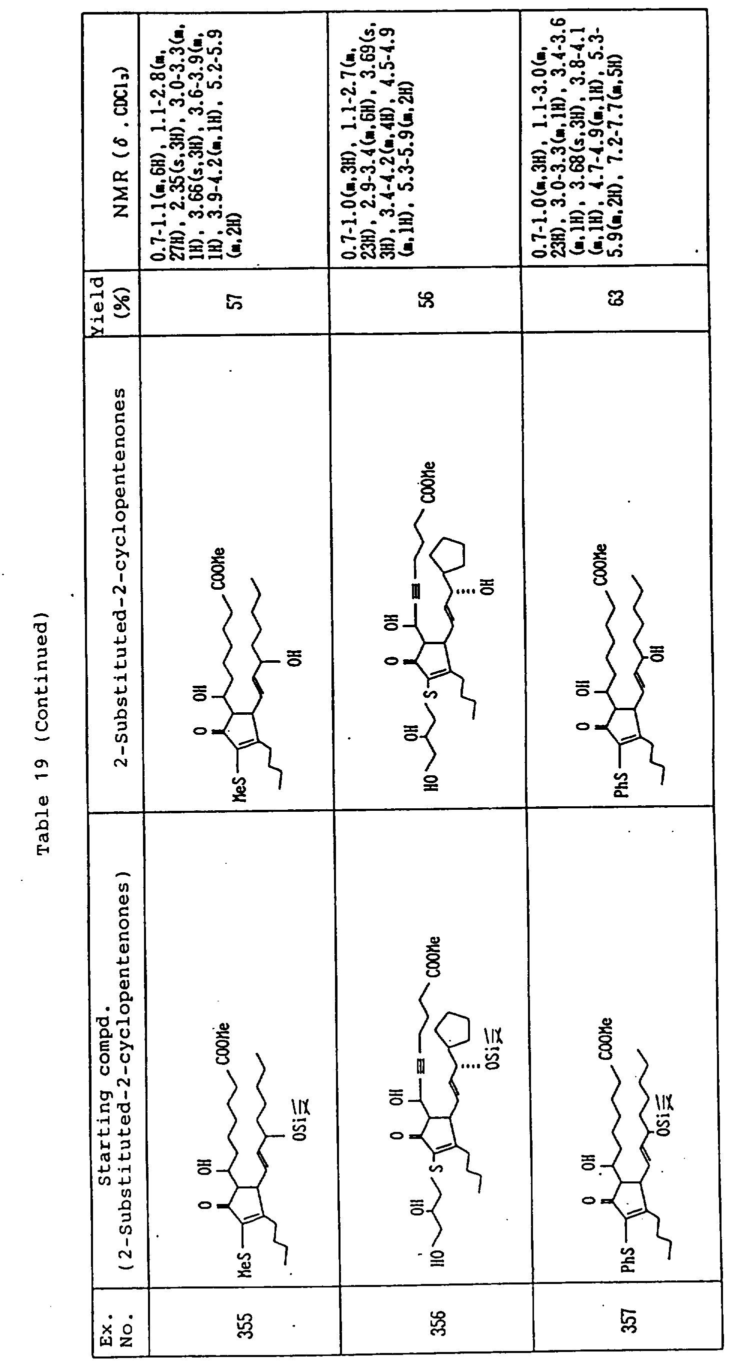

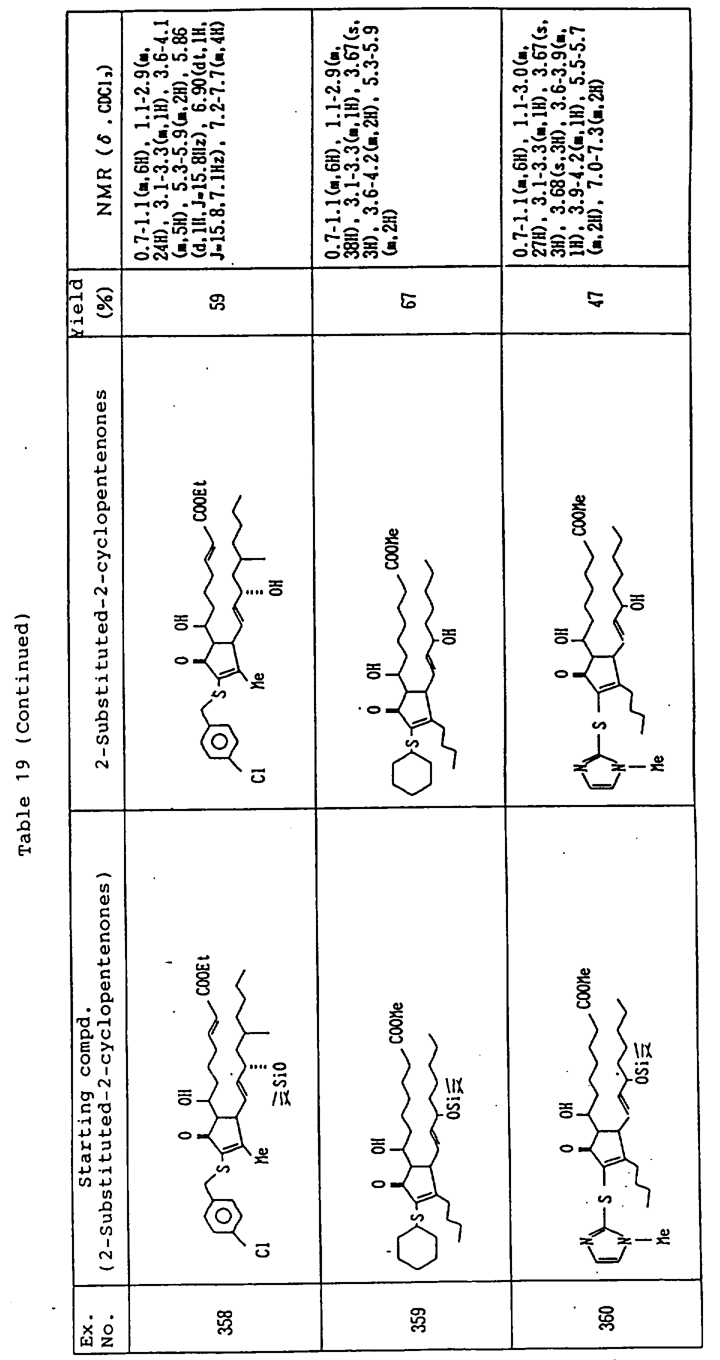

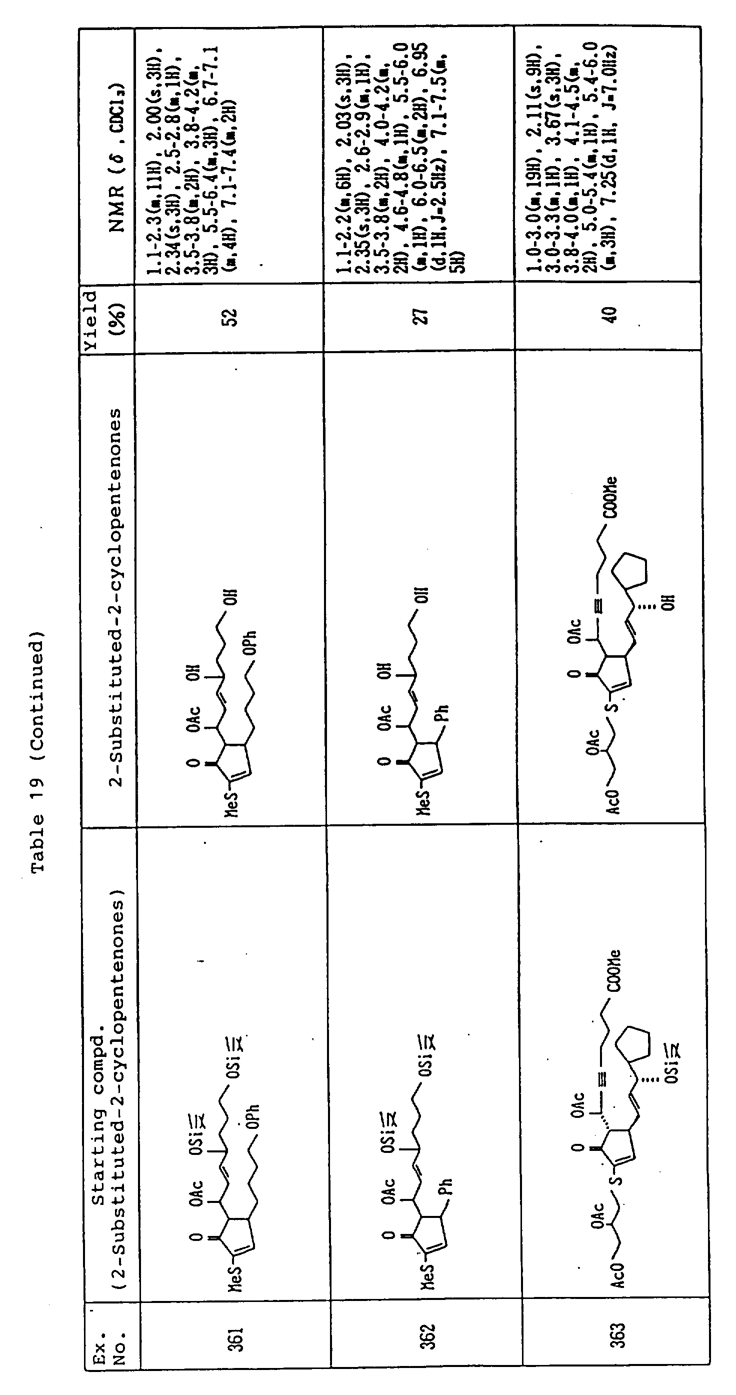

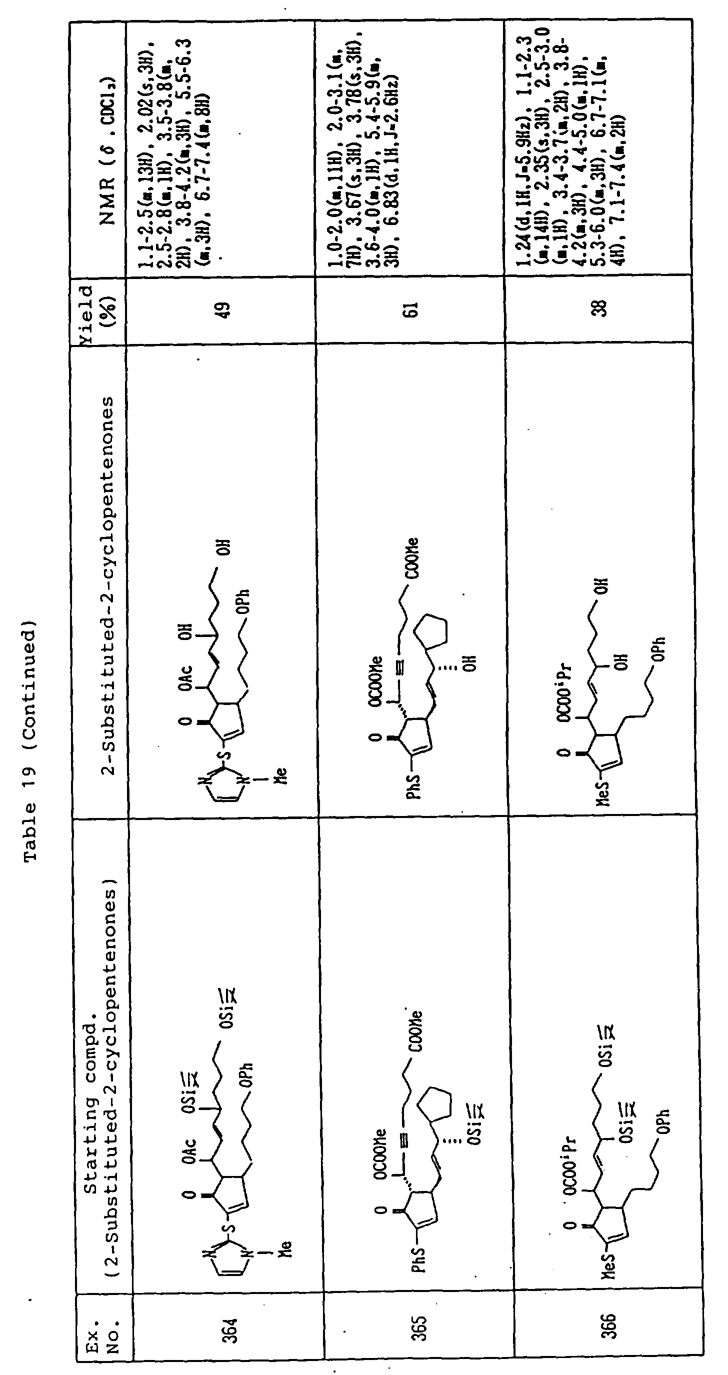

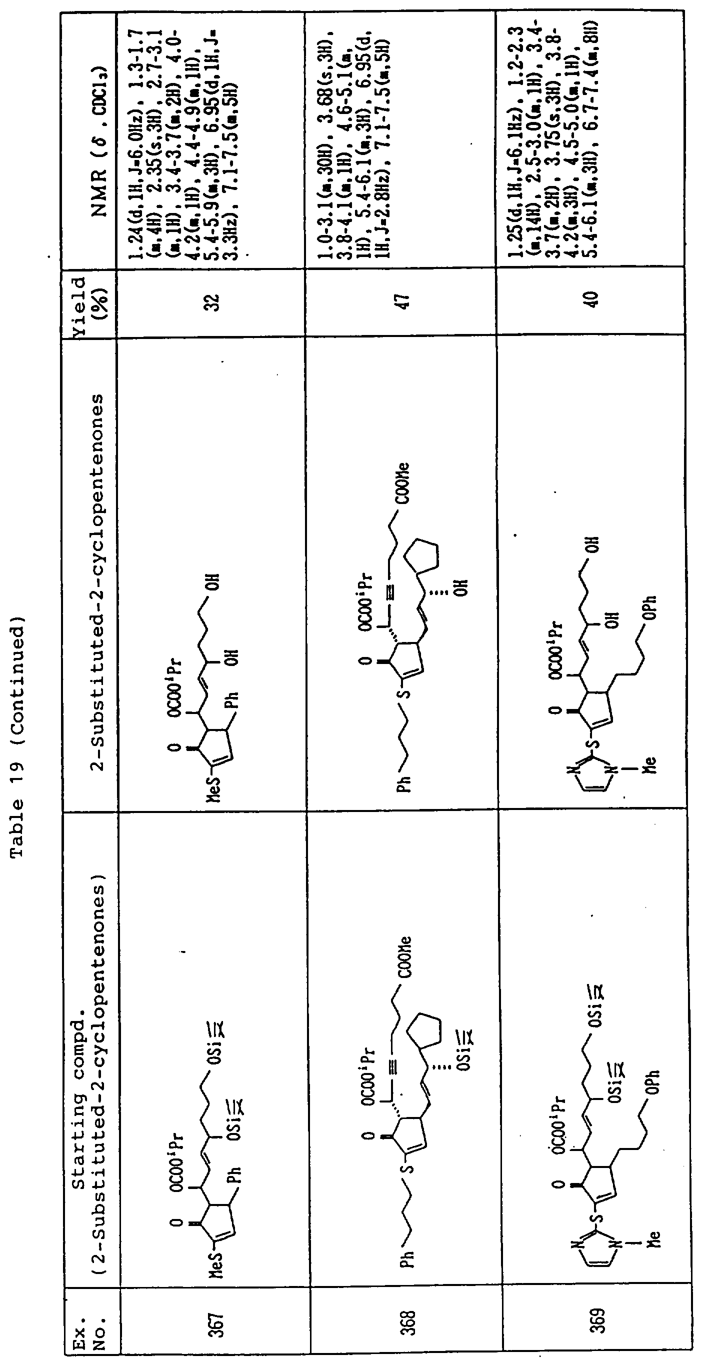

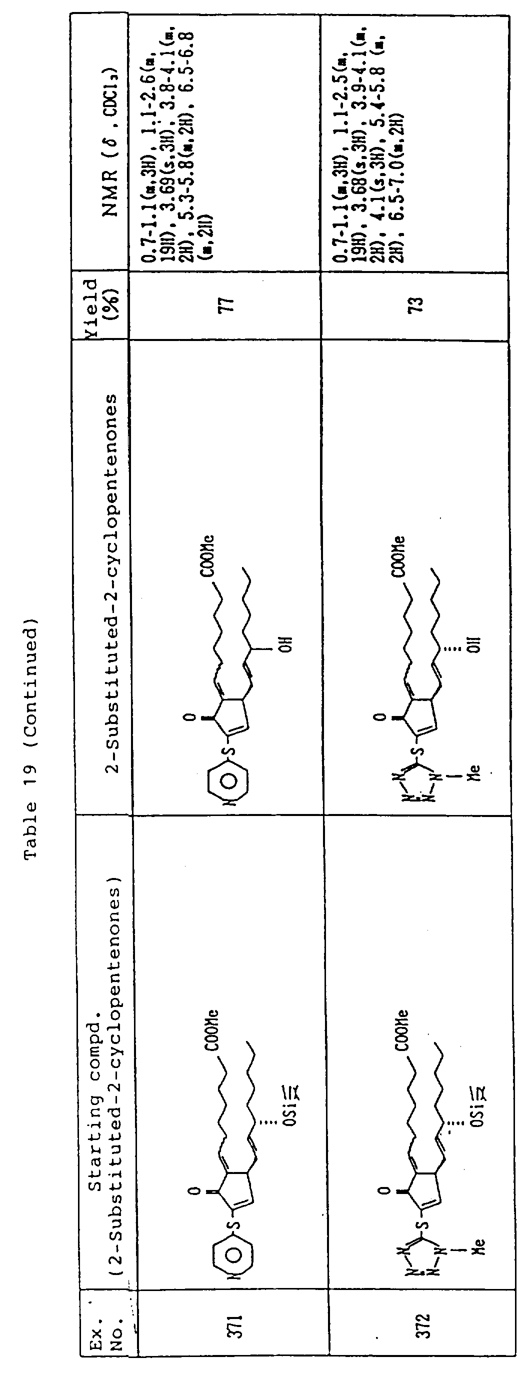

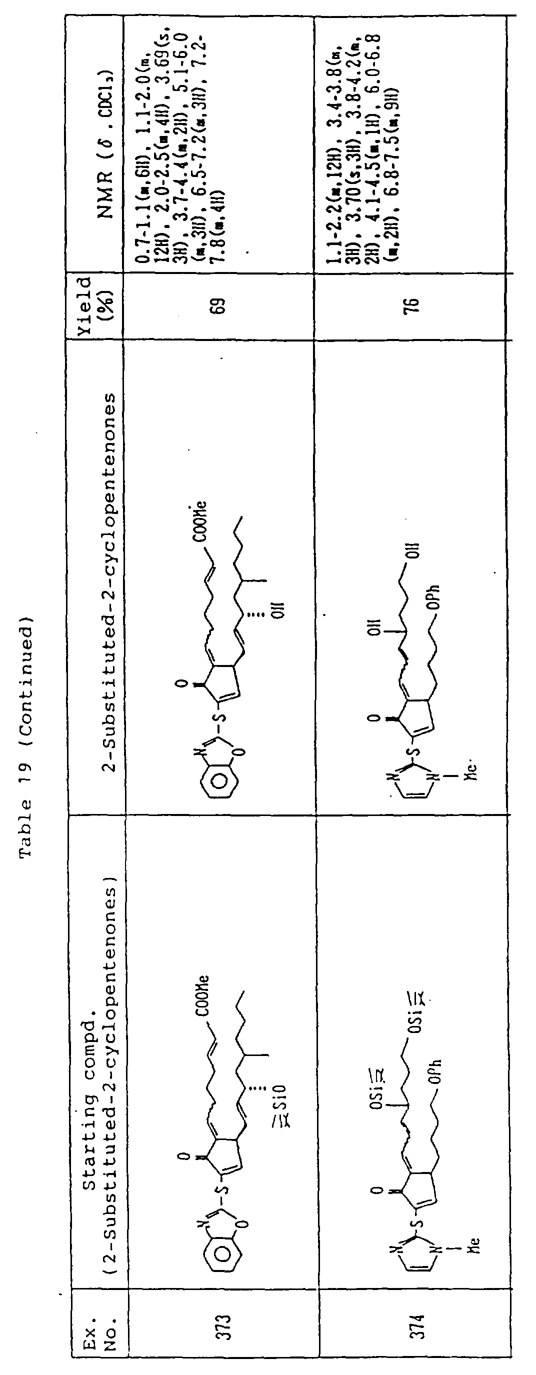

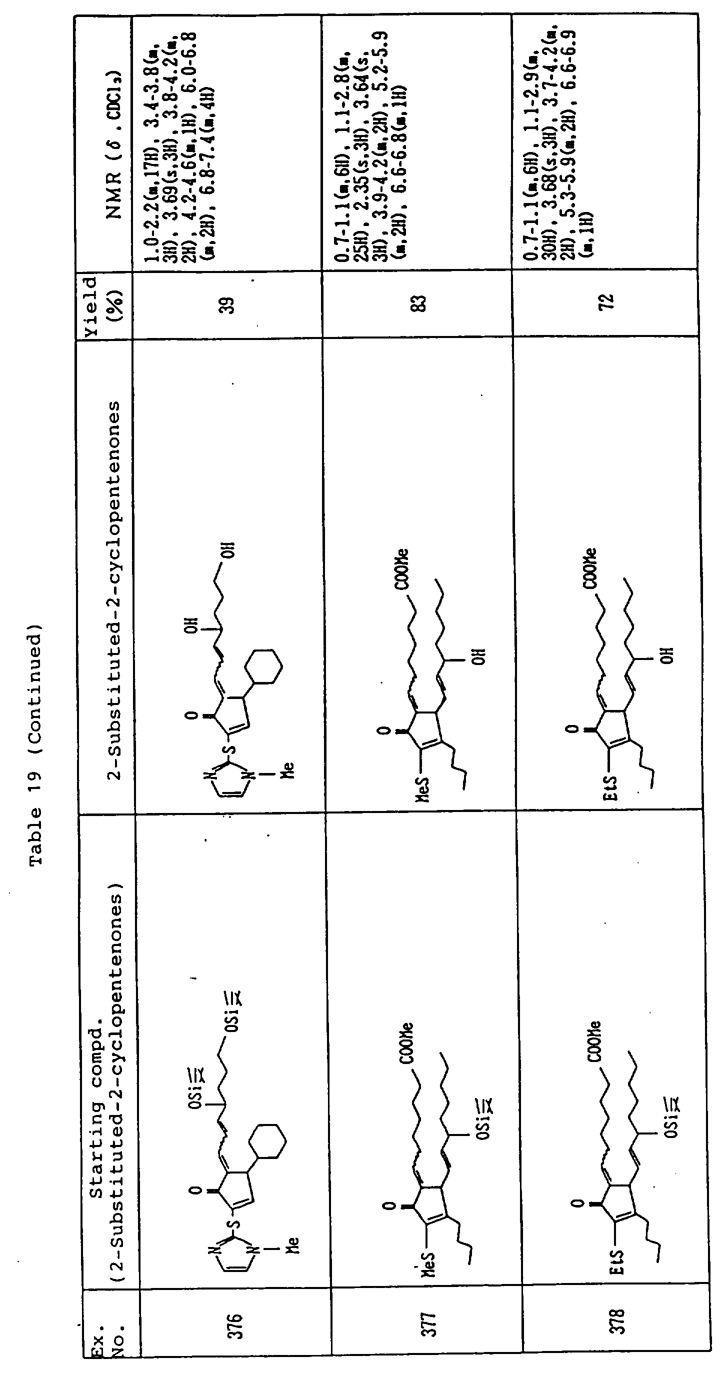

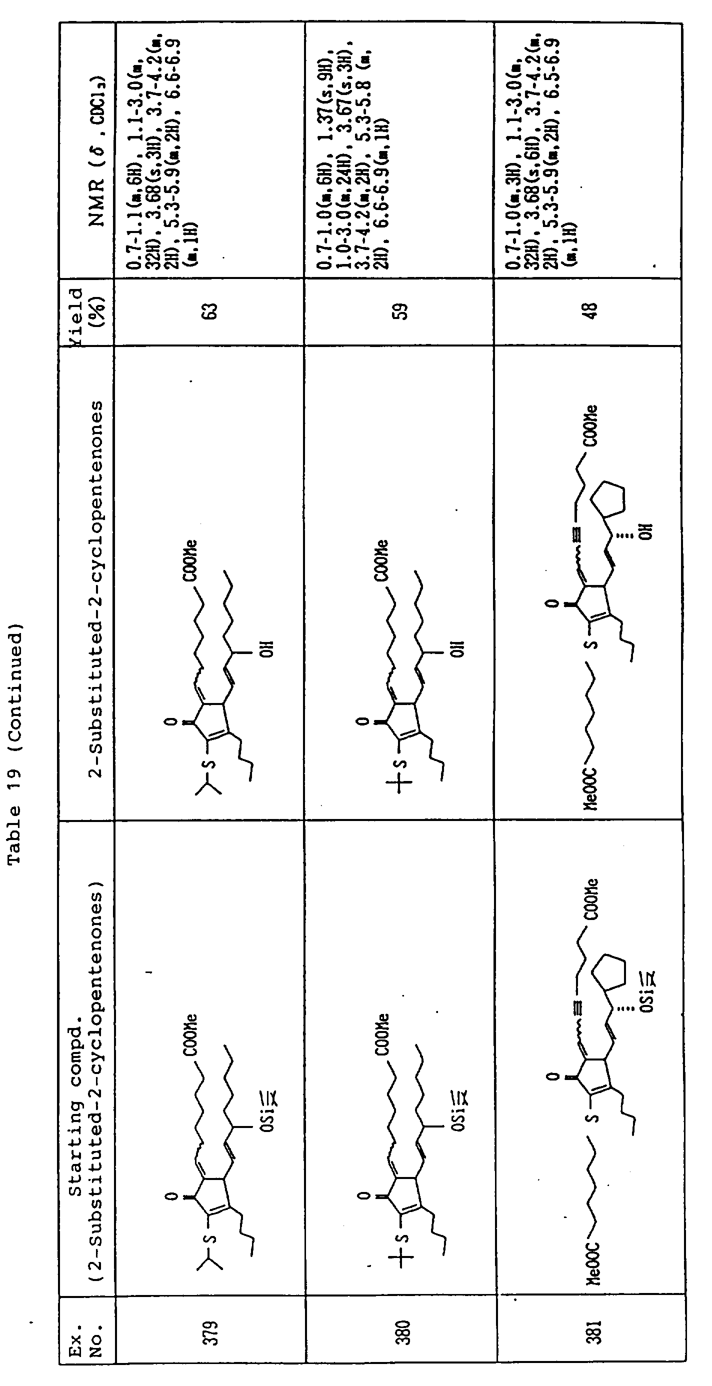

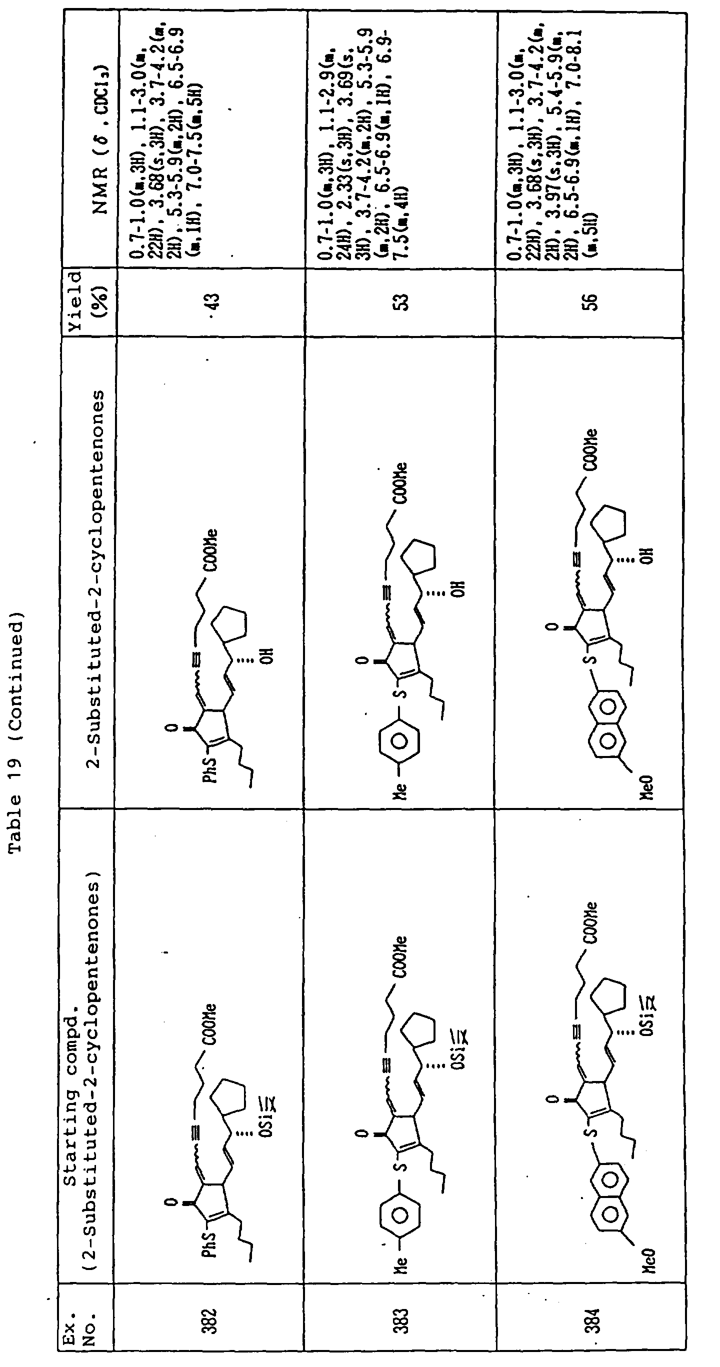

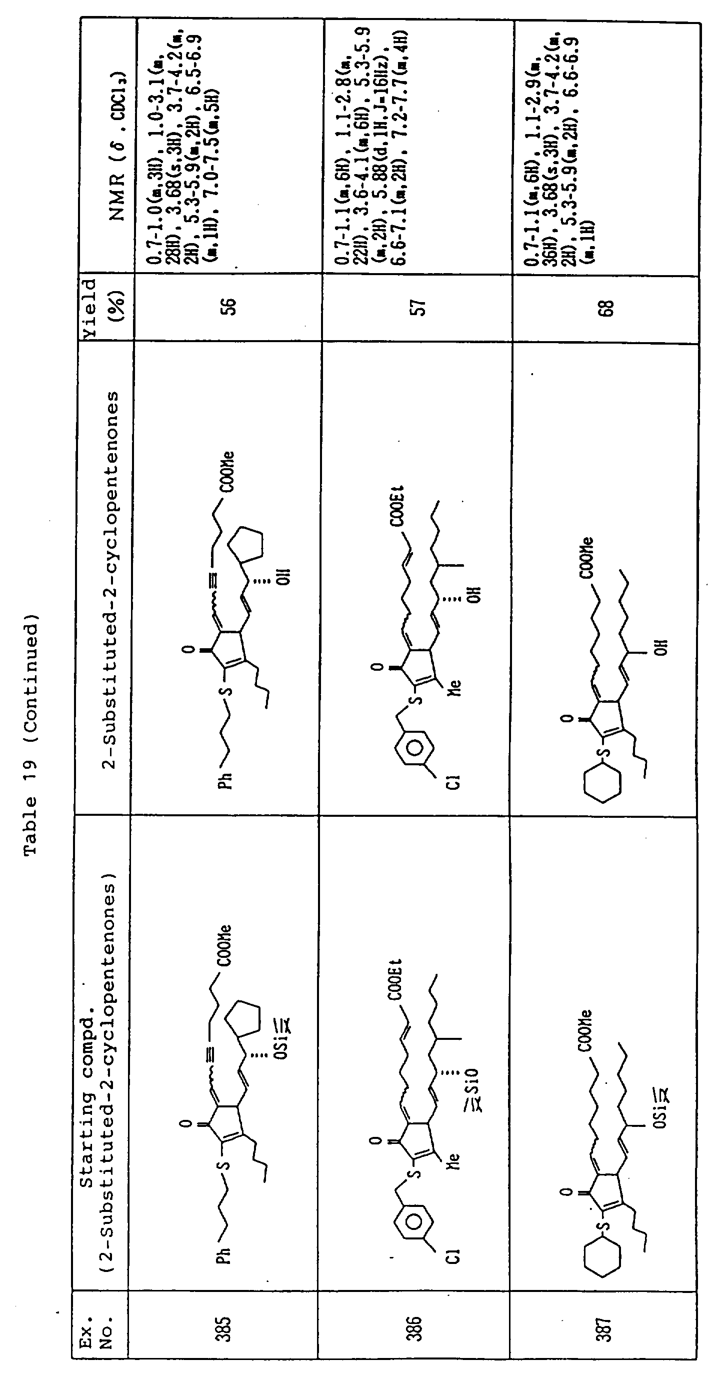

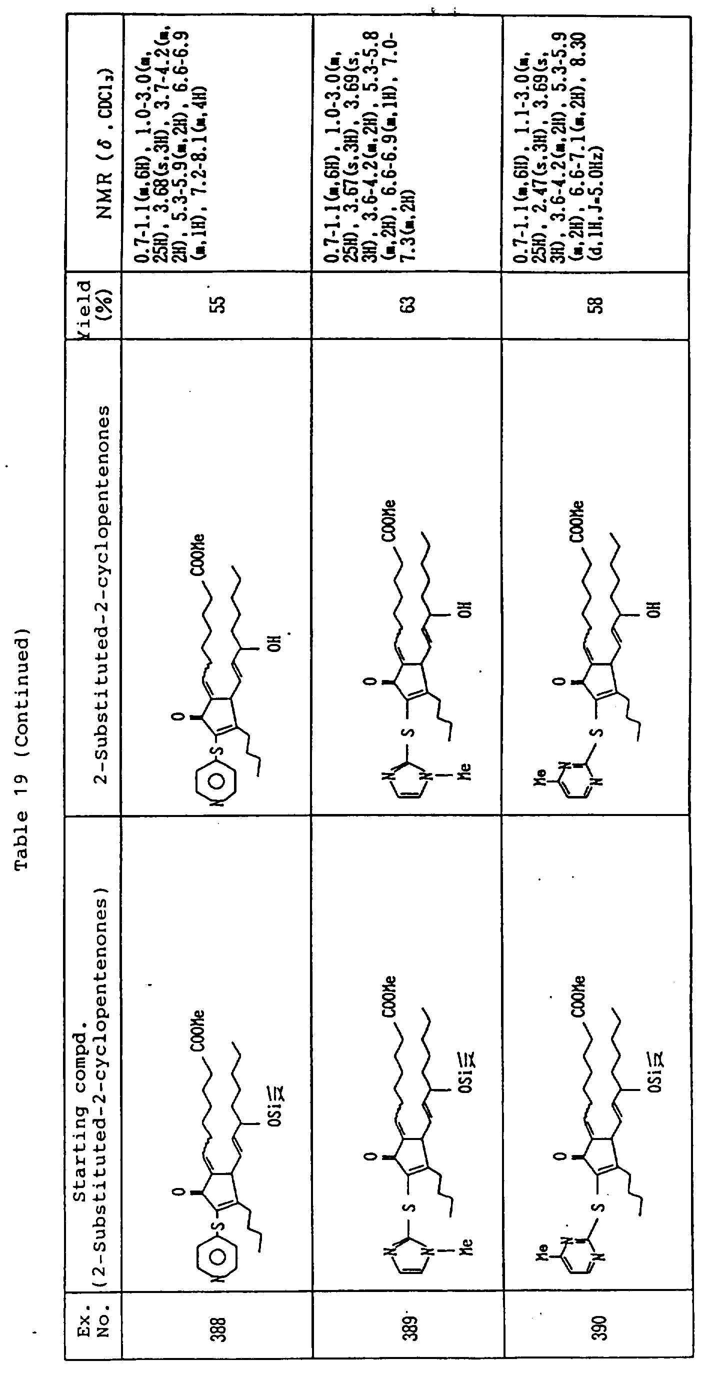

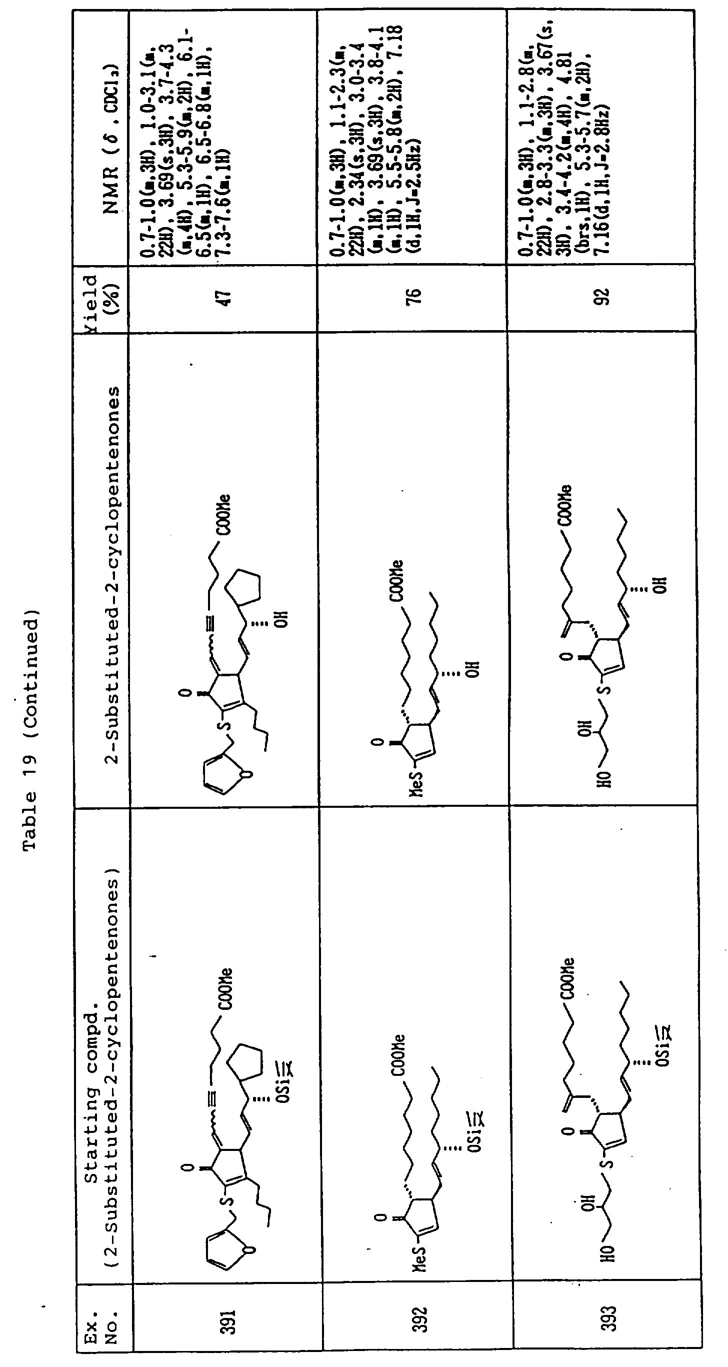

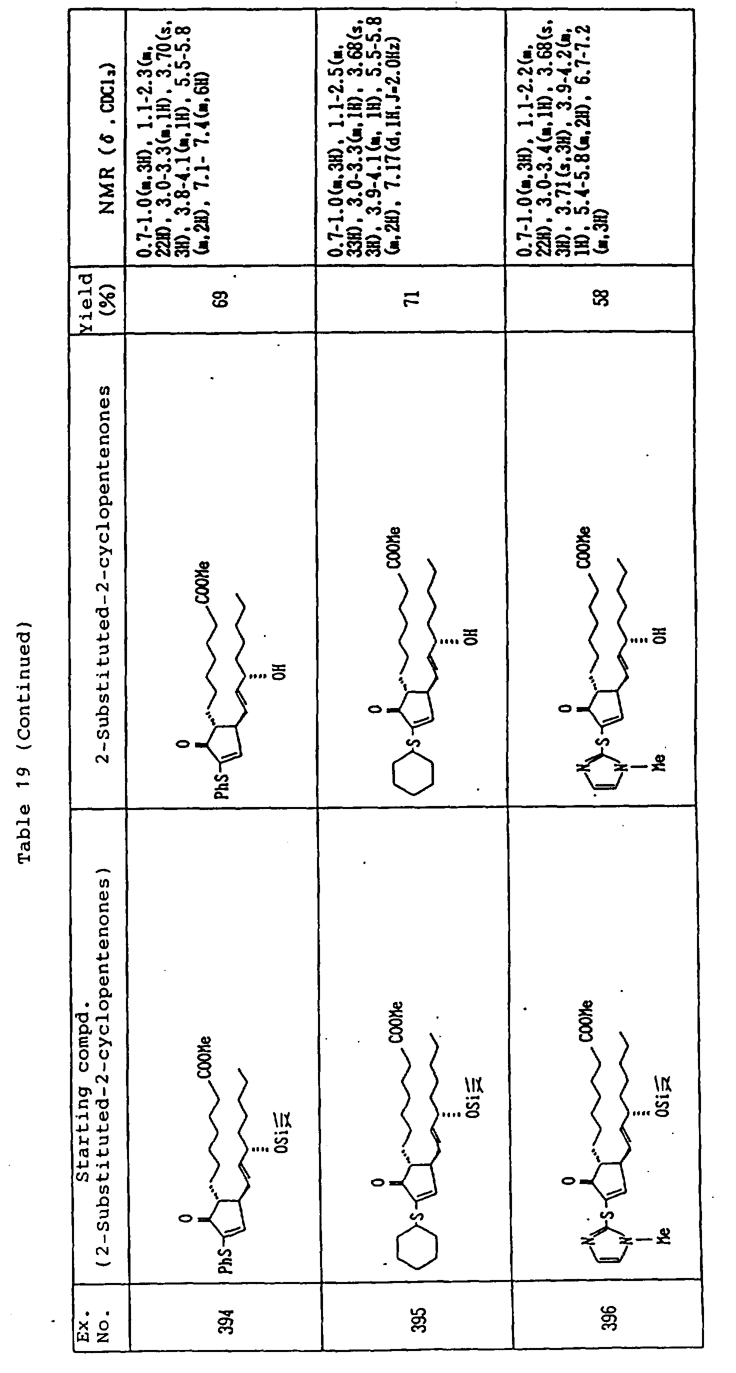

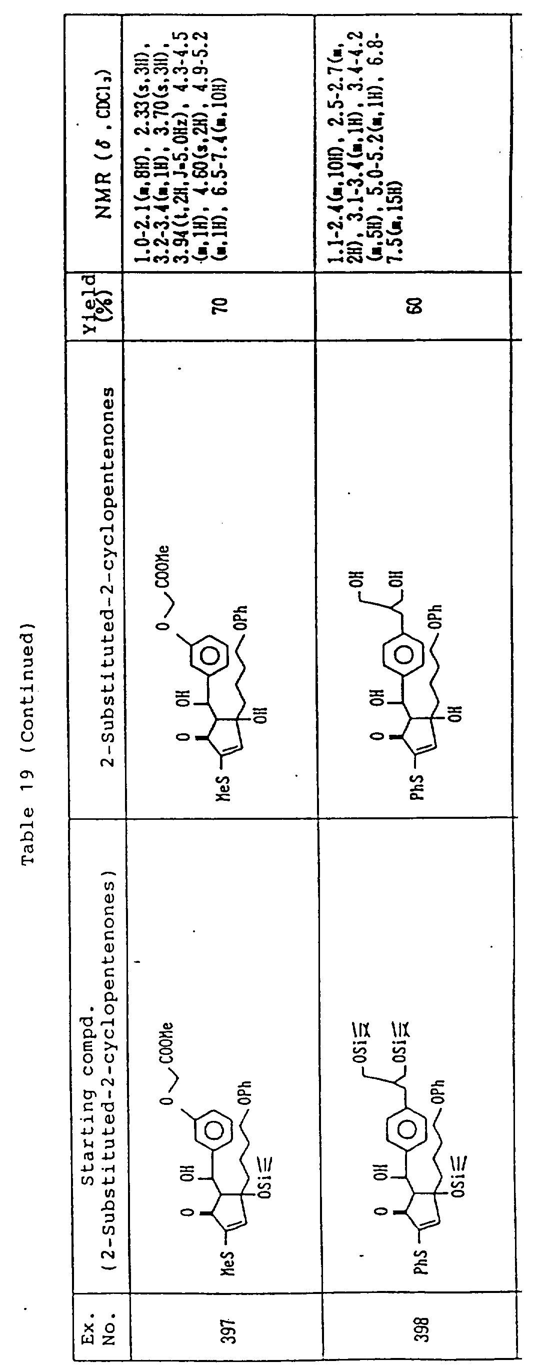

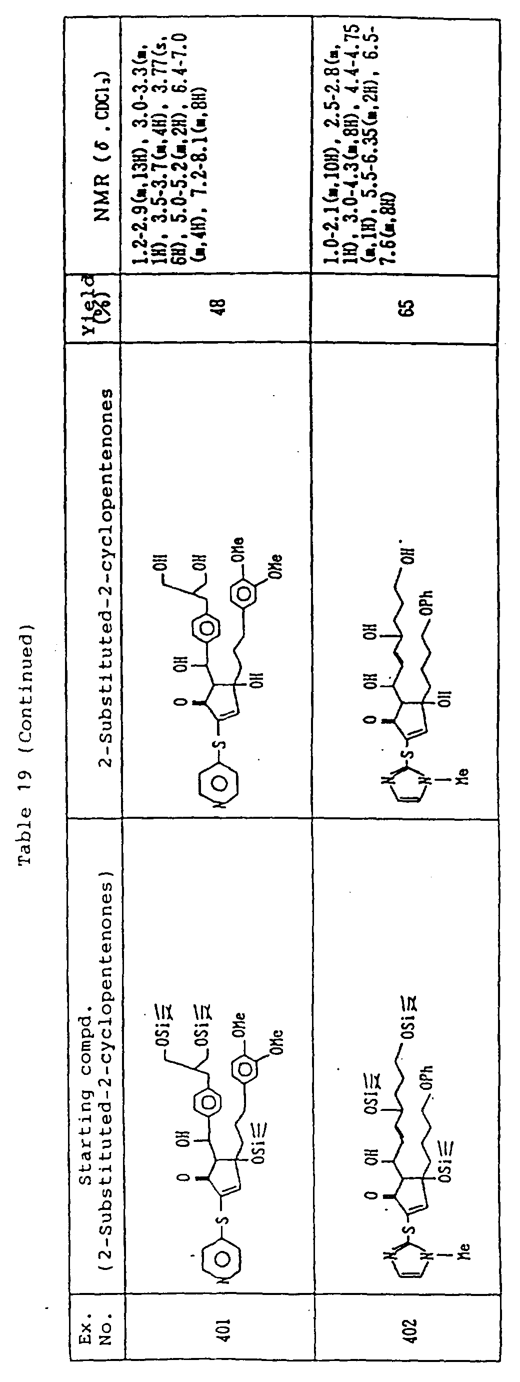

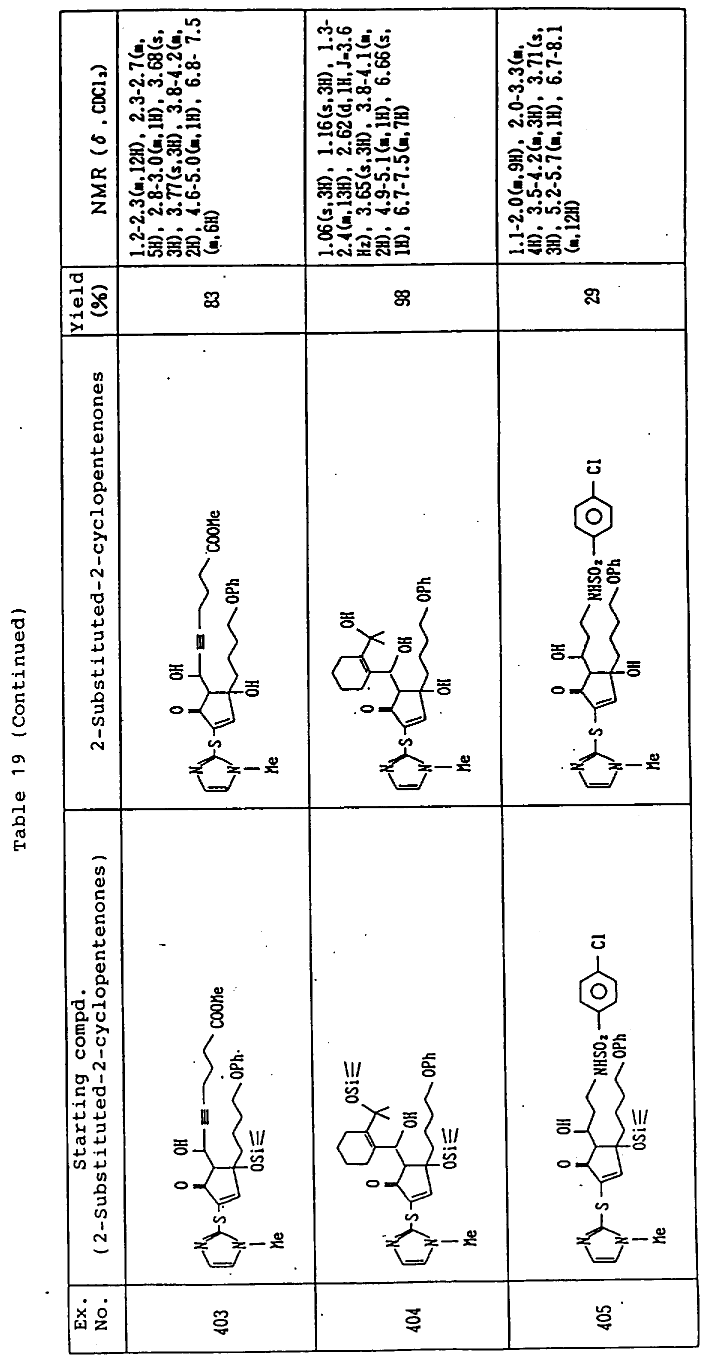

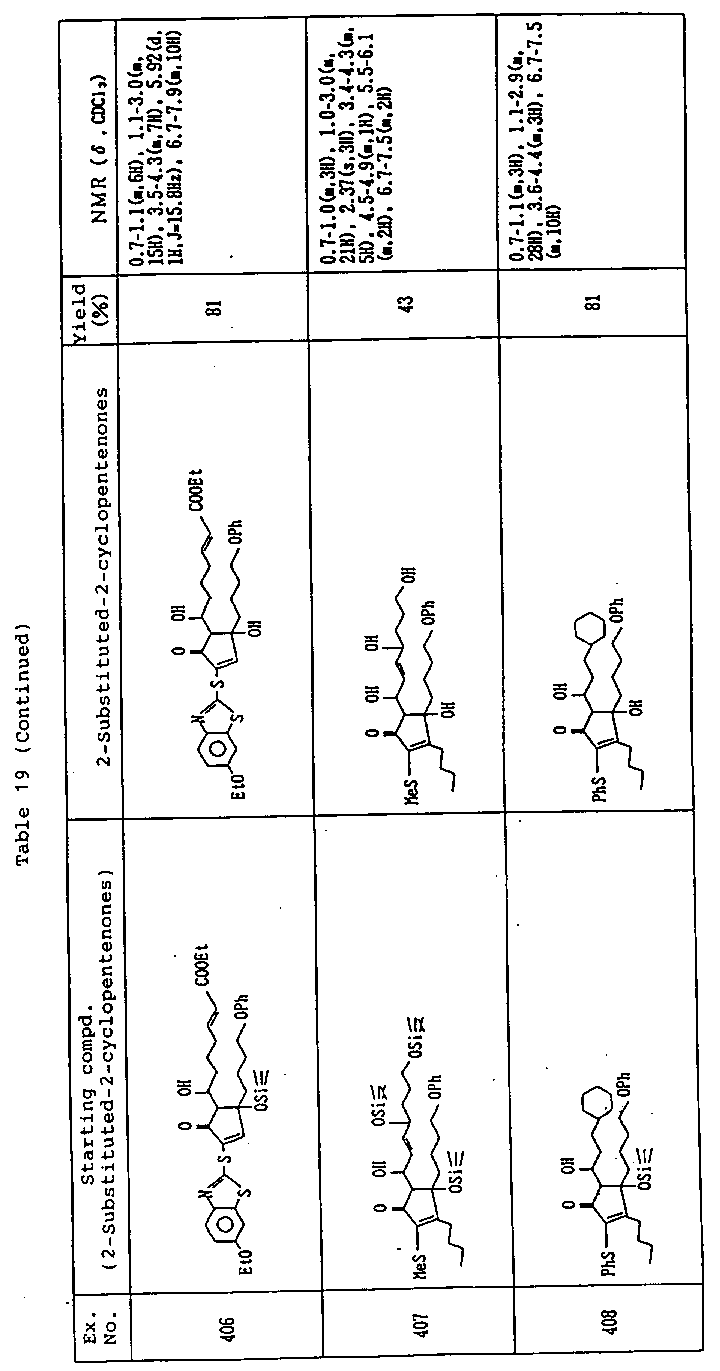

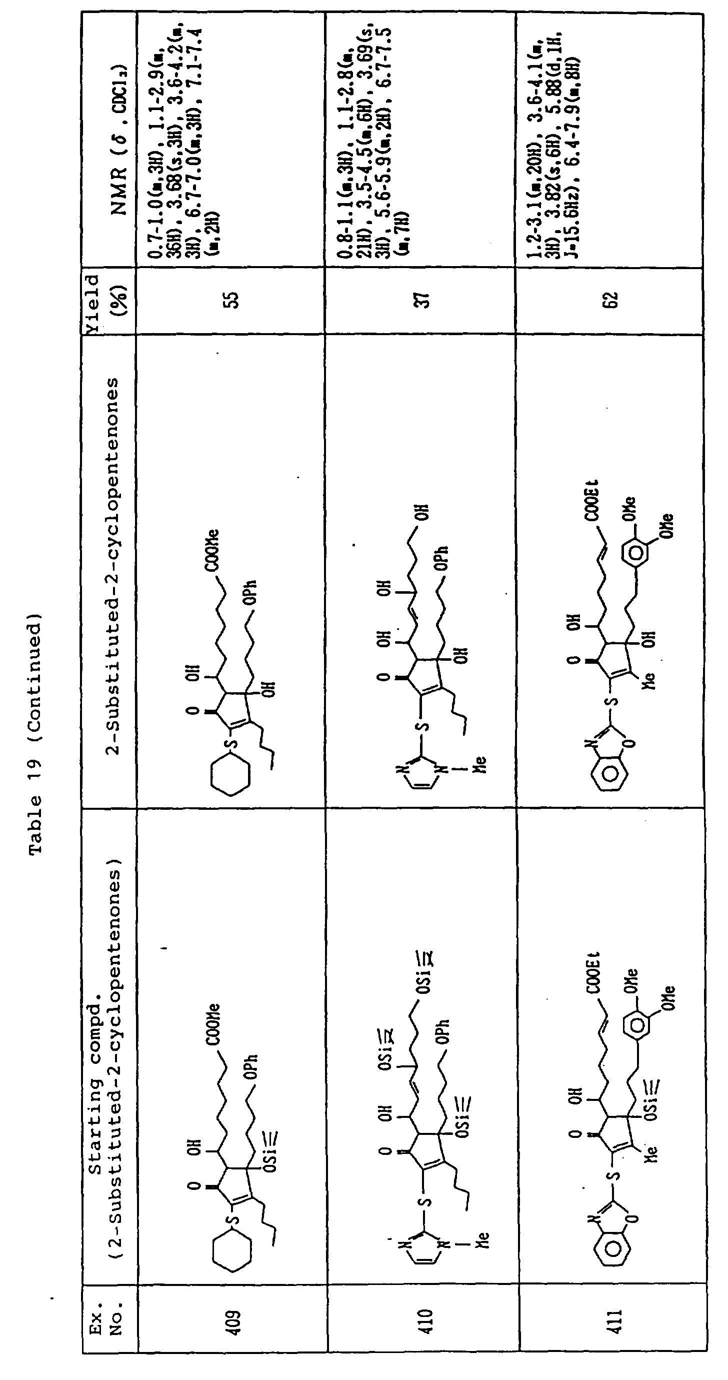

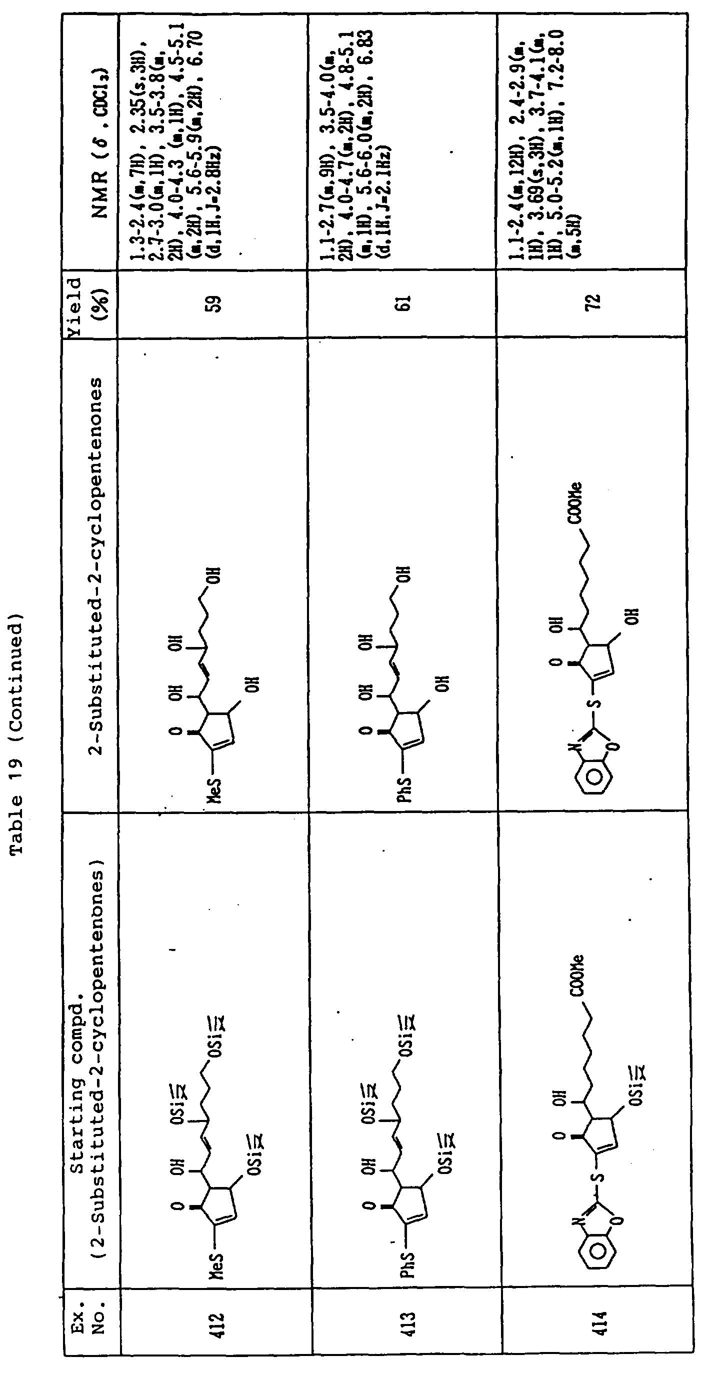

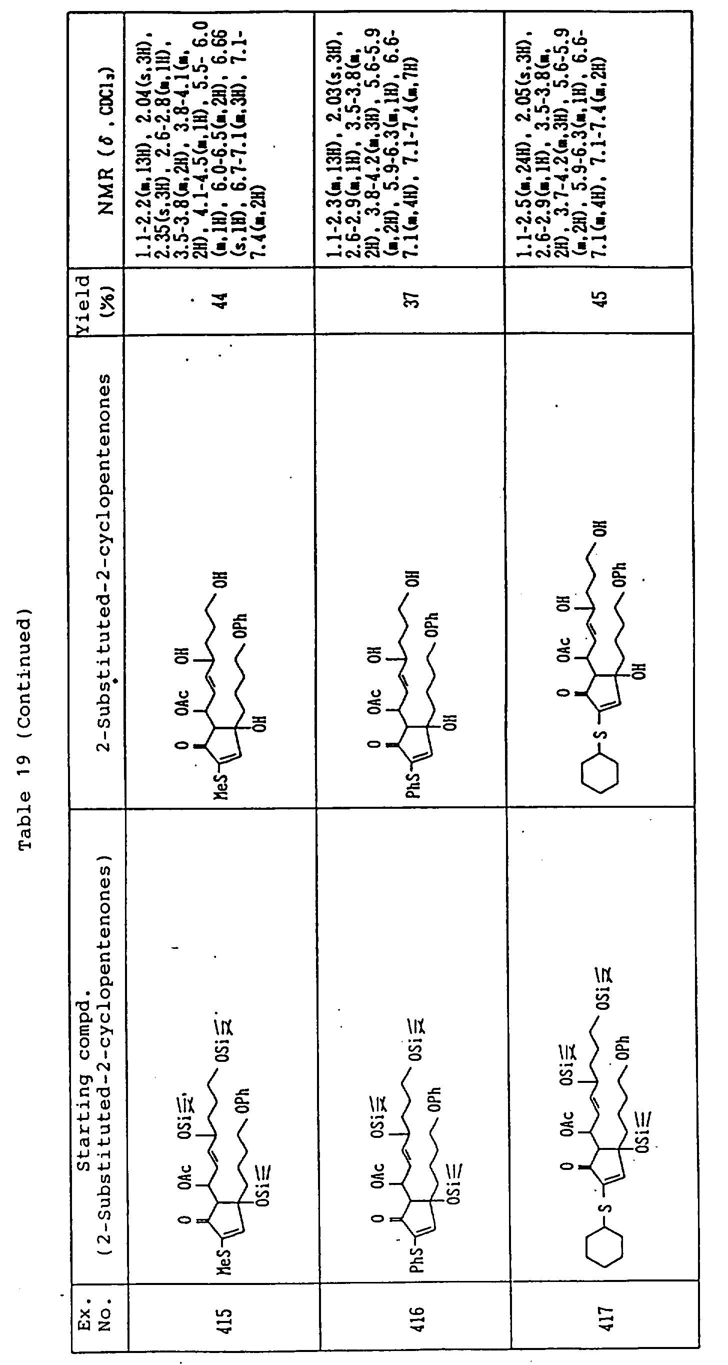

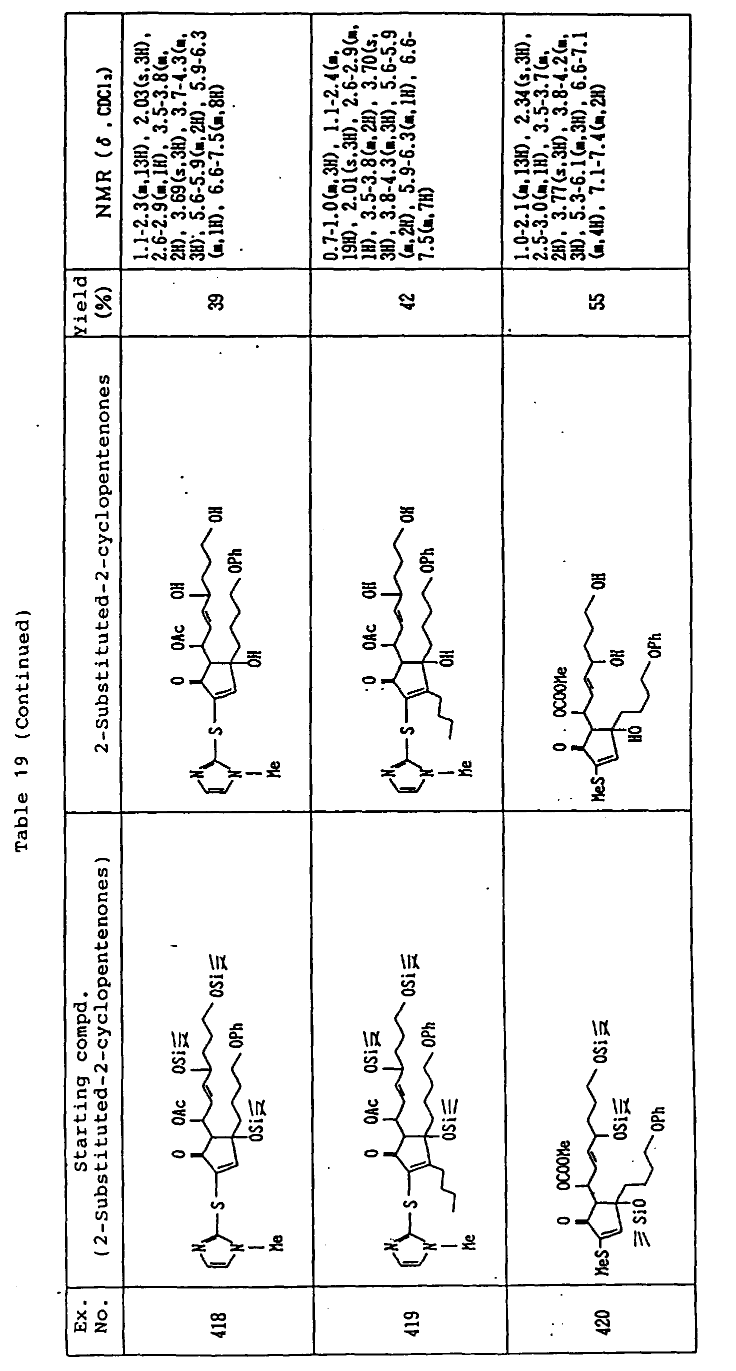

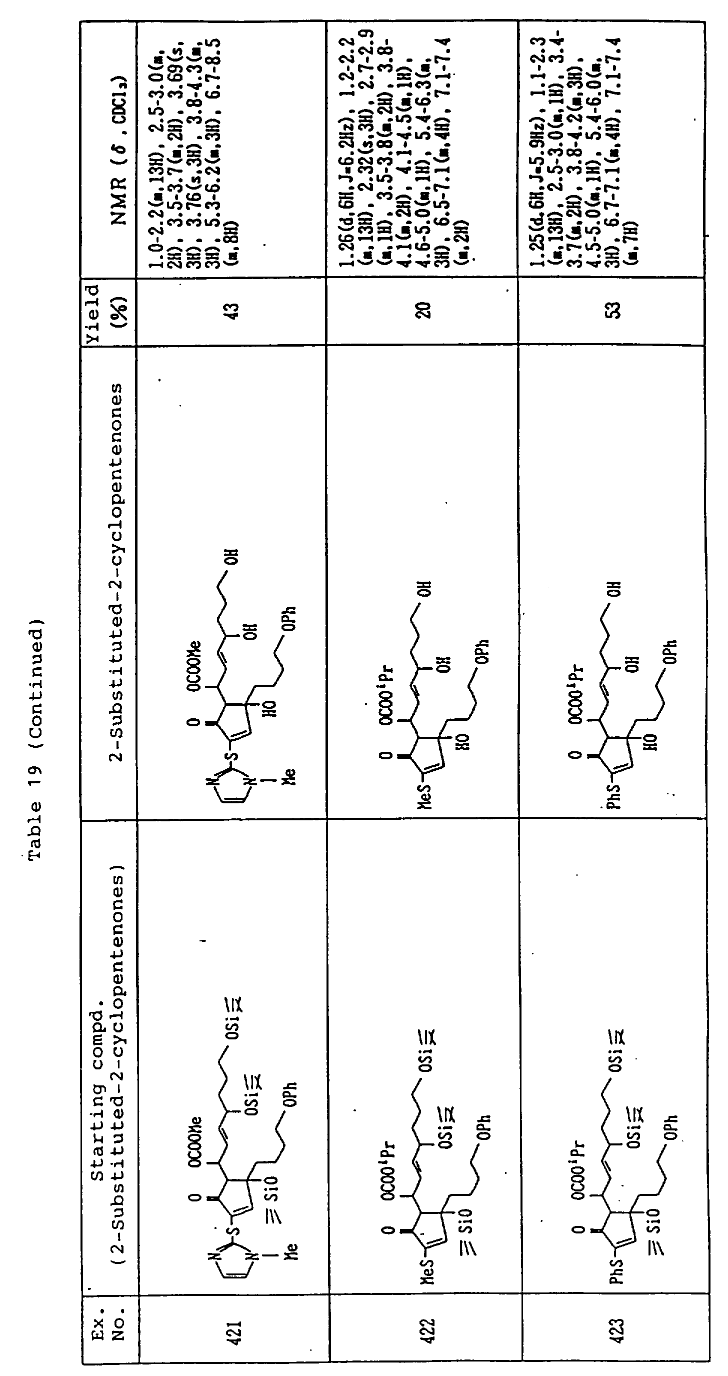

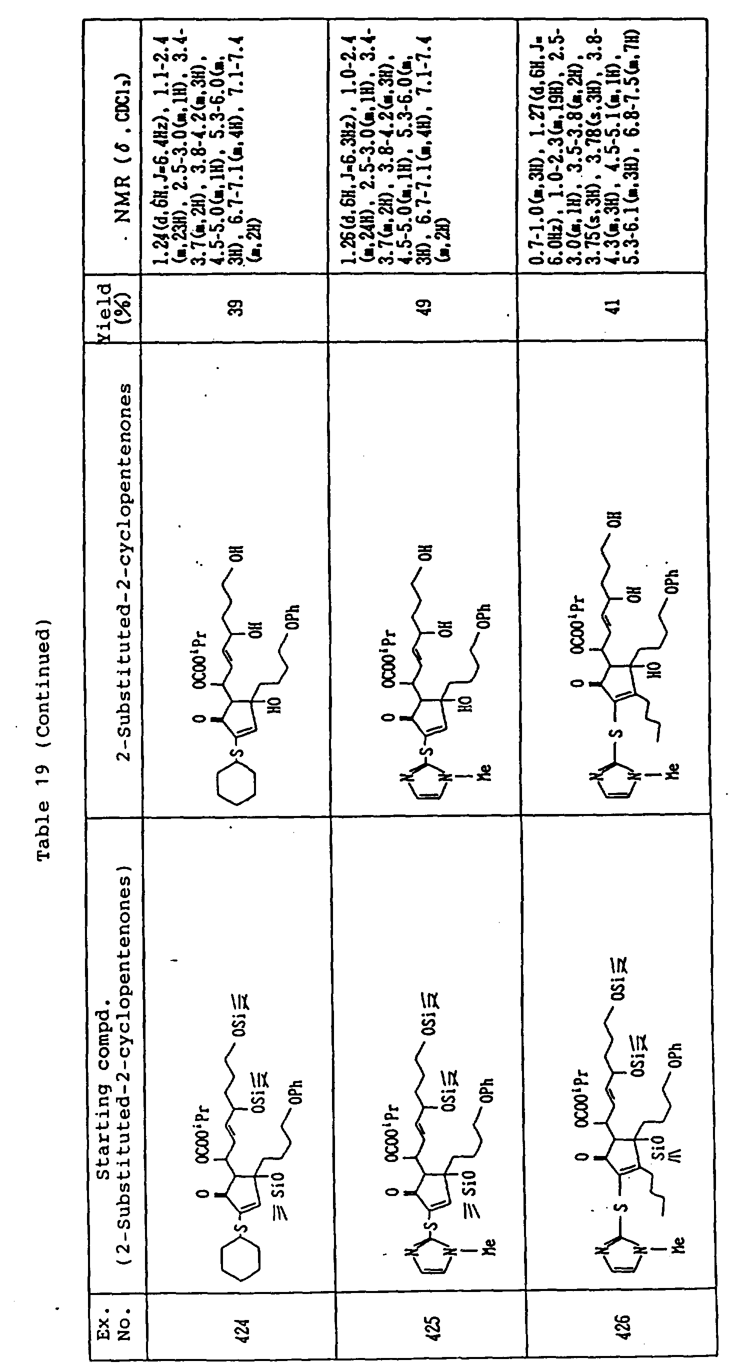

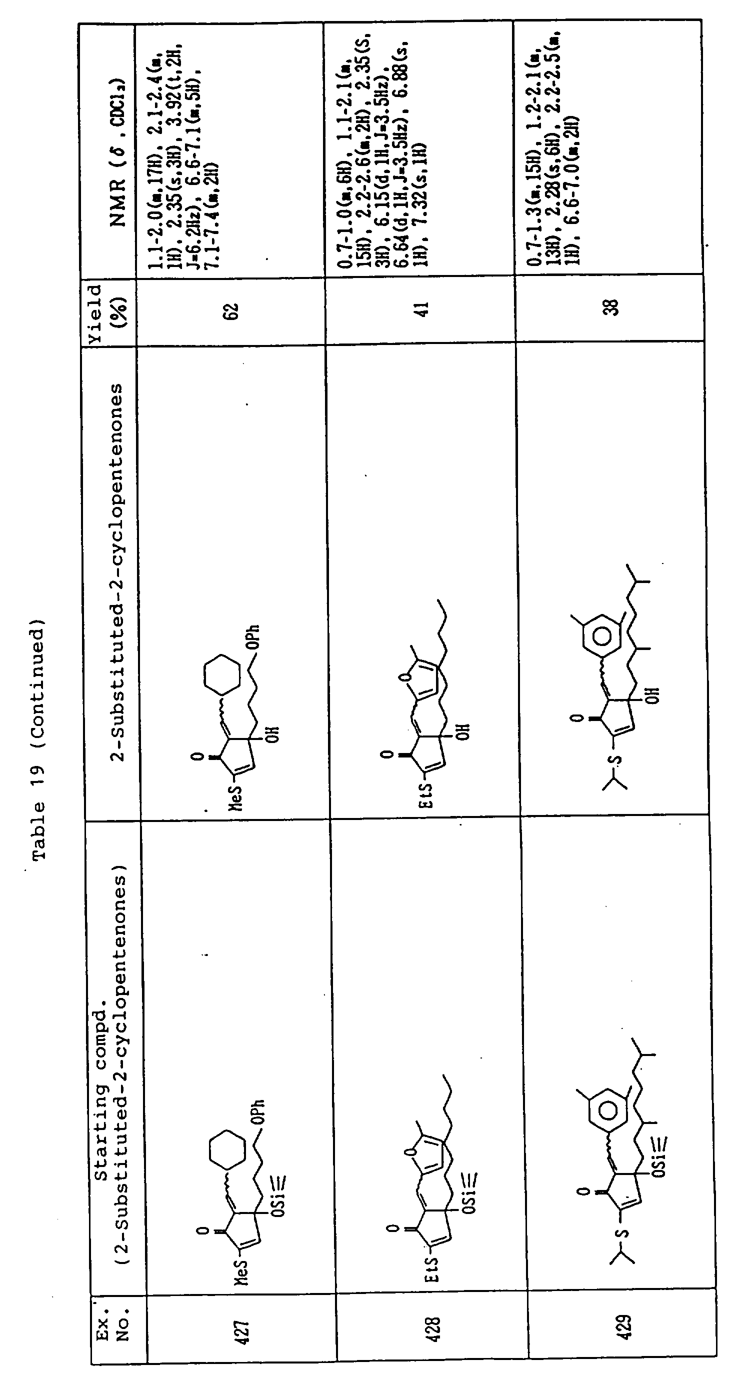

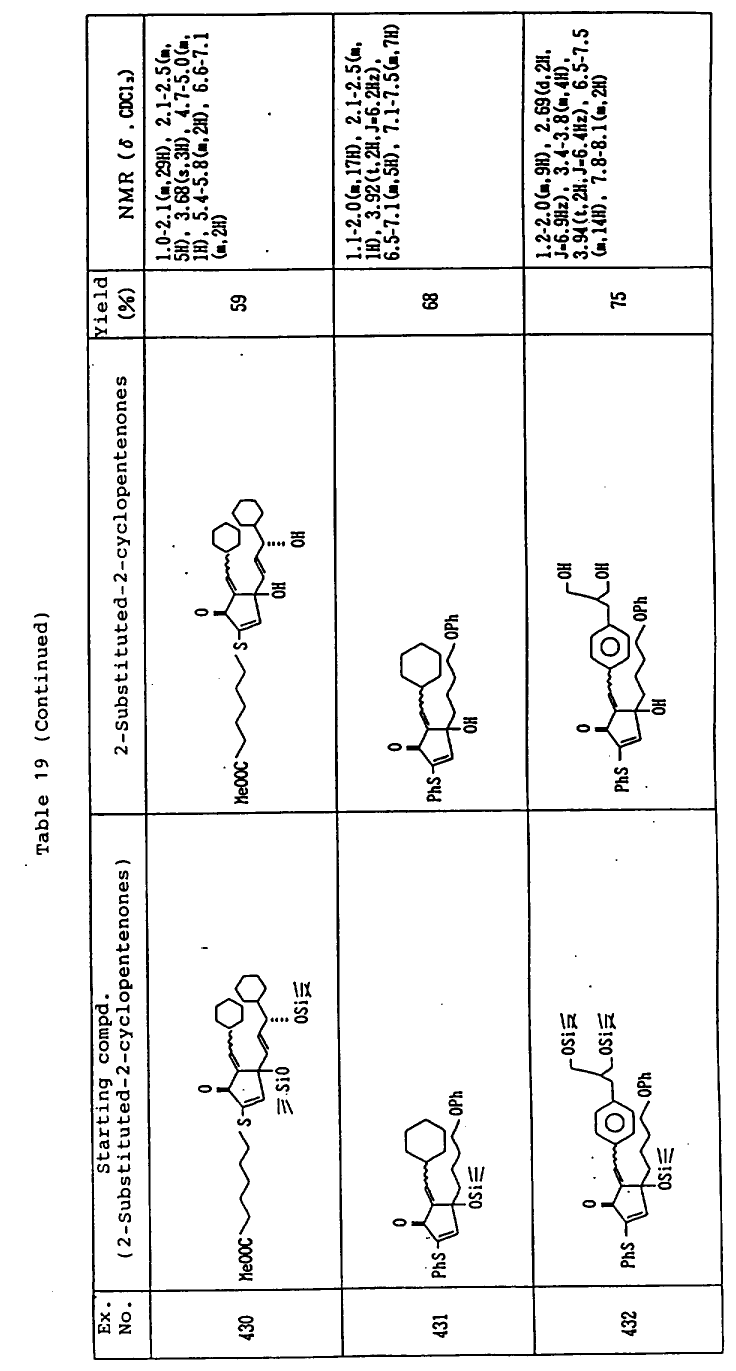

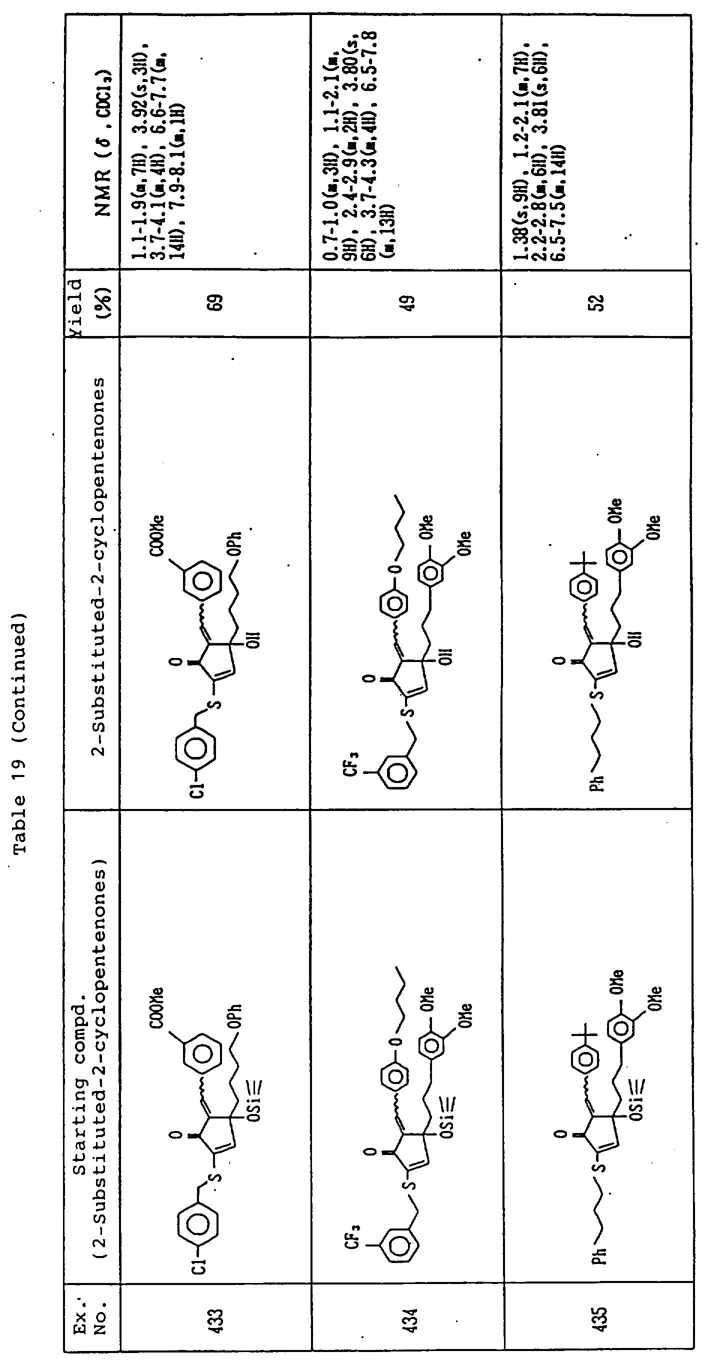

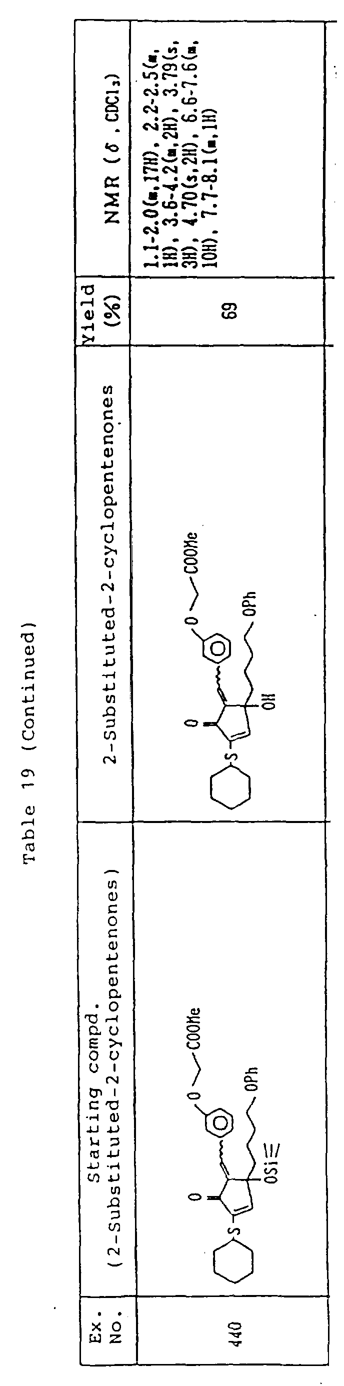

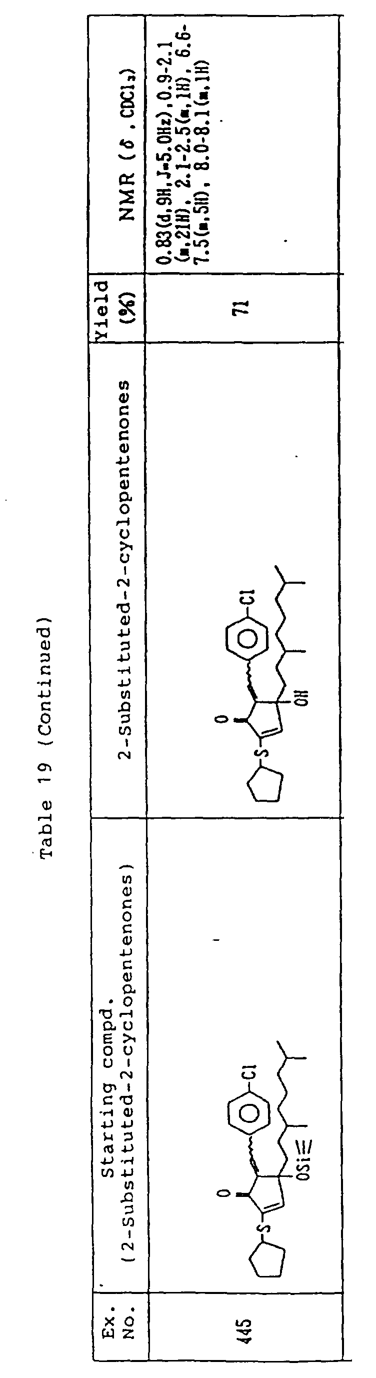

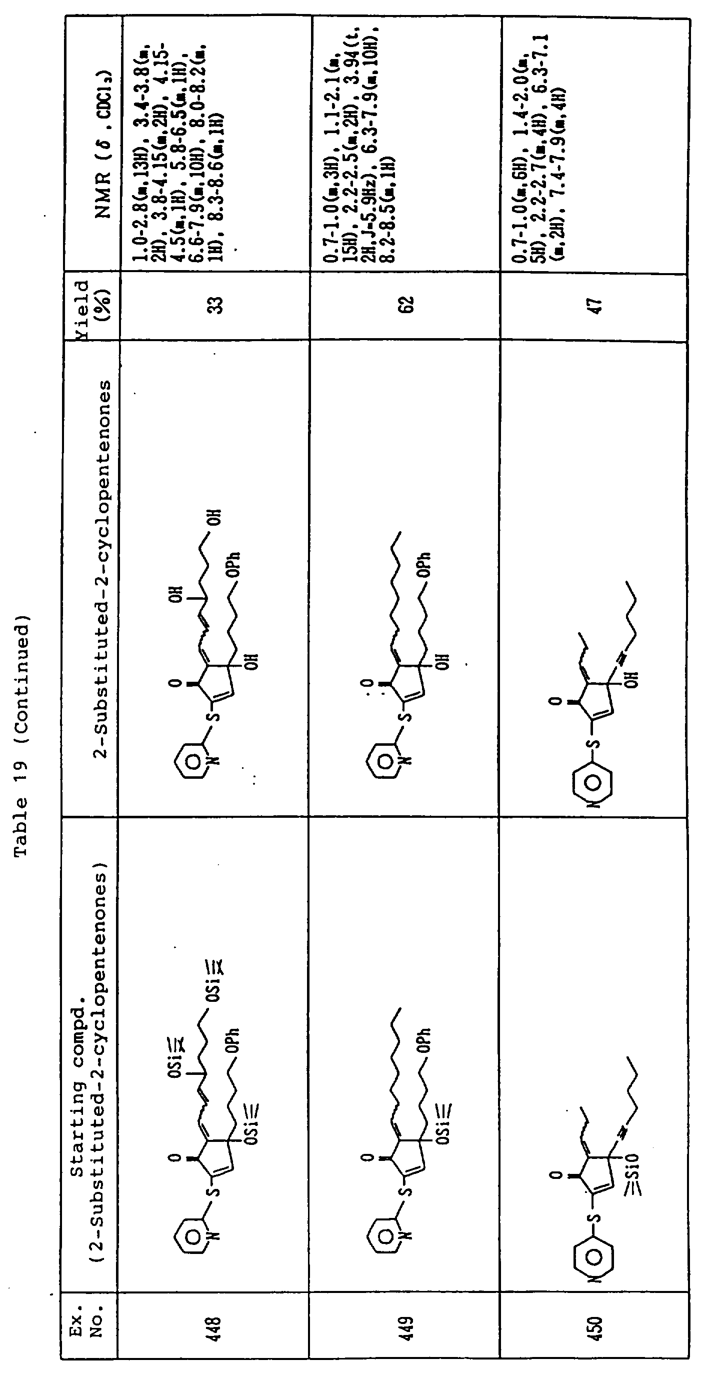

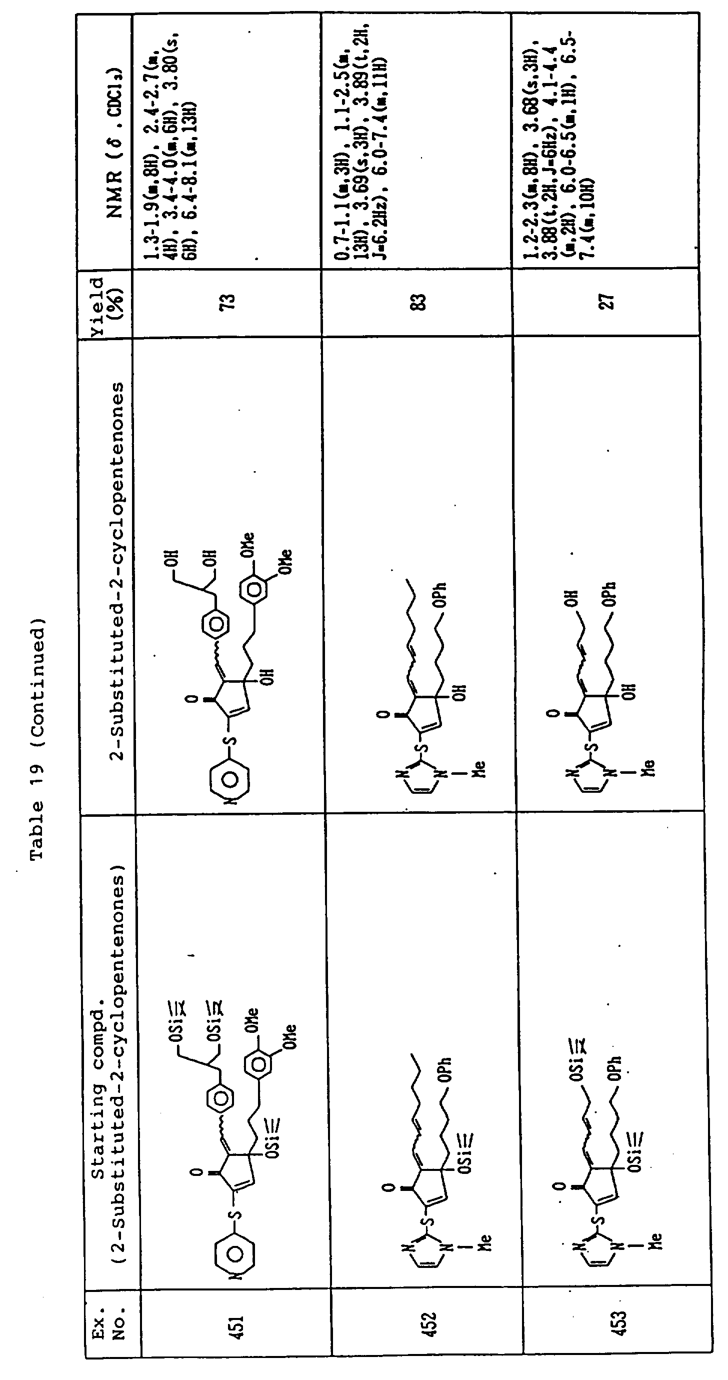

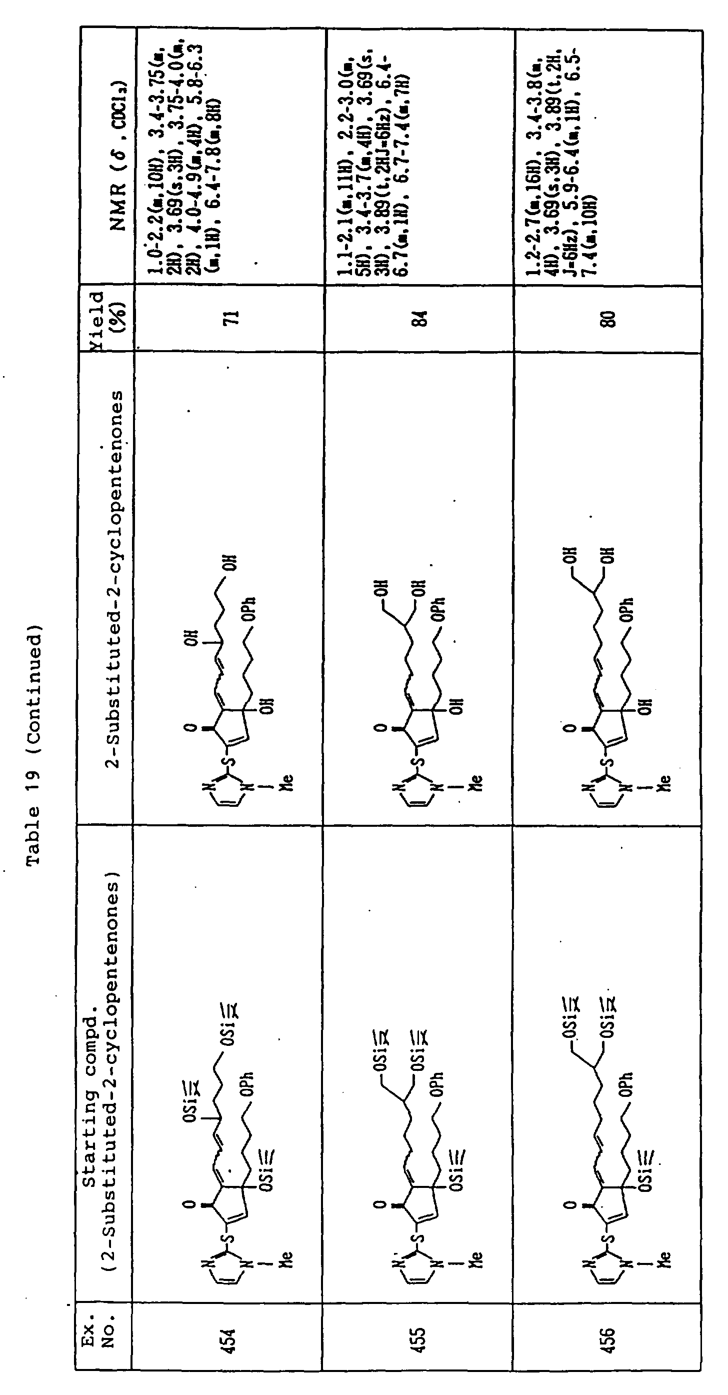

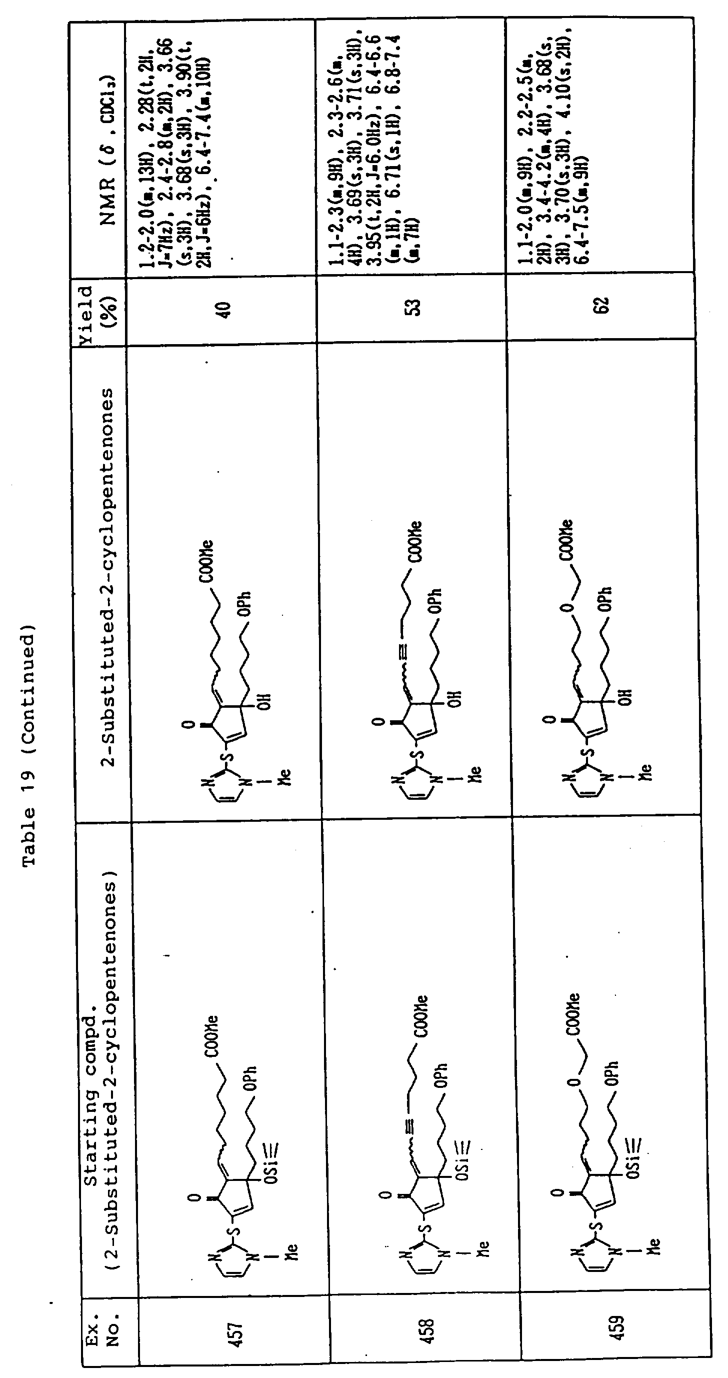

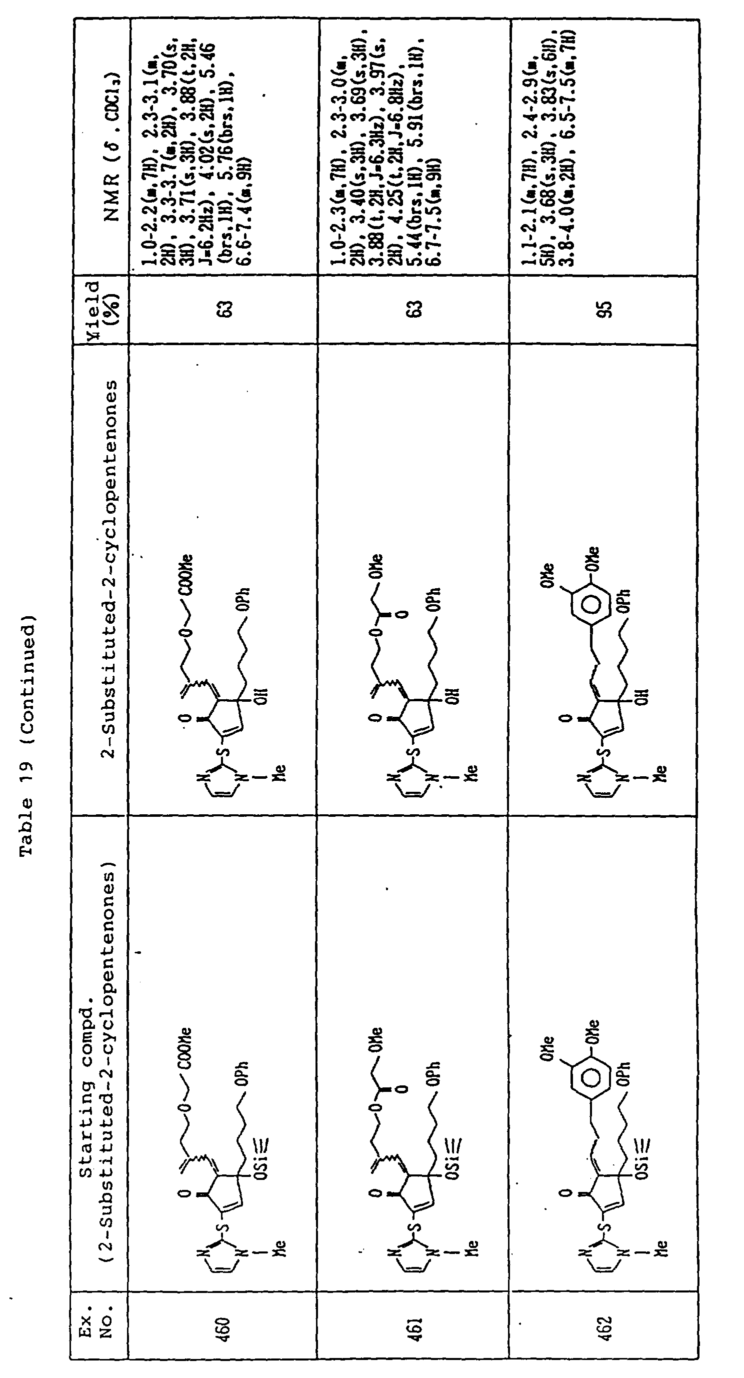

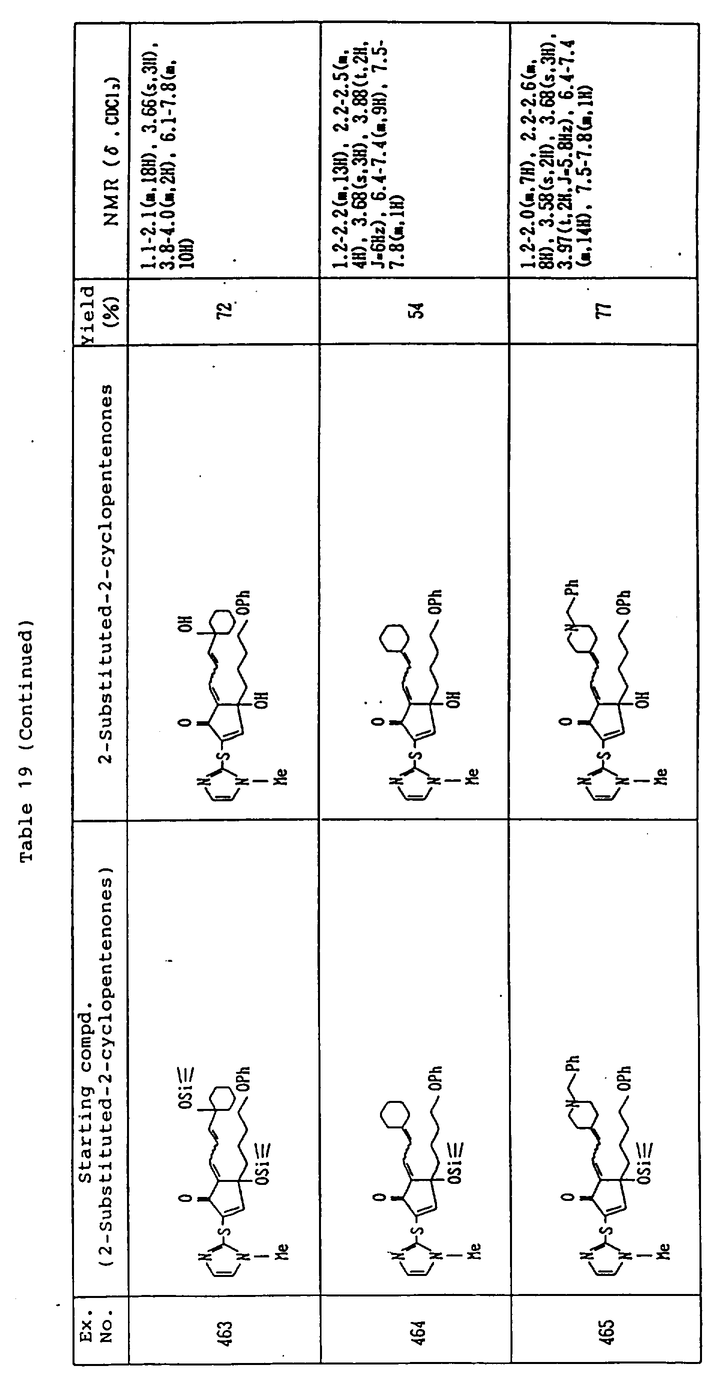

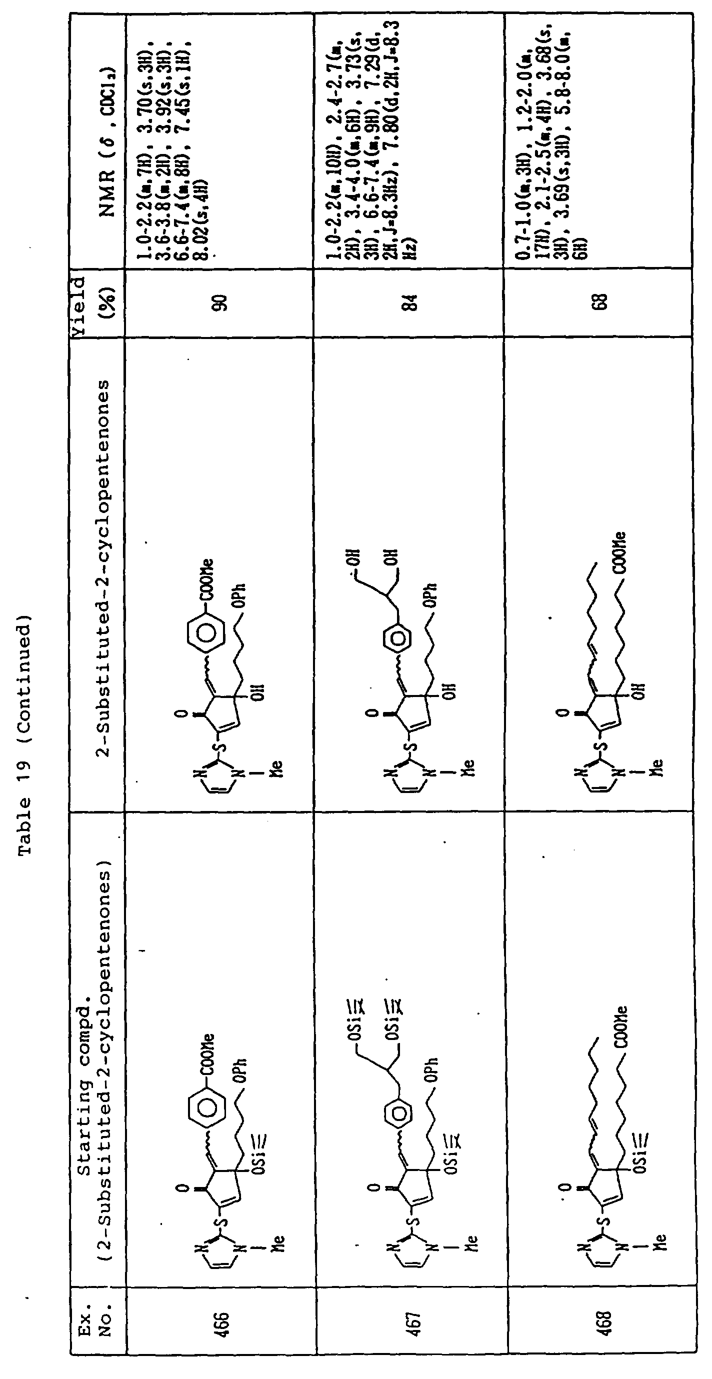

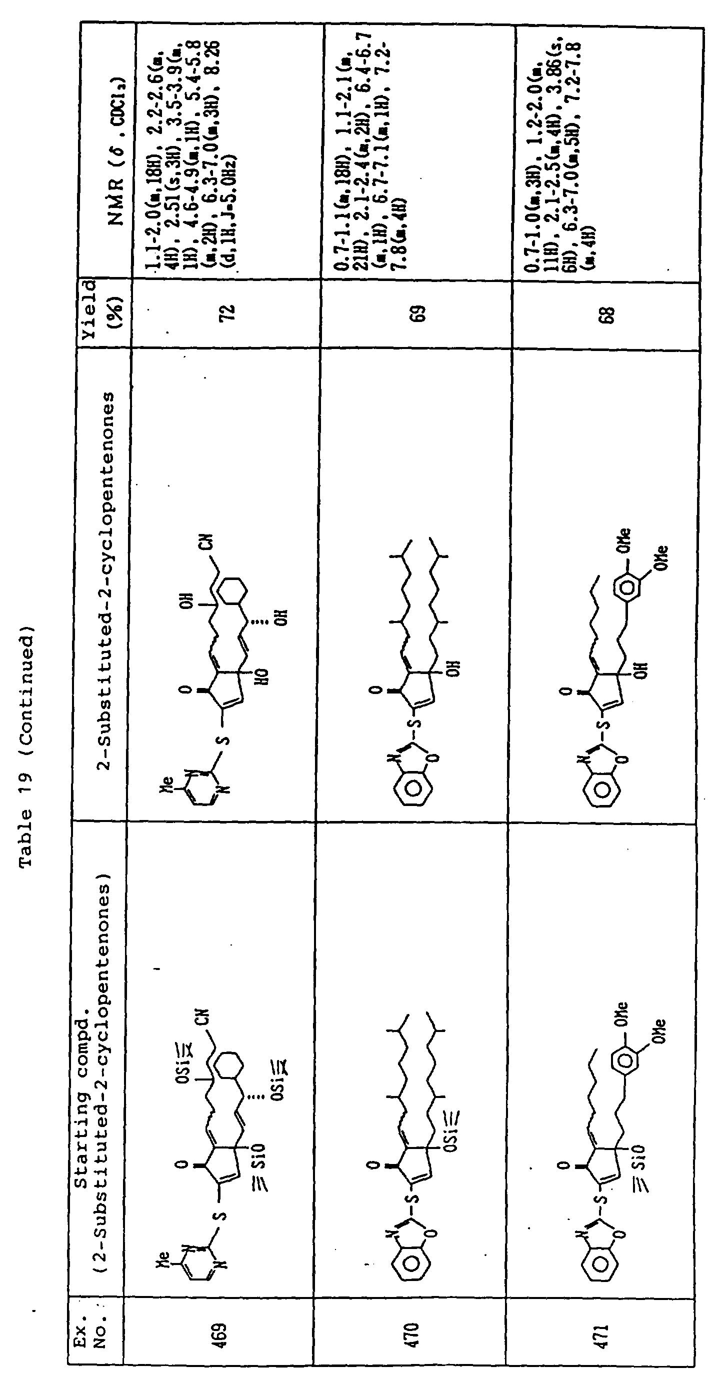

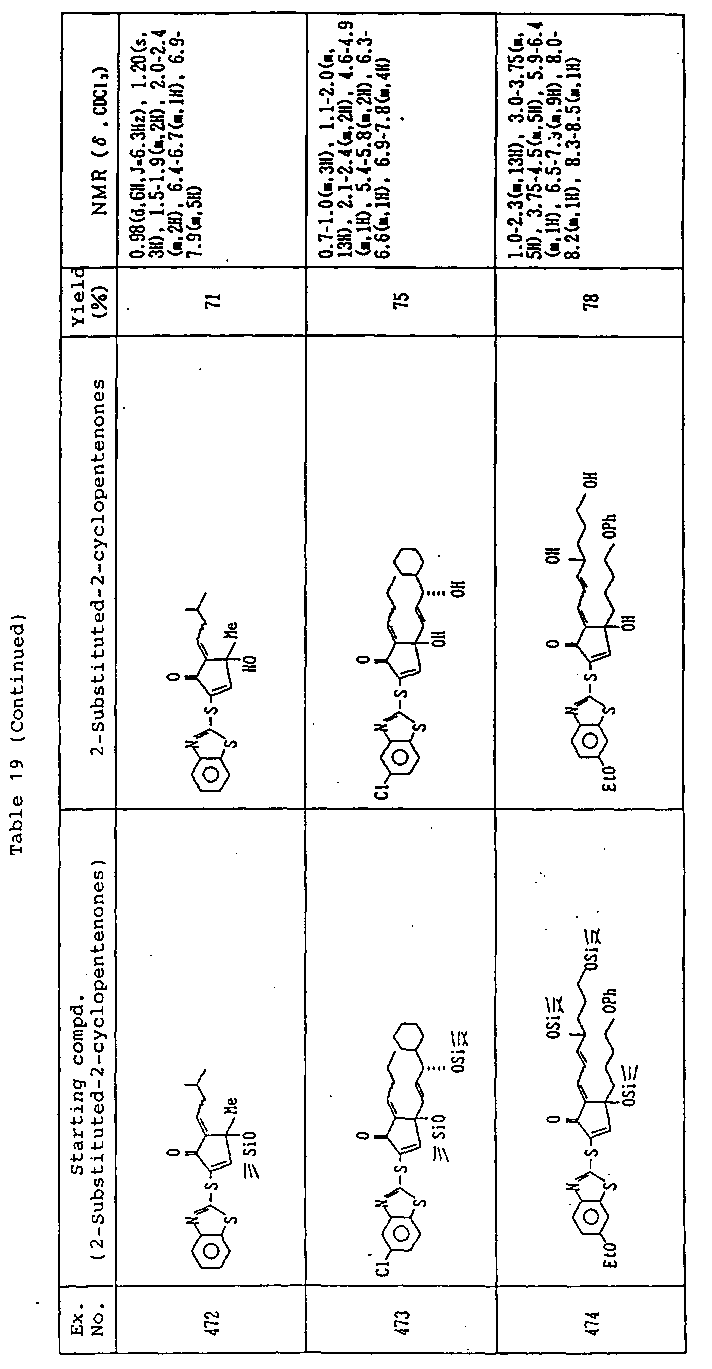

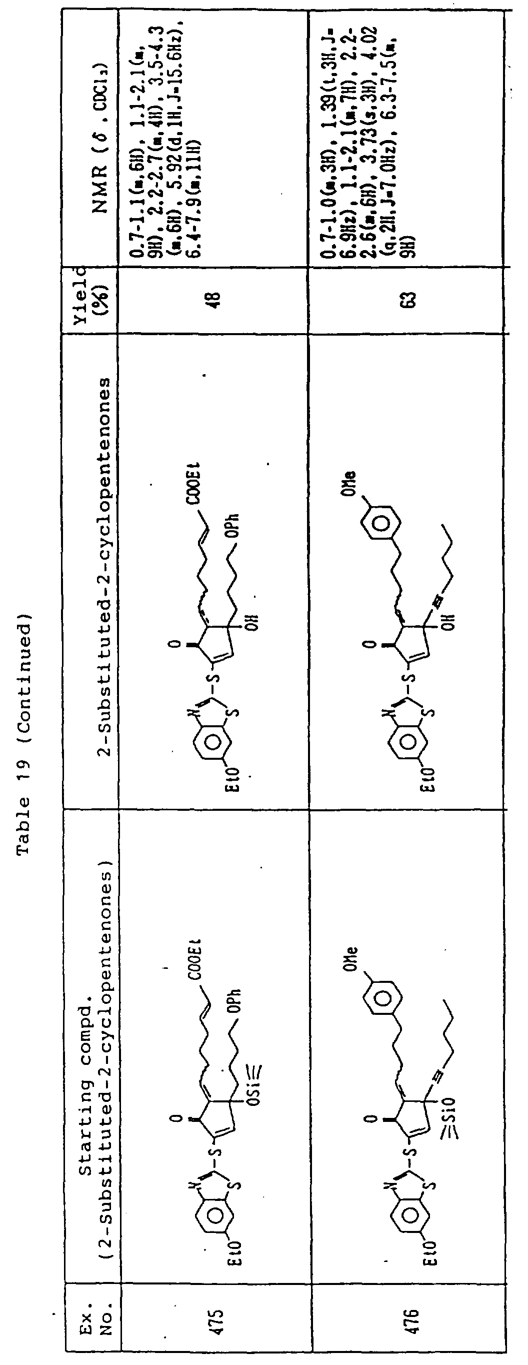

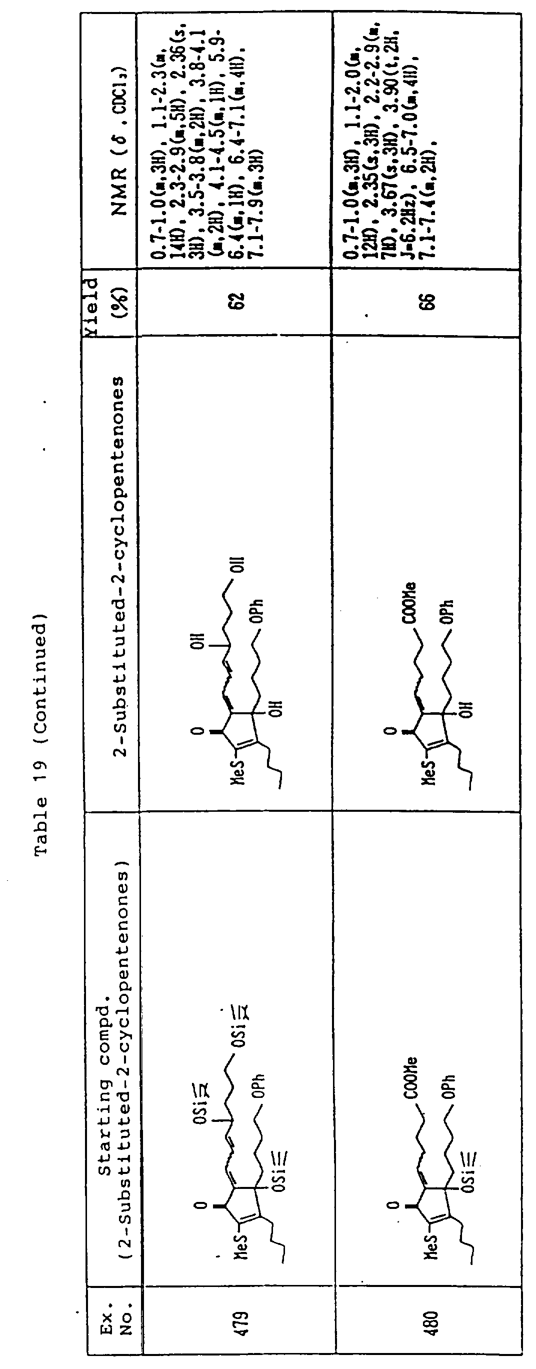

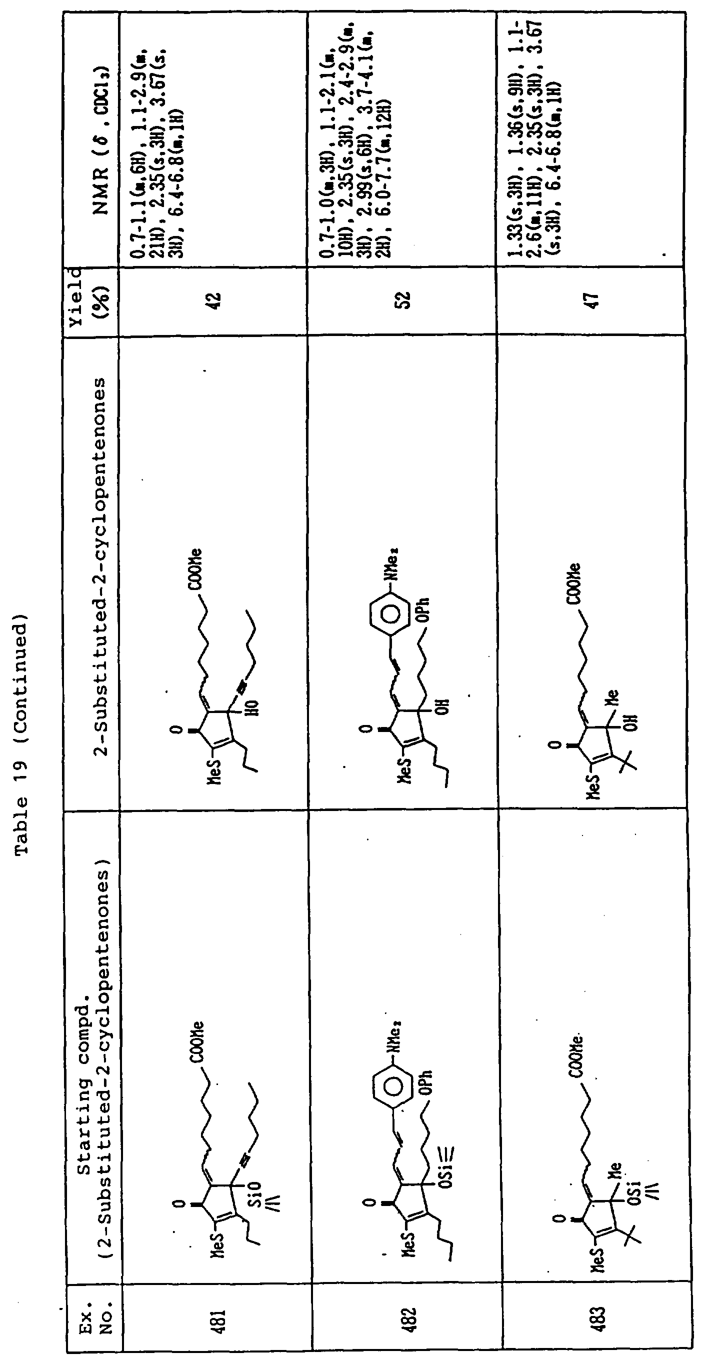

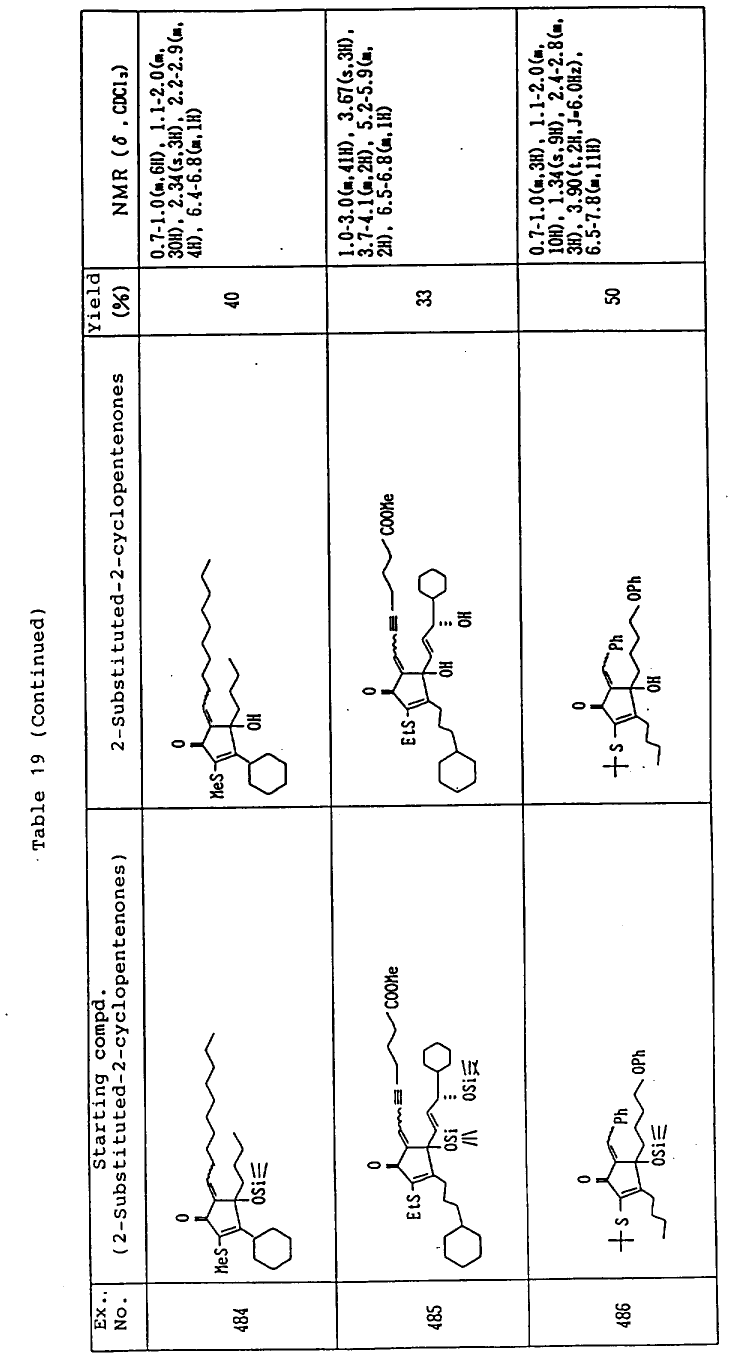

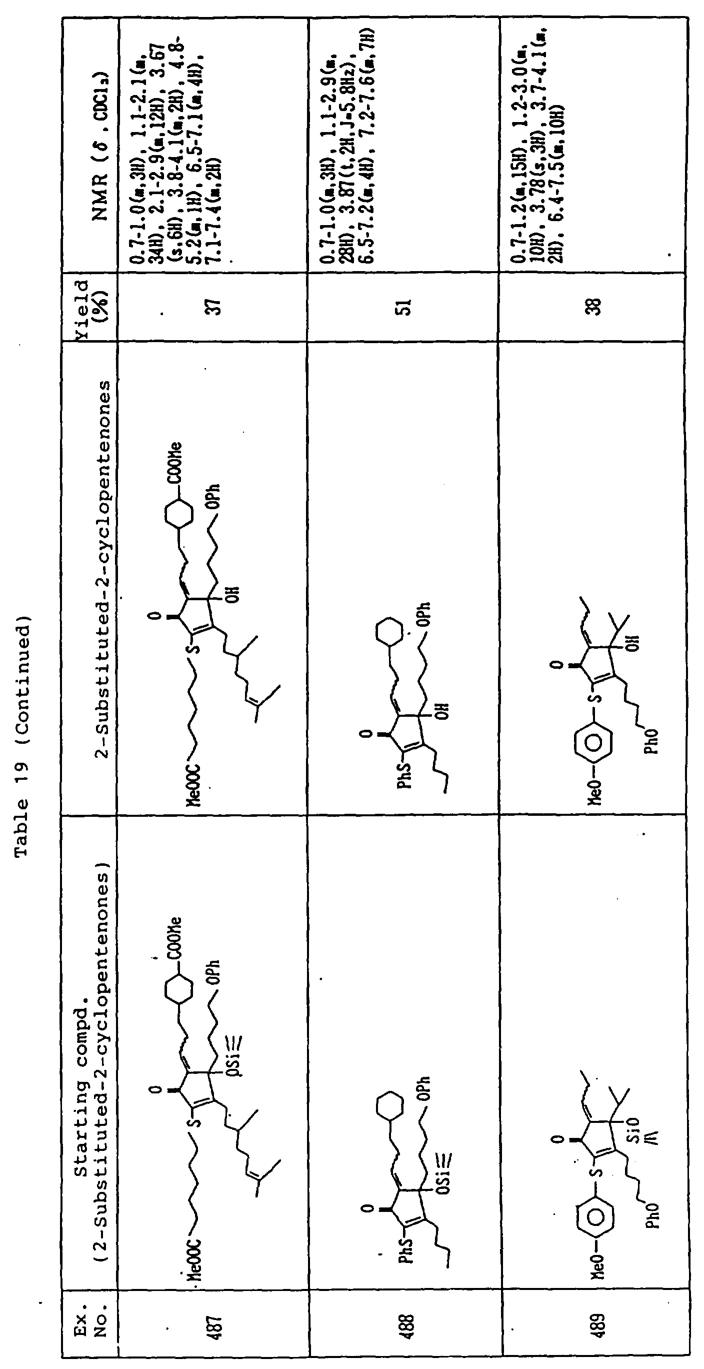

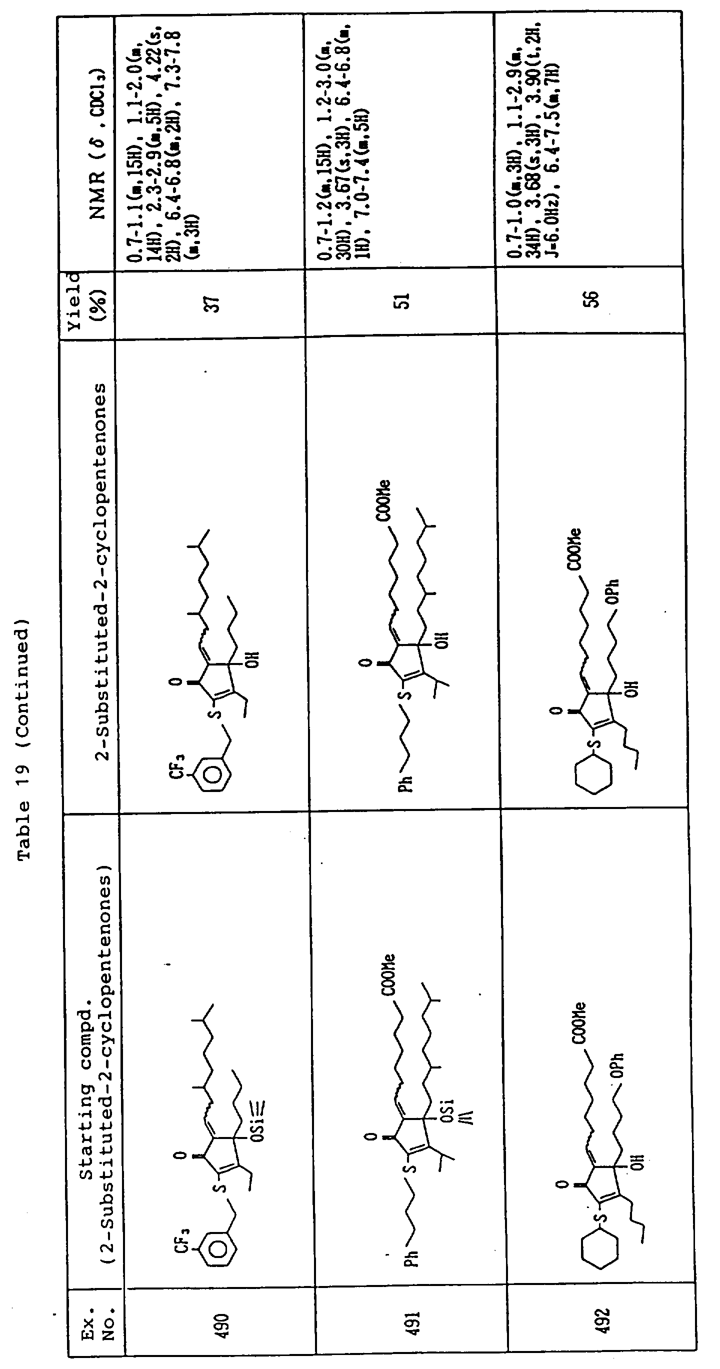

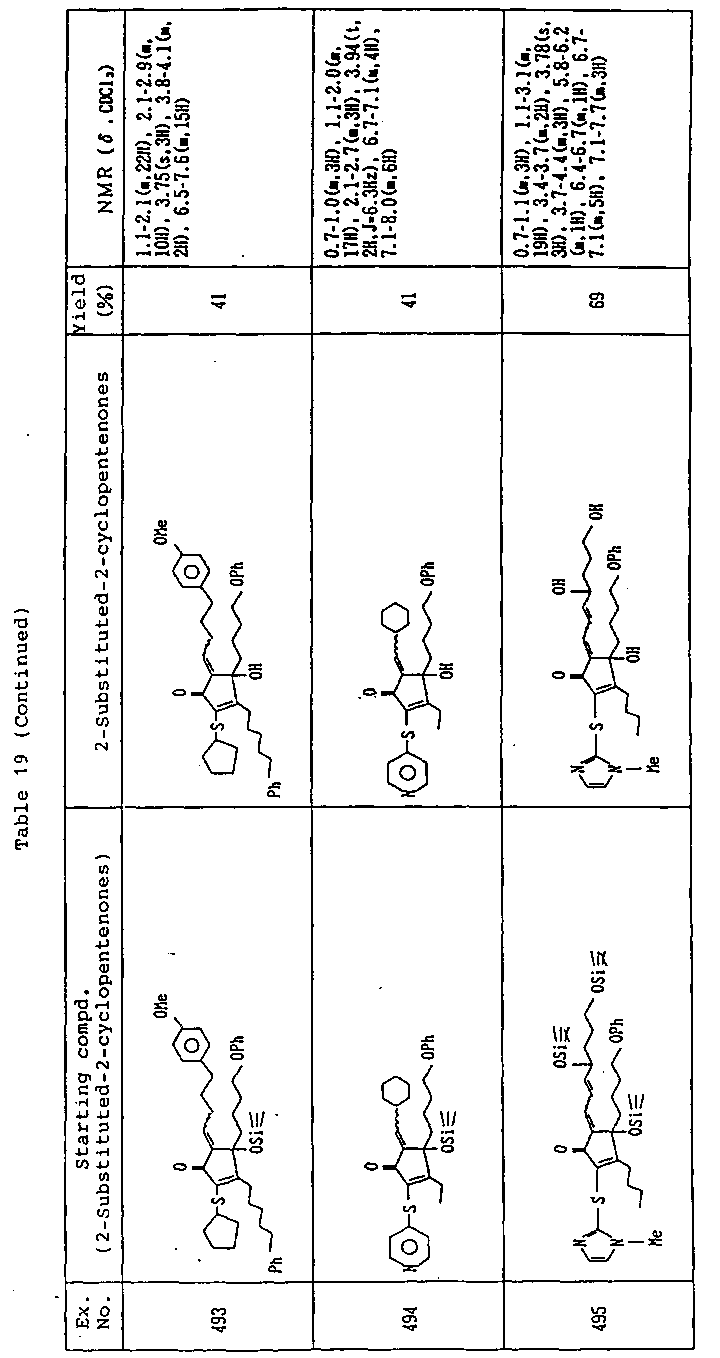

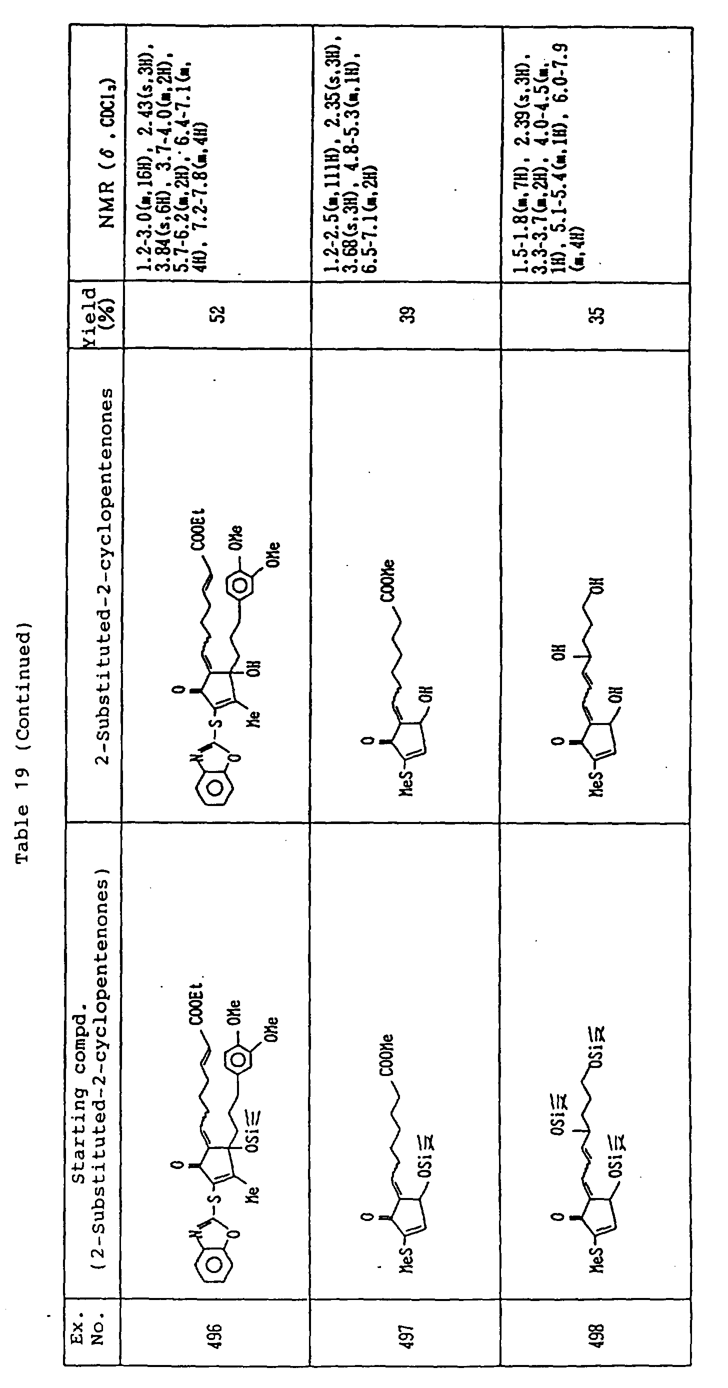

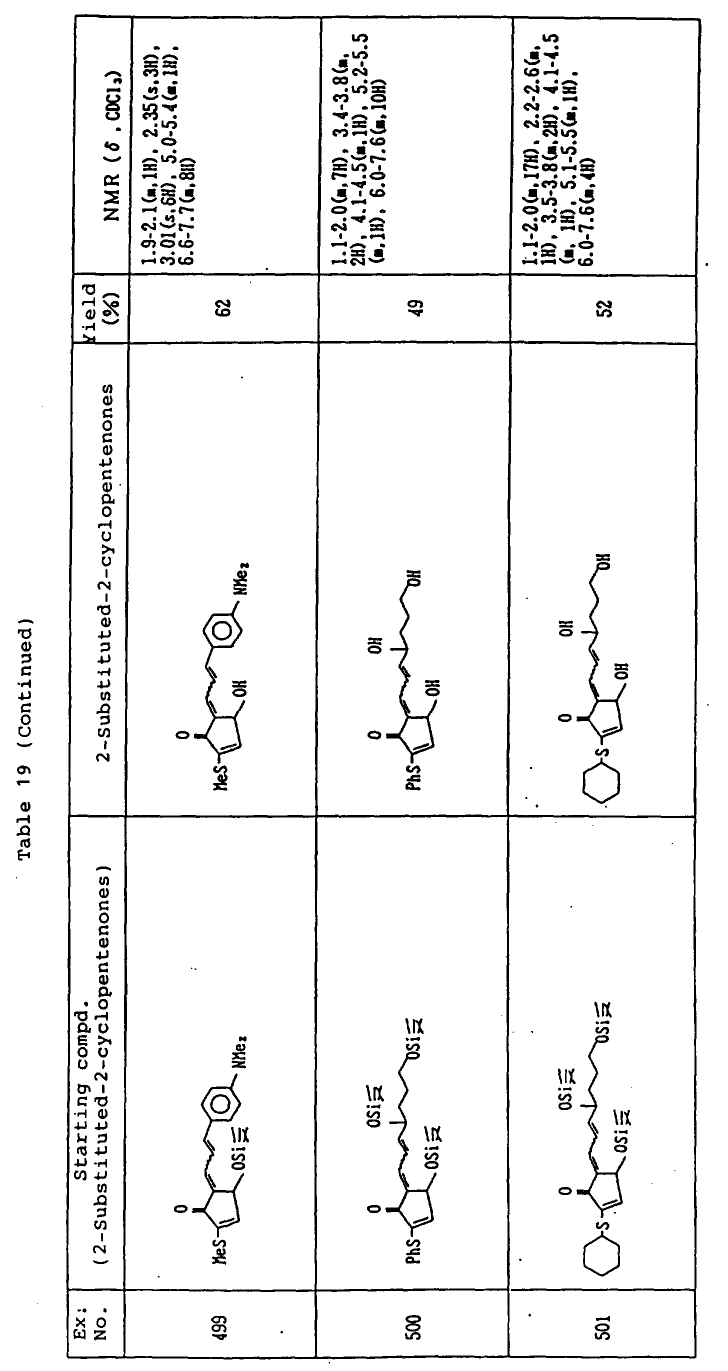

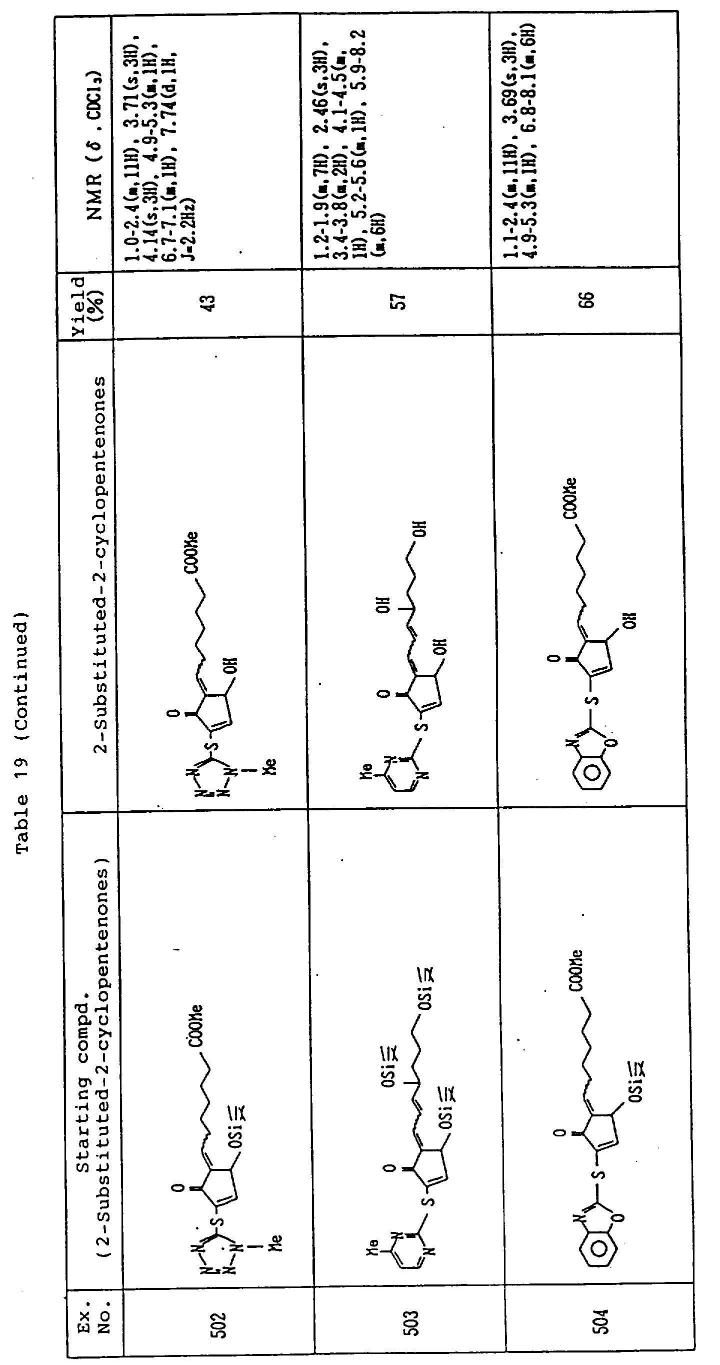

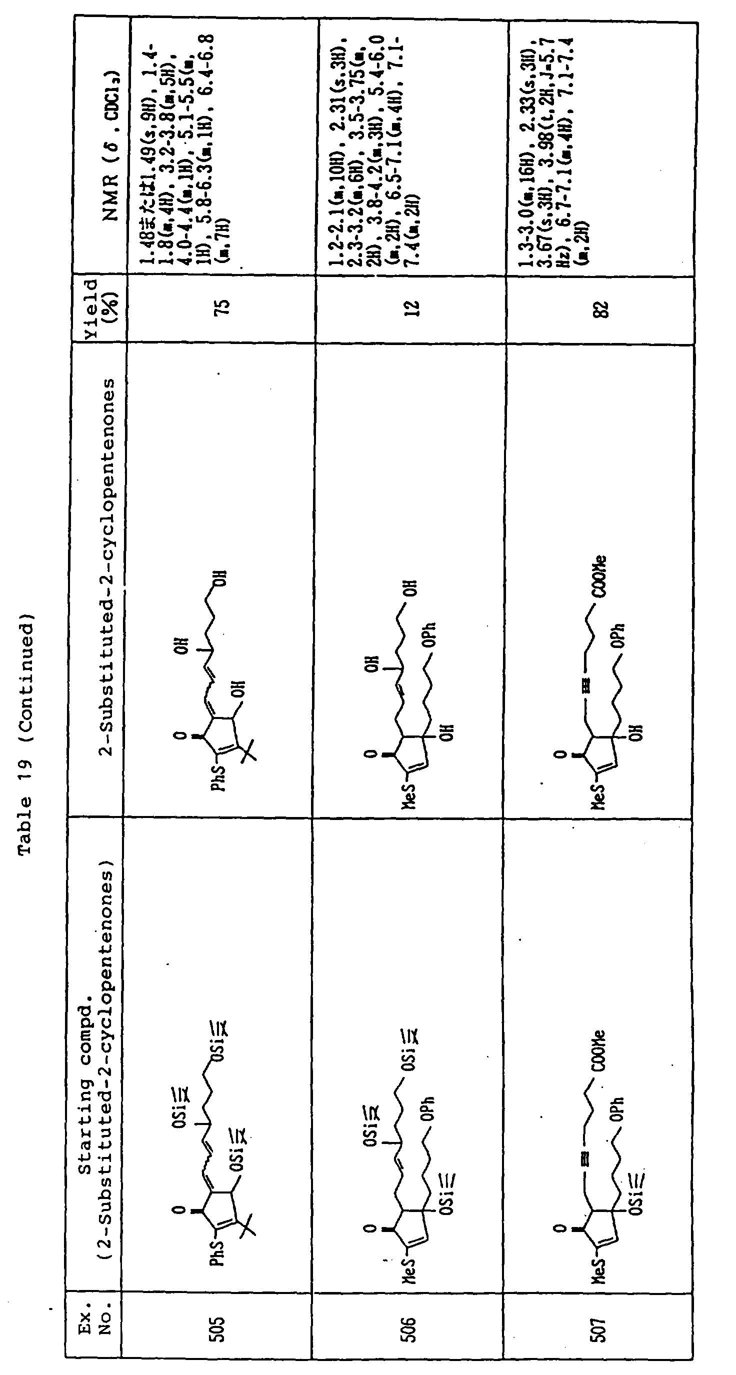

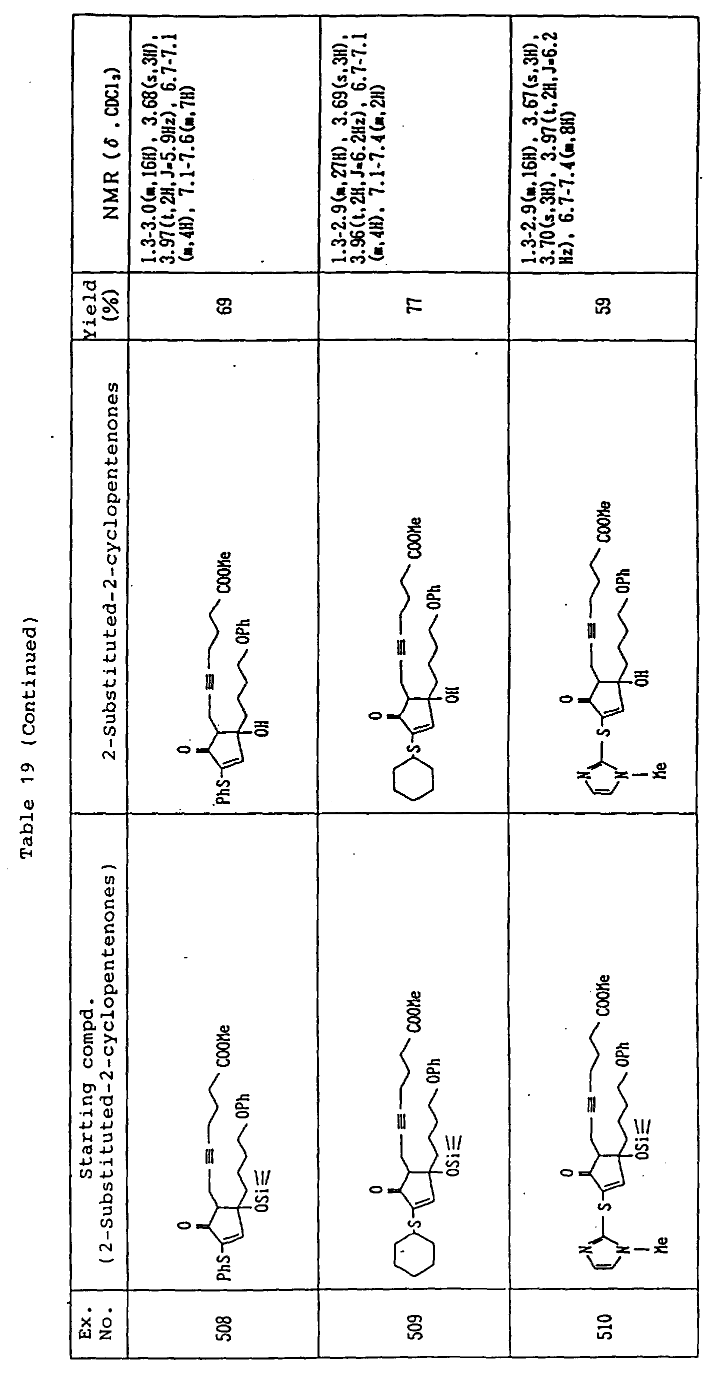

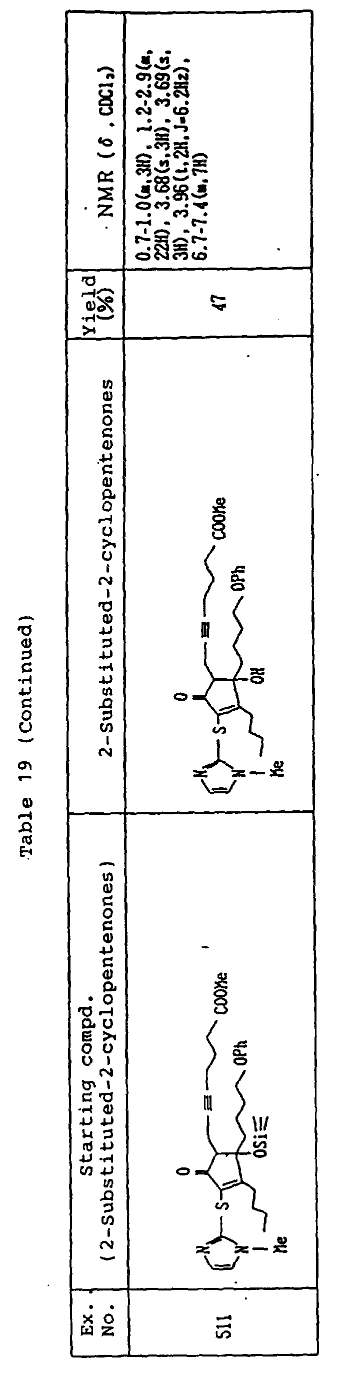

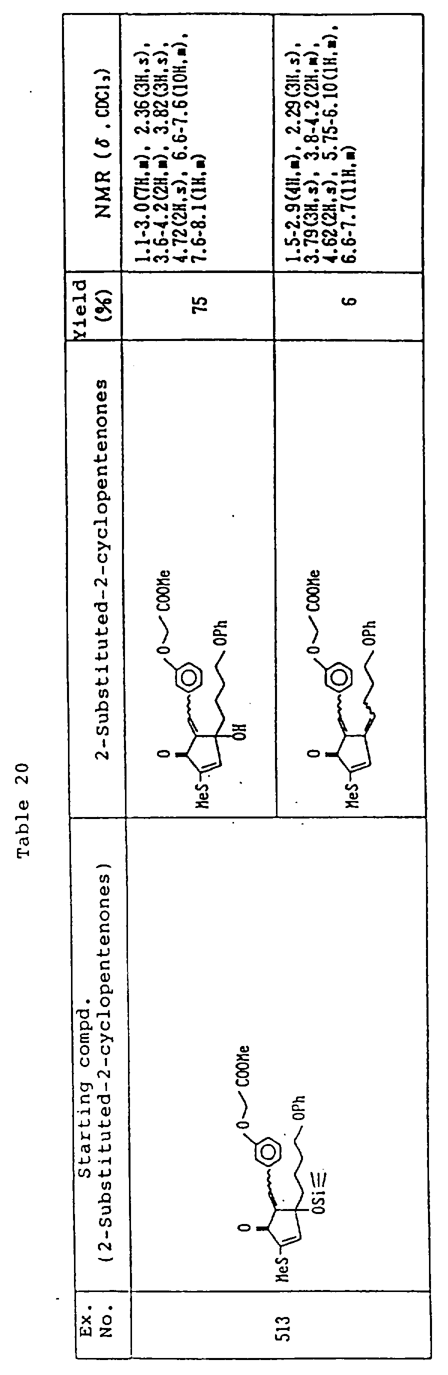

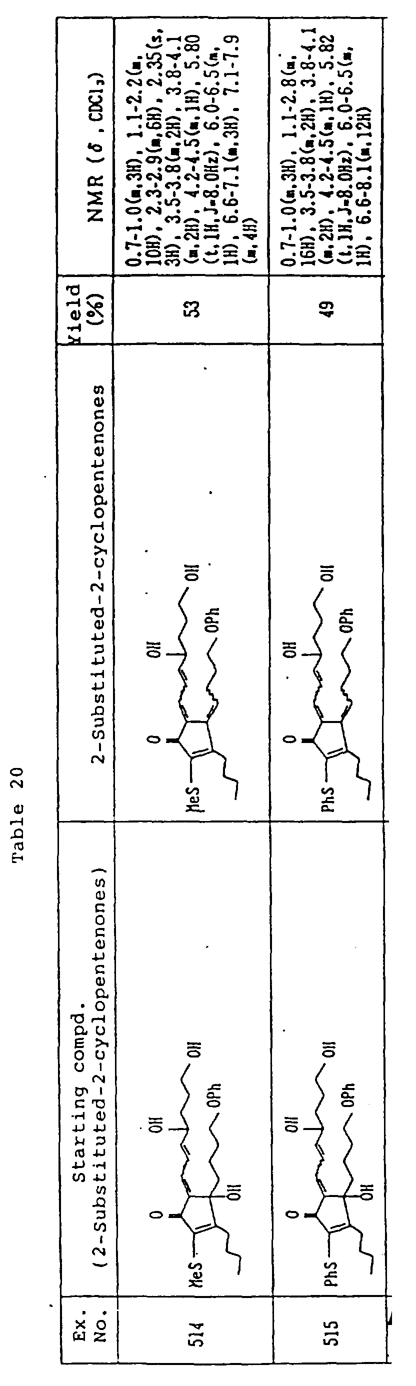

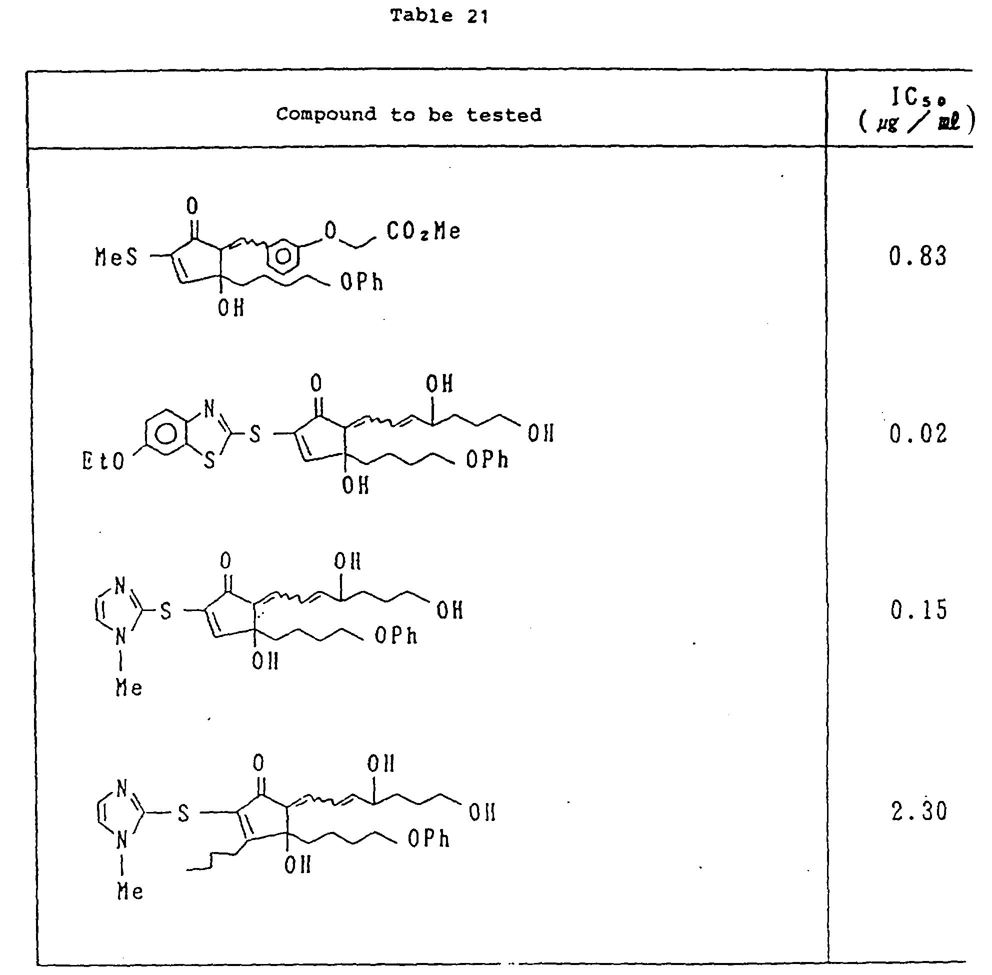

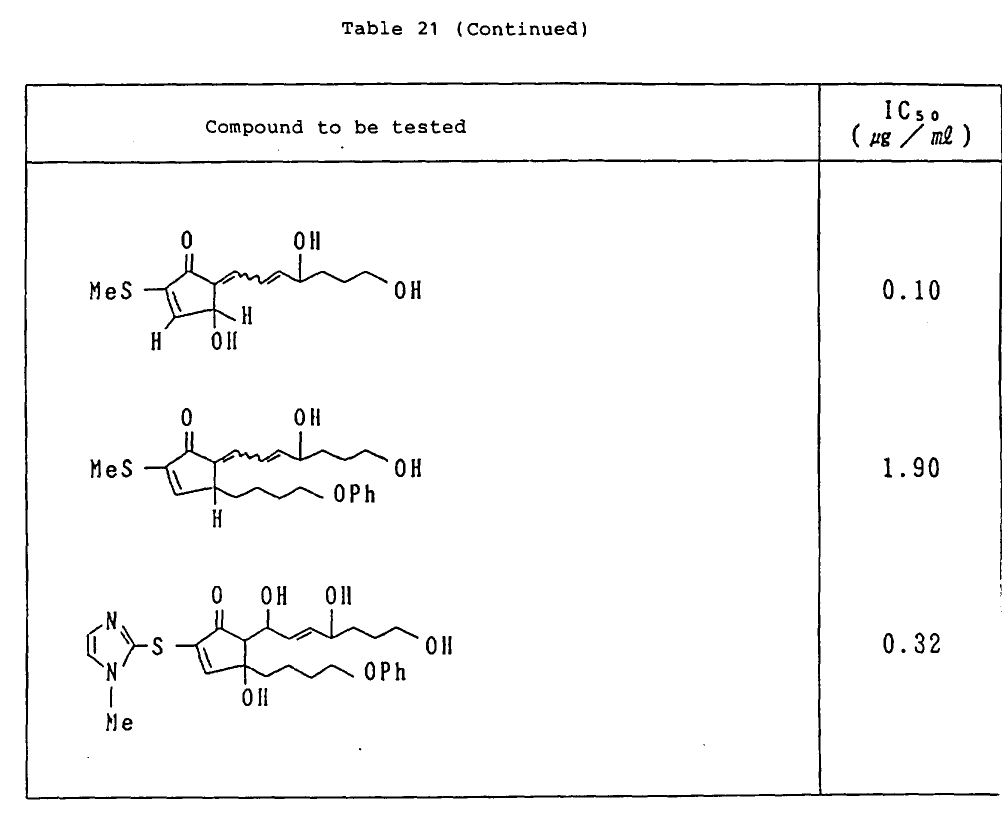

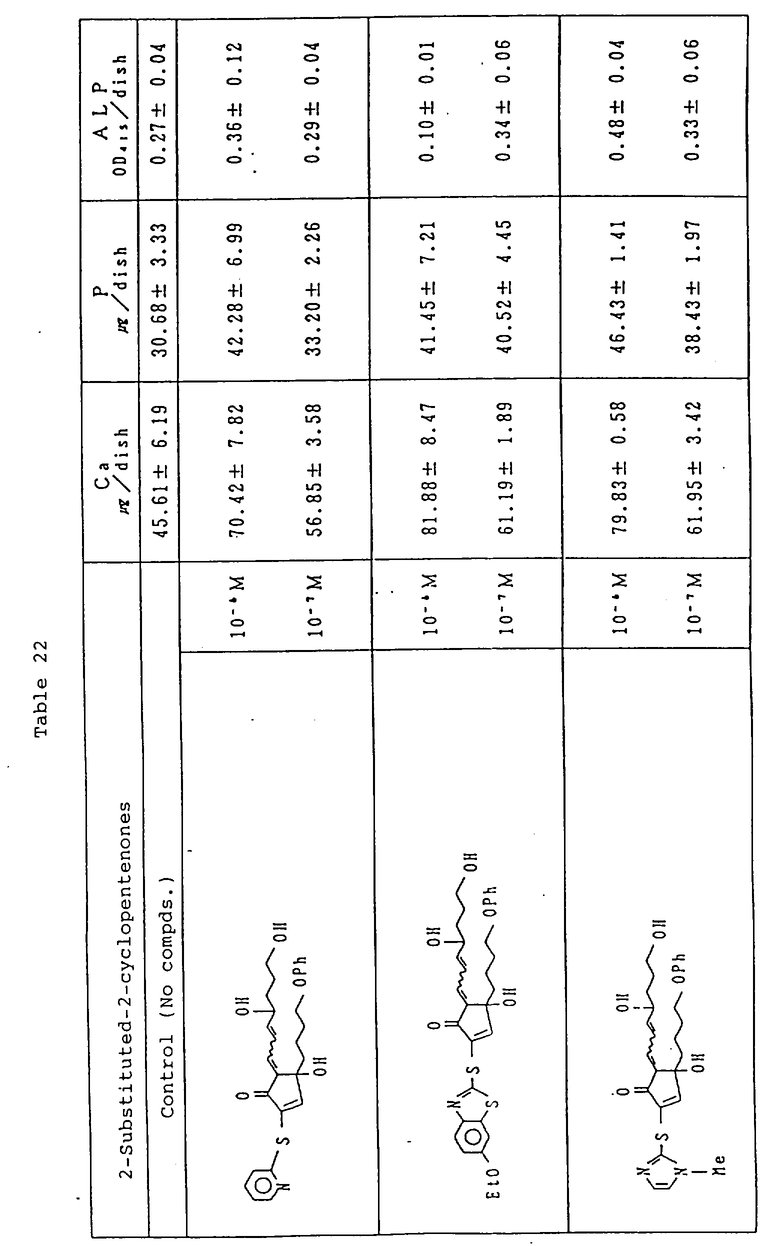

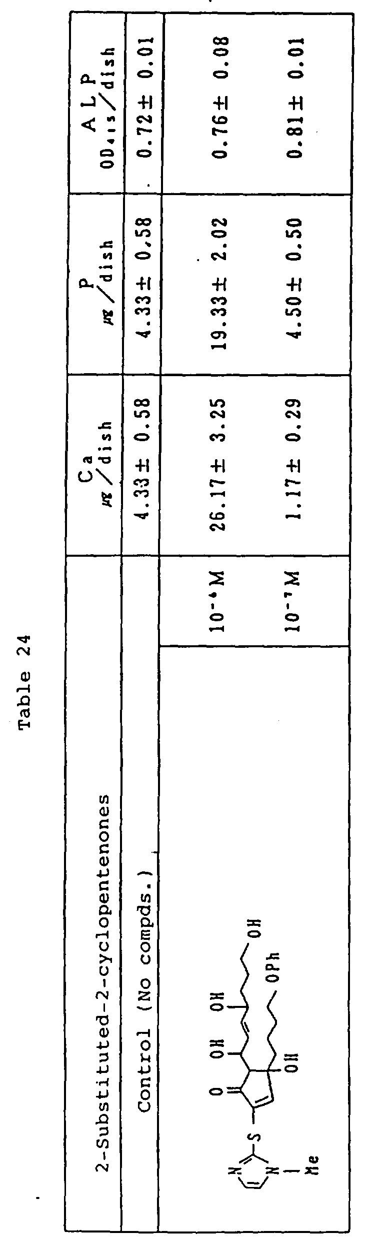

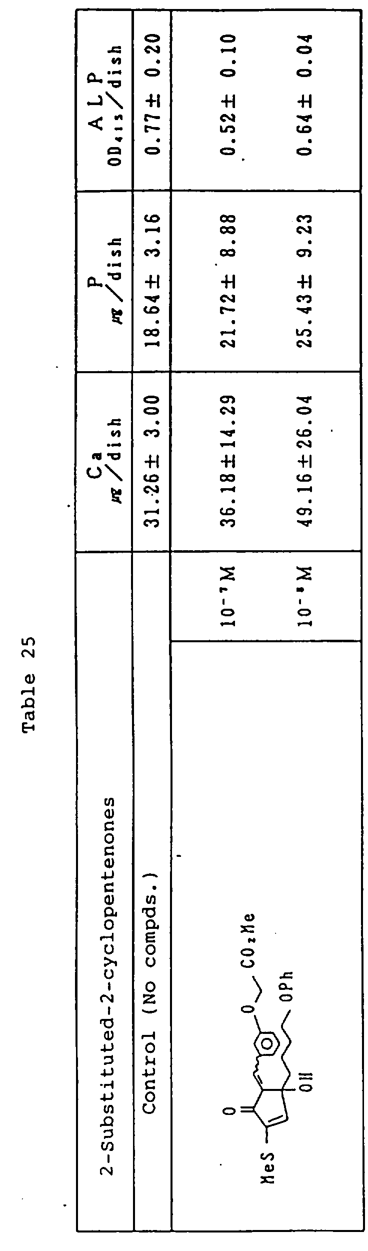

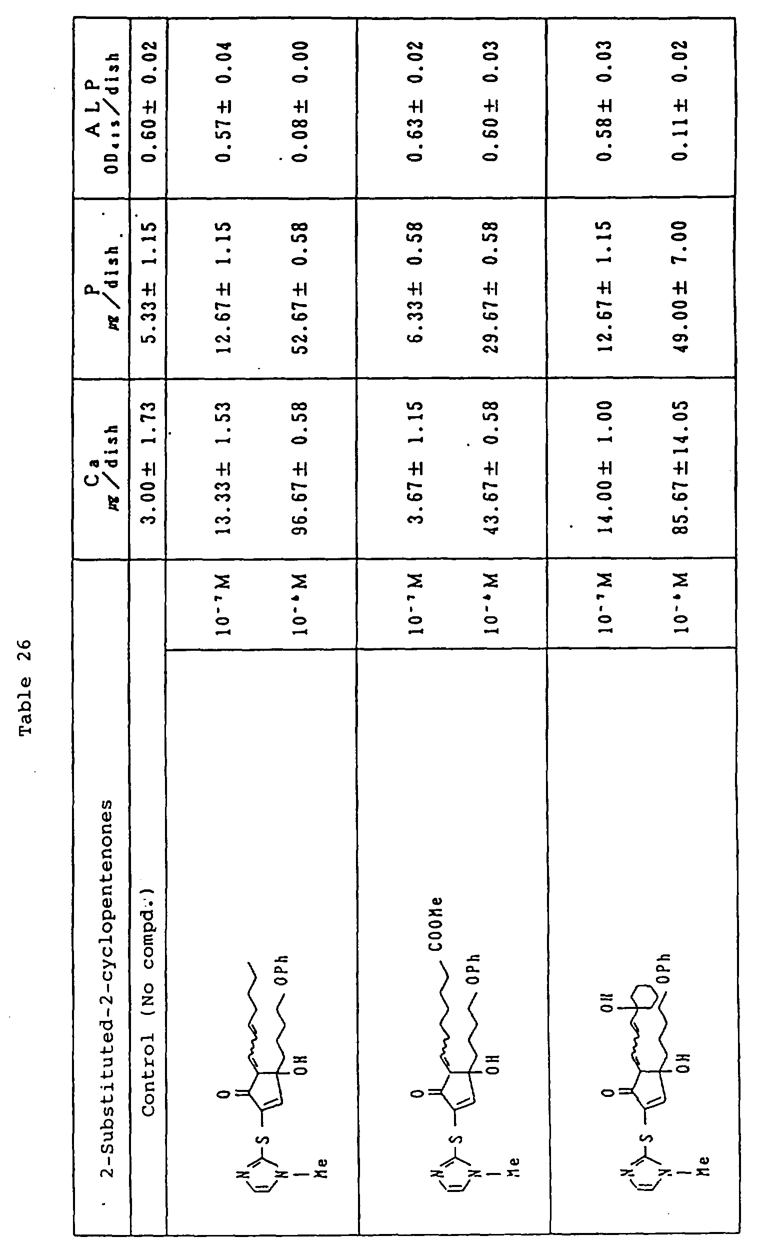

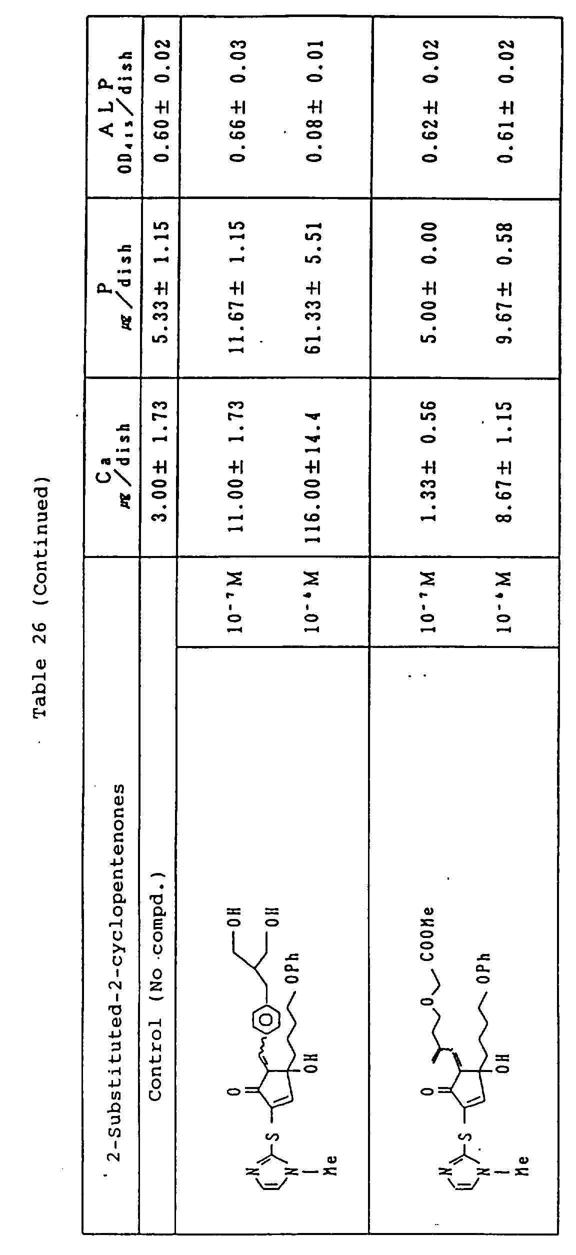

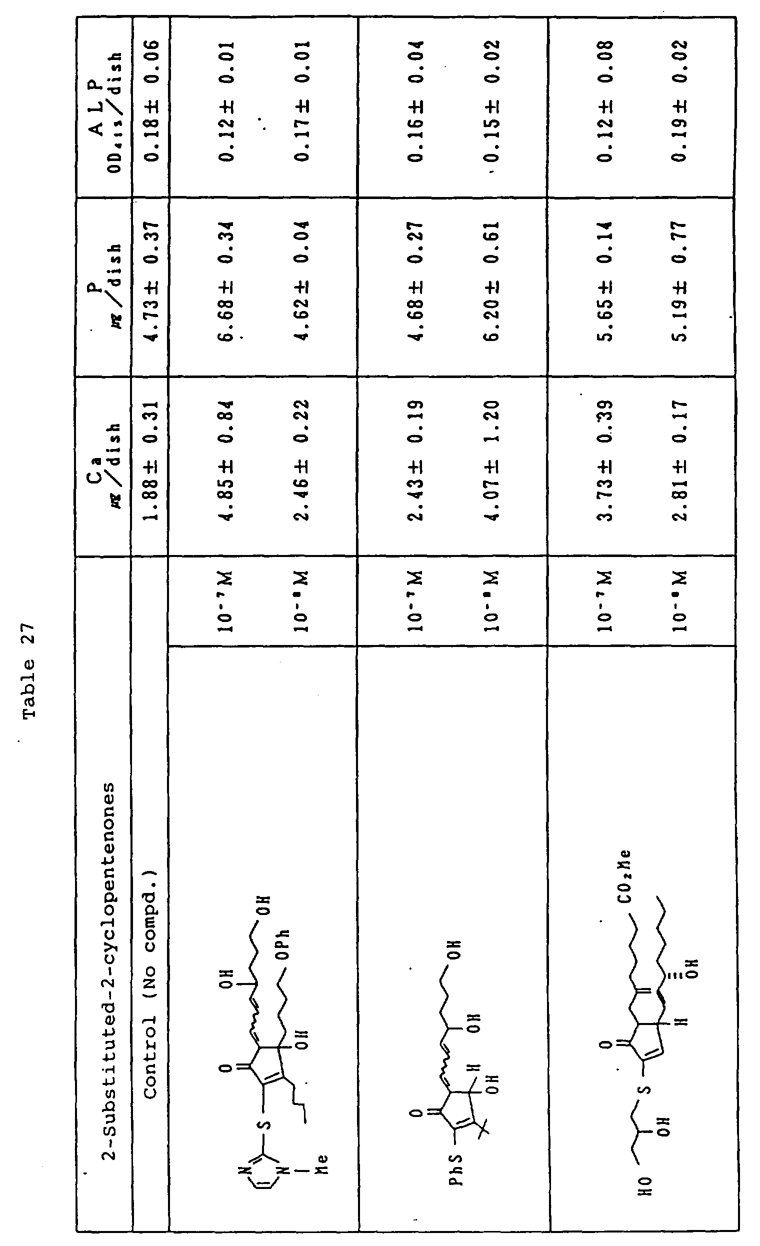

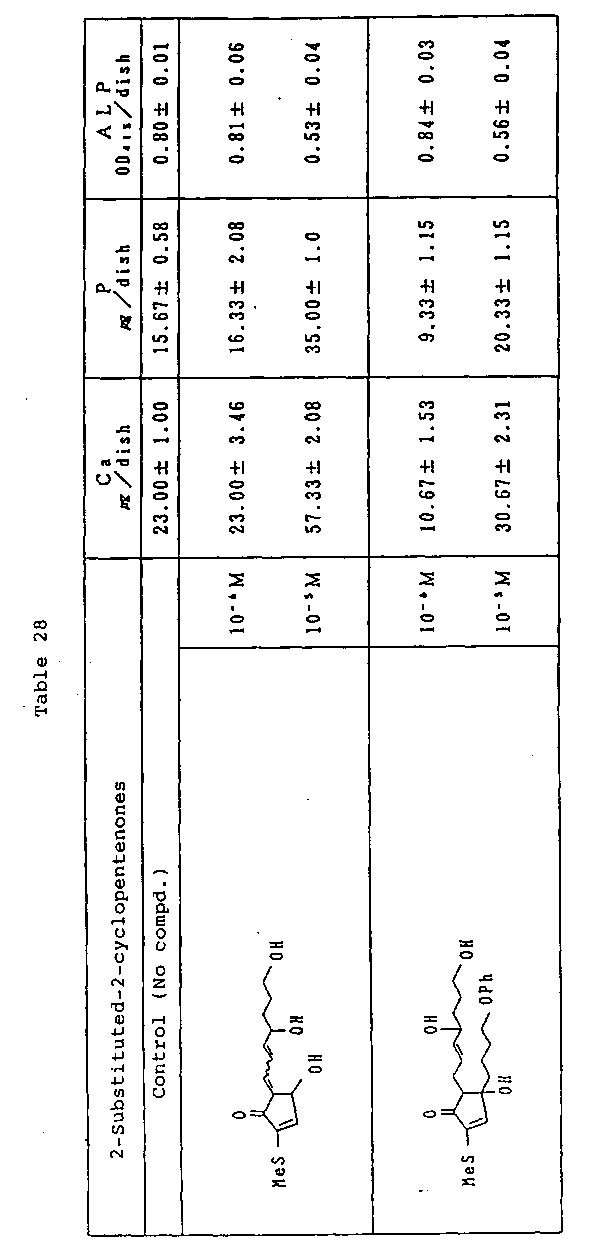

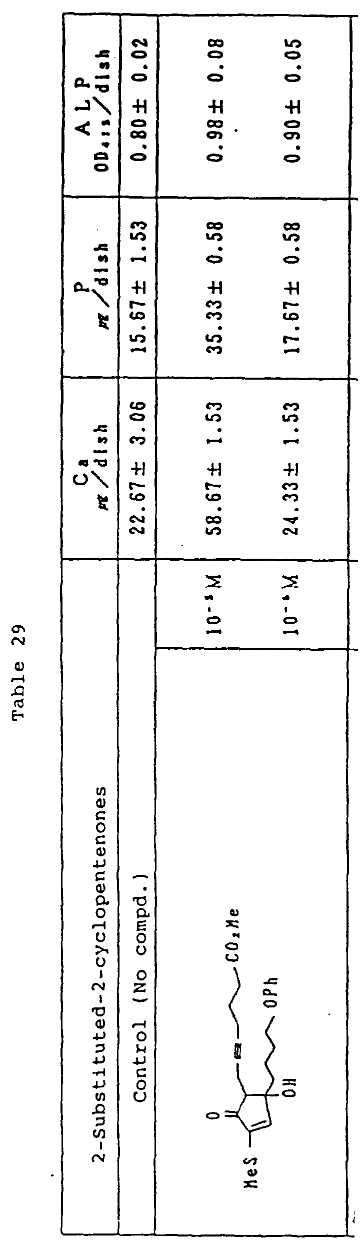

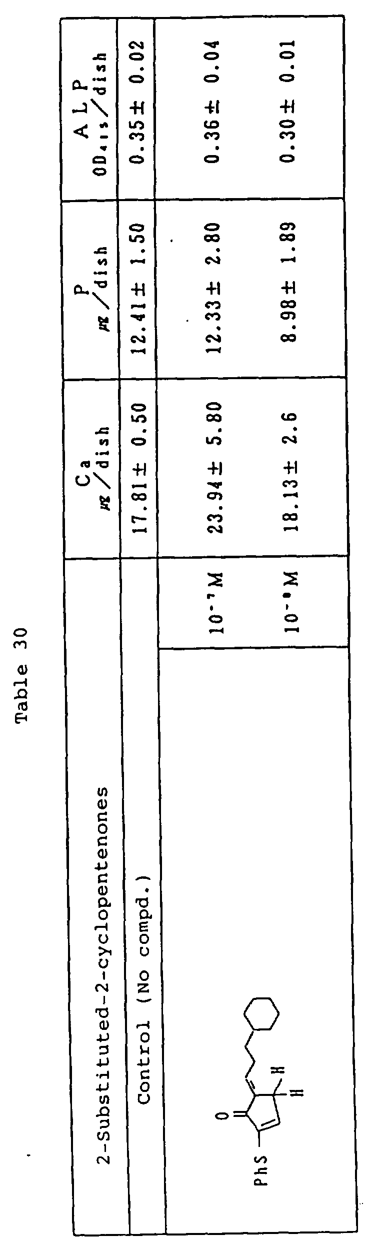

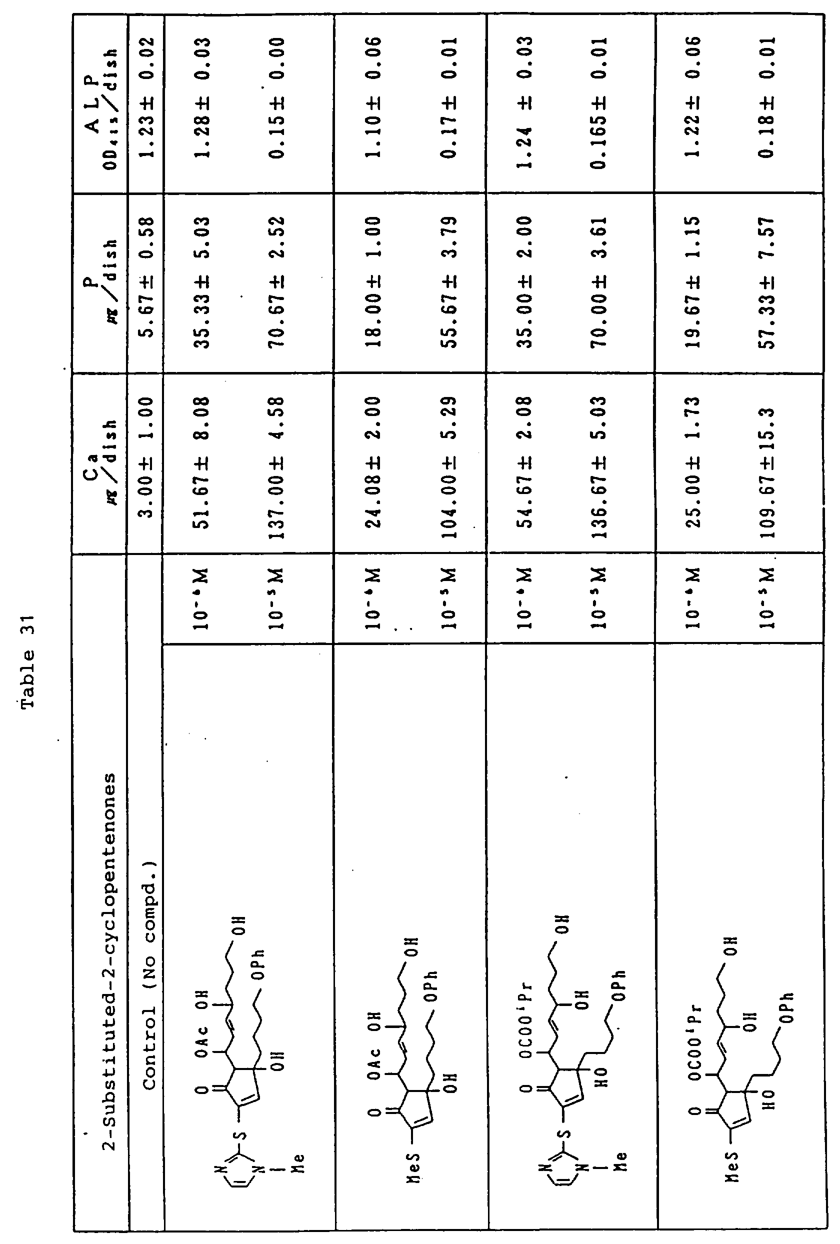

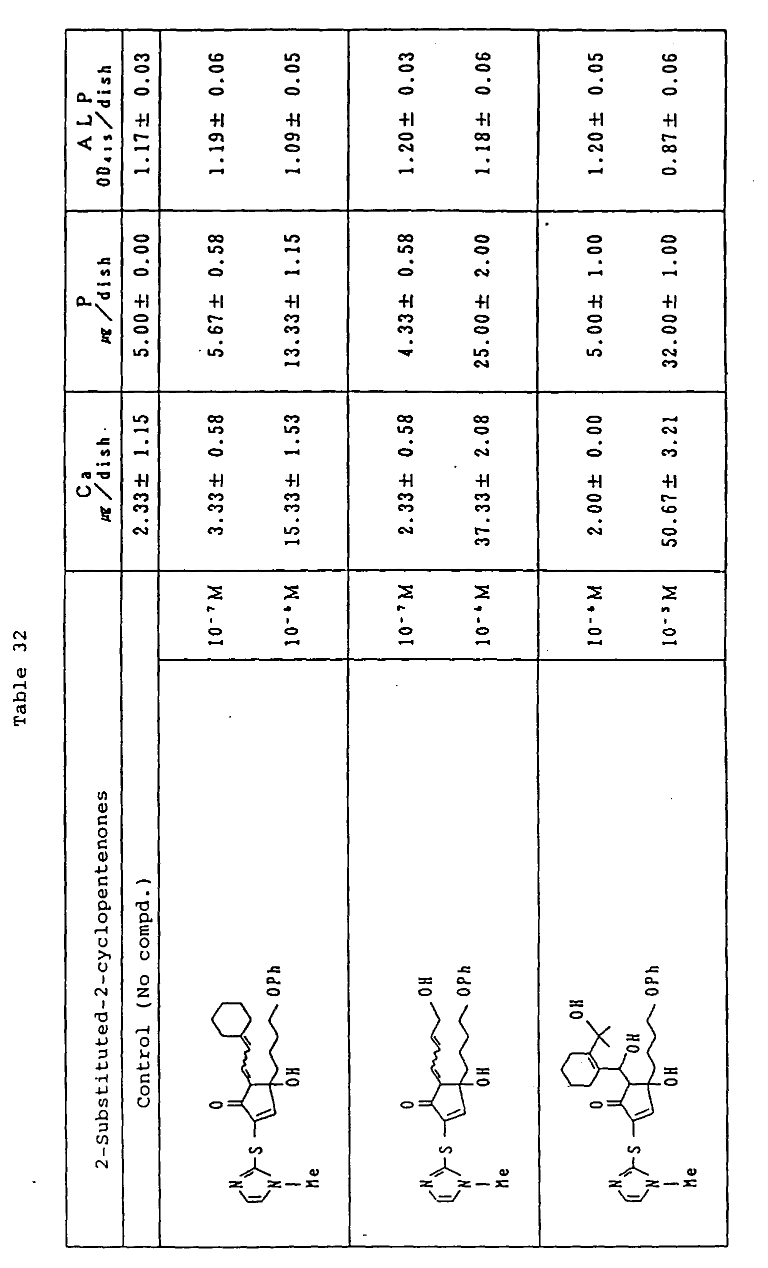

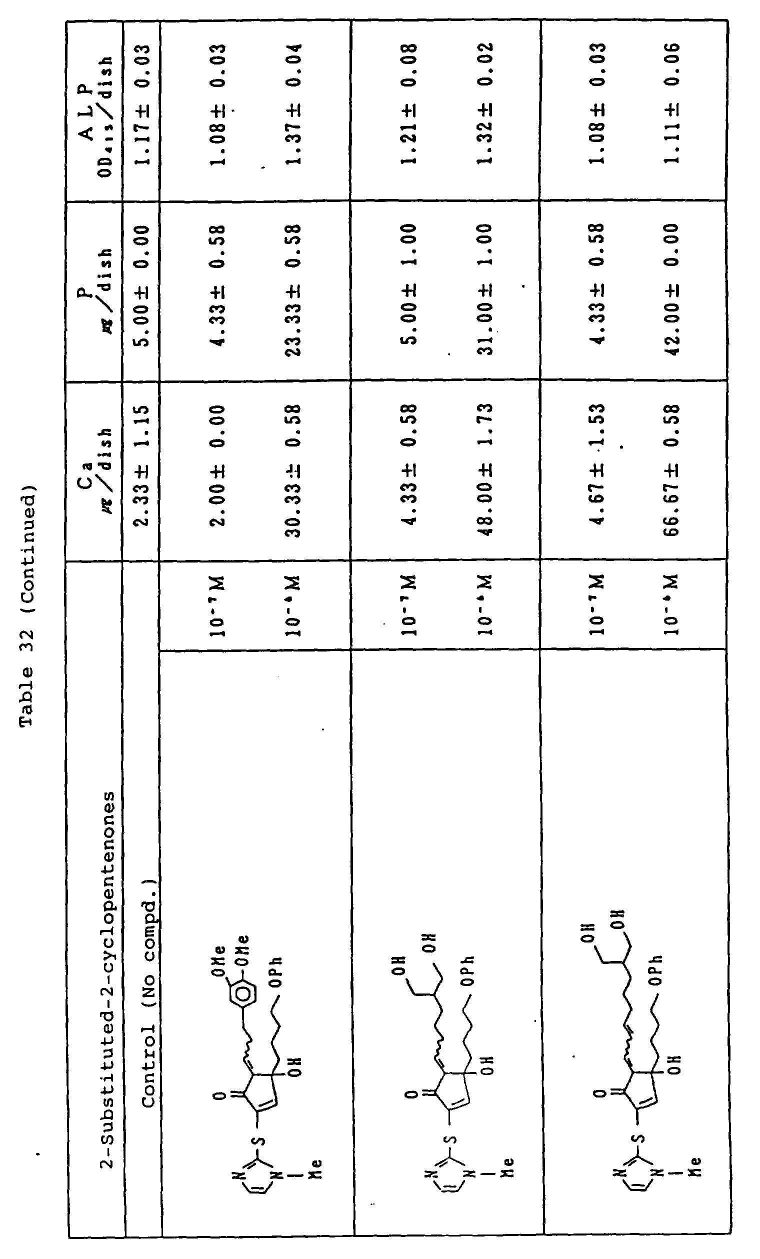

## Claims

1. A 2-substituted-2-cyclopentenone compound represented by the following formula (I): 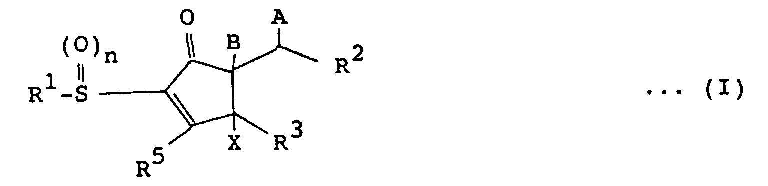 wherein
R¹ stands for a substituted or unsubstituted (i) acyclic aliphatic hydrocarbon group having 1 to 10 carbon atoms, (ii) alicyclic hydrocarbon group having 4 to 10 carbon atoms, (iii) aromatic hydrocarbon group having 6 to 10 carbon atoms or (iv) heterocyclic group having 1 to 9 carbon atoms;
R stands for a substituted or unsubstituted (i) acyclic aliphatic hydrocarbon group having 1 to 10 carbon atoms, (ii) alicyclic hydrocarbon group having 4 to 10 carbon atoms, (iii) aromatic hydrocarbon group having 6 to 10 carbon atoms or (iv) heterocyclic group having 1 to 9 carbon atoms;
R³ stands for a hydrogen atom or a substituted or unsubstituted (i) acyclic aliphatic hydrocarbon group having 1 to 10 carbon atoms, (ii) alicyclic hydrocarbon group having 4 to 10 carbon atoms or (iii) aromatic hydrocarbon group having 6 to 10 carbon atoms;
X stands for a hydrogen atom, or -OR⁴ (wherein R⁴ stands for a hydrogen atom, an alkyl group having 1 to 4 carbon atoms, an acyl group having 2 to 7 carbon atoms, an alkoxycarbonyl group having 2 to 5 carbon atoms, a tri(C₁ - C₇) hydrocarbon silyl group or a group capable of forming an acetal bond together with the oxygen atom attached to the R⁴), provided that X is absent when R³ is bonded to the carbon atom bonding thereto through a double bond;
R⁵ stands for a hydrogen atom, or a substituted or unsubstituted (i) acyclic aliphatic hydrocarbon group having 1 to 10 carbon atoms or (ii) an alicyclic hydrocarbon group having 4 to 10 carbon atoms;
B stands for a hydrogen atom when A stands for a hydrogen atom, a hydroxyl group, an acyloxy group having 2 to 7 carbon atoms, an alkoxycarbonyloxy group having 2 to 5 carbon atoms, a sulfonyloxy group having 1 to 7 carbon atoms or 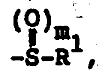 or A and B are combined with each other to form a bond line;
m and n which may be the same or different from each other stand for 0, 1 or 2,
exclusive of the cases where R¹ stands for a substituted or unsubstituted acyclic aliphatic hydrocarbon group, a substituted or unsubstituted alicyclic hydrocarbon group, or a substituted or unsubstituted aromatic hydrocarbon group, and R stands for a substituted or unsubstituted acyclic aliphatic hydrocarbon group, and R³ does not stand for a hydrogen atom, and R⁵ stands for a hydrogen atom, and either i) A stands for a hydroxyl group, a sulfonyloxy group or 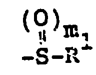 and B is a hydrogen atom or ii) A and B are combined with each other to form a bond line.

2. A 2-substituted-2-cyclopentenone compound according to claim 1, which is represented by the following formula (I_{A}): 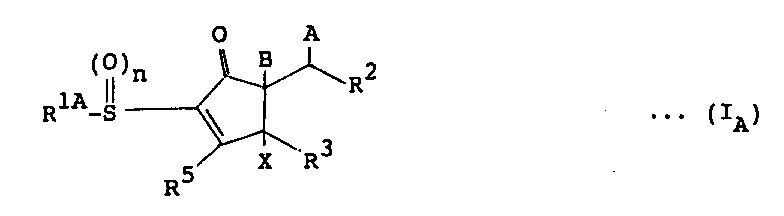 wherein
R^{1A} stands for a substituted or unsubstituted (i) alicyclic hydrocarbon group having 4 to 10 carbon atoms, (ii) heterocyclic group having 1 to 9 carbon atoms or (iii) an acyclic aliphatic hydrocarbon group having 1 to 10 carbon atoms substituted with an alicyclic hydrocarbon group having 4 to 10 carbon atoms or a heterocyclic group having 1 to 9 carbon atoms; and
R, R³, R⁵, X, A, B and n are as defined above.

3. A 2-substituted-2-cyclopentenone compound according to claim 1 or 2, which is represented by the following formula (I_{A}-A): 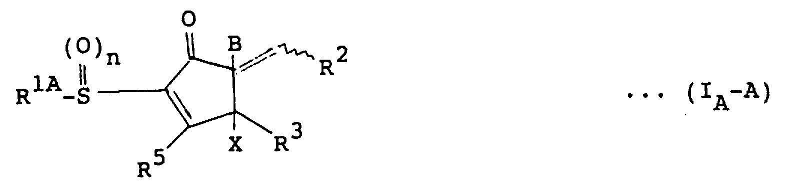 wherein R^{1A}, R, R³, R⁵, X and n are as defined above and 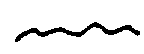 represents that the substituent attached to the double bond is in an E-configuration or a Z-configuration or a mixture thereof in any proportion.

4. A 2-substituted-2-cyclopentenone compound according to claim 1 or 2, which is represented by the following formula (I_{A}-B): 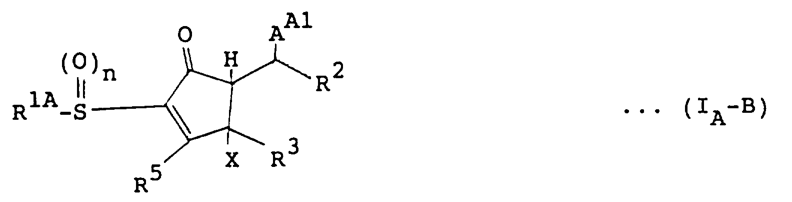 wherein
A^{A1} stands for a hydrogen atom, a hydroxyl group, an acyloxy group having 2 to 7 carbon atoms, an alkoxycarbonyloxy group having 2 to 5 carbon atoms, a sulfonyloxy group having 1 to 7 carbon atoms or 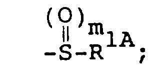 and
R^{1A}, R, R³, R⁵, X, m and n are as defined above.

5. A 2-substituted-2-cyclopentenone compound according to any one of claims 1 to 3, which is represented by the following formula (I_{A}-A-2): 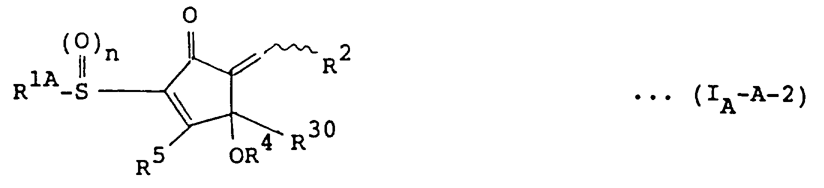 wherein
R^{1A}, R, R⁴, R⁵, n and 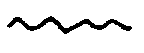 are as defined above; and
R³⁰ stands for a hydrogen atom or a substituted or unsubstituted (i) acyclic aliphatic hydrocarbon group having 1 to 10 carbon atoms, (ii) alicyclic hydrocarbon group having 4 to 10 carbon atoms or (iii) aromatic hydrocarbon group having 6 to 10 carbon atoms.

6. A 2-substituted-2-cyclopentenone compound according to any one of claims 1 to 3, which is represented by the following formula (I_{A}-A-3): 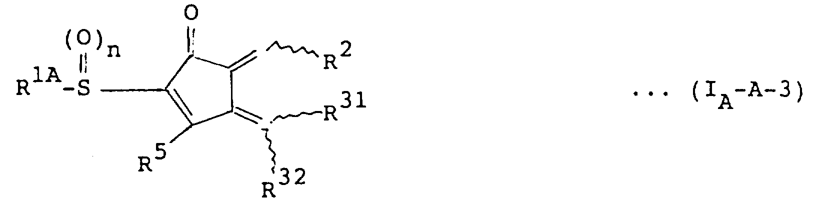 wherein
R^{1A}, R, R⁵, n and 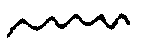 are as defined above; and
R³¹ and R³ which may be the same or different from each other stand for a hydrogen atom or a substituted, unsubstituted (i) acyclic aliphatic hydrocarbon group having 1 to 9 carbon atoms, (ii) alicyclic hydrocarbon group having 4 to 10 carbon atoms or (iii) aromatic hydrocarbon group having 6 to 10 carbon atoms, or a group wherein the R³¹ and R³ are combined with each other to form an alicyclic hydrocarbon group having a four to ten-membered ring.

7. A 2-substituted-2-cyclopentenone compound according to any one of claims 1, 2 and 4, which is represented by the following formula (I_{A}-B-2): 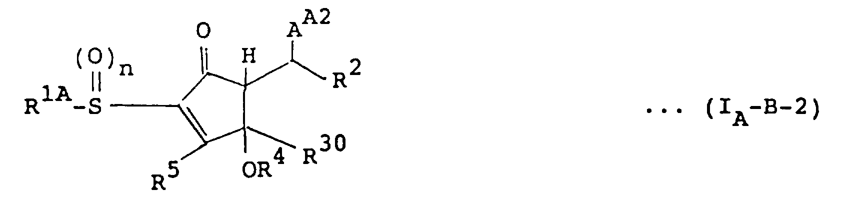 wherein
R^{1A}, R, R³⁰, R⁴, R⁵ and n are as defined above; and
A^{A2} stands for a hydrogen atom, a hydroxyl group, an acyloxy group having 2 to 7 carbon atoms or an alkoxycarbonyloxy group having 2 to 5 carbon atoms.

8. A 2-substituted-2-cyclopentenone compound according to any one of claims 1, 2, 3 and 5, which is represented by the following formula (I_{A0}-A-2): 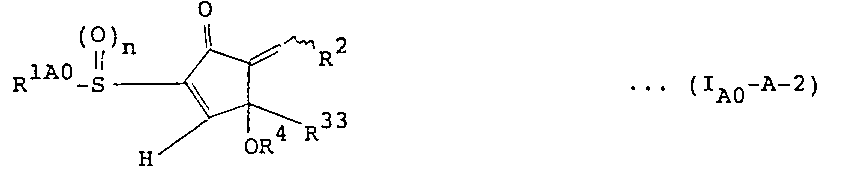 wherein
R, R⁴, n and 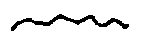 are as defined above;
R^{1A0} stands for a substituted or unsubstituted heterocyclic group having 1 to 9 carbon atoms; and
R³³ stands for a substituted or unsubstituted (i) acyclic aliphatic hydrocarbon group having 1 to 10 carbon atoms, (ii) alicyclic hydrocarbon group having 4 to 10 carbon atoms or (iii) aromatic hydrocarbon group having 6 to 10 carbon atoms.

9. A 2-substituted-2-cyclopentenone compound according to any one of claims 1, 2, 3 and 6, which is represented by the following formula (I_{A0}-A-3): 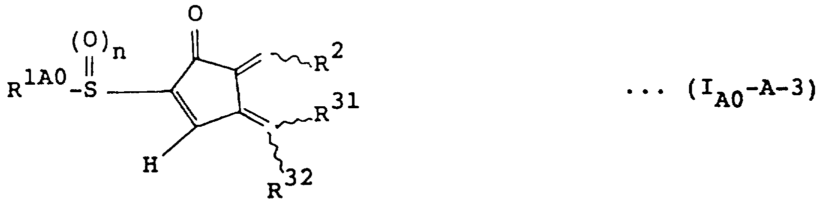 wherein R^{1A0}, R, R³¹, R³, n and 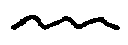 are as defined above.

10. A 2-substituted-2-cyclopentenone compound according to any one of claims 1, 2, 4 and 7, which is represented by the following formula (I_{A0}-B-2): 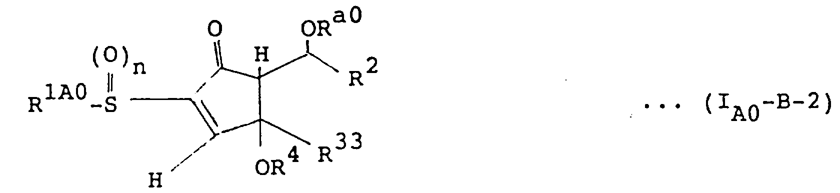 wherein
R^{1A0}, R, R³³, R⁴ and n are as defined above; and
R^{a0} stands for a hydrogen atom, an acyl group having 2 to 7 carbon atoms or an alkoxycarbonyl group having 2 to 5 carbon atoms.

11. A 2-substituted-2-cyclopentenone compound according to claim 1, which is represented by the following formula (I_{B}): 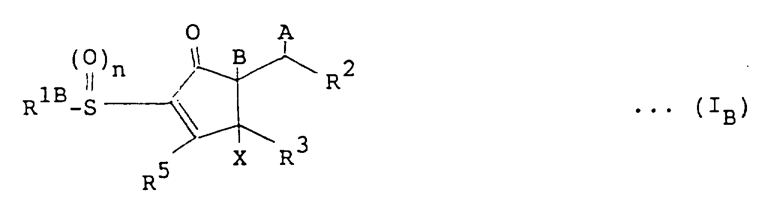 wherein
R^{1B} stands for a substituted or unsubstituted (i) acyclic aliphatic hydrocarbon group having 1 to 10 carbon atoms, or (ii) aromatic hydrocarbon group having 6 to 10 carbon atoms; and
R, R³, R⁵, X, A, B and n are as described above, provided that when the R⁵ stands for a hydrogen atom, the R³ stands for a hydrogen atom.

12. A 2-substituted-2-cyclopentenone compound according to claim 1 or 11, which is represented by the following formula (I_{B}-A): 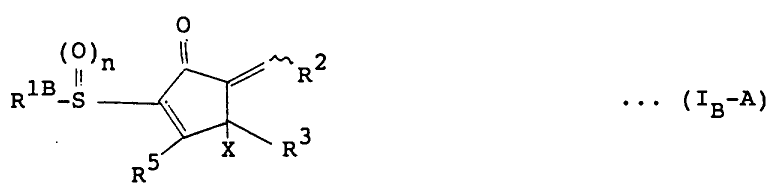 wherein R^{1B}, R, R³, R⁵, X, n and 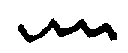 are as defined above, provided that when the R⁵ stands for a hydrogen atom, the R³ stands for a hydrogen atom.

13. A 2-substituted-2-cyclopentenone compound according to claim 1 or 11, which is represented by the following formula (I_{B}-B): 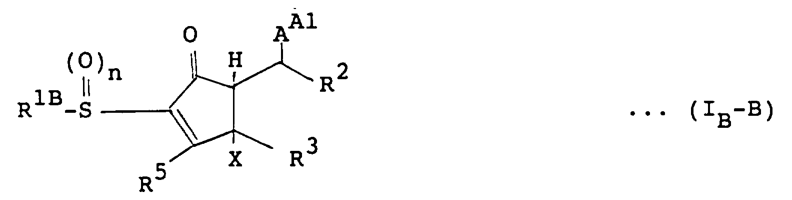 wherein R^{1B}, R, R³, R⁵, X, A^{A1} and n are as defined above, provided that when the R⁵ stands for a hydrogen atom, the R³ stands for a hydrogen atom.

14. A 2-substituted-2-cyclopentenone compound according to claim 1, 11 or 12, which is represented by the following formula (I_{B}-A-2): 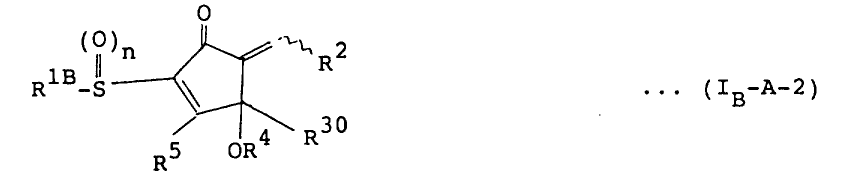 wherein R^{1B}, R, R³⁰, R⁴, R⁵, n and 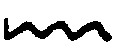 are as defined above, provided that when the R⁵ stands for a hydrogen atom, the R³⁰ stands for a hydrogen atom.

15. A 2-substituted-2-cyclopentenone compound according to claim 1, 11 or 12, which is represented by the following formula (I_{B}-A-3): 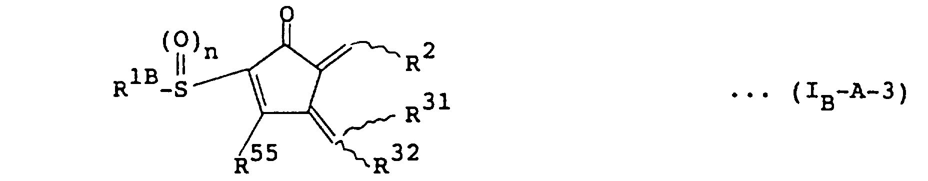 wherein
R^{1B}, R, R³¹, R³, n and 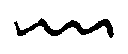 are as defined above; and
R⁵⁵ stands for a substituted or unsubstituted acyclic aliphatic hydrocarbon group having 1 to 10 carbon atoms or a substituted or unsubstituted alicyclic hydrocarbon group having 4 to 10 carbon atoms.

16. A 2-substituted-2-cyclopentenone compound according to claim 1, 11 or 13, which is represented by the following formula (I_{B}-B-2): 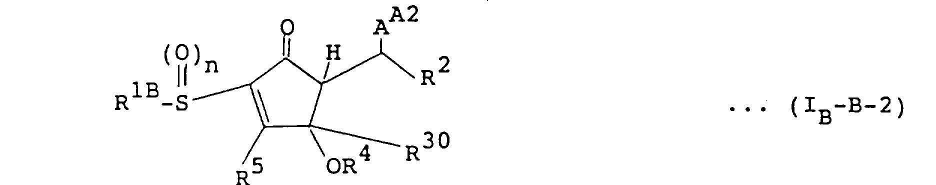 wherein R^{1B}, R, R³⁰, R⁴, R⁵, n and A^{A2} are as defined above, provided that when the R⁵ stands for a hydrogen atom, the R³⁰ stands for a hydrogen atom.

17. A 2-substituted-2-cyclopentenone compound according to claim 1, which is represented by the following formula (I_{C}): 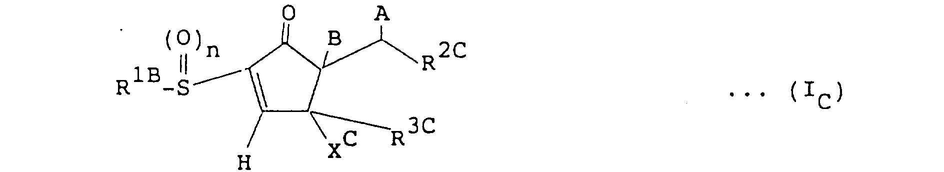 wherein
R^{1B}, A, B and n are as defined above;
R^{2C} stands for a substituted or unsubstituted (i) alicyclic hydrocarbon group having 4 to 10 carbon atoms, (ii) aromatic hydrocarbon group having 6 to 10 carbon atoms, (iii) heterocyclic group having 1 to 9 carbon atoms or (iv) acyclic aliphatic hydrocarbon group having 1 to 10 carbon atoms substituted with a heterocyclic group having 1 to 9 carbon groups;
R^{3C} stands for a substituted or unsubstituted (i) acyclic aliphatic hydrocarbon group having 1 to 10 carbon atoms, (ii) alicyclic hydrocarbon group having 4 to 10 carbon atoms or (iii) aromatic hydrocarbon group having 6 to 10 carbon atoms, provided that when the R^{3C} is bonded to the carbon atom attached thereto through a single bond, X^{C} stands for a hydrogen atom or -OR⁴ (wherein R⁴ is as defined above), while when R^{3C} is bonded to the carbon atom attached thereto through a double bond, the X^{C} is absent.

18. A 2-substituted-2-cyclopentenone compound according to claim 1 or 17, which is represented by the following formula (I_{C}-A): 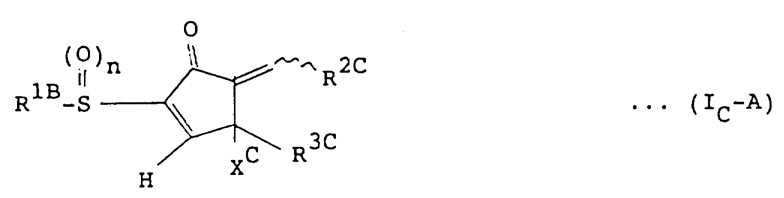 wherein R^{1B}, R^{2C}, R^{3C}, X^{C}, n and are 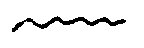 as defined above.

19. A 2-substituted-2-cyclopentenone compound according to claim 1 or 17, which is represented by the following formula (I_{C}-B): 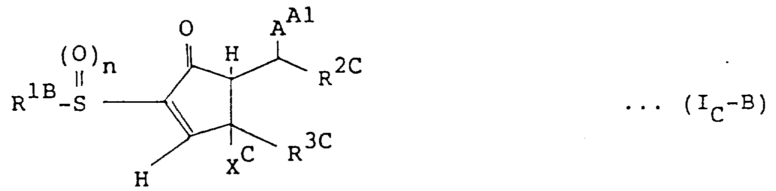 wherein R^{1B}, R^{2C}, R^{3C}, X^{C}, A^{A1} and n are as defined above.

20. A 2-substituted-2-cyclopentenone compound according to claim 1, 17 or 18, which is represented by the following formula (I_{C0}-A-2): 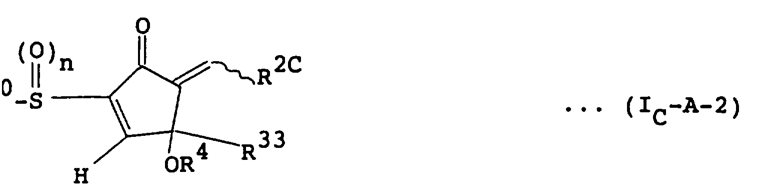 wherein
R^{2C}, R³³, R⁴, n and 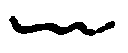 are as defined above; and
R^{1B0} stands for a substituted for unsubstituted aromatic hydrocarbon group having 6 to 10 carbon atoms or a methyl group.

21. A 2-substituted-2-cyclopentenone compound according to claim 1, 17 or 18, which is represented by the following formula (I_{C0}-A-3): 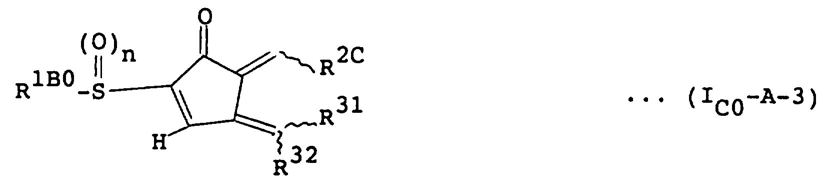 wherein R^{1B0}, R^{2C}, R³¹, R³, n and 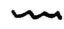 are as defined above.

22. A 2-substituted-2-cyclopentenone compound according to claim 1, 17 or 19, which is represented by the following formula (I_{C0}-B-2): 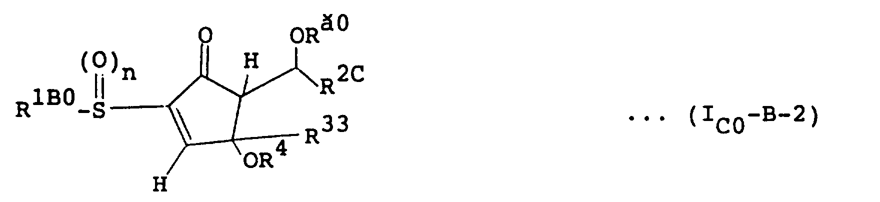 wherein R^{1B0}_{,} R^{2C}, R³³, R⁴, R^{a0} and n are as defined above.

23. A 2-substituted-2-cyclopentenone compound according to claim 1, which is represented by the following formula (I_{D}): 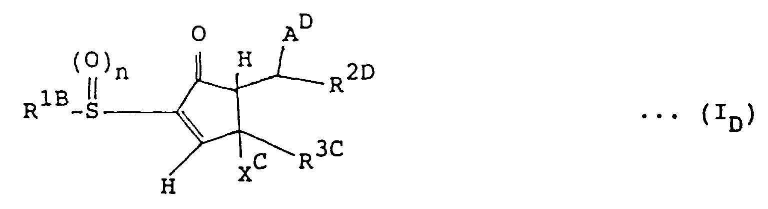 wherein
R^{1B}, R^{3C}, X^{C} and n are as defined above; and
R^{2D} stands for a substituted or unsubstituted (i) alicyclic hydrocarbon group having 4 to 10 carbon atoms or (ii) acyclic aliphatic hydrocarbon group having 1 to 10 carbon atoms which may be substituted with an aromatic hydrocarbon group having 6 to 10 carbon atoms; and
A^{D} stands for a hydrogen atom, an acyloxy group having 2 to 7 carbon atoms or an alkoxycarbonyloxy group having 2 to 5 carbon atoms.

24. A 2-substituted-2-cyclopentenone compound according to claim 1 or 23, which is represented by the following formula (I_{D0}-B-2): 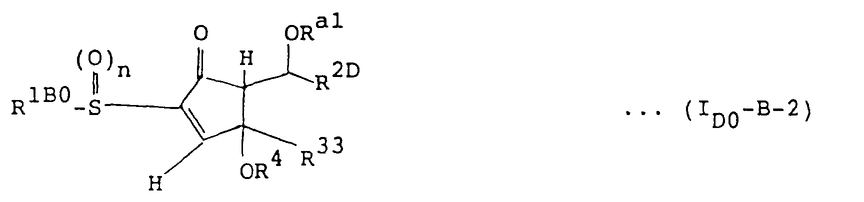 wherein
R^{1B0}, R^{2D}, R³³, R⁴ and n are as defined above; and
R^{a1} stands for an acyl group having 2 to 7 carbon atoms or an alkoxycarbonyl group having 2 to 5 carbon atoms.

25. An anticancer agent comprising as an effective active ingredient at least one compound selected from the group consisting of 2-substituted-2-cyclopentenone compounds represented by the formula (I) according to claim 1.

26. A bone formation accelerator comprising as an effective active ingredient at least one compound selected from the group consisting of 2-substituted-2-cyclopentenone cyclopentenone compounds represented by the formula (I) according to claim 1.

## Patentansprüche

1. In der 2-Position substituierte 2-Cyclopentenon-Verbindung gemäß der folgenden Formel (I): 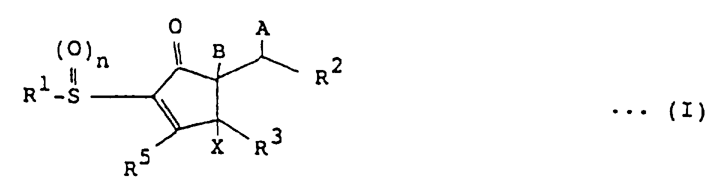 worin R¹ für eine substituierte oder unsubstituierte
(i) acyclische aliphatische Kohlenwasserstoff-Gruppe mit 1 bis 10 Kohlenstoff-Atomen,
(ii) alicyclische Kohlenwasserstoff-Gruppe mit 4 bis 10 Kohlenstoff-Atomen,
(iii) aromatische Kohlenwasserstoff-Gruppe mit 6 bis 10 Kohlenstoff-Atomen oder
(iv) heterocyclische Gruppe mit 1 bis 9 Kohlenstoff-Atomen steht;
worin R für eine substituierte oder unsubstituierte
(i) acyclische aliphatische Kohlenwasserstoff-Gruppe mit 1 bis 10 Kohlenstoff-Atomen,
(ii) alicyclische Kohlenwasserstoff-Gruppe mit 4 bis 10 Kohlenstoff-Atomen
(iii) aromatische Kohlenwasserstoff-Gruppe mit 6 bis 10 Kohlenstoff-Atomen oder
(iv) heterocyclische Gruppe mit 1 bis 9 Kohlenstoff-Atomen steht;
worin R³ für ein Wasserstoff-Atom oder eine substituierte oder unsubstituierte
(i) acyclische aliphatische Kohlenwasserstoff-Gruppe mit 1 bis 10 Kohlenstoff-Atomen,
(ii) alicyclische Kohlenwasserstoff-Gruppe mit 4 bis 10 Kohlenstoff-Atomen oder
(iii) aromatische Kohlenwasserstoff-Gruppe mit 6 bis 10 Kohlenstoff-Atomen steht;
worin X für ein Wasserstoff-Atom steht oder für -OR⁴ (worin R⁴ für ein Wasserstoff-Atom, eine Alkyl-Gruppe mit 1 bis 4 Kohlenstoff-Atomen, eine Acyl-Gruppe mit 2 bis 7 Kohlenstoff-Atomen, eine Alkoxycarbonyl-Gruppe mit 2 bis 5 Kohlenstoff-Atomen, eine Tri-(C₁-C₇)-Kohlenwasserstoff-Silyl-Gruppe oder für eine Gruppe steht, die zusammen mit dem an R⁴ gebundenen Sauerstoff-Atom zur Bildung einer Acetalbindung befähigt ist), unter der Voraussetzung, daß X nicht vorhanden ist, wenn R³ an das mit ihm verbundene Kohlenstoff-Atom durch eine Doppelbindung gebunden ist;
worin R⁵ für ein Wasserstoff-Atom oder für eine substituierte oder unsubstituierte
(i) acyclische aliphatische Kohlenwasserstoff-Gruppe mit 1 bis 10 Kohlenstoff-Atomen oder
(ii) alicyclische Kohlenwasserstoff-Gruppe mit 4 bis 10 Kohlenstoff-Atomen steht;
worin B für ein Wasserstoff-Atom steht, wenn A ein Wasserstoff-Atom, eine Hydroxyl-Gruppe, eine Acyloxy-Gruppe mit 2 bis 7 Kohlenstoff-Atomen, eine Alkoxycarbonyloxy-Gruppe mit 2 bis 5 Kohlenstoff-Atomen, eine Sulfonyloxy-Gruppe mit 1 bis 7 Kohlenstoff-Atomen oder 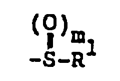 bedeutet oder A und B so miteinander verknüpft sind, daß sie eine Bindung bilden;
wobei m und n, welche gleich oder verschieden voneinander sein können, für 0, 1 oder 2 stehen,
mit Ausnahme solcher Fälle, in denen R¹ für eine substituierte oder unsubstituierte acyclische aliphatische Kohlenwasserstoff-Gruppe, eine substituierte oder unsubstituierte alicyclische Kohlenwasserstoff-Gruppe oder eine substituierte oder unsubstituierte aromatische Kohlenwasserstoff-Gruppe steht und R eine substituierte oder unsubstituierte acyclische aliphatische Kohlenwasserstoff-Gruppe bedeutet und R³ nicht für ein Wasserstoff-Atom steht und R⁵ ein Wasserstoff-Atom bedeutet und entweder (i) A für eine Hydroxyl-Gruppe, eine Sulfonyloxy-Gruppe oder für 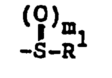 steht und B ein Wasserstoff-Atom ist oder ii) A und B so miteinander verknüpft sind, daß sie eine Bindung bilden.

2. In der 2-Position substituierte 2-Cyclopentenon-Verbindung nach Anspruch 1 gemäß der folgenden Formel (I_{A}): 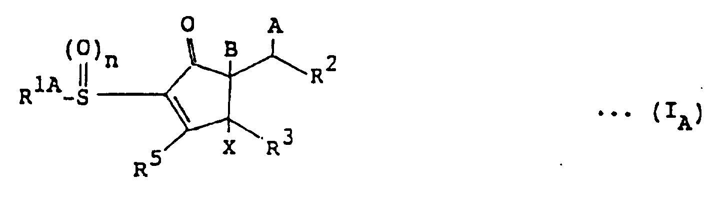 worin R^{1A} für eine substituierte oder unsubstituierte
(i) alicyclische Kohlenwasserstoff-Gruppe mit 4 bis 10 Kohlenstoff-Atomen,
(ii) heterocyclische Gruppe mit 1 bis 9 Kohlenstoff-Atomen oder
(iii) acyclische aliphatische Kohlenwasserstoff-Gruppe mit 1 bis 10 Kohlenstoff-Atomen, welche mit einer alicyclischen Kohlenwasserstoff-Gruppe mit 4 bis 10 Kohlenstoff-Atomen oder einer heterocyclischen Gruppe mit 1 bis 9 Kohlenstoff-Atomen substituiert ist, steht; und
worin R, R³, R⁵, X, A, B und n wie oben definiert sind.

3. In der 2-Position substituierte 2-Cyclopentenon-Verbindung nach Anspruch 1 oder 2, welche durch die folgende Formel (I_{A}-A) repräsentiert ist: 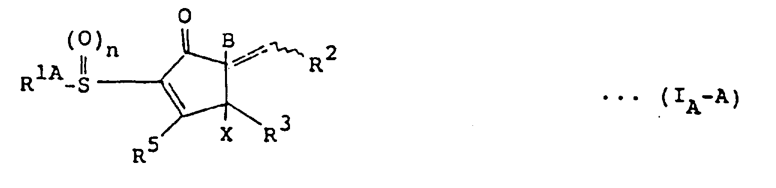 worin R^{1A}, R, R³, R⁵, X und n wie oben definiert sind und worin durch 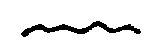 repräsentiert ist, daß der an die Doppelbindung gebundene Substituent in einer E-Konfiguration oder in einer Z-Konfiguration oder einer Mischung hiervon in einem beliebigen Verhältnis vorliegt.

4. In der 2-Position substituierte 2-Cyclopentenon-Verbindung nach Anspruch 1 oder 2, welche durch die folgende Formel (I_{A}-B) repräsentiert ist: 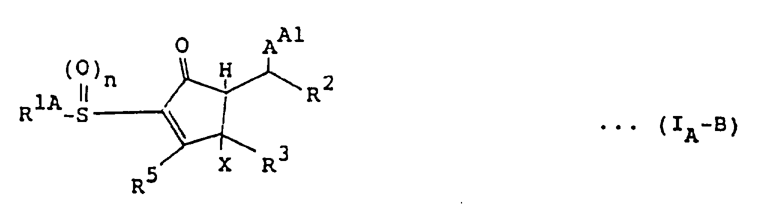 worin A^{A1} für ein Wasserstoff-Atom, eine Hydroxyl-Gruppe, eine Acyloxy-Gruppe mit 2 bis 7 Kohlenstoff-Atomen, eine Alkoxycarbonyloxy-Gruppe mit 2 bis 5 Kohlenstoff-Atomen, eine Sulfonyloxy-Gruppe mit 1 bis 7 Kohlenstoff-Atomen oder für 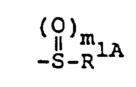 steht; und
worin R^{1A}, R, R³, R⁵, X, m und n wie oben definiert sind.

5. In der 2-Position substituierte 2-Cyclopentenon-Verbindung nach einem der Ansprüche 1 bis 3, welche durch die folgende Formel (I_{A}-A-2) repräsentiert ist: 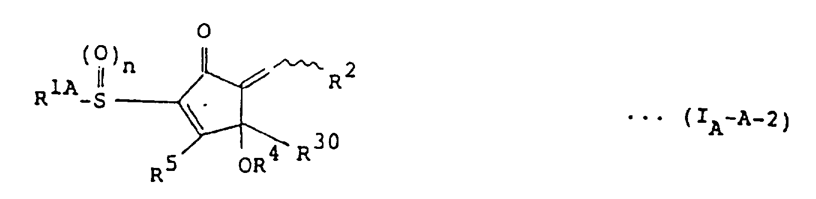 worin R^{1A}, R, R⁴, R⁵, n und
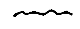 wie oben definiert sind; und
worin R³⁰ für ein Wasserstoff-Atom oder für eine substituierte oder unsubstituierte
(i) acyclische aliphatische Kohlenwasserstoff-Gruppe mit 1 bis 10 Kohlenstoff-Atomen,
(ii) alicyclische Kohlenwasserstoff-Gruppe mit 4 bis 10 Kohlenstoff-Atomen oder
(iii) aromatische Kohlenwasserstoff-Gruppe mit 6 bis 10 Kohlenstoff-Atomen steht.

6. In der 2-Position substituierte 2-Cyclopentenon-Verbindung nach einem der Ansprüche 1 bis 3, welche durch die folgende Formel (I_{A}-A-3) repräsentiert ist: 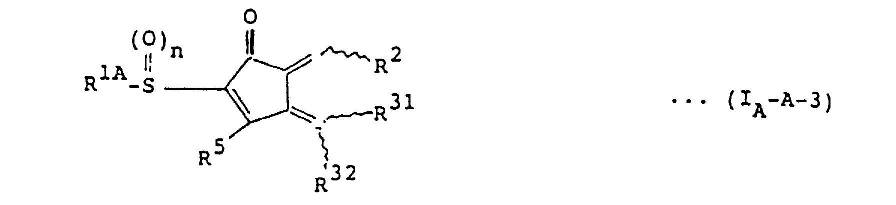 worin R^{1A}, R, R⁵, n und
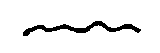 wie oben definiert sind; und
worin R³¹ und R³, welche gleich oder verschieden voneinander sein können, für ein Wasserstoff-Atom oder für eine substituierte, unsubstituierte
(i) acyclische aliphatische Kohlenwasserstoff-Gruppe mit 1 bis 9 Kohlenstoff-Atomen,
(ii) alicyclische Kohlenwasserstoff-Gruppe mit 4 bis 10 Kohlenstoff-Atomen oder
(iii) aromatische Kohlenwasserstoff-Gruppe mit 6 bis 10 Kohlenstoff-Atomen stehen oder
für eine Gruppe, worin R³¹ und R³ miteinander verknüpft sind, um eine alicyclische Kohlenwasserstoff-Gruppe zu bilden, die einen vier- bis zehngliedrigen Ring enthält.

7. In der 2-Position substituierte 2-Cyclopentenon-Verbindung nach einem der Ansprüche 1, 2 oder 4, welche durch die folgende Formel (I_{A}-B-2) repräsentiert ist: 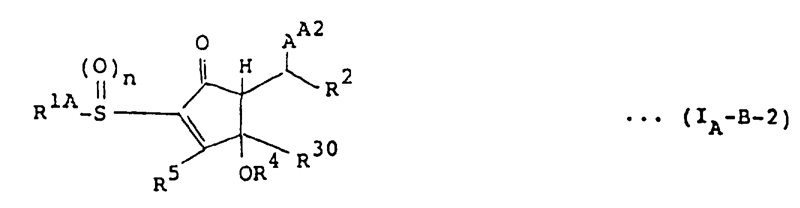 worin R^{1A}, R, R³⁰, R⁴, R⁵ und n wie oben definiert sind; und
worin A^{A2} für ein Wasserstoff-Atom, eine Hydroxyl-Gruppe, eine Acyloxy-Gruppe mit 2 bis 7 Kohlenstoff-Atomen oder eine Alkoxycarbonyloxy-Gruppe mit 2 bis 5 Kohlenstoff-Atomen steht.

8. In der 2-Position substituierte 2-Cyclopentenon-Verbindung nach einem der Ansprüche 1, 2, 3 oder 5, welche durch die folgende Formel (I_{A0}-A-2) repräsentiert ist: 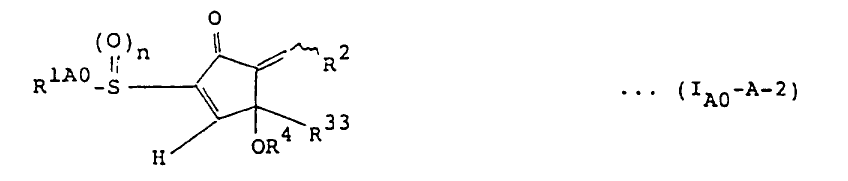 worin R, R⁴, n und 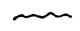
wie oben definiert sind; und
worin R^{1A0} für eine substituierte oder unsubstituierte heterocyclische Gruppe mit 1 bis 9 Kohlenstoff-Atomen steht; und
worin R³³ für eine substituierte oder unsubstituierte
(i) acyclische aliphatische Kohlenwasserstoff-Gruppe mit 1 bis 10 Kohlenstoff-Atomen,
(ii) alicyclische Kohlenwasserstoff-Gruppe mit 4 bis 10 Kohlenstoff-Atomen oder
(iii) aromatische Kohlenwasserstoff-Gruppe mit 6 bis 10 Kohlenstoff-Atomen steht.

9. In der 2-Position substituierte 2-Cyclopentenon-Verbindung nach einem der Ansprüche 1, 2, 3 oder 6, welche durch die folgende Formel (I_{A0}-A-3) repräsentiert ist: 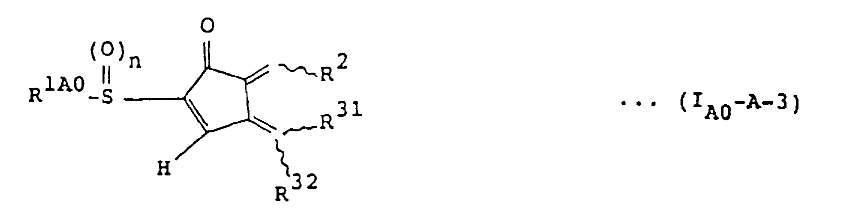 worin R^{1A0}, R, R³¹, R³, n und 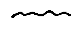
wie oben definiert sind.

10. In der 2-Position substituierte 2-Cyclopentenon-Verbindung nach einem der Ansprüche 1, 2, 4 oder 7, welche durch die folgende Formel (I_{A0}-B-2) repräsentiert ist: 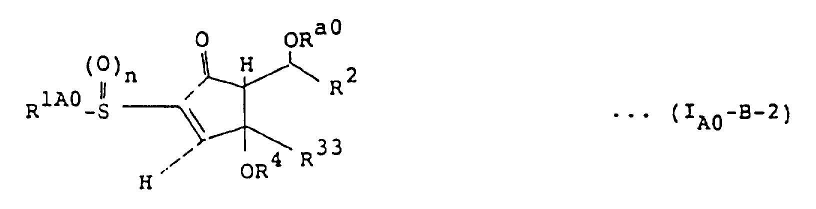 worin R^{1A0}, R, R³³, R⁴ und n wie oben definiert sind; und
worin R^{a0} für ein Wasserstoff-Atom, eine Acyl-Gruppe mit 2 bis 7.Kohlenstoff-Atomen oder eine Alkoxycarbonyl-Gruppe mit 2 bis 5 Kohlenstoff-Atomen steht.

11. In der 2-Position substituierte 2-Cyclopentenon-Verbindung nach Anspruch 1, welche durch die folgende Formel (I_{B}) repräsentiert ist: 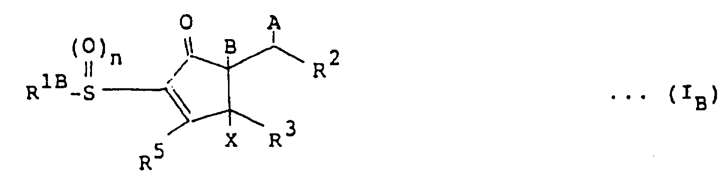 worin R^{1B} für eine substituierte oder unsubstituierte
(i) acyclische aliphatische Kohlenwasserstoff-Gruppe mit 1 bis 10 Kohlenstoff-Atomen oder
(ii) aromatische Kohlenwasserstoff-Gruppe mit 6 bis 10 Kohlenstoff-Atomen steht; und
worin R, R³, R⁵, X, A, B und n wie oben beschrieben sind, unter der Voraussetzung, daß wenn R⁵ ein Wasserstoff-Atom bedeutet, R³ für ein Wasserstoff-Atom steht.

12. In der 2-Position substituierte 2-Cyclopentenon-Verbindung nach Anspruch 1 oder 11, welche durch die folgende Formel (I_{B}-A) repräsentiert ist: 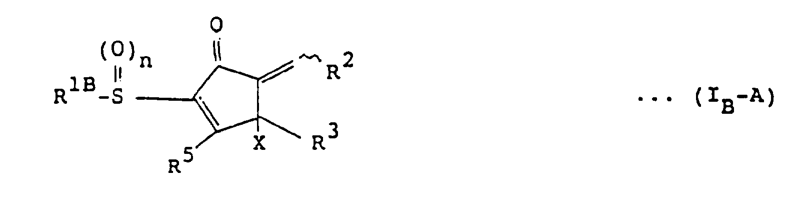 worin R^{1B}, R, R³, R⁵, X, n und 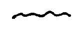
wie oben definiert sind, unter der Voraussetzung, daß wenn R⁵ ein Wasserstoff-Atom bedeutet, R³ für ein Wasserstoff-Atom steht.

13. In der 2-Position substituierte 2-Cyclopentenon-Verbindung nach Anspruch 1 oder 11, welche durch die folgende Formel (I_{B}-B) repräsentiert ist: 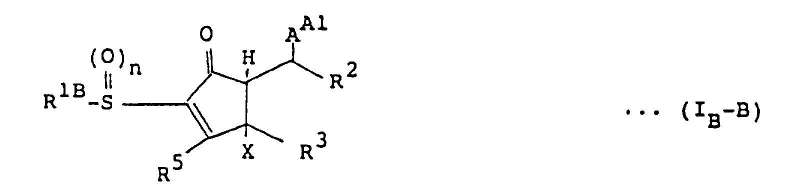 worin R^{1B}, R, R³, R⁵, X, A^{A1} und n wie oben definiert sind, unter der Voraussetzung, daß wenn R⁵ ein Wasserstoff-Atom bedeutet, R³ für ein Wasserstoff-Atom steht.

14. In der 2-Position substituierte 2-Cyclopentenon-Verbindung nach Anspruch 1, 11 oder 12, welche durch die folgende Formel (I_{B}-A-2) repräsentiert ist: 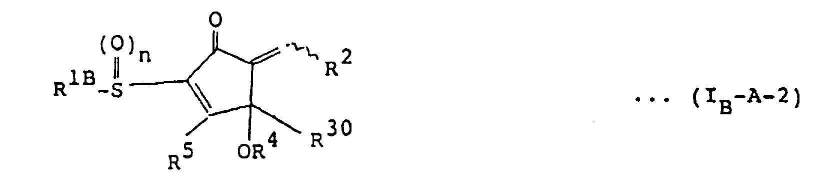 worin R^{1B}, R, R³⁰, R⁴, R⁵, n und 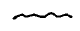
wie oben definiert sind, unter der Voraussetzung, daß wenn R⁵ für ein Wasserstoff-Atom steht, R³⁰ ein Wasserstoff-Atom bedeutet.

15. In der 2-Position substituierte 2-Cyclopentenon-Verbindung nach Anspruch 1, 11 oder 12, welche durch die folgende Formel (I_{B}-A-3) repräsentiert ist: 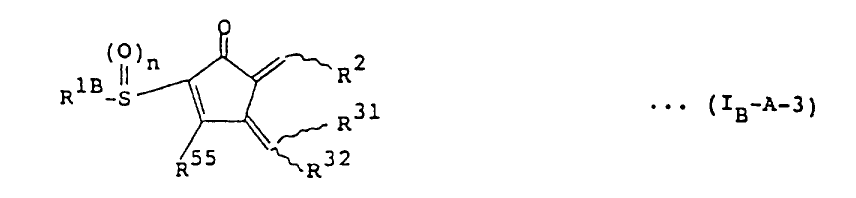 worin R^{1B}, R, R³¹, R³, n und 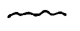
wie oben definiert sind; und
worin R⁵⁵ für eine substituierte oder unsubstituierte acyclische aliphatische Kohlenwasserstoff-Gruppe mit 1 bis 10 Kohlenstoff-Atomen oder für eine substituierte oder unsubstituierte alicyclische Kohlenwasserstoff-Gruppe mit 4 bis 10 Kohlenstoff-Atomen steht.

16. In der 2-Position substituierte 2-Cyclopentenon-Verbindung nach Anspruch 1, 11 oder 13, welche durch die folgende Formel (I_{B}-B-2) repräsentiert ist: 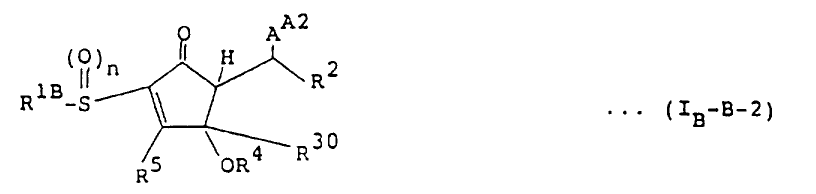 worin R^{1B}, R, R³⁰, R⁴, R⁵, n und A^{A2} wie oben definiert sind, unter der Voraussetzung, daß wenn R⁵ für ein Wasserstoff-Atom steht, R³⁰ ein Wasserstoff-Atom bedeutet.

17. In der 2-Position substituierte 2-Cyclopentenon-Verbindung nach Anspruch 1, welche durch die folgende Formel (I_{C}) repräsentiert ist: 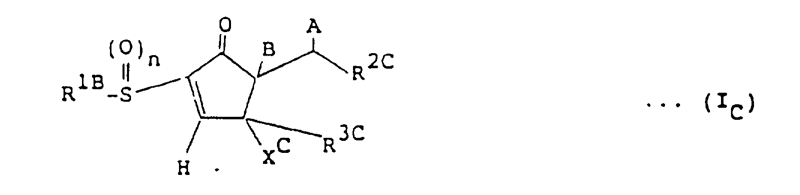 worin R^{1B}, A, B und n wie oben definiert sind;
worin R^{2C} für eine substituierte oder unsubstituierte
(i) alicyclische Kohlenwasserstoff-Gruppe mit 4 bis 10 Kohlenstoff-Atomen,
(ii) aromatische Kohlenwasserstoff-Gruppe mit 6 bis 10 Kohlenstoff-Atomen,
(iii) heterocyclische Gruppe mit 1 bis 9 Kohlenstoff-Atomen oder
(iv) acyclische aliphatische Kohlenwasserstoff-Gruppe mit 1 bis 10 Kohlenstoff-Atomen, welche mit einer heterocyclischen Gruppe mit 1 bis 9 Kohlenstoff-Atomen substituiert ist, steht;
worin R^{3C} für eine substituierte oder unsubstituierte
(i) acyclische aliphatische Kohlenwasserstoff-Gruppe mit 1 bis 10 Kohlenstoff-Atomen,
(ii) alicyclische Kohlenwasserstoff-Gruppe mit 4 bis 10 Kohlenstoff-Atomen oder
(iii) aromatische Kohlenwasserstoff-Gruppe mit 6 bis 10 Kohlenstoff-Atomen steht, unter der Voraussetzung, daß wenn R^{3C} an das mit ihm verbundene Kohlenstoffatom durch eine Einfachbindung gebunden ist, X^{C} für ein Wasserstoffatom oder für -OR⁴ steht (worin R⁴ wie oben definiert ist), während wenn R^{3C} an das mit ihm verbundene Kohlenstoffatom durch eine Doppelbindung gebunden ist, X^{C} nicht vorhanden ist.

18. In der 2-Position substituierte 2-Cyclopentenon-Verbindung nach Anspruch 1 oder 17, welche durch die folgende Formel (I_{C}-A) repräsentiert ist: 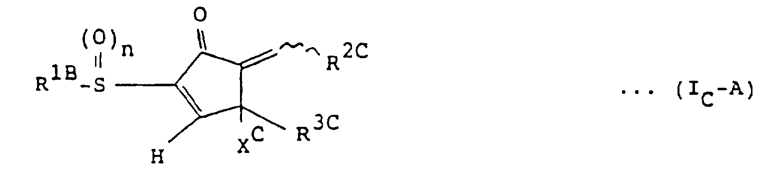 worin R^{1B}, R^{2C}, R^{3C}, X^{C}, n und 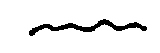
wie oben definiert sind.

19. In der 2-Position substituierte 2-Cyclopentenon-Verbindung nach Anspruch 1 oder 17, welche durch die folgende Formel (I_{C}-B) repräsentiert ist: 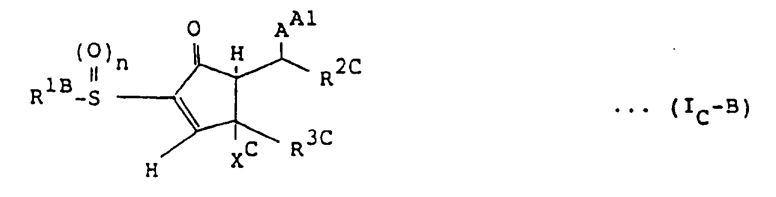 worin R^{1B}, R^{2C}, R^{3C}, X^{C}, A^{A1} und n wie oben definiert sind.

20. In der 2-Position substituierte 2-Cyclopentenon-Verbindung nach Anspruch 1, 17 oder 18, welche durch die folgende Formel (I_{C0}-A-2) repräsentiert ist: 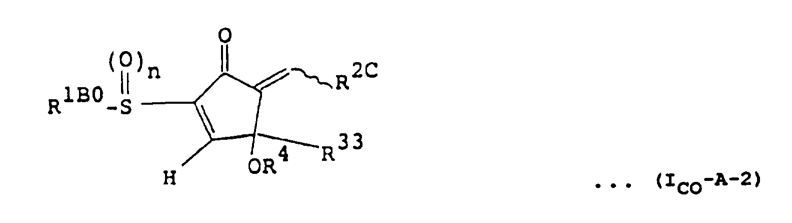 worin R^{2C}, R³³, R⁴, n und 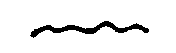
wie oben definiert sind; und
worin R^{1B0} für eine substituierte oder unsubstituierte aromatische Kohlenwasserstoff-Gruppe mit 6 bis 10 Kohlenstoff-Atomen oder eine Methyl-Gruppe steht.

21. In der 2-Position substituierte 2-Cyclopentenon-Verbindung nach Anspruch 1, 17 oder 18, welche durch die folgende Formel (I_{C0}-A-3) repräsentiert ist: 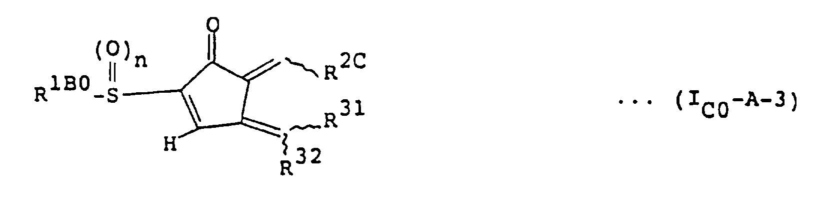 worin R^{1B0}, R^{2C}, R³¹, R³, n und 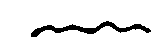
wie oben definiert sind.

22. In der 2-Position substituierte 2-Cyclopentenon-Verbindung nach Anspruch 1, 17 oder 19, welche durch die folgende Formel (I_{C0}-B-2) repräsentiert ist: 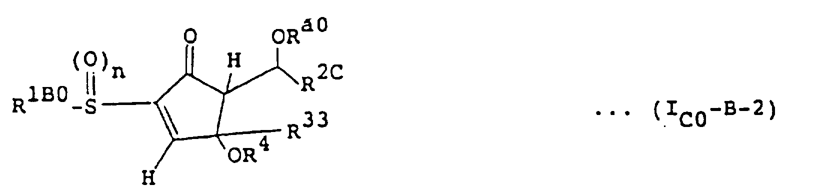 worin R^{1B0}, R^{2C}, R³³, R⁴, R^{a0} und n wie oben definiert sind.

23. In der 2-Position substituierte 2-Cyclopentenon-Verbindung nach Anspruch 1, welche der folgenden Formel (I_{D}) entspricht: 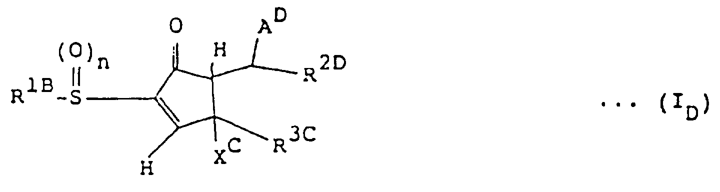 worin R^{1B}, R^{3C}, X^{C} und n wie oben definiert sind; und
worin R^{2D} für eine substituierte oder unsubstituierte
(i) alicyclische Kohlenwasserstoff-Gruppe mit 4 bis 10 Kohlenstoff-Atomen oder
(ii) acyclische aliphatische Kohlenwasserstoff-Gruppe mit 1 bis 10 Kohlenstoff-Atomen steht, welche mit einer aromatischen Kohlenwasserstoff-Gruppe mit 6 bis 10 Kohlenstoff-Atomen substituiert sein kann; und
worin A^{D} für ein Wasserstoff-Atom, eine Acyloxy-Gruppe mit 2 bis 7 Kohlenstoff-Atomen oder eine Alkoxycarbonyloxy-Gruppe mit 2 bis 5 Kohlenstoff-Atomen steht.

24. In der 2-Position substituierte 2-Cyclopentenon-Verbindung nach Anspruch 1 oder 23, welche durch die folgende Formel (I_{D0}-B-2) repräsentiert ist: 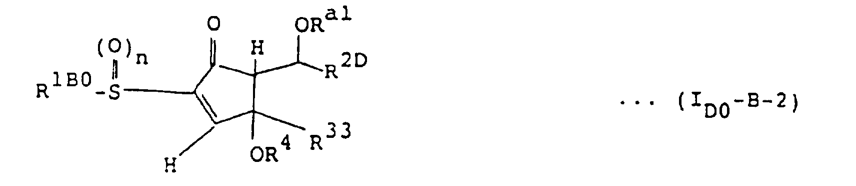 worin R^{1B0}, R^{2D}, R³³, R⁴ und n wie oben definiert sind; und
worin R^{a1} für eine Acyl-Gruppe mit 2 bis 7 Kohlenstoff-Atomen oder eine Alkoxycarbonyl-Gruppe mit 2 bis 5 Kohlenstoff-Atomen steht.

25. Antikrebs-Mittel, umfassend als eine wirksame Aktivsubstanz wenigstens eine Verbindung, welche ausgewählt ist aus der aus in der 2-Position substituierten 2-Cyclopentenon-Verbindungen gemäß Formel (I) nach Anspruch 1 gebildeten Gruppe.

26. Knochenbildungsbeschleuniger, welcher als eine wirksame Aktivsubstanz wenigstens eine Verbindung umfaßt, welche ausgewählt ist aus der aus durch die Formel (I) nach Anspruch 1 repräsentierten in der 2-Position substituierten 2-Cyclopentenonverbindungen gebildeten Gruppe.

## Revendications

1. Composé 2-cyclopenténone 2-substitué représenté par la formule (I) suivante : 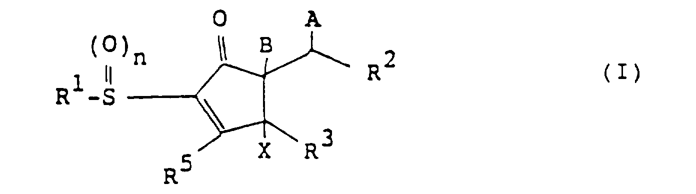 dans laquelle
R¹ représente (i) un groupe hydrocarboné aliphatique acyclique de 1 à 10 atomes de carbone, (ii) un groupe hydrocarboné alicyclique de 4 à 10 atomes de carbone, (iii) un groupe hydrocarboné aromatique de 6 à 10 atomes de carbone ou (iv) un groupe hétérocyclique de 1 à 9 atomes de carbone, substitués ou non substitués;
R représente (i) un groupe hydrocarboné aliphatique acyclique de 1 à 10 atomes de carbone, (ii) un groupe hydrocarboné alicyclique de 4 à 10 atomes de carbone, (iii) un groupe hydrocarboné aromatique de 6 à 10 atomes de carbone ou (iv) un groupe hétérocyclique de 1 à 9 atomes de carbone, substitués ou non substitués ;
R³ représente un atome d'hydrogène ou (i) un groupe hydrocarboné aliphatique acyclique de 1 à 10 atomes de carbone, (ii) un groupe hydrocarboné alicyclique de 4 à 10 atomes de carbone ou (iii) un groupe hydrocarboné aromatique de 6 à 10 atomes de carbone, substitués ou non substitués ;
X représente un atome d'hydrogène, ou -OR⁴ (où R⁴ représente un atome d'hydrogène, un groupe alcoyle de 1 à 4 atomes de carbone, un groupe acyle de 2 à 7 atomes de carbone, un groupe alkoxycarbonyle de 2 à 5 atomes de carbone, un groupe silyle tri(C₁-C₇) hydrocarboné ou un groupe capable de former une liaison acétale avec l'atome d'oxygène lié au radical R⁴), à la condition que X soit absent lorsque R³ forme une double liaison avec l'atome de carbone auquel il est lié ;
R⁵ représente un atome d'hydrogène, ou (i) un groupe hydrocarboné aliphatique acyclique de 1 à 10 atomes de carbone ou (ii) un groupe hydrocarboné alicyclique de 4 à 10 atomes de carbone, substitués ou non substitués ;
B représente un atome d'hydrogène lorsque A représente un atome d'hydrogène, un groupe hydroxyle, un groupe acyloxy de 2 à 7 atomes de carbone, un groupe alkoxycarbonyloxy de 2 à 5 atomes de carbone, un groupe sulfonyloxy de 1 à 7 atomes de carbone ou 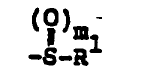 , ou A et B sont combinés l'un avec l'autre pour former une ligne de liaison ;
m et n, qui peuvent être identiques ou différents, représentent 0, 1 ou 2.
sauf dans les cas où R¹ représente un groupe hydrocarboné aliphatique acyclique substitué ou non substitué, un groupe hydrocarboné alicyclique substitué ou non substitué, ou un groupe hydrocarboné aromatique substitué ou non substitué, et R représente un groupe hydrocarboné aliphatique acyclique substitué ou non substitué, et R³ ne représente pas un atome d'hydrogène, et R⁵ représente un atome d'hydrogène, et i) A représente un groupe hydroxyle, un groupe sulfonyloxy ou 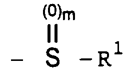 et B représente un atome d'hydrogène, ou ii) A et B sont combinés l'un à l'autre pour former une ligne de liaison.

2. Composé 2-cyclopenténone 2-substitué selon la revendication 1, qui est représenté par la formule (I_{A}) suivante : 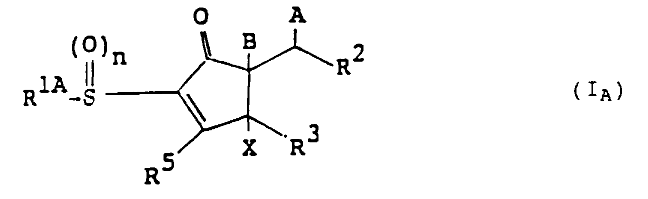 dans laquelle R^{1A} représente (i) un groupe hydrocarboné alicyclique de 4 à 10 atomes de carbone substitué ou non substitué,(ii) un groupe hétérocyclique de 1 à 9 atomes de carbone substitué ou non substitué ou (iii) un groupe hydrocarboné aliphatique acyclique, de 1 à 10 atomes de carbone substitué par un groupe hydrocarboné alicyclique de 4 à 10 atomes de carbone ou un groupe hétérocyclique de 1 à 9 atomes de carbone ; et
R, R³, R⁵, X, A, B et n sont tels que définis ci-dessus.

3. Composé 2-cyclopenténone 2-substitué selon la revendication 1 ou 2, qui est représenté par la formule (I_{A}-A) suivante : 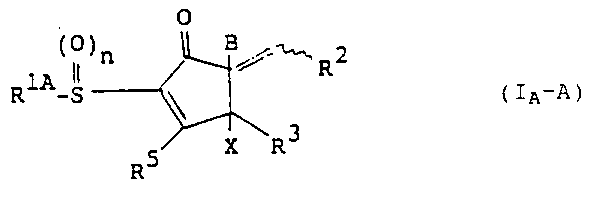 dans laquelle R^{1A}, R, R³, R⁵, X, et n sont tels que définis ci-dessus et 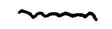 représente le fait que le substituant lié à la double liaison est dans une configuration E ou une configuration Z, ou est constitué d'un mélange de celles-ci en toutes proportions.

4. Composé 2-cyclopenténone 2-substitué selon la revendication 1 ou 2, qui est représenté par la formule (I_{A}-B) suivante : 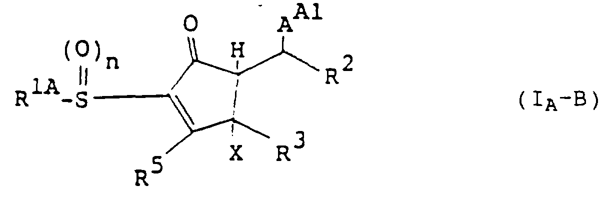 dans laquelle A^{A1} représente un atome d'hydrogène, un groupe hydroxyle, un groupe acyloxy de 2 à 7 atomes de carbone, un groupe alkoxycarbonyloxy de 2 à 5 atomes de carbone, un groupe sulfonyloxy de 1 à 7 atomes de carbone ou 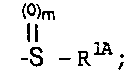 et
R^{1A}, R, R³, R⁵, X, m et n sont tels que définis ci-dessus.

5. Composé 2-cyclopenténone 2-substitué selon l'une quelconque des revendications 1 à 3, qui est représenté par la formule (I_{A}-A-2) suivante : 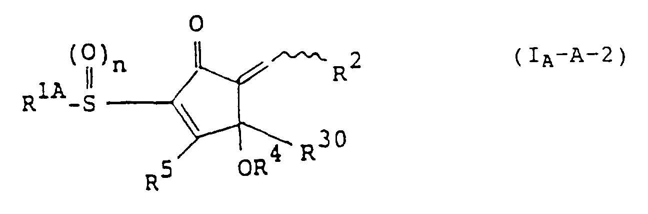 dans laquelle R^{1A}, R, R⁴, R⁵, n et 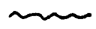 sont tels que définis ci-dessus ; et
R³⁰ représente un atome d'hydrogène ou (i) un groupe hydrocarboné aliphatique acyclique de 1 à 10 atomes de carbone, (ii) un groupe hydrocarboné alicyclique de 4 à 10 atomes de carbone ou (iii) un groupe hydrocarboné aromatique de 6 à 10 atomes de carbone, substitués ou non substitués.

6. Composé 2-cyclopenténone 2-substitué selon l'une quelconque des revendications 1 à 3, qui est représenté par la formule (I_{A}-A-3) suivante : 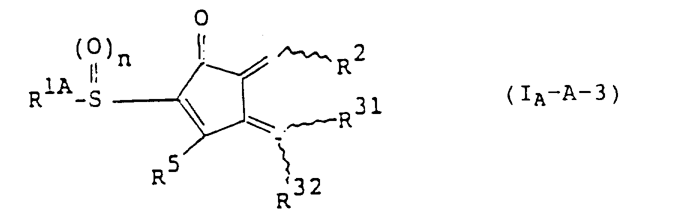 dans laquelle R^{1A}, R, R⁵, n et 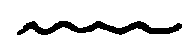 sont tels que définis ci-dessus ; et
R³¹ et R³, qui peuvent être identiques ou différents, représentent un atome d'hydrogène ou (i) un groupe hydrocarboné aliphatique acyclique de 1 à 9 atomes de carbone, (ii) un groupe hydrocarboné alicyclique de 4 à 10 atomes de carbone ou (iii) un groupe hydrocarboné aromatique de 6 à 10 atomes de carbone, substitués ou non substitués, ou un groupe dans lequel R³¹ et R³ sont combinés l'un avec l'autre pour former un groupe hydrocarboné alicyclique dont le cycle est constitué de quatre à dix membres.

7. Composé 2-cyclopenténone 2-substitué selon l'une quelconque des revendications 1, 2 et 4, qui est représenté par la formule (I_{A}-B-2) suivante : 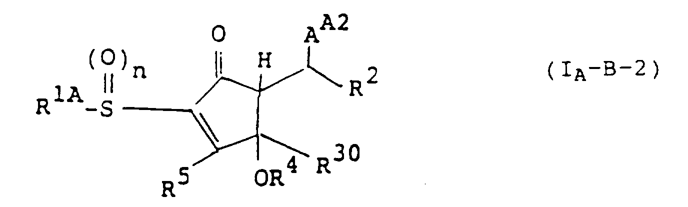 dans laquelle R^{1A}, R, R³⁰, R⁴, R⁵ et n sont tels que définis ci-dessus ; et
A^{A2} représente un atome d'hydrogène, un groupe hydroxyle, un groupe acyloxy de 2 à 7 atomes de carbone ou un groupe alkoxycarbonyloxy de 2 à 5 atomes de carbone.

8. Composé 2-cyclopenténone 2-substitué selon l'une quelconque des revendications 1, 2, 3 et 5, qui est représenté par la formule (I_{A0}-A-2) suivante : 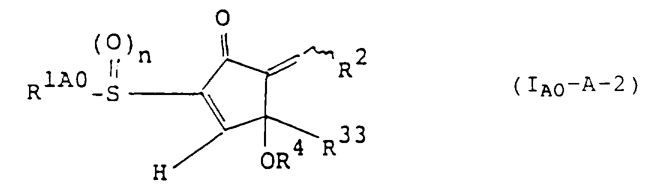 dans laquelle R, R⁴, n et 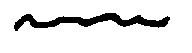 sont tels que définis ci-dessus ;
R^{1A0} représente un groupe hétérocyclique de 1 à 9 atomes de carbone substitué ou non substitué ; et
R³³ représente (i) un groupe hydrocarboné aliphatique acyclique de 1 à 10 atomes de carbone, (ii) un groupe hydrocarboné alicyclique de 4 à 10 atomes de carbone ou (iii) un groupe hydrocarboné aromatique de 6 à 10 atomes de carbone, substitués ou non substitués.

9. Composé 2-cyclopenténone 2-substitué selon l'une quelconque des revendications 1, 2, 3 et 6, qui est représenté par la formule (I_{A0}-A-3) suivante : 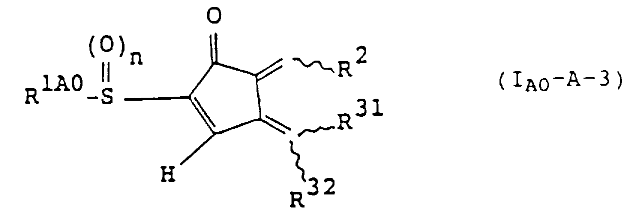 dans laquelle R^{1A0}, R, R³¹, R³, n et 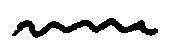 sont tels que définis ci-dessus.

10. Composé 2-cyclopenténone 2-substitué selon l'une quelconque des revendications 1, 2, 4 et 7, qui est représenté par la formule (I_{A0}-B-2) suivante : 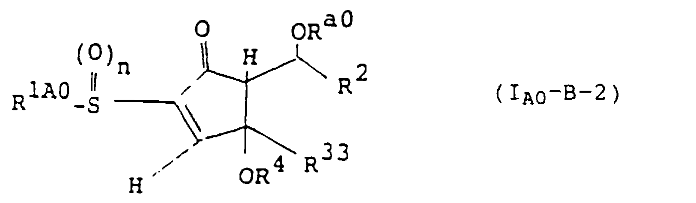 dans laquelle R^{1A0}, R, R³³, R⁴ et n sont tels que définis ci-dessus ; et
R^{a0} représente un atome d'hydrogène, un groupe acyle de 2 à 7 atomes de carbone ou un groupe alkoxycarbonyle de 2 à 5 atomes de carbone.

11. Composé 2-cyclopenténone 2-substitué selon la revendication 1, qui est représenté par la formule (I_{B}) suivante : 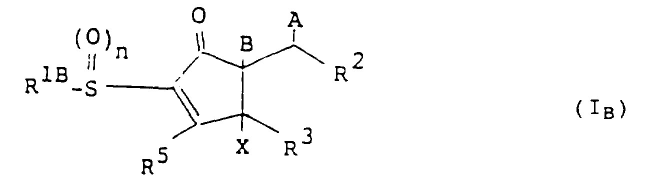 dans laquelle R^{1B} représente (i) un groupe hydrocarboné aliphatique acyclique de 1 à 10 atomes de carbone ou (ii) un groupe hydrocarboné aromatique de 6 à 10 atomes de carbone, substitués ou non substitués; et
R, R³, R⁵, X, A, B et n sont tels que définis ci-dessus, à la condition que lorsque R⁵ représente un atome d'hydrogène, R³ représente un atome d'hydrogène.

12. Composé 2-cyclopenténone 2-substitué selon la revendication 1 ou 11, qui est représenté par la formule (I_{B}-A) suivante : 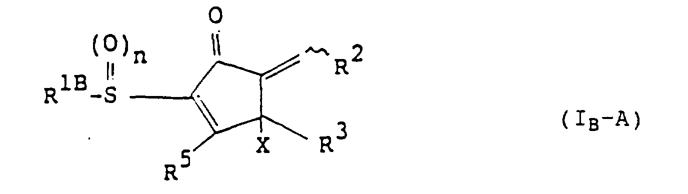 dans laquelle R^{1B}, R, R³, R⁵, X, n et 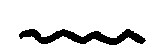 sont tels que définis ci-dessus, à la condition que R³ représente un atome d'hydrogène lorsque R⁵ représente un atome d'hydrogène.

13. Composé 2-cyclopenténone 2-substitué selon la revendication 1 ou 11, qui est représenté par la formule (I_{B}-B) suivante : 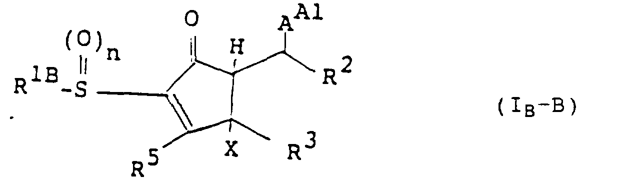 dans laquelle R^{1B}, R, R³, R⁵, X, A^{A1} et n sont tels que définis ci-dessus, à la condition que R³ représente un atome d'hydrogène lorsque R⁵ représente un atome d'hydrogène.

14. Composé 2-cyclopenténone 2-substitué selon la revendication 1, 11 ou 12, qui est représenté par la formule (I_{B}-A-2) suivante : 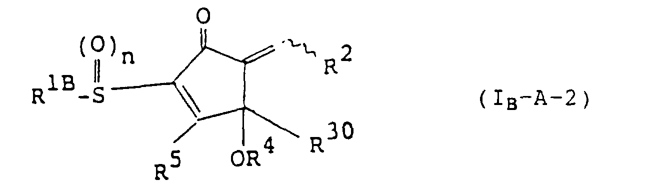 dans laquelle R^{1B}, R, R³⁰, R⁴, R⁵, n et 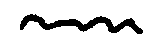 sont tels que définis ci-dessus, à la condition que R³⁰ représente un atome d'hydrogène lorsque R⁵ représente un atome d'hydrogène.

15. Composé 2-cyclopenténone 2-substitué selon la revendication 1, 11 ou 12, qui est représenté par la formule (I_{B}-A-3) suivante : 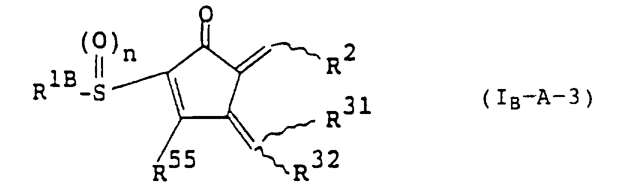 dans laquelle R^{1B}, R, R³¹, R³, n et 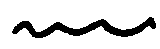 sont tels que définis ci-dessus ; et
R⁵⁵ représente un groupe hydrocarboné aliphatique acyclique de 1 à 10 atomes de carbone substitué ou non substitué ou un groupe hydrocarboné alicyclique de 4 à 10 atomes de carbone substitué ou non substitué.

16. Composé 2-cyclopenténone 2-substitué selon la revendication 1, 11 ou 13, qui est représenté par la formule (I_{B}-B-2) suivante : 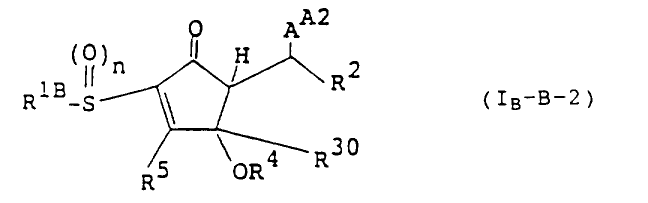 dans laquelle R^{1B}, R, R³⁰, R⁴, R⁵, n et A^{A2} sont tels que définis ci-dessus, à la condition que R³⁰ représente un atome d'hydrogène lorsque R⁵ représente un atome d'hydrogène.

17. Composé 2-cyclopenténone 2-substitué selon la revendication 1, qui est représenté par la formule (I_{C}) suivante : 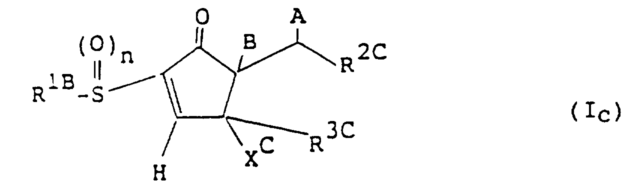 dans laquelle R^{1B}, A, B et n sont tels que définis ci-dessus ;
R^{2C} représente (i) un groupe hydrocarboné alicyclique de 4 à 10 atomes de carbone, (ii) un groupe hydrocarboné aromatique de 6 à 10 atomes de carbone, (iii) un groupe hétérocyclique de 1 à 9 atomes de carbone, substitués ou non substitués ou (iv) un groupe hydrocarboné aliphatique acyclique de 1 à 10 atomes de carbone substitué par un groupe hydrocarboné hétérocyclique de 1 à 9 atomes de carbone ;
R^{3C} représente (i) un groupe hydrocarboné aliphatique acyclique de 1 à 10 atomes de carbone, (ii) un groupe hydrocarboné alicyclique de 4 à 10 atomes de carbone ou (iii) un groupe hydrocarboné aromatique de 6 à 10 atomes de carbone, substitués ou non substitués, à la condition que lorsque R^{3C} forme une simple liaison avec l'atome de carbone auquel il est lié, X^{C} représente un atome d'hydrogène ou -OR⁴ (où R⁴ est tel que défini ci-dessus), et que lorsque R^{3C} forme une double liaison avec l'atome de carbone auquel il est lié, X^{C} est absent.

18. Composé 2-cyclopenténone 2-substitué selon la revendication 1 ou 17, qui est représenté par la formule (I_{C}-A) suivante : 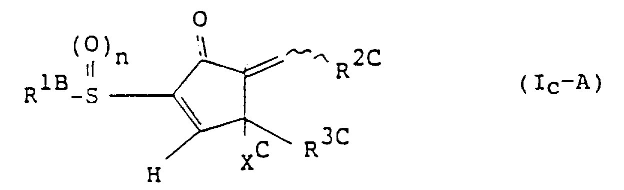 dans laquelle R^{1B}, R^{2C}, R^{3C}, R⁵, X^{C}, n et 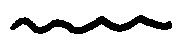 sont tels que définis ci-dessus.

19. Composé 2-cyclopenténone 2-substitué selon la revendication 1 ou 17, qui est représenté par la formule (I_{C}-B) suivante : 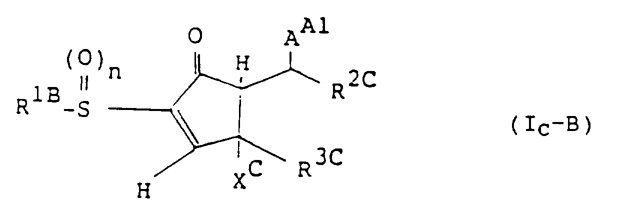 dans laquelle R^{1B}, R^{2C}, R^{3C}, X^{C}, A^{A1} et n sont tels que définis ci-dessus.

20. Composé 2-cyclopenténone 2-substitué selon la revendication 1, 17 ou 18, qui est représenté par la formule (I_{CO}-A-2) suivante : 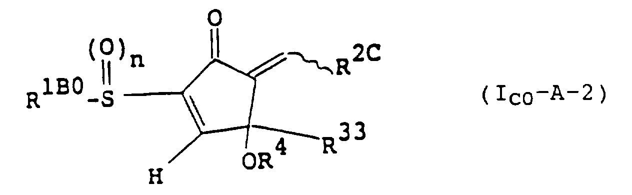 dans laquelle R^{2C}, R³³, R⁴, n et 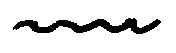 sont tels que définis ci-dessus ; et
R^{1B0} représente un groupe hydrocarboné aromatique de 6 à 10 atomes de carbone substitué ou non substitué, ou un groupe méthyle.

21. Composé 2-cyclopenténone 2-substitué selon la revendication 1, 17 ou 18, qui est représenté par la formule (I_{CO}-A-3) suivante : 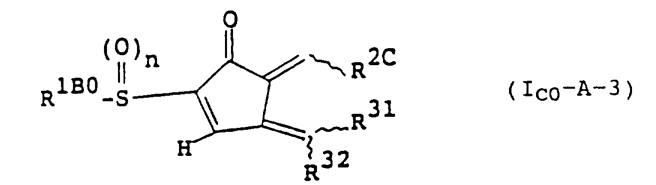 dans laquelle R^{1B0}, R^{2C}, R³¹, R³, n et 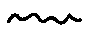 sont tels que définis ci-dessus.

22. Composé 2-cyclopenténone 2-substitué selon la revendication 1, 17 et 19, qui est représenté par la formule (I_{C0}-B-2) suivante : 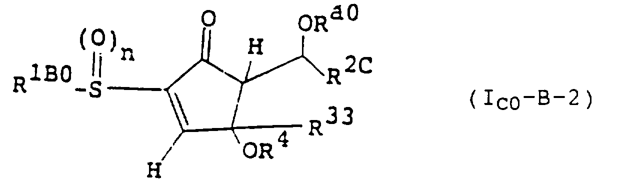 dans laquelle R^{1B0}, R^{2C}, R³³, R⁴, R^{a0} et n sont tels que définis ci-dessus.

23. Composé 2-cyclopenténone 2-substitué selon la revendication 1, qui est représenté par la formule (I_{D}) suivante : 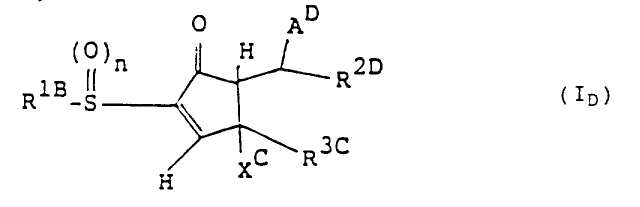 dans laquelle R^{1B}, R^{3C}, X^{C} et n sont tels que définis ci-dessus ; et
R^{2D} représente (i) un groupe hydrocarboné alicyclique de 4 à 10 atomes de carbone ou (ii) un groupe hydrocarboné aliphatique acyclique de 1 à 10 atomes de carbone substitués ou non substitués qui peut être substitué par un groupe hydrocarboné aromatique de 6 à 10 atomes de carbone ; et
A^{D} représente un atome d'hydrogène, un groupe acyloxy de 2 à 7 atomes de carbone ou un groupe alkoxycarbonyloxy de 2 à 5 atomes de carbone.

24. Composé 2-cyclopenténone 2-substitué selon la revendication 1 ou 23, qui est représenté par la formule (I_{D0}-B-2) suivante : 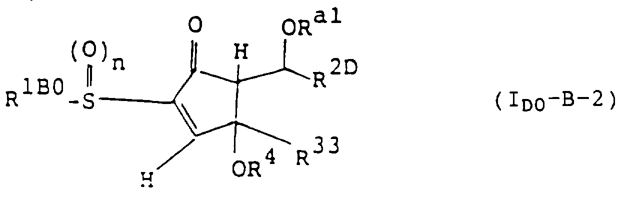 dans laquelle R^{1B0}, R^{2D}, R³³, R⁴ et n sont tels que définis ci-dessus ; et
R^{a1} représente un groupe acyle de 2 à 7 atomes de carbone ou un groupe alkoxycarbonyle de 2 à 5 atomes de carbone.

25. Agent anticancéreux comprenant en tant qu'ingrédient actif efficace au moins un composé choisi dans le groupe constitué des composés 2-cyclopenténone 2-substitués représentés par la formule (I) selon la revendication 1.

26. Accélérateur de l'ossification comprenant en tant qu'ingrédient actif efficace au moins un composé choisi dans le groupe constitué des composés 2-cyclopenténone 2-substitués représentés par la formule (I) selon la revendication 1.
